(19) Europäisches Patentamt
European Patent Office
Office européen des brevets

(11) EP 1 569 916 B1

(12) EUROPEAN PATENT SPECIFICATION

(45) Date of publication and mention of the grant of the patent:
**07.01.2009 Bulletin 2009/02**

(21) Application number: **03808341.6**

(22) Date of filing: **14.11.2003**

(51) Int Cl.:
*C07D 239/91* (2006.01) *C07D 279/16* (2006.01)
*C07D 413/04* (2006.01) *C07D 265/38* (2006.01)
*C07D 209/86* (2006.01) *C07D 215/14* (2006.01)
*C07D 209/08* (2006.01) *C07D 213/30* (2006.01)
*C07D 207/32* (2006.01) *C07D 235/06* (2006.01)
*C07D 409/04* (2006.01) *C07D 261/08* (2006.01)
*C07D 405/04* (2006.01) *A61K 31/421* (2006.01)
*A61K 31/422* (2006.01)

(86) International application number:
**PCT/IN2003/000358**

(87) International publication number:
**WO 2004/046119 (03.06.2004 Gazette 2004/23)**

(54) **SUBSTITUTED ARALKYL DERIVATIVES**
SUBSTITUIERTE ARALKYL VERBINDUNGEN
DERIVES D'ARALKYLE SUBSTITUES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **15.11.2002 IN MU15112002**
**12.08.2003 IN MU12082003**

(43) Date of publication of application:
**07.09.2005 Bulletin 2005/36**

(73) Proprietor: **CADILA HEALTHCARE LIMITED**
**Ahmedebad 380 015, Gujarat (IN)**

(72) Inventors:
- **LOHRAY, Braj, Bhushan, Cadila Healthcare Limited Ahmedabad 380 015, Gujarat (IN)**
- **LOHRAY, Vidya, Bhushan, Cadila Healthcare Limited Ahmedabad 380 015, Gujarat (IN)**
- **JAIN, Mukul, R., Cadila Healthcare Limited Ahmedabad 380 015, Gujarat (IN)**
- **BASU, Sujay, Cadila Healthcare Limited Ahmedabad 380 015, Gujarat (IN)**
- **PINGALI, Harikishore, Cadila Healthcare Limited Ahmedabad 380 015, Gujarat (IN)**
- **RAVAL, Saurin, K., Cadila Healthcare Limited Ahmedabad 380 015, Gujarat (IN)**
- **RAVAL, Preeti, S., Cadila Healthcare Limited Ahmedabad 380 015, Gujarat (IN)**

(74) Representative: **Spencer, Michael David et al**
**Bromhead Johnson**
**19 Buckingham Street**
**London**
**WC2 6EF (GB)**

(56) References cited:
**EP-A- 0 478 363** **EP-A- 0 930 299**
**WO-A-01/40170** **WO-A-91/19702**
**WO-A-96/38415** **WO-A-02/092084**
**WO-A-03/072102** **WO-A-20/04004665**
**WO-A-20/04031162** **JP-A- 51 149 265**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **EGBERTSON M S ET AL: "NON-PEPTIDE FIBRINOGEN RECEPTOR ANTAGONISTS. 2. OPTIMIZATION OF A TYROSINE TEMPLATE AS A MIMIC FOR ARG-GLY-ASP" JOURNAL OF MEDICINAL CHEMISTRY, AMERICAN CHEMICAL SOCIETY. WASHINGTON, US, vol. 37, no. 16, 1994, pages 2537-2551, XP000574969 ISSN: 0022-2623**

Remarks:

The file contains technical information submitted after the application was filed and not included in this specification

## Description

FIELD OF INVENTION

The present invention relates to novel antidiabetic, hypolipidaemic and hypocholesterolemic compounds, their derivatives, their analogs, their tautomeric forms, their stereoisomers, their pharmaceutically acceptable salts, their pharmaceutically acceptable solvates and pharmaceutically acceptable compositions containing them. More particularly, the present invention relates to novel substituted aralkyl derivatives of the general formula (I), their derivatives, their analogs, their tautomeric forms, their stereoisomers, their pharmaceutically acceptable salts, their pharmaceutically acceptable solvates, pharmaceutical compositions containing them, use of these compounds in medicine and the intermediates involved in their preparation.

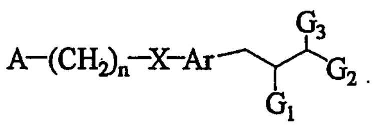

(I)

The present invention also relates to a process for the preparation of the above said novel compounds, their derivatives, their analogs, their tautomeric forms, their stereoisomers, their pharmaceutically acceptable salts, their pharmaceutically acceptable solvates, and pharmaceutical compositions containing them.

The present invention also discloses novel compounds of formula (IIIa) their derivatives, their analogs, their tautomeric forms, their stereoisomers, their pharmaceutically acceptable salts, their pharmaceutically acceptable solvates and pharmaceutically acceptable compositions containing them. The compounds of formula (IIIa) are useful as intermediates for the preparation of compounds of formula (I).

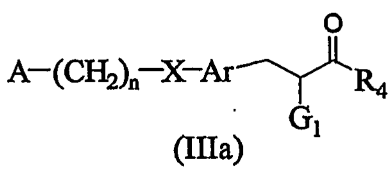

(IIIa)

The compounds of the general formula (I) & (IIIa) lower blood glucose, lower or modulate triglyceride levels and/or cholesterol levels and/or low-density lipoproteins (LDL) and raises the high-density lipoproteins (HDL) plasma levels and hence are useful in combating different medical conditions, where such lowering (and raising) is beneficial. Thus, it could be used in the treatment and/or prophylaxis of obesity, hyperlipidaemia, hypercholesteremia, hypertension, atherosclerotic disease events, vascular restenosis, diabetes and many other related conditions.

The compounds of general formula (I) & (IIIa) are useful to prevent or reduce the risk of developing atherosclerosis, which leads to diseases and conditions such as artereosclerotic cardiovascular diseases, stroke, coronary heart diseases, cerebrovascular diseases, peripheral vessel diseases and related disorders. These compounds of general formula (I) & (IIIa) are useful for the treatment and/or prophylaxis of metabolic disorders loosely defined as Syndrome X. The characteristic features of Syndrome X include initial insulin resistance followed by hyperinsulinemia, dyslipidemia and impaired glucose tolerance. The glucose intolerance can lead to non-insulin dependent diabetes mellitus (NIDDM, Type 2 diabetes), which is characterized by hyperglycemia, which if not controlled may lead to diabetic complications or metabolic disorders caused by insulin resistance. Diabetes is no longer considered to be associated only with glucose metabolism, but it affects anatomical and physiological parameters, the intensity of which vary depending upon stages/ duration and severity of the diabetic state. The compounds of this invention are also useful in prevention, halting or slowing progression or reducing the risk of the above mentioned disorders along with the resulting secondary diseases such as cardiovascular diseases, like arteriosclerosis, atherosclerosis; diabetic retinopathy, diabetic neuropathy and renal disease including diabetic nephropathy, glomerulonephritis, glomerular sclerosis, nephrotic syndrome, hypertensive nephrosclerosis and end stage renal diseases, like microalbuminuria and albuminuria, which may be result of hyperglycemia or hyperinsulinemia.

The compounds of the present invention can be useful as aldose reductase inhibitors; for improving cognitive functions in dementia, and in the treatment and/or prophylaxis of disorders such as psoriasis, polycystic ovarian syndrome (PCOS), cancer, osteoporosis, leptin resistance, inflammation and inflammatory bowel diseases, xanthoma, pancreatitis, myotonic dystrophy, endothelial cell dysfunction and hyperlipidemia.

The compounds of the present invention are useful in the treatment of the diseases mentioned herein, alone or in combination with one or more hypoglycemic, antihyperglycemic, hypolipidaemic, hypolipoproteinemic agents, antioxidants, antihypertensives, such as HMG CoA reductase inhibitor, fibrate, statins, glitazones, sulfonyl ureas, insulin, α-glycosidase inhibitors, nicotinic acid, cholestyramine, cholestipol or probucol, and the like.

BACKGROUND OF THE INVENTION

Hyperlipidaemia has been recognized as the major risk factor in causing cardiovascular diseases due to atherosclerosis. Atherosclerosis and other such peripheral vascular diseases affect the quality of life of a large population in the world. The therapy aims to lower the elevated plasma LDL cholesterol, low-density lipoprotein and plasma triglycerides in order to prevent or reduce the risk of occurrence of cardiovascular diseases. The detailed etiology of atherosclerosis and coronary artery diseases is discussed by Ross and Glomset [New Engl. J. Med., 295, 369-377 (1976)]. Plasma cholesterol is generally found esterified with various serum lipoproteins and numerous studies have suggested an inverse relationship between serum HDL-cholesterol level and risk for occurrence of cardiovascular disease. Many studies have suggested an increased risk of coronary artery diseases (CAD) due to elevated LDL and VLDL-cholesterol levels [Stampfer et al., N. Engl. J. Med., 325, 373-381(1991)]. The other studies illustrate protective effects of HDL against progression of atherosclerosis. Thus, HDL has become a crucial factor in treating diseases with increased levels of cholesterol [Miller et. al., Br. Med. J. 282, 1741-1744(1981); Picardo et al., Arteriosclerosis, 6, 434-441 (1986); Macikinnon et al., J. Biol. Chem. 261, 2548-2552 (1986)].

Diabetes is associated with a number of complications and also affect a large population. This disease is usually associated with other diseases such as obesity, hyperlipidemia, hypertension and angina. It is well established that improper treatment can aggravate impaired glucose tolerance and insulin resistance, thereby leading to frank diabetes. Further, patients with insulin resistance and type 2 diabetes often have raised triglycerides and low HDL-cholesterol concentrations and therefore, have greater risk of cardiovascular diseases. The present therapy for these diseases includes sulfonylureas and biguanides along with insulin. This type of drug therapy may lead to mild to severe hypoglycemia, which may lead to coma or in some cases may lead to death, as a result of unsatisfactory glycaemic control by these drugs. Recent addition of drugs in the treatment of diabetes are the thiazolidinediones, drugs having insulin-sensitizing action. Thiazolidinediones like troglitazone, rosiglitazone and pioglitazone are prescribed alone or in combination with other anti-diabetic agents.

These are useful in treating diabetes, lipid metabolism but are suspected to have tumor-inducing potential and cause hepatic dysfunction, which may lead to liver failure. Further, serious undesirable side-effects have occurred in animal and/or human studies which include cardiac hypertrophy, hema dilution and liver toxicity in a few glitazones progressing to advanced human trials. The drawback is considered to be idiosyncratic. Presently, there is a need for a safe and an effective drug, to treat insulin resistance, diabetes and hyperlipidemia.[ Exp. Clin. Endocrinol. Diabetes: 109(4), S548-9 (2001)]

Obesity is another major health problem being associated with increased morbidity and mortality. It is a metabolic disorder, in which excess of fat is accumulated in the body. Although, its etiology is unclear, the general feature includes excess of calorie intake than it is consumed. Various therapies such as dieting, exercise, appetite suppression, inhibition of fat absorption etc. have been used to combat obesity. However, more efficient therapies to treat this abnormality is essential as obesity is closely related to several diseases such as coronary heart disease, stroke, diabetes, gout, osteoarthritis, hyperlipidaemia and reduced fertility. It also leads to social and psychological problems [Nature Reviews: Drug Discovery: 1(4), 276-86 (2002)].

Peroxisome Proliferator Activated Receptor (PPAR) is a member of the steroid/ retinoid/ thyroid hormone receptor family. PPAR, PPARγ and PPARδ have been identified as subtypes of PPARs. Extensive reviews regarding PPAR, their role in different diseased conditions are widely published [Endocrine Reviews, 20(5), 649-688 (1999); J. Medicinal Chemistry, 43(4), 58-550 (2000); Cell, 55, 932-943 (1999); Nature, 405, 421-424 (2000); Trends in Pharmacological Sci., 469-473 (2000)]. PPARγ activation has been found to play a central role in initiating and regulating adipocyte differentiation [Endocrinology 135, 798-800, (1994)] and energy homeostasis, [Cell, 83, 803-812 (1995); Cell, 99, 239-242 (1999)]. PPARγ agonists would stimulate the terminal differentiation of adipocyte precursors and cause morphological and molecular changes characteristic of a more differentiated, less malignant state. During adipocyte differentiation, several highly specialized proteins are induced, which are being involved in lipid storage and metabolism. It is accepted that PPARγ activation leads to expression of CAP gene [Cell Biology, 95, 14751-14756, (1998)], however, the exact link from PPARγ activation to changes in glucose metabolism and decrease in insulin resistance in muscle has not been clear. PPARα is involved in stimulating β-oxidation of fatty acids [Trends Endocrine. Metabolism, 4, 291-296 (1993)] resulting in plasma circulating free fatty acid reduction [Current Biol., 5, 618-621 (1995)]. Recently, role of PPARγ activation in the terminal differentiation of adipocyte precursors has been implicated in the treatment of cancer. [Cell, 79, 1147-1156 (1994); Cell, 377-389 (1996); Molecular Cell, 465-470 (1998); Carcinogenesis, 1949-1953 (1998); Proc. Natl. Acad. Sci., 94, 237-241 (1997); Cancer Research, 58, 3344-3352 (1998)]. Since PPARγ is expressed in certain

cells consistently, PPAR$\gamma$ agonists would lead to nontoxic chemotherapy. There is growing evidence that PPAR agonists may also influence the cardiovascular system through PPAR receptors as well as directly by modulating vessel wall function [Med. Res. Rev., 20 (5), 350-366 (2000)].

PPAR $\alpha$ agonists have been found useful in the treatment of obesity (WO 97/36579). Dual PPAR $\alpha$ and $\gamma$ agonists have been suggested to be useful for Syndrome X (WO 97/25042). PPAR $\gamma$ agonists and HMG-CoA reductase inhibitors have exhibited synergism and indicated the usefulness of the combination in the treatment of atherosclerosis and xanthoma (EP 0753 298).

Leptin is a protein when bound to leptin receptors is involved in sending satiety signal to the hypothalamus. Leptin resistance would therefore lead to excess food in-take, reduced energy expenditure, obesity, impaired glucose tolerance and diabetes [Science, 269, 543-46(1995)]. It has been reported that insulin sensitizers lower plasma leptin concentration [Proc. Natl. Acad Sci. 93, 5793-5796 (1996): WO 98/02159)].

Phenalkyloxy-phenyl derivatives having general formula as given below, useful in the treatment of insulin resistance has been described in WO 01/40170 (AstraZeneca AB).

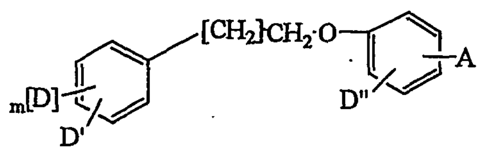

A typical example of these compounds is shown formula (IIa).

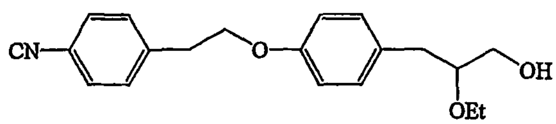

Aryl hydroxy propanol derivatives having the general formula given below

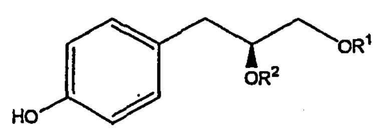

as agents for treatment of disorders associated with insulin resistance have been described in WO 03008362 (Dr. Reddy's Research Foundation)

A number of compounds belonging to the class of oxazole derivatives have been reported to be useful in the treatment of hyperlipidemia, hypercholesterolemia and hyperglycemia which includes
WO 02092084 (Hoffmann La Roche) describes oxazole compounds having the following general formula

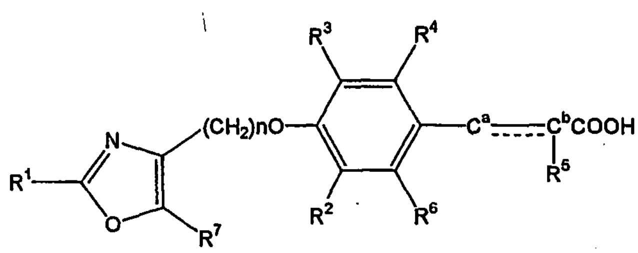

wherein, $R^1$ is aryl or heteroaryl; $R^2$, $R^3$, $R^4$ and $R^5$ are independently selected from the group consisting of hydrogen, hydroxy, lower-alkenyl, halogen, lower-alkyl and lower-alkoxy, wherein at least one of $R^2$, $R^3$, $R^4$ and $R^6$ is not hydrogen, or $R^3$ and $R^4$ are bonded to each other to form a ring together with the carbon atoms to which they are attached, and $R^3$ and $R^4$ together are -CH=CH-S-, -S-CH=CH-, - CH=CH-O-, -O-CH=CH-, -CH=CH-CH=CH-, $-(CH_2)_{3-5}-$, $-O-(CH_2)_{2-3}-$ or $-(CH_2)_{2-3}-O-$; $R^5$ is lower-alkoxy, lower-alkenyloxy, or

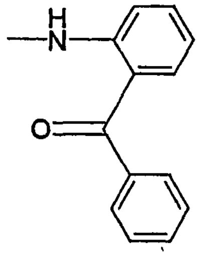

or

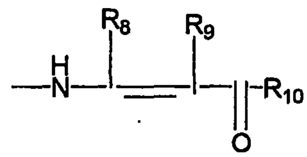

$R^7$ , $R^8$, $R^9$, each represent H or lower-alkyl; $R^{10}$ is aryl; n is 1, 2 or 3; the bond between $C_a$ & $C_b$ represent a carbon-carbon single or double bond;

WO 0216331(Eli Lilly & Co.) discloses oxazolyl-arylpropionic acid derivatives of the following general formula

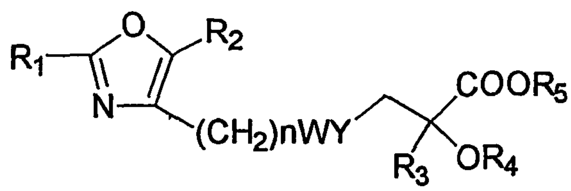

where $R_1$ is substituted or unsubstituted groups selected from aryl, heteroaryl, cycloalkyl, heterocycloalkyl, arylalkyl, heteroarylalkyl, cycloalkylalkyl, $(CH_3)_3C$-; n = 2,3, 4; W represents $CH_2$, CH(OH), CO, O; $R_2$ represents H, alkyl, haloalkyl, $C_6H_5$; Y represents substituted or unsubstituted group consisting of thiophen-2,5-diyl or phenylene; $R_3$ represents alkyl, haloalkyl; $R_4$ represents substituted or unsubstituted phenyl, naphthyl, 1,2,3,4-tetrahydronaphthyl, quinolyl, pyridyl, benzo[1,3]dioxol-5-yl; $R_5$ represents H, alkyl, aminoalkyl groups.

WO 9807699 (Japan Tobacco, Inc.) describes propionic acid derivative of the following general structure

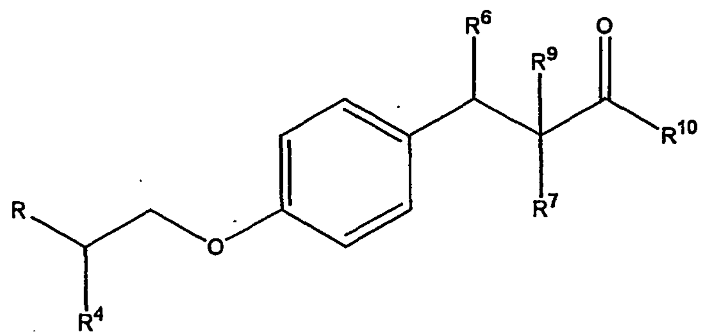

wherein R represents

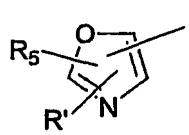

or

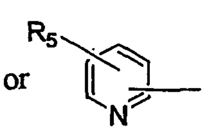

$R^1$ is an optionally substituted aromatic hydrocarbon, an optionally substituted alicyclic hydrocarbons, an optionally substituted heterocyclic group, or an optionally substituted fused heterocyclic group, $R^5$ is lower alkyl; $R^4$ = H or lower alkyl; $R^6$=H or forms together with $R^9$, a double bond; $R^7$ is a carboxy, an acyl, an optionally substituted alkoxycarbonyl, an optionally substituted lower alkyl, an optionally substituted carbamoyl, an optionally substituted aryloxycarbonyl, an optionally substituted aralkyloxycarbonyl or a group of the formula -Y-$R^8$ wherein Y is -NH- or an oxygen atom and $R^8$

is an optionally substituted acyl or an optionally substituted alkoxycarbonyl;
$R^9$ = H, an optionally substituted loweralkyl or an optionally substituted alkoxycarbonyl; $R^{10}$ is a hydroxy, an optionally substituted amino, an optionally substituted lower alkoxy, an optionally substituted lower alkyl, an optionally substituted aryloxy or an optionally substituted aralkyloxy, provided that when $R^7$ is an alkoxycarbonyl and $R^9$ is a hydrogen atom, $R^{10}$ is not a lower alkoxy.

WO 02100403 (Eli Lilly & Co.) discloses compounds of the following general formula suitable for the treatment of Syndrome X

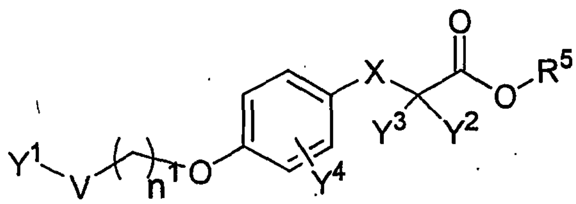

wherein $Y^1$ represents

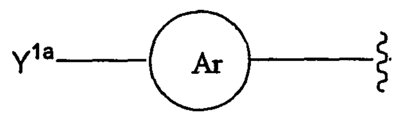

$Y^{1a}$ is H, $(C_0-C_3)$ alkyl-aryl, C(O)-aryl , heteroaryl, cycloalkyl, heterocycloalkyl, aryloxy, etc.;

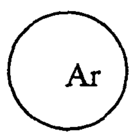

is aryl or heteroaryl; V is a bond or O; X is $CH_2$ or O, $R^5$ is H or $C_1$-$C_6$ alkyl; $Y^2$ and $Y^3$ are each independently H, $C_1$-$C_6$ alkyl or $C_1$-$C_6$ alkoxy; $Y^4$ is $(C_1-C_3)$ alkyl-$NR^5C(O)$ -$(C_0-C_5)$ alkyl-$Y^7$, $(C_1-C_3)$ alkyl-$NR^5C$ O) -$(C_2-C_5)$ alkenyl-$Y^7$, $(C_1-C_3)$ alkyl-$NR^5C(O)$ -$(C_2-C_5)$ alkynyl-$Y^7$, CN etc.; $Y^7$ is H, aryl heteroaryl, $C_1$-$C_{12}$ alkyl, $C_1$-$C_6$ alkoxy, cycloalkyl, heterocycloalkyl, aryloxy, C(O)-heteroaryl etc.; $n^1$ is 2,3,4 or 5;

US 5232945 (Pfizer Inc.), describes compounds of the following general formula

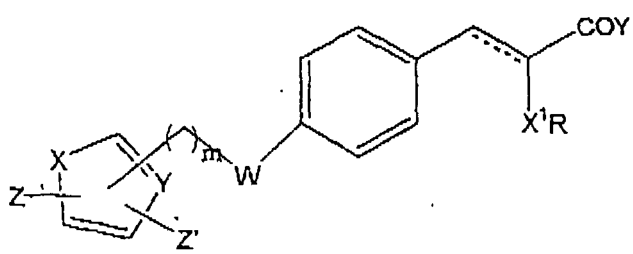

wherein Z= H, amino, $(C_1-C_7,)$alkyl, $(C_3-C_7)$cycloalkyl, phenyl or phenyl mono or disubstituted with $(C_1-C_3)$alkyl, $CF_3$, $(C_1-C_3)$alkoxy) phenyl, phenoxy, benzyl, benzyloxy, fluoro or chloro; $Z^1$ = H or $(C_1-C_3)$alkyl; R = (un)substituted alkyl, cycloalkyl, alkenyl, alkynyl, Ph, phenylalkyl alkanoyl; X = S, O, $NR_2$, -CH=CH, -CH=N, -N=CH; $R_2$ = H, alkyl, Ph, $CH_2Ph$; Y = CH, N; $X^1$ = O, S, SO, $SO_2$; $Y^1$ = OH, (un)substituted alkoxy, OPh, $OCH_2Ph$, $NH_2$ etc.; W = O, CO, $CH_2$, CH(OH), -CH=CH; m = 0 1, 2.

Several other oxazole derivatives useful in the treatment of diabetes, hyperlipidemia etc. (Syndrome X) have been reported for e.g. WO 03072100, WO 0320269, WO 0216332, WO 0218355, WO 0216331, WO 0216332, WO

0296895, WO 0296895, WO 0296894, WO 0296893, WO 0262774, WO 0250048, WO 0250047, WO 0276957, WO 0251820, WO 0214291, WO 0138325, WO 0116120, WO 0100403, WO 0116111, WO 0116120, WO 0179202, WO 0179197, WO 0008002, US 20010008898, JP 2002338555, JP 2001261612

However, very few of the compounds described above have reached the marker and so there therefore remains the need to develop newer medicines which are better and cost effective, are of better or comparable efficacy with the present treatment regimes, has lesser side effects and require a lower dosage regime

SUMMARY OF INVENTION

The objective of this invention is to develop novel compounds represented by the general formula (I) & (IIIa) used as hypocholesterolemic, hypolipidaemic, hypolipoproteinemic, anti-obesity and antihyperglycemic agents which may have additional body weight lowering effect and beneficial effect in the treatment and/or US 5232945 (Pfizer Inc.), describes compounds of the following general formula

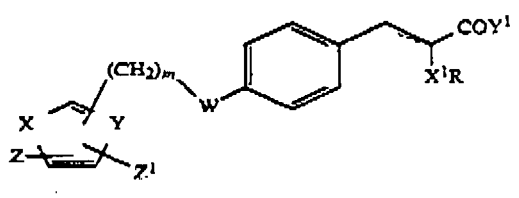

wherein Z= H, amino, ($C_1$-$C_7$) alkyl, ($C_3$-$C_7$) cycloalkyl, phenyl or phenyl mono or disubstituted with ($C_1$-$C_3$) alkyl, $CF_3$, ($C_1$-$C_3$)alkoxy, phenyl, phenoxy, benzyl, benzyloxy, fluoro or chloro; $Z^1$ = H or ($C_1$-$C_3$) alkyl; R = (un) substituted alkyl, cycloalkyl, alkenyl, alkynyl, Ph, phenylalkyl, alkanoyl; X = S, O, $NR_2$, -CH=CH, -CH=N, -N=CH: $R^2$ = H, alkyl, Ph, $CH_2Ph$; Y = CH, N; $X^1$ = O, S, SO, $SO_2$; $Y^1$ = OH, (un) substituted alkoxy, OPh, $OCH_2Ph$, $NH_2$ etc.; W = O, CO, $CH_2$, CH(OH), - CH=CH; m = 0, 1, 2.

WO 9119702 (Pfizer) discloses compounds with the general formula

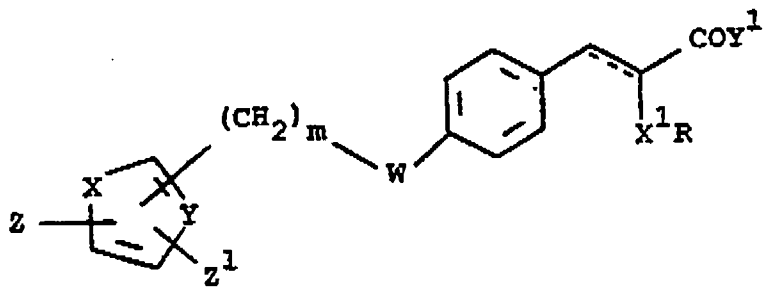

wherein Z= H, amino, ($C_1$-$C_7$) alkyl, ($C_3$-$C_7$)cycloalkyl, phenyl or phenyl mono or disubstituted with ($C_1$-$C_3$) alkyl, $CF_3$, ($C_1$-$C_3$) alkoxy, phenyl, phenoxy, benzyl, benzyloxy, fluoro or chloro; $Z^1$ = H or ($C_1$-$C_3$) alkyl; R = (un)substituted alkyl, cycloalkyl, alkenyl, alkynyl, Ph, phenylalkyl, alkanoyl; X = S, O, $NR_2$, -CH=CH, -CH=N, -N=CH; $R^2$ = H, alkyl, Ph, $CH_2Ph$; $X^1$ = O, S, SO, SO2; $Y^1$ = OH, (un) substituted alkoxy, OPh, OCH2Ph, NH2 etc.; W = O, CO, $CH_2$, CH(OH), -CH=CH; m = 0, 1, 2.

Journal of Medical Chemistry, American Chemical Society 1994, 37, 2537-2551 paper discloses the following compound suitable as a non-peptide fibrinogen receptor antagonist

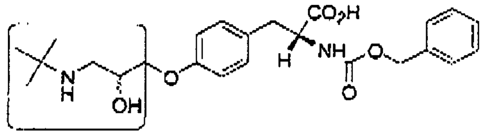

JP 51149265 (Yoshitomi Pharmaceutical) describes the preparation of compounds with the following general formula

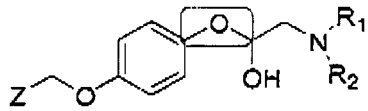

where 'z' represents 2-tetrahydrofuryl or 2-tetrahydropyranyl & $R_1$ & $R_2$ represents hydrogen, lower alkyl, cycloalkyl etc. groups.

EP 0478363 (Merck) discloses compounds with the following general structure and are useful as fibrinogen receptor antagonists:

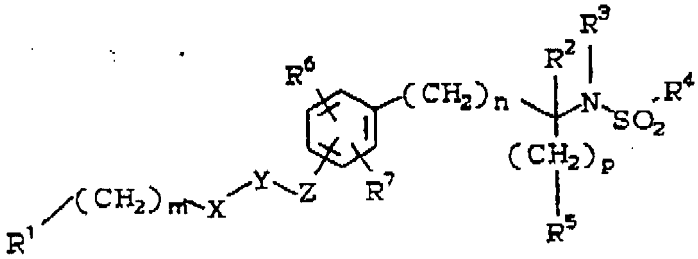

WO 9638415 (Sumitomo Metal Ind) discloses 2-amino-3-phenyl propanoic acid derivatives having the following general formula

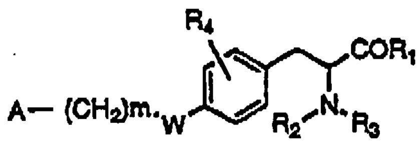

where $R_1$ represents hydroxy, lower alkoxy, phenoxy, phenyl alkoxy, amino, mono or dilakyl amino, substituted alkane sulfonyl amino, benzene sulfonyl amino etc;

$R_2$ & $R_3$ independently represents hydrogen, alkyl, arylalkyl, substituted or unsubstituted 5-6 membered heterocyclic radical, alkanoyl, benzoyl groups etc.; A represents any of the following hetero-ring containing radicals

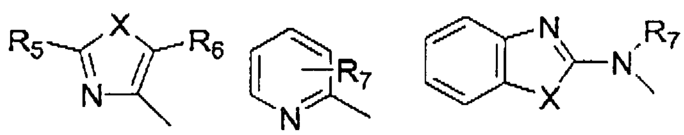

where $R_5$ & $R_6$ independently represent hydrogen, lower alkyl, phenyl, thienyl or furyl radical.

WO 0140170 (Astra Zeneca) discloses compounds with the general formula

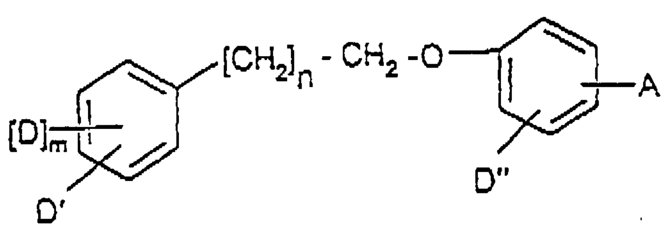

where 'A' represents

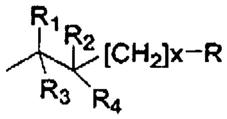

or

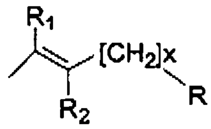

R is cyano when X=0 & when X = 1, then R = BRa or SCORa, where B is O, S, SO or SO2, wherein Ra represents hydrogen, alkyl, aryl or alkylaryl which may optionally be substituted; $R_1$, $R_2$, $R_3$ & $R_4$ are as defined in the specification. D represents alkyl, acyl, aryl, alkylaryl, halogen, -CN, - $NO_2$ each of which mey be substituted; D' represents hydrogen, alkyl, acyl, aryl etc. groups; while D" represents hydrogen, halogen, alkyl, acyl, aryl etc. groups.

WO 2004/031162 (Hoffman La Roche) discloses compounds with the general formula:

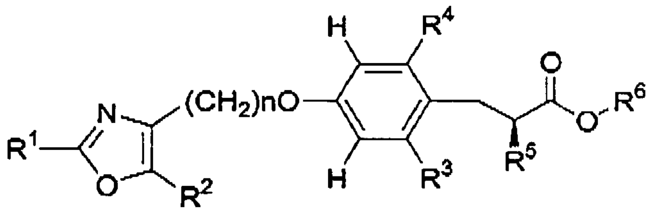

$R^1$ is aryl or heteroaryl;
$R^2$ is hydrogen, lower alkyl or fluoro-lower alkyl;
$R^3$ & $R^4$ are indepedently hydrogen, hydroxy, halogen, lower-alkyl, fluoro-lower-alkyl, hydroxy-lower-alkyl, lower-alkoxy-lower-alkyl etc. with the proviso that atleast one of $R^3$ & $R^4$ is not hydrogen;
$R^5$ is lower alkoxy, fluoro-lower-alkoxy, lower-alkenyloxy, fluoro-lower-alkenyloxy, aryloxy etc. groups
(this application thus, primarily claims the pure enantiomeric form of the compounds of formula (I); $R^6$ is hydrogen or lower alkyl; n =1-3.

WO 2004/004665 discloses a compound with the general formula:

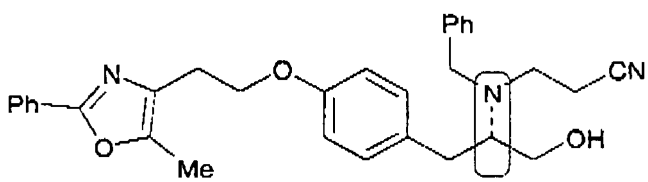

WO 03072102 (Eli Lily Co.) discloses compounds with the general structure:

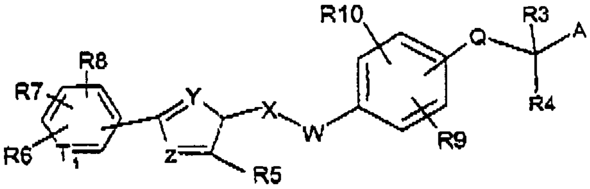

wherein $R_3$ is selected from hydrogen or alkoxy; $R_4$ is selected from hydrogen or alkyl; $R_b$ is selected from alkyl, alkenyl, aryl alkyl, aryloxy alkyl, arylthio alkyl; $R_6$ is selected from trifluoromethyl, trifluoromethoxy, hydroxy alkyl, alkyl, alkyl carbonyl etc.; $R_7$ & $R_8$ are independently selected from the group consisting of hydrogen, alkylor or trifluoromethyl; $R_9$ & $R_{10}$ are each independently selected from hydrogen, alkyl, alkenyl, halo and alkoxy; T1 is N or C; Q is selected from O, a single bond, O $(CH_2)$ q and C; W is selected from O, S, $SO_2$, $(CH_2)N(R_2O)$ $(CH_2)$ k etc; X is $CmH_2m$.

Several other oxazole derivatives useful in the treatment of diabetes, hyperlipidemia etc. (Syndrome X) have been reported for e.g. WO 03072100, WO 0320269, WO 0216332, WO 0218355, WO 0216331, WO 0216332, WO 0296895, WO 0296895, WO 0296894, WO 0296893, WO 0262774, WO 0250048, WO 0250047, WO 0276957, WO 0251820, WO 0214291, WO 0138325, WO 0116120, WO 0100403, WO 0116111, WO 0116120, WO 0179202, WO 0179197, WO 0008002, US 20010008898, JP 2002338555, JP 2001261612. However, very few of the compounds described above have reached the market and so there therefore remains the need to develop newer medicines which are better and cost effective, are of better or comparable efficacy with the present treatment regimes, has lesser side effects and require a lower dosage regime.

SUMMARY OF INVENTION

The objective of this invention is to develop novel compounds represented by the general formula (I) & (IIIa) used as hypocholesterolemic, hypolipidaemic, hypolipoproteinemic, anti-obesity and antihyperglycemic agents which may have additional body weight lowering effect and beneficial effect in the treatment and/or prophylaxis of diseases caused by hyperlipidaemia, diseases classified under syndrome X and atherosclerosis.

OBJECTIVES OF THE INVENTION

The main objective of the present invention is to provide novel substituted aralkyl derivatives represented by the general formula (I), their derivatives, their stereoisomers, their pharmaceutically acceptable salts, their pharmaceutically acceptable solvates, and pharmaceutical compositions containing them or mixtures thereof.

Another objective of the present invention is to provide novel substituted aralkyl derivatives represented by the general formula (I), their stereoisomers, their pharmaceutically acceptable salts, their pharmaceutically acceptable solvates, and pharmaceutical compositions containing them or mixtures thereof having enhanced activities, without toxic effects or with reduced toxic effect.

Yet another objective of this invention is to provide a process for the preparation of novel substituted aralkyl derivatives represented by the general formula (I), their derivatives, their stereoisomers, their pharmaceutically acceptable salts, their pharmaceutically acceptable solvates.

Still another objective of the present invention is to provide pharmaceutical compositions containing compounds of the general formula (I), their derivatives, their stereoisomers, their pharmaceutically acceptable salts, their pharmaceutically acceptable solvates or their mixtures in combination with suitable carriers, solvents, diluents and other media normally employed in preparing such compositions.

A further objective of the present invention is to provide novel substituted propanoic acid derivatives of formula (IIIa), their derivatives, their stereoisomers, their pharmaceutically acceptable salts, their pharmaceutically acceptable solvates, and pharmaceutical compositions containing them or mixtures thereof, useful as intermediates in the preparation of compounds of general formula (I).

Another objective of the present invention is to provide novel substituted propanoic acid derivatives represented by the general formula (IIIa), their derivatives, their stereoisomers, their pharmaceutically acceptable salts, their pharmaceutically acceptable solvates, and pharmaceutical compositions containing them or mixtures thereof having enhanced activities, without toxic effects or with reduced toxic effect.

Yet another objective of this invention is to provide a process for the preparation of novel substituted propanoic acid derivatives represented by the general formula (IIIa), their derivatives, their stereoisomers, their pharmaceutically acceptable salts, their pharmaceutically acceptable solvates.

Still another objective of the present invention is to provide pharmaceutical compositions containing compounds of the general formula (IIIa), their derivatives, their stereoisomers, their pharmaceutically acceptable salts, their pharmaceutically acceptable solvates or their mixtures in combination with suitable carriers, solvents, diluents and other media normally employed in preparing such compositions

**DETAILED DESCRIPTION OF THE INVENTION**

Accordingly, a first aspect of the present invention is directed to a compound of the general formula (I),

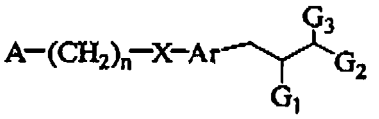

(I)

its derivatives, its stereoisomers, its pharmaceutically acceptable salts, its pharmaceutically acceptable solvates, wherein 'A' represents a substituted or unsubstituted, group selected from heteroaryl, heterocyclyl groups; 'n' is an integer from 1-3; 'X' represents oxygen; 'Ar' represents a substituted or unsubstituted phenyl group;

$G_1$ represents $OR_1$, $SR_1$, S (O) $R_3$, S $(O)_2R_3$, $N_3$, CN, COOH, tetrazolyl groups; $G_2$ represents $OR_1$, $NR_1R_2$, $SR_1$, S (O) R3, $S(O)_2R_3$, $N_3$, CN, COOH, tetrazolyl groups, $R_1$, $R_2$ represent hydrogen, substituted or unsubstituted groups selected from linear or branched $(C_1-C_8)$alkyl, acyl, alkoxycarbonyl, aryloxycarbonyl groups, aralkyloxycarbonyl groups; $R_3$ represents substituted or unsubstituted groups selected from alkyl, aryl groups; with the proviso that, when $G_2$ represents $NR_1R_2$, $G_1$ does not represent -OH group;

$G_3$ represents hydrogen or $(C_1-C_8)$ alkyl groups.

Suitable substituents on $R_1$, $R_2$ or $R_3$ may be same or different and independently selected from hydroxyl, oxo, halo, thio, nitro, amino, cyano, formyl, alkyl, haloalkyl, perhaloalkyl, alkoxy, aryl, aryloxy, aralkyl, aralkoxy, heterocylyl, heteroaryl, acyl, acyloxy, acylamino, monosubstituted or disubstituted amino, carbonylamino, hydroxyalkyl, alkylthio, thioalkyl groups.

When A is substituted, the substituents may be selected from hydroxyl, oxo, halo, thio, nitro, amino, cyano, formyl, or substituted or unsubstituted groups selected from amidino, guanidino, hydrazino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulfonylamino, alkylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfenyl derivatives, sulfonyl derivatives, sulfonic acid and its derivatives, phosphonic acid and its derivatives.

When the substituents on 'A' are further substituted, those substituents are selected from hydroxyl, oxo, halo, thio, nitro, amino, cyano, formyl, or substituted or unsubstituted groups selected from amidino, guanidino, hydrazino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulfonylamino, alkylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfenyl derivatives, sulfonyl derivatives, sulfonic acid and its derivatives, phosphonic acid and its derivatives

The substituents on the group represented by Ar represents substituted or unsubstituted linear or branched alkyl, alkoxy, thioalkyl, halogen, haloalkyl, haloalkoxy, acyl, amino, acylamino, thio or carboxylic or sulphonic acids and their derivatives, phosphonic acid and their derivatives.

Accordingly a second aspect of the present invention is directed to a compound of formula (IIIa), its tautomeric forms, its stereoisomers, its pharmaceutically acceptable salts, its pharmaceutically acceptable solvates,

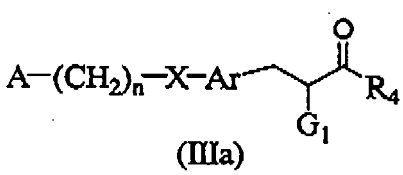

(IIIa)

wherein 'A' represents 4-oxazolyl group substituted with one or two substituents selected from substituted or unsubstituted linear or branched $(C_1-C_{12})$alkyl, substituted or unsubstituted single or fused heteroaryl or heterocyclic groups with the proviso that one of the substituants on "A" is always a heteroaryl or heterocyclic group; 'Ar' represents an unsubstituted

phenyl group; $G_1$ represents $OR_1$ where $R_1$ represents substituted or unsubstituted groups selected from linear or branched ($C_1$-$C_{12}$)alkyl groups; $R_4$ represents OH or alkoxy groups, 'n' is an integer from 1-3; X represents O.

Suitable substitutions on the substituents on 'A' are selected from hydroxyl, oxo, halo, thio, nitro, amino, cyano, formyl, or substituted or unsubstituted groups selected from amidino, guanidino, hydrazino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulfonylamino, alkylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulphenyl derivatives, sulphonyl derivatives, sulphonic acid and its derivatives, phosphonic acid and its derivatives; The compounds of formula (IIIa) are useful as intermediates for the preparation of compound of formula (I). In addition, compound of formula (IIIa) are also useful in presenting or reducing the risk of developing atherosclerosis, which leads to diseases and conditions such as artereosclerotic cardiovascular diseases, stroke, coronary heart diseases, cerebrovascular diseases, peripheral vessel diseases and related disorders. Also, the compounds of formula (IIIa) are useful in the treatment or prevention of diseases associated with Syndrome X.

The present invention also discloses novel processes for the preparation of compounds of formula (I) & (IIIa).

The various groups, radicals and substituents used anywhere in the specifications are described in the following paragraphs.

The term "alkyl" used herein, either alone or in combination with other radicals, denotes a linear or branched radical containing one to twelve carbons, such as methyl, ethyl, *n*-propyl, *iso*-propyl, *n*-butyl, *sec*-butyl, *tert*-butyl, amyl, *t*-amyl, *n*-pentyl, *n*-hexyl, *iso*-hexyl, heptyl, octyl and the like.

The term "alkenyl" used herein, either alone or in combination with other radicals, denotes a linear or branched radical containing two to twelve carbons such as vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl and the like. The term "alkenyl" includes dienes and trienes of straight and branched chains.

The term "alkynyl" used herein, either alone or in combination with other radicals, denotes a linear or branched radical containing two to twelve carbons, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 4-hexynyl, 5-hexynyl, and the like. The term "alkynyl" includes di- and tri-ynes.

The term "cycloalkyl" used herein, either alone or in combination with other radicals, denotes a radical containing three to seven carbons, such as cyclopropyl, cyclobutyl, cyclopentyl, cyclohexyl, cycloheptyl and the like.

The term "cycloalkenyl" used herein, either alone or in combination with other radicals, denotes a radical containing three to seven carbons, such as cyclopropenyl, 1-cyclobutenyl, 2-cylobutenyl, 1-cyclopentenyl, 2-cyclopentenyl, 3-cyclopentenyl, .1-cyclohexenyl, 2-cyclohexenyl, 3-cyclohexenyl, 1-cycloheptenyl, cycloheptadienyl, cycloheptatrienyl, and the like.

The term "alkoxy" used herein, either alone or in combination with other radicals, denotes an alkyl radical, as defined above, attached directly to an oxygen atom, such as methoxy, ethoxy, *n*-propoxy, *iso*-propoxy, *n*-butoxy, *t*-butoxy, *iso*-butoxy, pentyloxy, hexyloxy, and the like.

The term "alkenoxy" used herein, either alone or in combination with other radicals, denotes an alkenyl radical, as defined above, attached to an oxygen atom, such as vinyloxy, allyloxy, butenoxy, pentenoxy, hexenoxy, and the like.

The term "cycloalkoxy" used herein, either alone or in combination with other radicals, denotes a cycloalkyl radical as defined above, attached directly to an oxygen atom, such as cyclopropyloxy, cyclobutyloxy, cyclopentyloxy, cyclohexyloxy, cycloheptyloxy and the like.

The term "halo" or "halogen" used herein, either alone or in combination with other radicals, such as "haloalkyl", "perhaloalkyl" etc refers to a fluoro, chloro, bromo or iodo group. The term "haloalkyl" denotes a alkyl radical, as defined above, substituted with one or more halogens; such as perhaloalkyl, more preferably, perfluoro($C_1$-$C_6$)alkyl such as fluoromethyl, difluoromethyl, trifluoromethyl, fluoroethyl, difluoroethyl, trifluoroethyl, mono or polyhalo substituted methyl, ethyl; propyl, butyl, pentyl or hexyl groups. The term "haloalkoxy" denotes a haloalkyl, as defined above, directly attached to an oxygen atom, such as fluoromethoxy, chloromethoxy, fluoroethoxy chloroethoxy groups, and the like. The term "perhaloalkoxy" denotes a perhaloalkyl radical, as defined above, directly attached to an oxygen atom, trifluoromethoxy, trifluoroethoxy, and the like.

The term "aryl" or "aromatic" used herein, either alone or in combination with other radicals, denotes an aromatic system containing one, two or three rings wherein such rings may be attached together in a pendant manner or may be fused, such as phenyl, naphthyl, tetrahydronaphthyl, indane, biphenyl, and the like. The term 'aralkyl" denotes an alkyl group, as defined above, attached to an aryl, such as benzyl, phenethyl, naphthylmethyl, and the like. The term "aryloxy" denotes an aryl radical, as defined above, attached to an alkoxy group, such as phenoxy, naphthyloxy

and the like, which may be substituted. The term "aralkoxy" denotes an arylalkyl moiety, as defined above, such as benzyloxy, phenethyloxy, naphthylmethyloxy, phenylpropyloxy, and the like, which may be substituted.

The term "heterocyclyl" or "heterocyclic" used herein, either alone or in combination with other radicals, denotes saturated, partially saturated and unsaturated ring-shaped radicals, the heteroatoms selected from nitrogen, sulfur and oxygen. Examples of saturated heterocyclic radicals include aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, and the like; examples of partially saturated heterocyclic radicals include dihydrothiophene, dihydropyran, dihydrofuran, dihydrothiazole, and the like.

The term "heteroaryl" or "heteroaromatic" used herein, either alone or in combination with other radicals, denotes unsaturated 5 to 6 membered heterocyclic radicals containing one or more hetero atoms selected from O, N or S, such as pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzopyranyl, benzopyranonyl, benzofuranyl, benzothienyl, indolinyl, indolyl, azaindolyl, azaindolinyl, benzodihydrofuranyl, benzodihydrothienyl, pyrazolopyrimidinyl, pyrazolopyrimidonyl, azaquinazolinyl, azaquinazolinoyl, pyridofuranyl, pyridothienyl, thienopyrimidyl, thienopyrimidonyl, quinolinyl, pyrimidinyl, pyrazolyl, quinazolinyl, quinazolonyl, pyrimidonyl, pyridazinyl, triazinyl, benzoxazinyl, benzoxazinonyl, benzothiazinyl, benzothiazinonyl, benzoxazolyl, benzothiazolyl, benzimidazolyl, benzotriazolyl, phthalazynil, naphthylidinyl, purinyl, carbazolyl, phenothiazinyl, phenoxazinyl, and the like.

The term "heterocyclylalkyl" used herein, either alone or in combination with other radicals, represents a heterocyclyl group, as defined above, substituted with an alkyl group of one to twelve carbons, such as pyrrolidinealkyl, piperidinealkyl, morpholinealkyl, thiomorpholinealkyl, oxazolinealkyl, and the like, which may be substituted. The term "heteroaralkyl" used herein, either alone or in combination with other radicals, denotes a heteroaryl group, as defined above, attached to a straight or branched saturated carbon chain containing 1 to 6 carbons, such as (2-furyl)methyl, (3-furyl)methyl, (2-thienyl)methyl, (3-thienyl)methyl, (2-pyridyl)methyl, 1-methyl-1-(2-pyrimidyl)ethyl and the like. The terms "heteroaryloxy", "heteroaralkoxy", "heterocycloxy", "heterocylylalkoxy" denotes heteroaryl, heteroarylalkyl, heterocyclyl, heterocylylalkyl groups respectively, as defined above, attached to an oxygen atom.

The term "acyl" used herein, either alone or in combination with other radicals, denotes a radical containing one to eight carbons such as formyl, acetyl, propanoyl, butanoyl, iso-butanoyl, pentanoyl, hexanoyl, heptanoyl, benzoyl and the like, which may be substituted.

The term "acyloxy" used herein, either alone or in combination with other radicals, denotes a radical acyl, as defined above, directly attached to an oxygen atom, such as acetyloxy, propionyloxy, butanoyloxy, iso-butanoyloxy, benzoyloxy and the like.

The term "acylamino" used herein, either alone or in combination with other radicals, denotes an acyl group as defined earlier, may be $CH_3CONH$, $C_2H_5CONH$, $C_3H_7CONH$ $C_4H_9CONH$, $C_6H_5CONH$ and the like, which may be substituted.

The term "mono-substituted amino" used herein, either alone or in combination with other radicals, denotes an amino group, substituted with one group selected from $(C_1\text{-}C_6)$alkyl, substituted alkyl, aryl, substituted aryl or arylalkyl groups. Examples of monoalkylamino group include methylamine, ethylamine, *n*-propylamine, *n*-butylamine, *n*-pentylamine and the like.

The term 'disubstituted amino" used herein, either alone or in combination with other radicals, denotes an amino group, substituted with two radicals that may be same or different selected from $(C_1\text{-}C_6)$alkyl, substituted alkyl, aryl, substituted aryl, or arylalkyl groups, such as dimethylamino, methylethylamino, diethylamino, phenylmethyl amino and the like.

The term "arylamino" used herein, either alone or in combination with other radicals, denotes an aryl group, as defined above, linked through amino having a free valence bond from the nitrogen atom, such as phenylamino, naphthylamino, N-methyl anilino and the like.

The term "aralkylamino" used herein, either alone or in combination with other radicals, denotes an arylalkyl group as defined above linked through amino having a free valence bond from the nitrogen atom e.g. benzylamino, phenethylamino, 3-phenylpropylamino, 1-napthylmethylamino, 2-(1-napthyl)ethylamino and the like.

The term "oxo" or "carbonyl" used herein, either alone (-C=O-) or in combination with other radicals, such as "alkylcarbonyl", denotes a carbonyl radical (-C=O-) substituted with an alkyl radical such as acyl or alkanoyl, as described above.

The term "carboxylic acid" used herein, alone or in combination with other radicals, denotes a -COOH group, and includes derivatives of carboxylic acid such as esters and amides. The term "ester" used herein, alone or in combination with other radicals, denotes -COO- group, and includes carboxylic acid derivatives, where the ester moieties are alkoxycarbonyl, such as methoxycarbonyl, ethoxycarbonyl, and the like, which may be substituted; aryloxycarbonyl group such as phenoxycarbonyl, napthyloxycarbonyl, and the like, which may be substituted; aralkoxycarbonyl group such as benzyloxycarbonyl, phenethyloxycarbonyl, napthylmethoxycarbonyl, and the like, which may be substituted; heteroaryloxycarbonyl, heteroaralkoxycarbonyl, wherein the heteroaryl group, is as defined above, which may be substituted; heterocyclyloxycarbonyl, where the heterocyclic group, as defined earlier, which may be substituted.

The term "amide" used herein, alone or in combination with other radicals, represents an aminocarbonyl radical ($H_2N$-C=O-), wherein the amino group is mono- or di-substituted or unsubstituted, such as methylamide, dimethylamide, ethylamide, diethylamide, and the like. The term "aminocarbonyl" used herein, either alone or in combination with other radicals, with other terms such as 'aminocarbonylalkyl", "n-alkylaminocarbonyl", "N-arylaminocarbonyl", "N,N-dialkylaminocarbonyl", "N-alkyl-N-arylaminocarbonyl", "N-alkyl-N-hydroxyaminocarbonyl", and "N-alkyl-N-hydroxyaminocarbonylalkyl", substituted or unsubstituted. The terms "N-alkylaminocabonyl" and "N,N-dialkylaminocarbonyl" denotes aminocarbonyl radicals, as defined above, which have been substituted with one alkyl radical and with two alkyl radicals, respectively. Preferred are "lower alkylaminocarbonyl" having lower alkyl radicals as described above attached to aminocarbonyl radical. The terms "N-arylaminocarbonyl" and "N-alkyl-N-arylaminocarbonyl" denote amiocarbonyl radicals substituted, respectively, with one aryl radical, or one alkyl, and one aryl radical. The term "aminocarbonylalkyl" includes alkyl radicals substituted with aminocarbonyl radicals.

The term "hydroxyalkyl" used herein, either alone or in combination with other radicals, denotes an alkyl group, as defined above, substituted with one or more hydroxy radicals, such as hydroxymethyl, hydroxyethyl, hydroxypropyl, hydroxybutyl, hydroxypentyl, hydroxyhexyl and the like.

The term "aminoalkyl" used herein, alone or in combination with other radicals, denotes an amino (-$NH_2$) moiety attached to an alkyl radical, as defined above, which may be substituted, such as mono- and di-substituted aminoalkyl. The term "alkylamino" used herein, alone or in combination with other radicals, denotes an alkyl radical, as defined above, attached to an amino group, which may be substituted, such as mono- and di-substituted alkylamino.

The term "alkoxyalkyl" used herein, alone or in combination with other radicals, denotes an alkoxy group, as defined above, attached to an alkyl group, such as methoxymethyl, ethoxymethyl, methoxyethyl, ethoxyethyl and the like. The term "aryloxyalkyl" used herein, alone or in combination with other radicals, includes phenoxymethyl, napthyloxymethyl, and the like. The term "aralkoxyalkyl" used herein, alone or in combination with other radicals, includes $C_6H_5CH_2OCH_2$, $C_6H_5CH_2OCH_2CH_2$, and the like.

The term "alkylthio" used herein, either alone or in combination with other radicals, denotes a straight or branched or cyclic monovalent substituent comprising an alkyl group of one to twelve carbon atoms, as defined above, linked through a divalent sulfur atom having a free valence bond from the sulfur atom, such as methylthio, ethylthio, propylthio, butylthio, pentylthio and the like. Examples of cyclic alkylthio are cyclopropylthio, cyclobutylthio, cyclopentylthio, cyclohexylthio and the like, which may be substituted.

The term "thioalkyl" used herein, either alone or in combination with other radicals, denotes an alkyl group, as defined above, attached to a group of formula -SR', where R' represents hydrogen, alkyl or aryl group, e.g. thiomethyl, methylthiomethyl, phenylthiomethyl and the like, which may be substituted.

The term "arylthio' used herein, either alone or in combination with other radicals, denotes an aryl group, as defined above, linked through a divalent sulfur atom, having a free valence bond from the sulfur atom such as phenylthio, napthylthio and the like.

The term "alkoxycarbonylamino" used herein, alone or in combination with other radicals, denotes an alkoxycarbonyl group, as defined above, attached to an amino group, such as methoxycarbonylamino, ethoxycarbonylamino, and the like. The term "aryloxycarbonylamino" used herein, alone or in combination with other radicals, denotes an aryloxycarbonyl group, as defined above, attached to the an amino group, such as $C_6H_5OCONH$, $C_6H_5OCONCH_3$, $C_6H_5OCONC_2H_5$, $C_6H_4(CH_3O)CONH$, $C_6H_4(OCH_3)OCONH$, and the like. The term "aralkoxycarbonylamino" used herein, alone or in combination with other radicals, denotes an aralkoxycarbonyl group, as defined above, attached to an amino group $C_6H_5CH_2OCONH$, $C_6H_5CH_2CH_2CH_2OCONH$, $C_6H_5CH_2OCONHCH_3$, $C_6H_5CH_2OCONC_2H_5$, $C_6H_4(CH_3)CH_2OCONH$, $C_6H_4(OCH_3)CH_2OCONH$, and the like.

The term "aminocarbonylamino", "alkylaminocarbonylamino", "dialkylaminocarbonylamino" used herein, alone or in combination with other radicals, denotes a carbonylamino (-$CONH_2$) group, attached to amino($NH_2$), alkylamino group or dialkylamino group respectively, where alkyl group is as defined above.

The term "amidino" used herein, either alone or in combination with other radicals, denotes a -C(=NH)-$NH_2$ radical. The term "alkylamidino" denotes an alkyl radical, as discussed above, attached to an amidino group.

The term "hydrazino" used herein, either alone or in combination with other radicals, denotes NHNH-, suitably substituted with other radicals, such as alkyl hydrazino, where an alkyl group, as defined above is attached to a hydrazino group.

The term "alkoxyamino" used herein, alone or in combination with other radicals, denotes an alkoxy group, as defined above, attached to an amino group. The term "hydroxyamino" used herein, alone or in combination with other radicals, denotes -NHOH moiety, and may be substituted.

The term "sulfenyl" or "sulfenyl and its derivatives" used herein, alone or in combination with other radicals, denotes a bivalent group, -SO- or $R_xSO$, where $R_x$ is substituted or unsubstituted alkyl, aryl, heteroaryl, heterocyclyl, and the like.

The term "sulfonyl" or "sulfones and its derivatives" used herein, either alone or in combination with other

radicals, with other terms such as alkylsulfonyl, denotes divalent radical $-SO_2-$, or $R_xSO_2-$, where $R_x$ is substituted or unsubstituted groups selected from alkyl, aryl, heteroaryl, heterocyclyl, and the like. "Alkylsulfonyl" denotes alkyl radicals, as defined above, attached to a sulfonyl radical, such as methylsulfonyl, ethylsulfonyl, propylsulfonyl and the like. The term "arylsulfonyl" used herein, either alone or in combination with other radicals, denotes aryl radicals, as defined above, attached to a sulfonyl radical, such as phenylsulfonyl and the like.

The term "substituted" used alone or in combination with other radicals, denotes suitable substituents on that radical such as substituted alkyl, substituted alkenyl, substituted alkynyl, substituted cycloalkyl, substituted aryl, etc, mentioned anywhere in the specification. The suitable substituents include, but are not limited to the following radicals, alone or in combination with other radicals, such as, hydroxyl, oxo, halo, thio, nitro, amino, cyano, formyl, amidino, guanidino, hydrazino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulfonylamino, alkylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfenyl derivatives, sulfonyl derivatives, sulfonic acid and its derivatives, phosphonic acid and its derivatives.

Suitable groups and substituents on the groups may be selected from those described anywhere in the specification.

Particularly useful compounds according to the present invention includes
Ethyl (2S)-ethoxy-3-[4-(5-methyl-2-thiophen-2-yl-oxazol-4-ylmethoxy)-phenyl]-propanoate;
Ethyl (2S)-ethoxy-3-{4-[2-(5-methyl-2-thiophen-2-yl-oxazol-4-yl)-ethoxy]-phenyl}-propanoate;
Ethyl (2S)-ethoxy-3-{4-[5-methyl-2-(5-methyl-thiophen-2-yl)-oxazol-4-ylmethoxy]-phenyl}-propanoate;
Ethyl (2S)-ethoxy-3-(4-{2-[5-methyl-2-(5-methyl-thiophen-2-yl)-oxazol-4-yl]-ethoxy}-phenyl)-propanoate;
Ethyl (2S)-ethoxy-3-{4-[5-methyl-2-(3-methyl-thiophen-2-yl)-oxazol-4-ylmethoxy]-phenyl}-propanoate;
Ethyl (2S)-ethoxy-3-(4-{2-[5-methyl-2-(3-methyl-thiophen-2-yl)-oxazol-4-yl]-ethoxy}-phenyl)-propanoate;
Ethyl (2S)-ethoxy-3-[4-(5-methyl-2-thiophen-3-yl-oxazol-4-ylmethoxy)-phenyl]-propanoate;
Ethyl (2S)-ethoxy-3-{4-[2-(5-methyl-2-thiophen-3-yl-oxazol-4-yl)-ethoxy]-phenyl}-propanoate;
Ethyl 3-[4-(2-benzo[b]thiophen-2-yl-5-methyl-oxazol-4-ylmethoxy)-phenyl]-(2S)-ethoxy-propanoate;
Ethyl 3-{4-[2-(2-benzo[b]thiophen-2-yl-5-methyl-oxazol-4-yl)-ethoxy]-phenyl}-(2S)-ethoxy- propanoate;
Ethyl (2S)-ethoxy-3-[4-(2-furan-2-yl-5-methyl-oxazol-4-ylmethoxy)-phenyl]-propanoate;
Ethyl (2S)-ethoxy-3-{4-[2-(2-furan-2-yl-5-methyl-oxazol-4-yl)-ethoxy]-phenyl}-propanoate;
Ethyl (2S)-ethoxy-3-[4-(5-methyl-2-quinolin-2-yl-oxazol-4-ylmethoxy)-phenyl]-propanoate and its pharmaceutically acceptable salts;
Ethyl (2S)-ethoxy-3-{4-[2-(5-methyl-2-quinolin-2-yl-oxazol-4-yl)-ethoxy]-phenyl}-propanoate and its pharmaceutically acceptable salts;
Ethyl (2S)-ethoxy-3-{4-[3-(5-methyl-2-thiophen-2-yl-oxazol-4-yl)-propoxy]-phenyl}-propanoate;
Ethyl (2S)-ethoxy-3-{4-[5-methyl-2-(5-phenyl-thiophen-2-yl)-oxazol-4-ylmethoxy]-phenyl}-propanoate;
Ethyl (2S)-ethoxy-3-{4-[5-methyl-2-(5-chloro-thiophen-2-yl)-oxazol-4-ylmethoxy]-phenyl}-propanoate;
Ethyl (2S)-ethoxy-3-{4-[5-methyl-2-(5-bromo-thiophen-2-yl)-oxazol-4-ylmethoxy]-phenyl}-propanoate;
Ethyl (2S)-ethoxy-3-{4-[5-methyl-2-(5-methyl-furan-2-yl)-oxazol-4-ylmethoxy]-phenyl}-propanoate;
Ethyl (2S)-ethoxy-3-(4-{2-[5-methyl-2-(5-phenyl-thiophen-2-yl)-oxazol-4-yl]-ethoxy}-phenyl)-propanoate;
Ethyl (2S)-ethoxy-3-(4-{2-[5-methyl-2-(5-chloro-thiophen-2-yl)-oxazol-4-yl]-ethoxy}-phenyl)-propanoate;
Ethyl (2S)-ethoxy-3-(4-{2-[5-methyl-2-(5-bromo-thiophen-2-yl)-oxazol-4-yl]-ethoxy}-phenyl)-propanoate;
Ethyl (2S)-ethoxy-3-[4-(5-methyl-2-pyridin-2-yl-oxazol-4-ylmethoxy)-phenyl]-propanoate and its pharmaceutically acceptable salts;
Ethyl (2S)-ethoxy-3-[4-(5-methyl-2-pyridin-4-yl-oxazol-4-ylmethoxy)-phenyl]-propanoate and its pharmaceutically acceptable salts;
Ethyl (2S)-ethoxy-3-[4-{2-(5-methyl-2-pyridin-3-yl-oxazol-4-yl)-ethoxy}-phenyl]-propanoate and its pharmaceutically acceptable salts;
Ethyl (2S)-ethoxy-3-[4-(5-methyl-2-pyridin-3-yl-oxazol-4-ylmethoxy)-phenyl]-propanoate and its pharmaceutically acceptable salts;
(2S)-Ethoxy-3-[4-(5-methyl-2-thiophen-2-yl-oxazol-4-ylmethoxy)-phenyl]-propanoic acid and its pharmaceutically acceptable salts;
(2S)-Ethoxy-3-{4-[2-(5-methyl-2-thiophen-2-yl-oxazol-4-yl)-ethoxy]-phenyl}-propanoic acid and its pharmaceutically acceptable salts;
(2S)-Ethoxy-3-{4-[5-methyl-2-(5-methyl-thiophen-2-yl)-oxazol-4-ylmethoxy]-phenyl}- propanoic acid and its pharmaceutically acceptable salts;

(2S)-Ethoxy-3-(4-{2-[5-methyl-2-(5-methyl-thiophen-2-yl)-oxazol-4-yl]-ethoxy}-phenyl)-propanoic acid and its pharmaceutically acceptable salts;
(2S)-Ethoxy-3-{4-[5-methyl-2-(3-methyl-thiophen-2-yl)-oxazol-4-ylmethoxy]-phenyl}-propanoic acid and its pharmaceutically acceptable salts;
(2S)-Ethoxy-3-(4-{2-[5-methyl-2-(3-methyl-thiophen-2-yl)-oxazol-4-yl]-ethoxy}-phenyl)-propanoic acid and its pharmaceutically acceptable salts;
(2S)-Ethoxy-3-[4-(5-methyl-2-thiophen-3-yl-oxazol-4-ylmethoxy)-phenyl]-propanoic acid and its pharmaceutically acceptable salts;
(2S)-Ethoxy-3-{4-[2-(5-methyl-2-thiophen-3-yl-oxazol-4-yl)-ethoxy]-phenyl}-propanoic acid and its pharmaceutically acceptable salts;
3-[4-(2-Benzo[b]thiophen-2-yl-5-methyl-oxazol-4-ylmethoxy)-phenyl]- (2S)-ethoxy-propanoic acid and its pharmaceutically acceptable salts;
3-{4-[2-(2-Benzo[b]thiophen-2-yl-5-methyl-oxazol-4-yl)-ethoxy]-phenyl}-(2S)-ethoxy-propanoic acid and its pharmaceutically acceptable salts
(2S)-Ethoxy-3-[4-(2-furan-2-yl-5-methyl-oxazol-4-ylmethoxy)-phenyl]-propanoic acid and its pharmaceutically acceptable salts;
(2S)-Ethoxy-3-{4-[2-(2-furan-2-yl-5-methyl-oxazol-4-yl)-ethoxy]-phenyl}-propanoic acid and its pharmaceutically acceptable salts;
(2S)-Ethoxy-3-[4-(5-methyl-2-quinolin-2-yl-oxazol-4-ylmethoxy)-phenyl]-propanoic acid and its pharmaceutically acceptable salts;
(2S)-Ethoxy-3-{4-[2-(5-methyl-2-quinolin-2-yl-oxazol-4-yl)-ethoxy]-phenyl}-propanoic acid and its pharmaceutically acceptable salts;
(2S)-Ethoxy-3-{4-[3-(5-methyl-2-thiophen-2-yl-oxazol-4-yl)-propoxy]-phenyl}-propanoic acid and its pharmaceutically acceptable salts;
(2S)-Ethoxy-3-{4-[5-methyl-2-Benzofuran-2-yl)-oxazol-4-ylmethoxy]-phenyl}-propanoic acid and its pharmaceutically acceptable salts;
(2S)-Ethoxy-3-{4-[5-methyl-2-(5-chloro-thiophen-2-yl)-oxazol-4-ylmethoxy]-phenyl}-propanoic acid and its pharmaceutically acceptable salts;
(2S)-Ethoxy-3-{4-[5-methyl-2-(5-bromo-thiophen-2-yl)-oxazol-4-ylmethoxy]-phenyl}-propanoic acid and its pharmaceutically acceptable salts;
(2S)-Ethoxy-3-{4-[5-methyl-2-(5-methyl-furan-2-yl)-oxazol-4-ylmethoxy]-phenyl}-propanoic acid and its pharmaceutically acceptable salts;
(2S)-Ethoxy-3-(4-{2-[5-methyl-2-(5-phenyl-thiophen-2-yl)-oxazol-4-yl]-ethoxy}-phenyl)- propanoic acid and its pharmaceutically acceptable salts;
(2S)-Ethoxy-3-(4-{2-[5-methyl-2-(5-chloro-thiophen-2-yl)-oxazol-4-yl]-ethoxy}-phenyl)-propanoic acid and its pharmaceutically acceptable salts;
(2S)-Ethoxy-3-(4-{2-[5-methyl-2-(5-bromo-thiophen-2-yl)-oxazol-4-yl]-ethoxy}-phenyl)-propanoic acid and its pharmaceutically acceptable salts;
(2S)-Ethoxy-3-(4-{2-[5-methyl-2-(5-methyl-furan-2-yl)-oxazol-4-yl]-ethoxy}-phenyl)-propanoic acid and its pharmaceutically acceptable salts;
2(S)-Ethoxy-3-[4-(5-methyl-2-pyridin-2-yl-oxazol-4-ylmethoxy)-phenyl]- propanoic acid and its pharmaceutically acceptable salts;
2(S)-Ethoxy-3-[4-(5-methyl-2-pyridin-4-yl-oxazol-4-ylmethoxy)-phenyl]- propanoic acid and its pharmaceutically acceptable salts;
2(S)-Ethoxy-3-[4-(5-methyl-2-pyridin-3-yl-oxazol-4-ylmethoxy)-phenyl]- propanoic acid and its pharmaceutically acceptable salts;
3-(6-Benzyloxy-naphthalen-2-yl)-2-ethoxy-propan-1-ol;
(2S)-Ethoxy-3-{4-[2-(5-methyl-2-phenyl-oxazol-4-yl)-ethoxy]-phenyl}-propan-1-ol;
(2S)-Ethoxy-3-{4-(4-hydroxy-3-methyl-3,4-dihydro-quinazolin-2yl-methoxy)-phenyl}-propan-1-ol;
2-Hydroxy-3-{4-[2-(5-methyl-2-phenyl-oxazol-4-yl)-ethoxy]-phenyl}-propan-1-ol;
3-{4-[2-(2,3-Dihydro-benzo[1,4]thiazin-4-yl)-ethoxy]-phenyl}-(2S)-ethoxy-propan-1-ol;
(2S)-Ethoxy-3-[4-(2-(phenoxazin-10-yl)-ethoxy)-phenyl]-propan-1-ol;
3-[4-(2-(Carbazol-9-yl)-ethoxy)-phenyl]-(2S)-ethoxy-propan-1-ol;
3-{4-[2-(3,4-Dihydro-2H-quinolin-1-yl)-ethoxy]-phenyl}-(2S)-ethoxy-propan-1-ol;
(2S)-Ethoxy-3-[4-(2-(indol-1-yl)-ethoxy)-phenyl]-propan-1-ol;
(2S)-Ethoxy-3-[4-(2-(phenothiazin-10-yl)-ethoxy)-phenyl]-propan-1-ol;
3-{4-[2-(2,3-Dihydro-benzo[1,4]oxazin-4-yl)-ethoxy]-phenyl}-(2S)-ethoxy-propan-1-ol;
3-{4-[2-(5-Methyl-2-phenyl-oxazol-4-yl)-ethoxy]-phenyl}-2-phenylsulfanyl-propan-1-ol;

(2S)-Ethoxy-3-{4-[2-(methyl-pyridin-2-yl-amino)-ethoxy]-phenyl}-propan-1-ol and its pharmaceutically acceptable salts;
(2S)-Ethoxy-3-{4-[2-(5-ethyl-pyridin-2-yl)-ethoxy]-phenyl}-propan-1-ol and its pharmaceutically acceptable salts;
(2S)-Ethoxy-3-[4-(5-methyl-2-thiophen-2-yl-oxazol-4-ylmethoxy)-phenyl]-propan-1-ol;
(2S)-Ethoxy-3-{4-[2-(5-methyl-2-thiophen-2-yl-oxazol-4-yl)-ethoxy]-phenyl}-propan-1-ol;
(2S)-Ethoxy-3-(4-{2-[2-methyl-5-(4-methylsulfanyl-phenyl)-pyrrol-1-yl]-ethoxy}-phenyl)-propan-1-ol;
(3S)-Ethoxy-4-{4-[2-(5-methyl-2-phenyl-oxazol-4-yl)-ethoxy]-phenyl}-butan-1-ol;
(2S)-Ethoxy-3-(4-{2-[5-methyl-2-(4-methylsulfanyl-phenyl)-oxazol-4-yl]-ethoxy}-phenyl)-propan-1-ol;
(2S)-Amino-3-{4-[2-(5-methyl-2-phenyl-oxazol-4-yl)-ethoxy]-phenyl}-propan-1-ol and its pharmaceutically acceptable salts;
(2S)-tert-butoxycarbonylamino-3-[4-(5-methyl-2-phenyl-oxazol-4-yl-methoxy)-phenyl]- propan-1-ol;
(2S)-tert-butoxycarbonylamino-3-{4-[2-(5-methyl-2-phenyl-oxazol-4-yl)-ethoxy]-phenyl}-propan-1-ol;
(2S)-Ethoxy-3-(4-{2-[2-methyl-5-(benzofuran-2-yl)-pyrrol-1-yl]-ethoxy}-phenyl)-propan-1-ol;
(2S)-Ethoxy-3-(4-{2-[2-methyl-5-(benzo[1,3]dioxol-5-yl)-pyrrol-1-yl]-ethoxy}-phenyl)-propan-1-ol;
(2S)-Ethoxy-3-[4-(1-methyl-1H-benzoimidazol-2-yl methoxy)-phenyl]-propan-1-ol;
(2S)-Ethoxy-3-[4-(5-methyl-3-phenyl-isoxazol-4-ylmethoxy)-phenyl]-propan-1-ol;
(2S)-Ethoxy-3-{4-[2-(5-ethyl-pyridin-2-yl)-2-hydroxy-ethoxy]-phenyl}-propan-1-ol;
(2S)-Ethoxy-3-[4-(2-benzoimidazol-1-yl-ethoxy)-phenyl]-propan-1-ol;
(2S)-Ethoxy-3-[4-(5-methyl-2-phenyl-oxazol-4-ylmethoxy)-phenyl]-propan-1-ol;
(2S)-Ethoxy-3-(4-{2-[2-methyl-5-(5-methyl-thiophen-2-yl)-pyrrol-1-yl]-ethoxy}-phenyl)-propan-1-ol;
(2S)-Ethoxy-3-[4-(5-methyl-2-phenyl-oxazol-4-ylmethoxy)-phenyl]-propan-1,2-diol;
1-Ethoxy-(2S)-ethoxy-3-[4-{2-(5-methyl-2-phenyl-oxazol-4-yl)-ethoxy}-phenyl]-propane;
2-((2S)-Ethoxy-3-{4-[2-(5-methyl-2-phenyl-oxazol-4-yl)-ethoxy]-phenyl}-propoxy)-ethanol;
2-((2S)-Ethoxy-3-{4-[2-(5-methyl-2-phenyl-oxazol-4-yl)-ethoxy]-phenyl}-propoxy)-benzoic acid and its pharmaceutically acceptable salts;
(2S)-Ethoxy-3-{4-[2-(5-methyl-2-phenyl-oxazol-4-yl)-ethoxy]-phenyl}-propyl bromo acetate;
1-Ethoxy-(2S)-ethoxy-3-[4-{2-(-3,4-Dihydro-2H-benzo[1,4]thiazin-lyl) ethoxy}phenyl]- propane;
1-Propoxy-(2S)-ethoxy-3-[4-{2-(5-methyl-2-phenyl-oxazol-4-yl)ethoxy}-phenyl]-propane;
2-((2S)-Ethoxy-3-[4-(5-methyl-2-phenyl-oxazol-4-ylmethoxy)-phenyl]-propoxy)-benzoic acid and its pharmaceutically acceptable salts;
1-Ethoxy-(2S)-ethoxy-3-[4-(5-methyl-2-phenyl-oxazol-4-ylmethoxy)-phenyl]-propane;
(2S)-Ethoxy-3-[4-(5-methyl-2-phenyl-oxazol-4-ylmethoxy)-phenyl]-1-phenoxy propane;
(2S)-Ethoxy-1-ethyl sulfinyl-3-{4-(5-methyl-2-phenyl-oxazol-4-ylmethoxy)-phenyl}-propane;
(2S)-Ethoxy-1-ethyl sulfanyl-3-{4-(5-methyl-2-phenyl-oxazol-4-ylmethoxy)-phenyl}-propane;
(2S)-Ethoxy-3-[4-(5-methyl-2-phenyl-oxazol-4-ylmethoxy)-phenyl]-1-isopropoxy propane;
(3S)-Ethoxy-4-[4-(5-methyl-2-phenyl-oxazol-4-ylmethoxy)-phenyl]-butyronitrile;
(2S)-Ethoxy-1H-tetrazole-3-[4-(5-methyl-2-phenyl-oxazol-4-ylmethoxy)-phenyl]-propane;
2-Ethoxy-1-[4-(5-methyl-2-phenyl-oxazol-4-ylmethoxy)-phenyl]- pentane-3-ol;
2,3-Diethoxy-1-[4-(5-methyl-2-phenyl-oxazol-4-ylmethoxy)-phenyl]- pentane;
2-Ethoxy-1-{4-[2-(5-methyl-2-phenyl-oxazol-4-yl)-ethoxy]-phenyl}- pentane-3-ol;
2,3-Diethoxy-1-{4-[2-(5-methyl-2-phenyl-oxazol-4-yl)-ethoxy]-phenyl}- pentane;
((2S)-Ethoxy-3-{4-[2-(5-methyl-2-phenyl-oxazol-4-yl)-ethoxy]-phenyl}-propoxy)-acetic acid and its pharmaceutically acceptable salts;.
3-(4-Benzyloxy-phenyl)-(2S)-ethoxy-propyl-methanesulfonate;
(2S)-ethoxy-3-[4-(5-methyl-2-phenyl-oxazol-4-ylmethoxy)-phenyl]-propyl methane sulfonate;
(2S)-Ethoxy-3-{4-[2-(2-methyl-5-(5-methyl-thiophen-2-yl)-pyrrol-1-yl)-ethoxy]-phenyl}-propyl-methane sulfonate;
(2S)-Ethoxy-3-{4-[2-(5-methyl-2-phenyl-oxazol-4-yl)-ethoxy]-phenyl}-propyl-methane sulfonate;
(2S)-ethoxy-3-[4-(5-methyl-2-thiophen-2-yl-oxazol-4-ylmethoxy)-phenyl]}-propyl methanesulfonate;
(2S)-ethoxy-3-{4-[2-(5-ethyl-pyridin-2-yl)-ethoxy]-phenyl}- propyl methanesulfonate;
(2S)-Ethoxy-1-methoxy-3-{4-[2-(5-methyl-2-thiophen-2-yl-oxazol-4-yl)-ethoxy]-phenyl}- propane;
1-Ethoxy-(2S)-ethoxy-3-(4-{2-[5-methyl-2-(4-methylsulfanyl-phenyl)-oxazol-4-yl]-ethoxy}-phenyl)- propane;
1-Ethoxy-(2S)-ethoxy -3-{4-[2-(5-methyl-2-thiophen-2-yl-oxazol-4-yl)-ethoxy]-phenyl}- propane;
(2S)-Ethoxy-3-[4-(5-methyl-2-thiophen-2-yl-oxazol-4-ylmethoxy)-phenyl]-1-ethoxy propane;
(2S)-Ethoxy-3-{4-[2-(2-methyl-5-benzofuran-2-yl-pyrrol-1-yl)-ethoxy]-phenyl}-propyl-methanesulfonate;
(2S)-Ethoxy-3-{4-[2-(2-methyl-5-benzo[1,3]dioxol-5-yl-pyrrol-1-yl)-ethoxy]-phenyl}-propyl-methanesulfonate;
(2S)-Ethoxy-3-{4-[2-(2-methyl-5-benzofuran-2-yl-pyrrol-1-yl)-ethoxy]-phenyl}-propyl-(4-methyl phenyl)-sulfonate;
(2S)-Ethoxy-3-{4-[2-(2-methyl-5-benzo[1,3]dioxol-5-yl-pyrrol-1-yl)-ethoxy]-phenyl}-propyl-(4-methyl phenyl)-sulfonate;
1-Ethoxy-(2S)-ethoxy-3-[4-(1-methyl-1H-benzoimidazol-2-ylmethoxy)-phenyl]-propane;
(2S)-Ethoxy-3-{4-(5-methyl-2-phenyl-oxazol-4-ylmethoxy)-phenyl}-1-propoxy propane;

1-Ethoxy-(2S)-ethoxy-3-[4-(5-methyl-3-phenyl-isoxazol-4-yl methoxy)-phenyl]-propane;
(2S)-tert-Butoxycarbonylamino-3-[4-(5-methyl-2-phenyl-oxazol-4-ylmethoxy)-phenyl]- propyl methanesulfonate;
(2S)-Ethoxy-3-{4-[2-(5-methyl-2-phenyl-oxazol-4-yl)-ethoxy]-phenyl}-propyl amine and its pharmaceutically acceptable salts;
(2S)-Ethoxy-3-[4-{3-methyl-3H-quinazolin-4-on-2yl methoxy}phenyl]propyl amine and its pharmaceutically acceptable salts;
((2S)-Ethoxy-3-{4-[2-(5-methyl-2-phenyl-oxazol-4-yl)-ethoxy]-phenyl}-propyl)-isopropyl-amine and its pharmaceutically acceptable salts;
3-{4-[2-(2,3-Dihydro-benzo[1,4]thiazin-4-yl)-ethoxy]-phenyl}-(2S)-ethoxy-propyl amine and its pharmaceutically acceptable salts;
3-(4-Benzyloxy-phenyl)-(2S)-ethoxy-propyl amine and its pharmaceutically acceptable salts;
(2S)-Ethoxy-3-[4-(5-methyl-2-thiophen-2-yl-oxazol-4-ylmethoxy)-phenyl]-propylamine and its pharmaceutically acceptable salts.
(2S)-Ethoxy-3-[4-(5-methyl-2-phenyl-oxazol-4-ylmethoxy)-phenyl]-propylamine and its pharmaceutically acceptable salts;
N-tert-Butoxycarbonyl-(2S)-ethoxy-3-[4-(5-methyl-2-phenyl-oxazol-4-ylmethoxy)-phenyl]-propyl amine;
(2S)-Ethoxy-3-{4-[2-(2-methyl-5-(5-methyl-thiophen-2-yl)-pyrrol-1-yl)-ethoxy]-phenyl}-propylamine and its pharmaceutically acceptable salts;
N-((2S)-Ethoxy-3-{4-(5-methyl-2-phenyl-oxazol-4-ylmethoxy)-phenyl}-propyl)-methane sulfonamide;
(2S)-Ethoxy-3-[4-(5-methyl-2-phenyl-oxazol-4-ylmethoxy)-phenyl]-propylamine and its pharmaceutically acceptable salts;
[(2S)-Ethoxy-3-[4-(5-methyl-2-phenyl-oxazol-4-ylmethoxy)-phenyl]-propyl]-ethylamine and its pharmaceutically acceptable salts;
[(2S)-Ethoxy-3-[4-(5-methyl-2-phenyl-oxazol-4-ylmethoxy)-phenyl]-propyl]-isopropyl-amine and its pharmaceutically acceptable salts;
(2S)-Ethoxy-3-{4-[2-(2-methyl-5-benzo[1,3]dioxol-5-yl-pyrrol-1-yl)-ethoxy]-phenyl}-propylamine and its pharmaceutically acceptable salts;
(2S)-Ethoxy-3-{4-[2-(2-methyl-5-benzofuran-2-yl-pyrrol-1-yl)-ethoxy]-phenyl}-propylamine and its pharmaceutically acceptable salts;
N-[(2S)-Ethoxy-3-[4-(5-methyl-2-phenyl-oxazol-4-ylmethoxy)-phenyl]-propyl]-2,2,2-trifluoro-acetamide;
N-Ethoxycarbonyl-((2S)-ethoxy-3-{4-(5-methyl-2-phenyl-oxazol-4-ylmethoxy)-phenyl}-propyl)amine;
N-Benzyloxycarbonyl-((2S)-ethoxy-3-{4-(5-methyl-2-phenyl-oxazol-4-ylmethoxy)-phenyl}-propyl)amine;
N-[(2S)-Ethoxy-3-[4-(5-methyl-2-phenyl-oxazol-4-ylmethoxy)-phenyl]-propyl]-acetamide;
(2S)-Hydroxy-3-{4-[2-(5-methyl-2-phenyl-oxazol-4-yl)-ethoxy]-phenyl}-propyl azide;
3-(4-Benzyloxy-phenyl)-(2S)-ethoxy-propyl azide;
(2S)-Ethoxy-3-{4-[2-(5-methyl-2-phenyl-oxazol-4-yl)-ethoxy]-phenyl}-propyl azide;
(2S)-Ethoxy-3-{4-[2-(5-ethyl-pyridin-2-yl)-ethoxy]-phenyl}- propyl azide and its pharmaceutically acceptable salts;
(2S)-Ethoxy-3-[4-(5-methyl-2-phenyl-oxazol-4-ylmethoxy)-phenyl]- propyl azide;
(2S)-Ethoxy-3-{4-[2-(2-methyl-5-(5-methyl-thiophen-2-yl)-pyrrol-1-yl)-ethoxy]-phenyl}- propyl azide;
(2S)-Ethoxy-3-{4-[2-(5-ethyl-pyridine-2-yl)-2-(tert-butyldimethyl-silanyloxy)-ethoxy]-phenyl}- propyl azide and its pharmaceutically acceptable salts;
(2S)-Ethoxy-3-{4-[2-(5-ethyl-pyridine-2-yl)-2-hydroxy-ethoxy]-phenyl}- propyl azide and its pharmaceutically acceptable salts;
(2S)-Ethoxy-3-{4-[2-(methyl-pyridin-2-yl-amino)-ethoxy]-phenyl}- propyl azide and its pharmaceutically acceptable salts;
(2S)-Ethoxy-3-[4-(5-methyl-2-thiophen-2-yl-oxazol-4-ylmethoxy)-phenyl]- propyl azide;
(2S)-Ethoxy-3-{4-[2-(5-methyl-2-thiophen-2-yl-oxazol-4-yl)-ethoxy]-phenyl}-propyl azide;
(2S)-Hydroxy-3-[4-(5-methyl-2-phenyl-oxazol-4-yl)-methoxy)-phenyl]- propyl azide;
(2S)-Amino-3-{4-[2-(5-methyl-2-phenyl-oxazol-4-yl)-ethoxy]-phenyl}- propyl azide and its pharmaceutically acceptable salts;
(2S)-Amino-3-{4-[2-(5-methyl-2-phenyl-oxazol-4-yl)-methoxy]-phenyl}- propyl azide and its pharmaceutically acceptable salts;
(2S)-tert-butoxycarbonylamino-3-[4-(5-methyl-2-phenyl-oxazol-4-ylmethoxy)-phenyl]-propyl azide;
(2S)-tert-butoxycarbonylamino-3-[4-(5-methyl-2-phenyl-oxazol-4-yl-ethoxy)-phenyl]-propyl azide;
(2S)-Ethoxy-3-{4-[2-(2-methyl-5-benzo[1,3]dioxol-5-yl-pyrrol-1-yl)-ethoxy]-phenyl}-propyl azide;
(2S)-Ethoxy-3-{4-[2-(2-methyl-5-benzofuran-2-yl-pyrrol-1-yl)-ethoxy]-phenyl}-propyl azide;
N-Benzyloxycarbonyl-(2S)-ethoxy-3-{4-[2-(5-methyl-2-phenyl-oxazol-4-yl)-ethoxy]-phenyl}-propyl amine;
N-tert-Butoxycarbonyl-(2S)-Ethoxy-3-{4-[2-(5-methyl-2-phenyl-oxazol-4-yl)-ethoxy]-phenyl}- propyl amine;
N-tert-Butoxycarbonyl-3-{4-[2-(2,3-dihydro-benzo[1,4]thiazin-4-yl)-ethoxy]-phenyl}-(2S)-ethoxy-propyl amine;

N-((2S)-Ethoxy-3-{4-[2-(5-methyl-2-phenyl-oxazol-4-yl)-ethoxy]-phenyl}-propyl)-acetamide;
3-(4-Benzyloxy-phenyl)-N-tert-butoxycarbonyl-(2S)-ethoxy-propyl amine;
N-tert-Butoxycarbonyl-(2S)-ethoxy-3-(4-hydroxy-phenyl)- -propyl amine;
N-tert-Butoxycarbonyl-(2S)-ethoxy-3-{4-[2-(5-ethyl-pyridin-2-yl)-ethoxy]-phenyl}-propyl amine;
(2S)-Ethoxy-1-ethylsulfanyl-3-{4-[2-(5-methyl-2-phenyl-oxazol-4-yl)-ethoxy]-phenyl}-propane;
(2S)-Ethoxy-1-ethylsulfonyl-3-{4-[2-(5-methyl-2-phenyl-oxazol-4-yl)-ethoxy]-phehyl}-propane;

The compounds of the general formula (I), namely (Ia), (Ib), (Ic), (Id) and (Ie) may be prepared by one or more methods described in scheme I.

Scheme I:

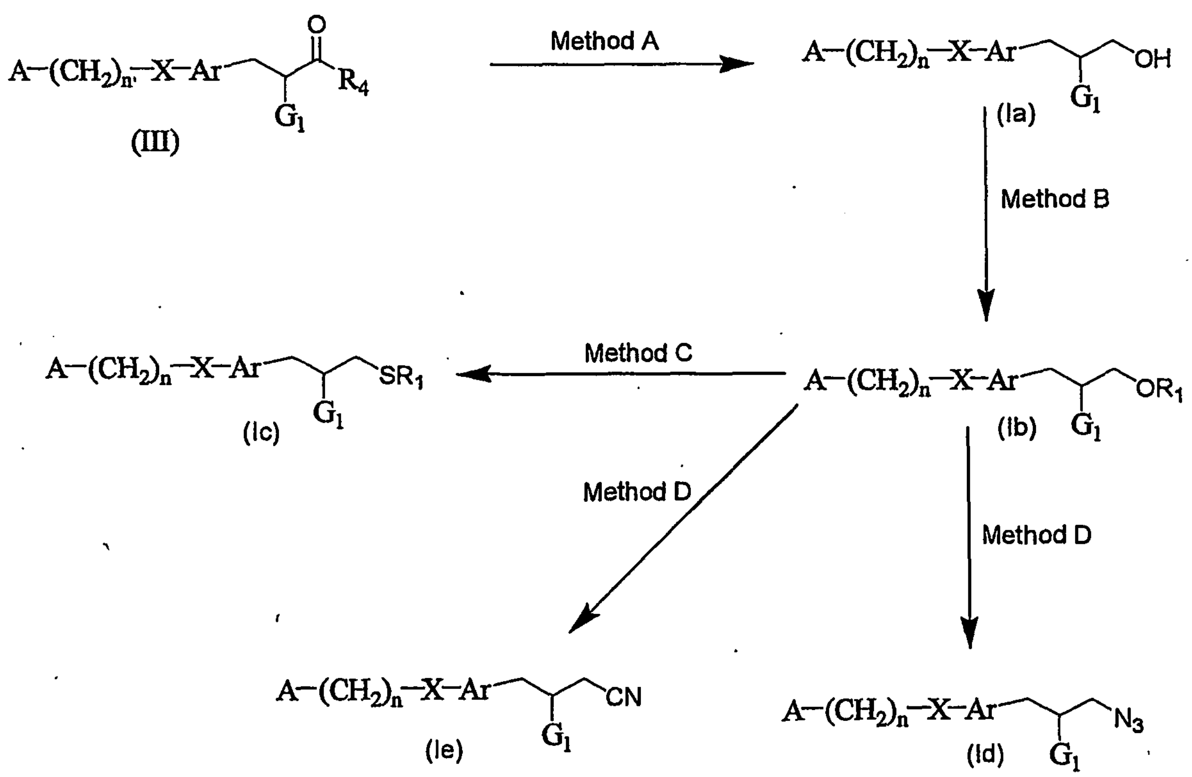


i) The compounds of general formula (Ia) wherein all the symbols are as defined earlier may be prepared by reduction of compounds of general formula (III) wherein all the symbols are as defined earlier and $R_4$ represents OH, alkoxy, aryloxy or aralkoxy and the like.

ii) The compounds of general formula (Ia) wherein all the symbols are as defined earlier may be converted to compounds of general formula (Ib) wherein all the symbols are as defined earlier by alkylation, acylation or sulfonation.

iii) The compounds of general formula (Ib) wherein all the symbols are as defined earlier and $OR_1$ represents leaving groups such as mesylate, tosylate and the like are converted to compounds of general formula (Ic), (Id), or (Ie) wherein all the symbols are as defined earlier by reacting with metal salts of alcohols, phenols, thiols, sodium azide or sodium or potassium cyanide respectively.

Method A: The compounds of general formula (III) are reduced to compounds of general formula (Ia) by using suitable reducing agents such as $LiAlH_4$, $NaBH_4$, diborane, $NaBH_4/BF_3OEt_2$, $LiBH_4$, DIBAH, and the like. Suitable solvents appropriate for the reducing agent used may be employed for e.g. with $LiAlH_4$, $NaBH_4$, diborane, $NaBH_4/BF_3OEt_2$ aprotic solvents such as THF, ether and the likes are preferred. With $NaBH_4$, $LiBH_4$ etc. alcoholic solvents may also be used. Reaction may be carried out at temperatures ranging from 0 °C to the reflux temperature of the solvent(s) used. Inert atmosphere may be maintained using $N_2$, He, or argon gas. Reaction time may range from 1 to 48 hours.

Method B: The compounds of general formula (Ia) may be alkylated, acylated or sulfonated to corresponding compounds of general formula (Ib). Alkyl halides, mesylates or tosylates and the like may be employed for alkylation.

Acyl halides or anhydrides and suitable sulfonyl halides may be used for acylation and sulfonation respectively. Suitable bases like metal hydrides e.g NaH and the like, alkali metal carbonates e.g. potassium carbonate, sodium carbonate and the like, sodium hydroxide, potassium hydroxide, organic bases e.g. trialkyl amines and the like may be used. Reaction may be carried out in suitable solvents like DMF, DMSO, THF, acetone, dichloromethane, toluene and the like or mixtures thereof. Inert atmosphere may be maintained using $N_2$, He, or argon gas. reaction time may range from 1 to 48 hours.

Method C: The compounds of general formula (Ib) where $OR_1$ represents leaving groups such as mesyl, tosyl and the like may be converted to compounds of general formula (Ic) by reacting with thiols in the presence of bases like NaH, KH, Na metal, potassium carbonate, sodium hydroxide, potassium hydroxide and the like. Reaction may be carried out in solvents like DMF, DMSO, toluene, acetone, THF and the like or mixtures thereof. Reaction temperatures may range from 0 °C to the reflux temperature of the solvent(s) used. Inert atmosphere may be maintained using $N_2$, He, or argon gas. reaction time may range from 1 to 48 hours.

Method D: The compounds of general formula (Ib) where $OR_1$ represents leaving groups such as mesyl, tosyl and the like may be converted to the compounds of general formula (Id) or (Ie) by reacting with metal azides e.g. sodium azide or the like or cyanides e.g. sodium cyanide or potassium cyanide and the like respectively. Reaction may be carried out in solvents like DMF, DMSO, Toluene, THF and the like or fixtures thereof. Reaction temperatures may range from 0 °C to the reflux temperature of the solvent(s) used. Inert atmosphere may be maintained using $N_2$, He, or argon gas.

Reaction time may range from 1 to 72 hours.

The compounds of the general formula (If), and (Ig) may be prepared by one or more methods described in scheme II.

Scheme II:

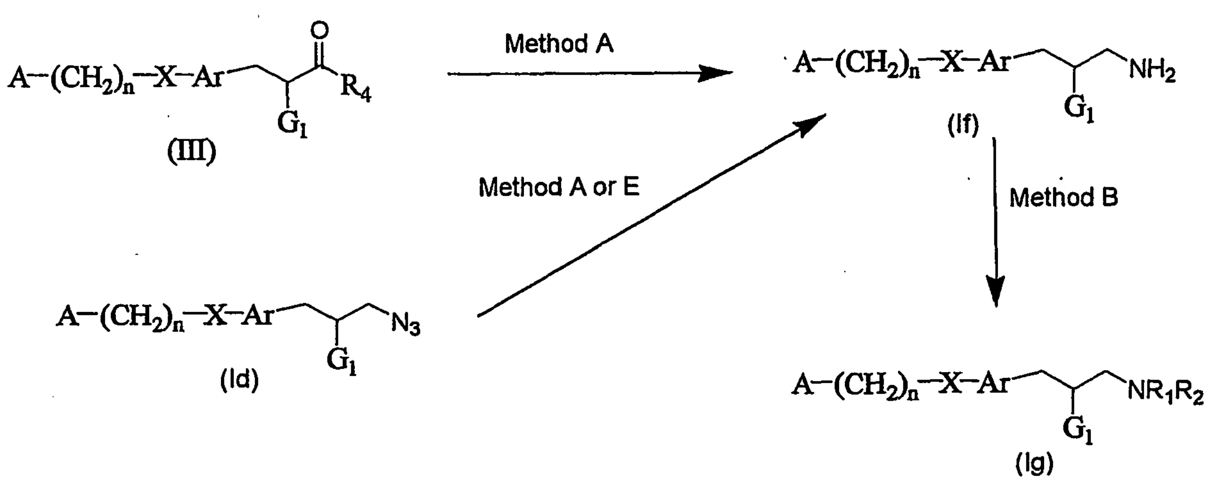


i) The compounds of general formula (III) wherein all the symbols are as defined earlier and $R_4$ represents $NH_2$, $NR_1R_2$ where $R_1$ and $R_2$ are as defined earlier are reduced to compounds of general formula (If) where in all the symbols are as defined earlier.

ii) The compounds of general formula (If) wherein all the symbols are as defined earlier are converted to compounds of general formula (Ig), wherein all the symbols are as defined earlier by alkylation or acylation.

iii) The compounds of general formula (Id) wherein all the symbols are as defined earlier are reduced to compounds of general formula (If) wherein all the symbols are as defined earlier.

Method A: The compounds of general formula (III) where $R_4$ represents $NH_2$ or $NR_1R_2$ where $R_1$ and $R_2$ are as defined earlier may be reduced to compounds of general formula (If) by a procedure similar to that described in method A of scheme I

Method B: The compounds of the general formula (If) may be converted to compounds of general formula (Ig) by a procedure similar to that described in method B of scheme I.

Method E: The compounds of general formula (Id) may be reduced to compounds of general formula (If), using

suitable reducing agents e.g. Pd on charcoal, Raney Ni and the like. Suitable solvents like alcohols, ethyl acetate and the like or the mixtures thereof may be used. Reaction may be carried out under a pressure of hydrogen gas. Reaction temperature may range from 0 °C to the reflux temperature of the solvent(s) used. Reaction may also be carried out using the presence of $PPh_3$ in moist solvents such as moist THF.

The compounds of the general formula (I) may also be prepared by the general method described in scheme III

Scheme III

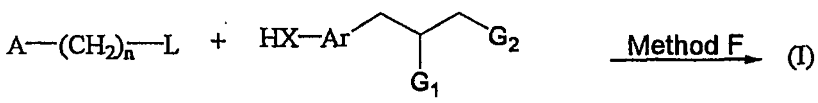


(IV) (V)

Further the compounds of the general formula (If), and (Ig) may be prepared by one or more methods described in scheme III (a)

Scheme III(a):

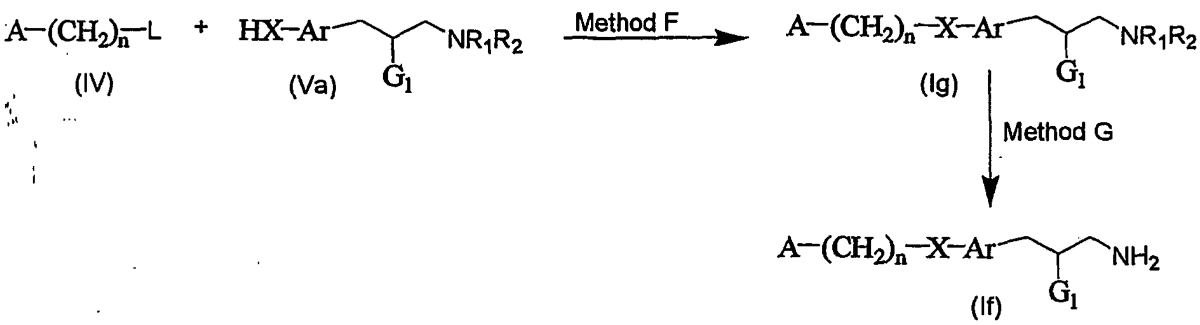


(IV) (Va) (Ig) (If)

i) The compound of the general formula (Ig) wherein all the symbols are as defined earlier may be prepared by reacting compounds of general formula (IV) with compounds of general formula (Va), wherein all symbols are as defined earlier and L represents a leaving group such as halogen, mesylate, tosylate, triflate & the like.
ii) The compound of general formula (Ig), when one of $R_1$ and $R_2$ is hydrogen and the other is acyl e.g. tert butoxy carbonyl or benzyloxy carbonyl and the like may optionally be converted to the compounds of general formula (If).
Compounds of the general formula (Ia) and (Ib) may be prepared by one or more methods described in scheme III (b)

Scheme III(b):

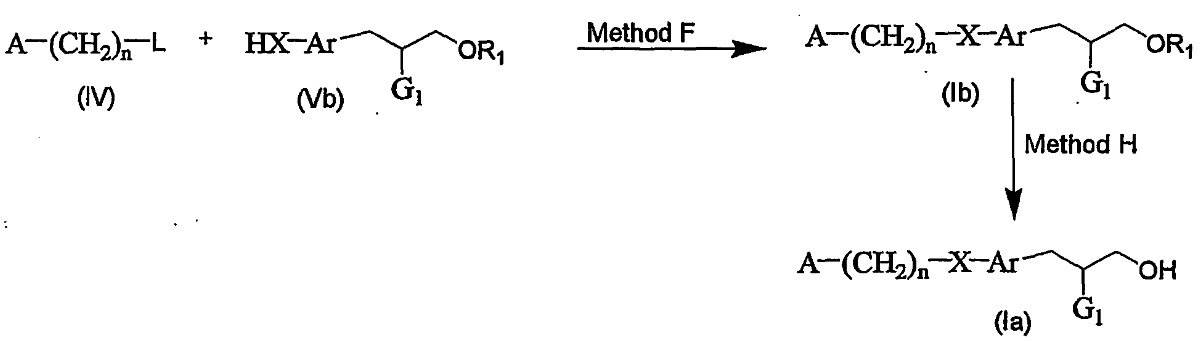


(IV) (Vb) (Ib) (Ia)

i) The compound of the general formula (Ib) wherein all the symbols are as defined earlier may be prepared by reacting compounds of general formula (IV) with compounds of general formula (Vb), wherein all symbols are as

defined earlier and L represents a leaving group such as halogen, mesylate, tosylate, triflate & the like.
ii) The compound of general formula (Ib), when $R_1$ represents acyl, benzyl, alkoxycarbonyl, aralkoxycarbonyl and the like may optionally be converted to the compounds of general formula (Ia).
Method F: The compound of the general formula (Ig) or (Ib) may be prepared by reacting compounds of general formula (IV) where L represents a leaving group such as halogen, mesylate, tosylate, triflate & the like, with compounds of general formula (Va) or (Vb) respectively. Suitable bases like metal hydrides e.g NaH and the like, alkali metal carbonates e.g. potassium carbonate, sodium carbonate and the like, sodium hydroxide, potassium hydroxide, organic bases e.g. trialkyl amines and the like may be used. Reaction may be carried out in suitable solvents like DMF, DMSO, THF, acetone, dichloromethane, toluene and the like or the mixture thereof. Reaction temperature may range from 0 ˚C to the reflux temperature of the solvent(s) used. Inert atmosphere may be maintained using $N_2$, He, or argon gas. Reaction time may range from 1 to 72 hours.
Method G: The compound of general formula (Ig) wherein one of the $R_1$ and $R_2$ is hydrogen and the other is acyl e.g. tert butoxycarbonyl or benzyloxycarbonyl and the like may optionally be converted to the compounds of general formula (If) by using suitable deacylation methods e.g trifluroacetic acid to deprotect tertbutoxycarbonyl or hydrogenation using Pd/C and the like under hydrogen pressure to deprotect benzyloxy carbonyl groups. Suitable solvents appropriate for the reagent used may be used .e.g chlorinated hydrocarbons like dichloromethane and the like may be used along with trifluoroacetic acid. Alcohols are preferred for hydrogenation. Reaction temperature may range from 0 ˚C to the reflux temperature of the solvent(s) used. Reaction time may range from 1 to 72 hours.
Method H: The compound of general formula (Ib) wherein $R_1$ is acyl, benzyl, alkoxycarbonyl, aralkoxycarbonyl and the like may optionally be converted to the compounds of general formula (Ia) by using suitable deacylation or debenzylation methods e.g acidic or alkaline hydrolysis to deprotect acyl group or hydrogenation using Pd/C and the like under hydrogen pressure to deprotect benzyl group. Suitable solvents appropriate for the reagent used may be used .e.g aqueous alcohols are used for hydrolysis reactions. Alcohols, ester solvents or dioxane are preferred for hydrogenation. Reaction temperature may range from 0 ˚C to the reflux temperature of the solvent(s) used. Reaction time may range from 1 to 72 hours.

The compounds of formula (IIIa) may be prepared according to the following general scheme

Scheme III(c)

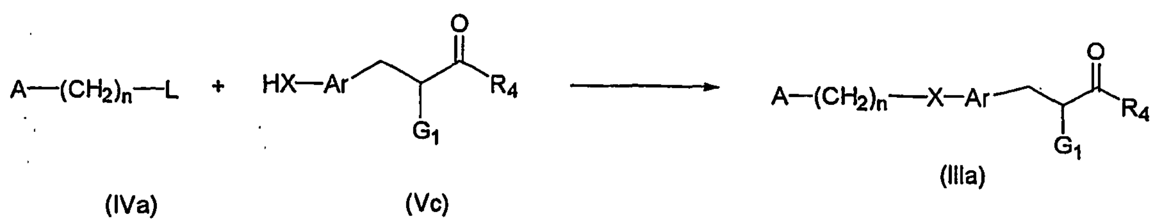

(IVa) (Vc) (IIIa)

i. reacting a compound of formula (IVa) wherein 'A' represents 4-oxazolyl group substituted with one or two substitutents selected from substituted or unsubstituted linear or branched ($C_1$-$C_{12}$)alkyl, substituted or unsubstituted single or fused heteroaryl or heterocyclic groups; 'Ar' represents unsubstituted divalent phenyl; $G_1$ represents $OR_1$ or $SR_1$, where $R_1$ represents hydrogen, perfluoro($C_1$-$C_{12}$)alkyl, substituted or unsubstituted groups selected from linear or branched ($C_1$-$C_{12}$)alkyl, cyclo($C_1$-$C_{12}$)alkyl, aryl, ar($C_1$-$C_{12}$)alkyl, heteroaryl, heteroar($C_1$-$C_{12}$)alkyl, heterocyclyl, alkoxyalkyl, aryloxyalkyl, alkoxycarbonyl, aryloxycarbonyl, cycloalkyloxycarbonyl, alkylaminocarbonyl, arylaminocarbonyl or acyl groups; $R_4$ represents OH, alkoxy or aryloxy, aralkoxy or $NR_1R_2$ groups, where $R_1$ & $R_2$ are as defined earlier; 'n' is an integer from 1-3; X represents O or S, by a process similar to that described in Method F above.

ii. optionally hydrolysing the compound of formula (IIIa) wherein $R_4$ represents alkoxy, aralkoxy, aryloxy or $NR_1R_2$ groups, where $R_1$ & $R_2$ are as defined earlier, to a further compound of formula (IIIa) wherein $R_4$ represents OH.

The compounds (I) & (IIIa) of the present invention may have asymmetric centers and may occur either as racemates or racemic mixtures as well as individual stereoisomers, including optical isomers, being included in the present invention Mixture of stereoisomers may be resolved by conventional methods such as microbial resolution,

resolving the diastereomeric salts formed with chiral acids or chiral bases. Chiral acids may be tartaric acid, mandelic acid, lactic acid, camphorsulfonic acid, amino acids and the like. Chiral bases may be cinchona alkaloids, (+) or (-) brucine, α-methyl benzylamine, (+) or (-) phenyl glycinol, ephedrine, amino sugars such as glucosamines or a basic amino acid such as lysine, arginine and the like.

The compounds (I) & (IIIa) of the present invention may have asymmetric centers and may occur either as racemates or racemic mixtures as well as individual diastereomers of any of the possible isomers, including optical isomers, being included in the present invention These can be isolated using conventional techniques known to persons skilled in the art (Jaques et al. "Enantiomers, Racemates and Resolution", Wiley Interscience, 1981; R A. Sheldon, in "Chirotechnology", Marcel Dekker, Inc. NY, Basel, 1993, 173-204 and references therein; A. N. Collins, G. N. Sheldrack and J Crosby, in "Chirality in Industry II", John Wiley & Sons, Inc, 1997, 81-98 and references therein; E. L. Eliel and S. H. Wilen, in "Stereochemistry of Organic Compound", John Wiley & Sons, Inc, 1999, 297-464 and references therein).

It will be appreciated that in any of the above mentioned reactions any reactive group in the substrate molecule may be protected, according to conventional chemical practice. Suitable protecting groups in any of the above mentioned reactions are those used conventionally in the art. The methods of formation and removal in such protecting groups are those conventional methods appropriate to the molecule being protected. T. W. Greene and P. G. M.. Wuts "Protective groups in Organic Synthesis", John Wiley & Sons, Inc, 1999, 3rd Ed., 201-245 along with references therein.

It will be appreciated that the above-mentioned preparation of the compounds of Formula (I) or (IIIa), or pharmaceutically acceptable salts thereof, and/or pharmaceutically acceptable solvate thereof is a stereoselective procedure and that the compounds of formula (I) or (IIIa), is a single stereoisomer. Favorably, a compound of formula (I) or (IIIa), is present in admixture with less than 50% w/w of its racemic isomer, suitably 80 - 100 % and preferably 90 - 100 % pure, such as 90 - 95 %, most preferably 95-100 %, for example 95 %, 96 %, 97 %, 98 %, 99 % and 99.99 % optically pure.

Preferably the compounds of Formula (I) or (IIIa), or a pharmaceutically acceptable salt thereof, and/or pharmaceutically acceptable solvate thereof is in optically pure form.

The absolute stereochemistry of the compounds may be determined using conventional methods, such as X-ray crystallography.

It will be appreciated that when substituents have different sites where they can be attached, such differently attached substituents are also included in the present invention.

"Pharmaceutically acceptable salt", where such salts are possible, includes both pharmaceutically acceptable acid and base addition salts. The pharmaceutically acceptable base addition salts forming a part of this invention may be prepared by treating suitable compounds of the invention with 1-6 equivalents of a base such as sodium hydride, sodium methoxide, sodium ethoxide, sodium hydroxide, potassium tert-butoxide, calcium hydroxide, calcium acetate, calcium chloride, magnesium hydroxide, magnesium chloride, magnesium acetate, magnesium alkoxide and the like. Solvents such as water, acetone, ether, THF, methanol, ethanol, t-butanol, 2-butanone, dioxane, propanol, butanol, isopropanol, diisopropyl ether, tert-butyl ether or mixtures thereof may be used. Organic bases such as lysine, arginine, methyl benzylamine, ethanolamine, diethanolamine, tromethamine, choline, guanidine and their derivatives may be used. Acid addition salts, wherever applicable may be prepared by treatment with acids such as tartaric acid, mandelic acid, fumaric acid, malic acid, lactic acid, maleic acid, salicylic acid, citric acid, ascorbic acid, benzene sulfonic acid, p-toluene sulfonic acid, hydroxynaphthoic acid, methane sulfonic acid, acetic acid, benzoic acid, succinic acid, palmitic acid, hydrochloric acid, hydrobromic acid, sulfuric acid, nitric acid and the like in solvents such as water, alcohols, ethers, ethyl acetate, dioxane, THF, acetonitrile, DMF or a lower alkyl ketone such as acetone, or mixtures thereof.

Another aspect of the present invention comprises a pharmaceutical composition, containing at least one of the compounds of the general formula (I) or (IIIa), their derivatives, their analogs, their tautomeric forms, their stereoisomers, their pharmaceutically acceptable salts, their pharmaceutically acceptable solvates thereof as an active ingredient, together with pharmaceutically employed carriers diluents and the like.

Pharmaceutical compositions containing a compound of the present invention may be prepared by conventional techniques, e.g. as described in Remington: the Science and Practice of Pharmacy, 19th Ed., 1995. The compositions may be in the conventional forms, such as capsules, tablets, powders, solutions, suspensions, syrups, aerosols or topical applications. They may contain suitable solid or liquid carriers or in suitable sterile media to form injectable solutions or suspensions. The compositions may contain 0.5 to 20 %, preferably 0.5 to 10 % by weight of the active compound, the remaining being pharmaceutically acceptable carriers, excipients, diluents, solvents and the like.

Typical compositions containing a compound of formula (I) or (IIIa) or a pharmaceutically acceptable acid addition salt thereof, associated with a pharmaceutically acceptable excipients which may be a carrier or a diluent or be diluted by a carrier, or enclosed within a carrier which can be in the form of a capsule, sachet, paper or other container. When the carrier serves as a diluent, it may be a solid, semisolid, or liquid material, which acts as a vehicle, excipients or medium for the active compound. The active compound can be absorbed on a granular solid container for example in a sachet. Some of suitable carriers are water, salt solutions, alcohols, polyethylene glycols, polyhydroxyethoxylated castor oil, peanut oil, olive oil, gelatin, lactose, terra alba, sucrose, cyclodextrin, amylose, magnesium sterate, talc, gelatin, agar, pectin, acacia, stearic acid or lower alkyl ethers of cellulose, silicic acid, fatty acids, fatty acid amines, fatty

acids monoglycerides and diglycerides, pentaerythritol fatty acids esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preservatives, sweetening agents or flavoring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, buffers and/or coloring substances and the like, which do not deleteriously react with the active compounds.

The route of administration may be any route, which effectively transports the active drug to the appropriate or desired site of action effectively, such as oral, nasal, transdermal, pulmonary or parental e.g. rectal, depot, subcutaneous, intravenous, intraurethral, intramuscular, intranasal, ophthalmic solution or an ointment, preferably through oral route.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of formula (I) or (IIIa) dissolved or suspended in a liquid carrier, in particular an aqueous carrier, for aerosol application. The carrier may contain additives such as solubilizing agent, e.g. propylene glycol, surfactants, absorption enhancers such as lecithin (phosphatidylcholine) or cyclodextrin, or preservatives such as parabens.

For parental application, particularly suitable are injectable solutions or suspensions, preferably aqueous solutions with the active compound dissolved in polyhydroxylated castor oil.

Tablet, dragees or capsules having talc and/or a carbohydrate carrier or binder or the like are particularly suitable for oral application Preferably, carriers for tablets, dragees or capsules include lactose, corn starch and/or potato starch. A syrup or elixir can be used in cases where a sweetened vehicle can be employed.

A typical tablet which may be prepared by conventional tabletting techniques may contain:

Core:

| | |
|---|---|
| Active ingredient (as free compound or salt thereof) | 100 g |
| Wheat starch | 45 g |
| Maize starch | 55 g |
| Microcrystalline cellulose | 12 g |
| Ethyl cellulose | 8 g |
| Magnesium stearate | 5 g |

The coating may compose of the following ingredients in varying compositions
Lac
Gelatin
Gum arabic
Sucrose
Titanium dioxide
Beeswax
Carnauba wax
Ethyl vanilin

The compounds of general formula (I) or (IIIa) or the compositions thereof are useful for the treatment and/or prophylaxis of disease caused by metabolic disorders such as hyperlipidemia, insulin resistance, Leptin resistance, hyperglycemia, obesity, or inflammation.

These compounds are useful for the treatment of hypercholesteremia, familial hypercholesteremia, hypertriglyceridemia, type 2 diabetes, dyslipidemia, disorders related to syndrome X such as hypertension, obesity, insulin resistance, coronary heart disease, atherosclerosis, xanthoma, stroke, peripheral vascular diseases and related disorders, diabetic complications, certain renal diseases such as glomerulonephritis, glomerulosclerosis, nephrotic syndrome, hypertensive nephrosclerosis, retinopathy, nephropathy, psoriasis, polycystic ovarian syndrome, osteoporosis, inflammatory bowel diseases, myotonic dystrophy, arteriosclerosis, Xanthoma, pancreatitis and for the treatment of cancer.

The compounds of the invention may be administered to a mammal, especially, a human in need of such

treatment, prevention, elimination, alleviation or amelioration of diseases mentioned above.

The compounds of the present invention are effective over a wide dosage range, however, the exact dosage, mode of administration and form of composition depends upon the subject to be treated and is determined by the physician or veterinarian responsible for treating the subject. Generally, dosages from about 0.025 to about 200 mg preferably from about 0.1 to about 100 mg, per day may be used. Generally, the unit dosage form comprises about 0.01 to 100 mg of the compound of formula (I) or (IIIa), as an active ingredient together with a pharmaceutically acceptable carrier. Usually suitable dosage forms for nasal, oral, transdermal or pulmonary administration comprises from about 0.001 mg to about 100 mg, preferably from 0.01 mg to about 50 mg of the active ingredient mixed with a pharmaceutically acceptable carrier or diluent.

In another aspect of the present invention, the use of the present invention for the preparation of a medicament for the treatment and/or prevention of the diseases mentioned above.

In a further aspect of the present invention, the use of one or more compounds of the general formula (I) or (IIIa) or pharmaceutically acceptable salts, for the preparation of a medicament for the treatment and/or prevention of diseases mentioned in this document.

In still further aspect of the present invention use of the compounds of the present invention for the preparation of a medicament to be given alone or in combination with statins, glitazones, biguanides, angiotensin II inhibitors, aspirin, insulin secretagogue, sitosterol inhibitor, sulphonylureas, insulin, fibric acid derivatives, nicotinic acid, cholestyramine, cholestipol or probucol, α-glycosidase inhibitors or antioxidants, which additional ingredient may be administered together with the medicament or within such a period as to act synergistically together.

The invention is explained in detail by the examples given below, which are provided by way of illustration only and therefore should not be construed as limiting the scope of the claims.

*$^1$H NMR spectral data given in the tables (vide infra) were recorded using a 300 MHz spectrometer (Bruker AVANCE-300) and reported in δ scale. Until and otherwise mentioned the solvent used for NMR is $CDCl_3$ using Tetramethyl silane as the internal standard.*

Preparation 1

Ethyl (2S)-ethoxy-3-[4-(5-methyl-2-thiophen-2-yl-oxazol-4-ylmethoxy)-phenyl]-propanoate (compound No.1)

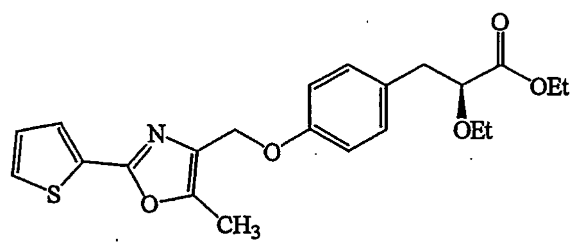

A mixture of Ethyl (2S)-ethoxy-3-(4-hydroxyphenyl)-propanoate (4.09 g), and anhydrous potassium carbonate (3.33 g) in DMF (40 mL) was heated at 80˚C for 1hr. The mixture was cooled to 50 ˚C and 4-chloromethyl-5-methyl-2-thiophen-2yl-oxazole (4.4 g) was added. The reaction mixture was continued heating at 80 ˚C for 6 hrs. Later it was cooled to 20 ˚C - 25 ˚C and water (80 mL) was added and the crude product was extracted with ethyl acetate (2 x 40 mL), washed with water (2 x 50 mL), brine (50mL) and was dried over anhydrous sodium sulfate. The solvent was evaporated under reduced pressure to obtain an oily product. The crude oily product was chromatographed over silica gel using ethyl acetate:petroleum ether (60-80) (1:9) as an eluent to afford the title product as a colourless solid.

Preparation 2

Ethyl (2S)-ethoxy-3-{4-[2-(5-methyl-2-thiophen-2-yl-oxazol-4-yl)-ethoxy]-phenyl}- propanoate (compound No.2)

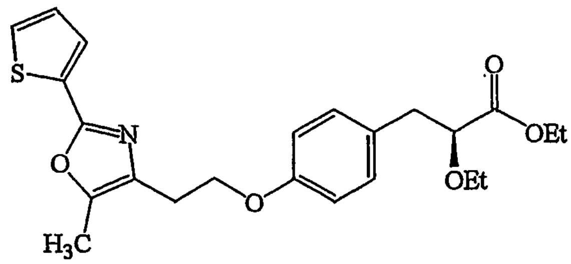

A mixture of Ethyl (2S)-ethoxy-3-(4-hydroxyphenyl)-propanoate (1.9 g), and potassium carbonate (1.51 g) in toluene (15 mL) was heated at 80 ˚C for 1hr. The mixture was cooled to 50 ˚C and Methyl 2-[5-methyl-2-thiophen-2-yl-oxazol-4yl]-ethylsulfonate (2.56 g) was added. The reaction mixture was continued heating at 80 ˚C for 16 hrs. Later it was cooled to 20 ˚C - 25 ˚C, water (20 mL) was added and the crude product was extracted with ethyl acetate (2 x 25 mL). The organic extract was washed with water (2 x 20 mL), brine (25 mL) and dried over anhydrous sodium sulfate. The solvent was evaporated under reduced pressure to obtain an oily product. The crude oily product was chromatographed over silica gel using ethyl acetate: petroleum ether (60-80) (1:9) as an eluent to afford the title product as a yellow oil.

In like manner the following compounds in the table 1 are prepared following a method similar to that described in preparations 1& 2.

Table 1:

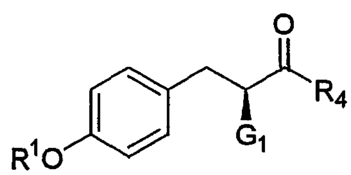

| Ex. No | $R^1$ | $G_1$ | $R_4$ | Mol.Wt | % yield |
|---|---|---|---|---|---|
| 1. | 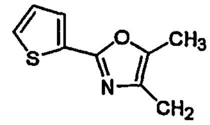 | OEt | OEt | 415 | 51 |
| | $^1$H : 1.16 (3H, t, J=7.0 Hz), 1.22 (3H, t, J=7.14 Hz), 2.40 (3H, s), 2.95 (2H, d, J=6.6 Hz), 3.32-3.37 (1H, m ),3.57-3.62 (1H, m ), 3.97 (1H, t, J=6.3 Hz), 4.15 (2H, q, J=7.12 Hz), 4.95 (2H, s ), 6.91 (2H, d, J=8.58 Hz), 7.08-7.11 (1H, m), 7.15 (2H, d, J=8.52 Hz), 7.40 (1H, dd, J=4.14 Hz & 0.82 Hz), 7.65 (1H, dd, J=2.82 & 0.75 Hz). | | | | |
| 2. | 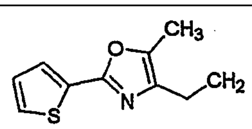 | OEt | OEt | 429 | 78 |
| | $^1$H : 1.15 (3H, t, J=6.93 Hz), 1.2 (3H, t, J=7.14 Hz), 2.34 (3H, s), 2.92-2.96 (4H, m), 3.30-3.61 (2H, m), 3.95 (1H, t, J=6.6 Hz), 4.12-4.21 (4H, m) , 6.81 (2H, d, J=8.64 Hz), 7.06-7.09 (1H, m), 7.12 (2H, d, J=8.6 Hz), 7.35 (1H, dd, J=1.11& 5.05 Hz), 7.57 (1H, dd, J=1.14 & 3.69 Hz). | | | | |
| 3. | 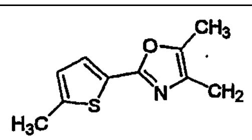 | OEt | OEt | 429 | 82 |
| | $^1$H : 1.16 (3H, t, J=7.0 Hz), 1.24 (3H, t, J=7.14 Hz), 2.38 (3H, s) 2.52 (3H, s), 2.96 (2H, d, J=6.63 Hz), 3.32-3.37 (1H, m), 3.57-3.62 (1H, m), 3.97 (1H, t, J=6.66 Hz), 4.17 (2H, q, J=7.11 Hz), 4.92 (2H, s), 6.73-6.76 (1H, m), 6.92 (2H, d, J=8.61 Hz), 7.17 (2H, d, J=8.55 Hz), 7.42 (1H, d, J=3.6 Hz). | | | | |

(continued)

| Ex. No | $R^1$ | $G_1$ | $R_4$ | Mol.Wt | % yield |
|---|---|---|---|---|---|
| 4. | 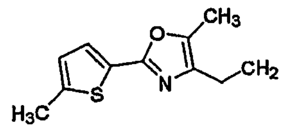 | OEt | OEt | 443 | 75 |
| | $^{1}H$ : 1.15 (3H, t, J=6.9 Hz), 1.2 (3H, t, J=7.12 Hz), 2.3 (3H, s), 2.5 (3H, s), 2.90-2.94 (4H, m), 3.33-3.36 (1H, m), 3.56-3.58 (1H, m), 3.94 (1H, t, J=6.67 Hz), 4.1-4.2 (4H, m), 6.71-6.73 (1H, m), 6.79 (2H, d,J=8.4 Hz), 7.14 (2H, d, J=8.61 Hz), 7.36 (1H, d, J=3.6 Hz). | | | | |
| 5. | 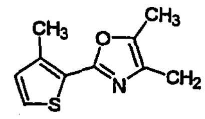 | OEt | OEt | 429 | 82 |
| | $^{1}H$ : 1.16 (3H, t, J=7.0 Hz), 1.22 (3H, t, J=7.12 Hz), 2.4 (3H, s), 2.5 (3H, s), 2.96 (2H, d, J=6.66 Hz), 3.32-3.37 (1H, m), 3.57-3.62 (1H, m), 3.97 (1H, t, J=6.64 Hz), 4.17 (2H, q, J=7.11 Hz), 4.95 (2H, s), 6.88-6.92 (1H, m), 6.94 (2H, d, J=8.61 Hz), 7.17 (2H, d, J=8.55 Hz), 7.26 (1H, d,J=4.0 Hz). | | | | |
| 6. | 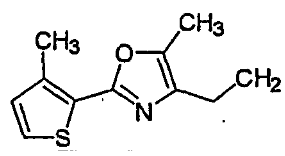 | OEt | OEt | 443 | 30 |
| | $^{1}H$ : 1.15 (3H, t, J=7.0 Hz), 1.21 (3H, t, J=7.10 Hz), 2.34 (3H, s), 2.55 (3H, s), 2.92-2.96 (4H, m), 3.31-3.36 (1H, m), 3.56-3.59 (1H, m), 3.95 (1H, t, J=6.66 Hz), 4.12-4.18 (2H, m), 4.19-4.22 (2H, m), 6.82 (2H, d, J=8.58 Hz), 6.89 (1H, d, J=5.01 Hz), 7.14 (2H, d, J=8.58 Hz), 7.23 (1H, d, J=5.01 Hz). | | | | |
| 7. | 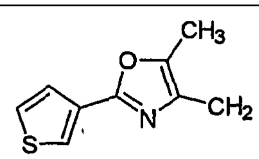 | OEt | OEt | 415 | 78 |
| | $^{1}H$ : 1.16 (3H, t, J=7.01 Hz), 1.21 (3H, t, J=7.14 Hz), 2.40 (3H, s) 2.97 (2H, d, J=6.60 Hz), 3.32-3.40 (1H, m), 3.55-3.62 (1H, m), 3.97 (1H, t, J=6.65 Hz), 4.19 (2H, q, J=7.12 Hz), 4.94 (2H, s), 6.93 (2H, d, J=8.5 Hz), 7.18 (2H, d, J=8.5 Hz), 7.35-7.37 (1H, m), 7.61 (1H, d, J=5.02 Hz), 7.88-7.89 (1H, m). | | | | |
| 8. | 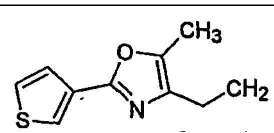 | OEt | OEt | 429 | 63 |
| | $^{1}H$ : 1.15 (3H, t, J=7.0 Hz), 1.21 (3H, t, J=7.10 Hz), 2.34 (3H, s), 2.92-2.96 (4H, m), 3.30-3.36 (1H, m), 3.56-3.61 (1H, m), 3.92 (1H, t, J=6.64 Hz) 4.12-4.16 (2H, m), 4.17-4.22 (2H, m), 6.82 (2H, d, J=8.58 Hz), 7.14 (2H, d, J=8.55 Hz), 7.33-7.36 (1H, m), 7.55-7.57 (1H, m), 7.83-7.84 (1H, m). | | | | |
| 9. | 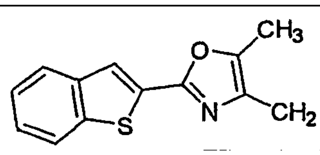 | OEt | OEt | 465 | 62 |
| | $^{1}H$ : 1.16 (3H, t, J=6.99 Hz), 1.22 (3H, t, J=7.12 Hz), 2.44 (3H, s), 2.96(2H, d, J=6.69 Hz), 3.32-3.38(1H, m), 3.57-3.63 (1H, m), 3.97(1H, t, J=6.63 Hz), 4.16(2H, q, J=7.12 Hz), 4.97 (2H, s), 6.93(2H, d, J=8.61 Hz), 7.18(2H, d, J=8.58 Hz), 7.36-7.51(2H, m), 7.79-7.87(3H, m). | | | | |
| 10. | 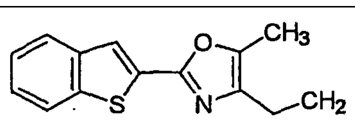 | OEt | OEt | 479 | 21 |

(continued)

| Ex. No | $R^1$ | $G_1$ | $R_4$ | Mol.Wt | % yield |
|---|---|---|---|---|---|
| | $^1$H: 1.14 (3H, t, J=7.14 Hz), 1.21 (3H, t, J=7.12 Hz), 2.39 (3H, s), 2.92-2.99 (4H, m), 3.33-3.36 (1H, m), 3.55-3.59 (1H, m), 3.94 (1H, t, J=6.7 Hz), 4.14 (2H, t, J=7.12 Hz), 4.22 (2H, t, J=6.33 Hz), 6.81 (2H, d, J=8.61 Hz), 7.13 (2H, d, J=8.55 Hz), 7.34-7.4 (2H, m), 7.79-7.86 (3H, m). | | | | |
| 11. | 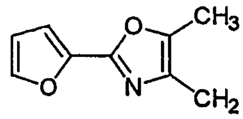 | OEt | OEt | 399 | 78 |
| 12. | 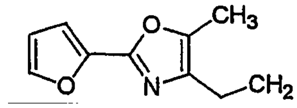 | OEt | OEt | 413 | 66 |
| | $^1$H: 1.15 (3H, t, J=6.93 Hz), 1.2 (3H, t, J=7.14 Hz), 2.34 (3H, s), 2.92-2.96 (4H, m), 3.3-3.4 (1H, m), 3.5-3.62 (1H, m), 3.95 (1H, t, J=7.1 Hz), 4.14 (2H, t, J=7.12 Hz), 4.22 (2H, t, J=6.65 Hz), 6.49-6.51 (1H, m), 6. 81 (2H, d, J=8.6 Hz), 6.9 (1H, d, J=3.2 Hz), 7.14 (2H, d, J=8.6 Hz), 7.5 (1H, m). | | | | |
| 13. | 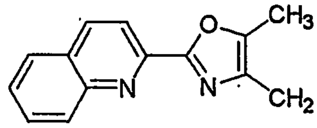 | OEt | OEt | 460 | 51 |
| 14. | 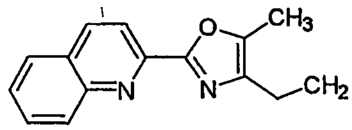 | OEt | OEt | 474 | 32 |
| 15. | 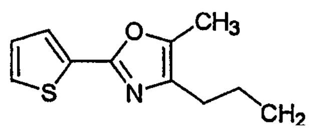 | OEt | OEt | 443 | 34 |
| | $^1$H : 1.16 (3H, t, J=6.93 Hz), 1.22 (3H, t, J=7.14 Hz), 2.02-2.1 (2H, qui), 2.24 (3H, s), 2.67 (2H, t, J=7.18 Hz), 2.95 (2H, d, J=7.02 Hz), 3.35-3.37 (1H, m), 3.57-3.59 (1H, m), 3.91-3.98 (3H, m), 4.17 (2H, q, J=7.1 Hz), 6.81 (2H, d, J=8.5 Hz), 7.06-7.08 (1H, m), 7.12 (2H, d, J=8.5 Hz), 7.3 (1H, d, J=5.0 Hz), 7.59 (1H, d, J=3.48 Hz). | | | | |
| 16. | 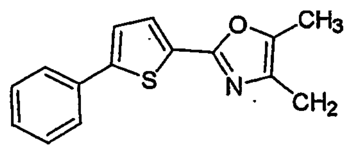 | OEt | OEt | 491 | 34 |
| | $^1$H: 1.16 (3H, t, J=6.99 Hz), 1.22 (3H, t, J=6.98 Hz), 2.41 (3H, s), 2.96 (2H, d, J=6.66 Hz), 3.32-3.63 (2H, m), 3.97 (1H, t, J=6.66 Hz), 4.16 (2H, q, J=7.05& 7.14 Hz), 4.95 (2H, s), 6.92 (2H, d, J=8.64 Hz), 7.17 (2H, d, J=8.58 Hz), 7.26-7.43 (4H, m), 7.58 (1H, d, J=3.87 Hz), 7.63 (2H, d, 7.14 Hz). | | | | |
| 17. | 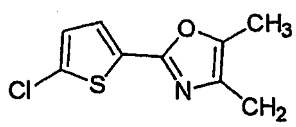 | OEt | OEt | 449.5 | 55 |
| | $^1$H: 1.16 (3H, t, J=7.00 Hz), 1.22 (3H, t, J=7.15 Hz), 2.39 (3H, s), 2.95 J=6.6 Hz), 3.32-3.62 (2H, m), 3.94 (1H, t, J=6.63 Hz), 4.12-4.19 (2H, q, 7.11 Hz), 4.92 (2H, s), 6.89-6.92 (3H, m), 7.17 (2H, d, J=8.58 Hz), 7.37 J=3.96 Hz). (2H, d, J=7.11& (1H; d, | | | | |

(continued)

| Ex. No | $R^1$ | $G_1$ | $R_4$ | Mol.Wt | % yield |
|---|---|---|---|---|---|
| 18 | 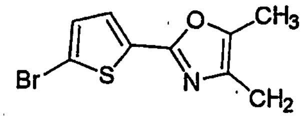 | OEt | OEt | 494 | 40 |
| | $^1$H: 1.16 (3H, t, J=7.0 Hz), 1.22 (3H, t, J=7.14 Hz), 2.39 (3H, s), 2.95 (2H, d, J=6.57 Hz), 3.32-3.63 (2H, m), 3.97 (1H, t, J=6.64 Hz), 4.12-4.19 (2H, q, J=7.11&7.14 Hz), 4.92 (2H, s), 6.91 (2H, d, J=8.58 Hz), 7.04 (1H, d, J=3.93 Hz), 7.17 (2H, d, J=8.54 Hz), 7.35 (1H, d, J=3.93 Hz). | | | | |
| 19. | 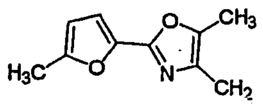 | OEt | OEt | 413 | 31 |
| | $^1$H: 1.16 (3H, t, J=6.99 Hz), 1.21 (3H, t, J=6.26 Hz), 2.41 (6H, s), 2.95 (2H, d, J=6.48 Hz), 3.35-3.37 (2H, m), 3.59 (1H, t, J=4.55 Hz), 4.11-4.19 (2H, m), 4.98 (2H, s), 6.13 (1H, d, J=2.68 Hz), 6.75 (2H, d, J=8.46 Hz), 6.96 (1H, d, J=3.91 Hz), 7.10 (2H, d, J=8.46 Hz). | | | | |
| 20. | 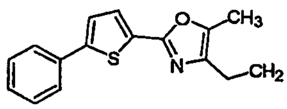 | OEt | OEt | 505 | 48 |
| | $^1$H: 1.15 (3H, t, J=6.99 Hz), 1.21 (3H, t, J=6.94 Hz), 2.35 (3H, s), 2.92-2.97 (4H, m), 3.31-3.61 (2H, m); 3.95 (1H, t, J=6.61 Hz), 4.08-4.23 (4H, m), 6.81 (2H, d, J=8.61 Hz), 7.13 (2H, d, J=8.57 Hz), 7.26-7.64 (7H, m). | | | | |
| 21. | 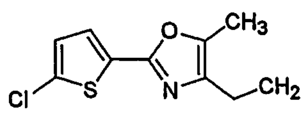 | OEt | OEt | 463.5 | 39 |
| | $^1$H: 1.14 (3H, t, J=6.96 Hz), 1.21 (3H, t, J=5.6 Hz), 2.33 (3H, s), 2.89-2.94 (4H, m), 3.28-3.63 (2H, m), 3.93 (1H, t, J=6.62 Hz), 4.08-4.20 (4H, m), 6.80 (2H, d, J=7.39 Hz), 6.89 (1H, d, J=3.96 Hz), 7.13 (2H, d, J=8.58 Hz), 7.33 (1H, d, J=3.96 Hz). | | | | |
| 22. | 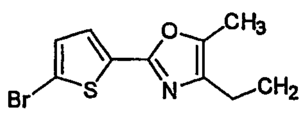 | OEt | OEt | 507 | 26 |
| | $^1$H: 1.18 (3H, t, J=6.15 Hz), 1.22 (3H, t, J=7.06 Hz), 2.33 (3H, s), 2.90-2.94 (4H, m), 3.28-3.63 (2H, m), 3.95 (1H, t, J=6.63 Hz), 4.12-4.20 (4H, m), 6.80 (2H, d, J=8.60 Hz), 7.03 (1H, d, J=3.92 Hz), 7.13 (2H, d, J=8.53 Hz), 7.30 (1H, d, J=3.93 Hz). | | | | |
| 23. | 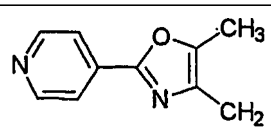 | OEt | OEt | 410 | 32 |
| | $^1$H: 1.16 (3H, t, J=6.9 Hz), 1.24 (3H, t, J=7.1 Hz), 2.55 (3H, s), 2.95-2.98 (2H, m), 3.3-3.6 (2H, m), 3.96-4.0 (1H, m), 4.14-4.21 (2H, q, J=7.14&7.12 Hz), 5.03 (2H, s), 6.90 (2H, d, J=8.5 Hz), 7.19 (2H, d, J=8.5 Hz), 8.4 (2H, m), 8.8 (2H, m). | | | | |
| 24. | 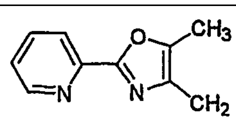 | OEt | OEt | 410 | 35 |
| | $^1$H: 1.16 (3H, t, J=6.99), 1.23 (3H, t, J=7.1 Hz), 2.10 (3H, s), 2.48 (2H, m), 3.35 (1H, m), 3.6 (1H, m), 3.97 (1H, m),4.1-4.2 (2H, q, J=7.1 Hz), 5.05 (2H, s), 6.92 (2H, d, J=8.6 Hz), 7.18 (2H, d, J=8.5 Hz), 7.8 (1H, m), 7.8 (1H, t, J=7.8 Hz), 8.1 (1H, d, J=7.9 Hz) 8.7 (1H, d, J=4.4 Hz). | | | | |

(continued)

| Ex. No | $R^1$ | $G_1$ | $R_4$ | Mol.Wt | % yield |
|---|---|---|---|---|---|
| 25. | 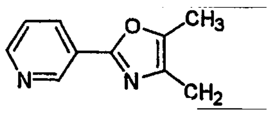 | OEt | OEt | 410 | 68 |
| | $^1$H: 1.2 (3H, t, J= 6.9 Hz), 1.22 (3H, t, J=7.0 Hz), 2.46 (3H, s), 2.96 (2H, d, J=6.5 Hz), 3.35 (1H, m), 3.6 (1H, m), 3.97 (1H, t, J=6.8 Hz), 4.17 (2H, q, J=7.14 Hz), 4.98 (2H, s), 6.92 (2H, d, J=8.5 Hz), 7.17 (2H, d, J=8.5 Hz), 7.47 (1H, dd, J=5.0&7.8 Hz), 8.39 (1H, d, J=8.0 Hz), 8.68 (1H, d, J=4.3 Hz), 9.25 (1H, s). | | | | |
| 26. | 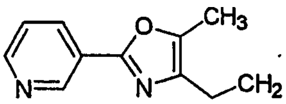 | OEt | OEt | 424 | 22 |
| | $^1$H: 1.15 (3H, t, J=7.0.Hz), 1.22 (3H, t, J=7.1Hz), 2.4 (3H, s), 2.95 (4H, m), 3.5 (2H, m), 3.95 (1H, t, J=6.61 Hz), 4.17 (2H, q, J=14.4 & 7.18 Hz), 4.23 (2H,t, J=6.6 Hz), 6.82 (2H, d, J=8.58 Hz), 7.13 (2H, d, J=8.52 Hz), 7.36 (1H, dd, J=7.8 & 4.8 Hz), 8.23 (1H, d, J=7.98 Hz), 8.63 (1H, d, J=3.21 Hz) 9.2 (1H, s). | | | | |

Preparation 3

(2S)-Ethoxy-3-[4-(5-methyl-2-thiophen-2-yl-oxazol-4-ylmethoxy)-phenyl]- propanoic acid (compound No.27)

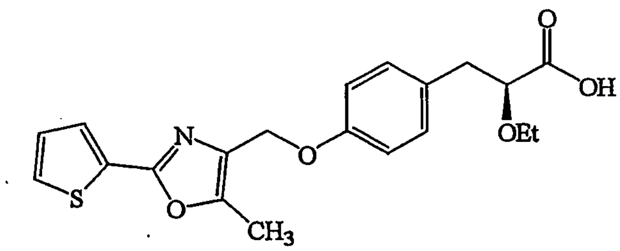

A mixture of Ethyl (2S)-ethoxy-3-[4-(5-methyl-2-thiophen-2-yl-oxazol-4-ylmethoxy)-phenyl]-propionate (0.5 g), sodium hydroxide (0.062g in 5 mL water) in methanol (10 mL) was stirred at 20 ˚C to 25 ˚C for 16 h. Solvents were evaporated under reduced pressure. The residue was diluted with water (10 mL) and was acidified with dilute hydrochloric acid. The product was extracted with ethyl acetate (2 x 25 mL), washed with water (2 x 25 mL), brine (30 mL) and dried over sodium sulfate. The solvent was evaporated under reduced pressure to obtain 0.4 g of title compound. In like manner following compounds in table 2 were prepared following a procedure similar to that described in preparation 3.

Table 2:

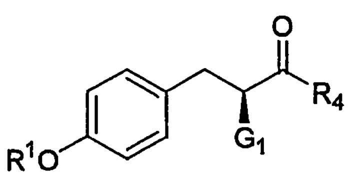

(continued)

| Ex. No | $R^1$ | $G_1$ | $R_4$ | Mol.Wt | % yield |
|---|---|---|---|---|---|
| 27. | 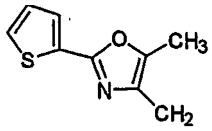 | OEt | OH | 387 | 95 |
| | $^1H$: 1.16 (3H, t, J=7.0 Hz), 2.40 (3H, s), 2.91-2.98 (2H, m), 3.35-3.49 (1H, m), 3.54-3.64 (1H, m), 4.03 (1H, m), 4.95 (2H, s), 6.93 (2H, d, J=8.47 Hz), 7.01- (1H, t, J=3.69 Hz), 7.15 (2H, d, J=8.47 Hz), 7.40 (1H, d, J=4.89 Hz), 7.65 (1H, d, J=3.45 Hz). | | | | |
| 28. | 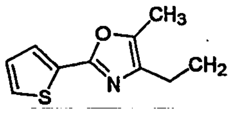 | OEt | OH | 401 | 85 |
| | $^1H$: 1.17 (3H, t, J=6.99 Hz), 2.35 (3H, s), 2.92-2.97 (1H, m), 2.95 (2H, t, J= 6.6 Hz), 3.04-3.09 (1H, m), 3.41-3.47 (1H, m), 3.55-3.60 (1H, m), 4.01-4.05 (1H, m), 4.18 (2H, t, J=6.6 Hz), 6.81 (2H, d, J=8.6 Hz), 7.06-7.09 (1H, m), 7.13 (2H, d, J=8.55 Hz), 7.37 (1H, dd, J=1.0 Hz & 4.22 Hz), 7.48 (1H, dd, J=1.0 Hz&2.64 Hz). | | | | |
| 29. | 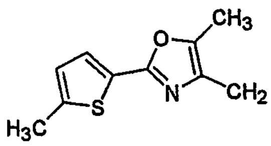 | OEt | OH | 401 | 73 |
| | $^1H$ : 1.16 (3H, t, J=6.96 Hz), 2.38 (3H, s), 2.51 (3H, s), 2.90-2.97 (1H, m), 3.03-3.09 (1H, m), 3.38-3.43 (1H, m), 3.57-3.62 (1H, m), 4.00-4.04 (1H, m), 4.92 (2H, s), 6.73-6.75 (1H, m), 6.92 (2H, d, J=8.58 Hz), 7.18 (2H, d, J=8.58 Hz), 7.44 (1H, d, J=3.6 Hz). | | | | |
| 30. | 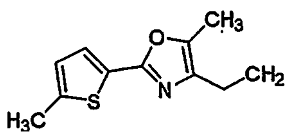 | OEt | OH | 415 | 76 |
| | $^1H$ : 1.15 (3H, t, J=6.96 Hz), 2.32(3H, s), 2.50(3H, s), 2.89-2.96(4H, m), 3.40-3.43 (1H, m), 3.56-3.59 (1H, m), 3.99-4.03 (1H, m), 4.16 (2H, t, J=6.58 Hz), 6.72 (1H, d, J=3.54 Hz), 6.81 (2H, d, J=8.5 Hz),7.15 (2H, d, J=8.5 Hz), 7.39 (1H, d, J=3.6 Hz). | | | | |
| 31. | 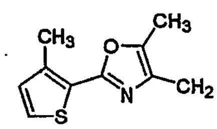 | OEt | OH | 401 | 97 |
| | $^1H$ : 1.16 (3H, t, J=7.0 Hz), 2.4 (3H, s), 2.57 (3H, s), 2.90-2.98 (1H, m), 3.05-3.10 (1H, m), 3.41-3.46 (1H, m), 3.58-3.61 (1H, m), 4.02-4.13 (1H, m), 4.95 (2H, s), 6.9 (1H, d, J=5.0 Hz), 6.94 (2H, d, J=8.6 Hz), 7.18 (2H, d, J=8.55 Hz), 7.27 (1H, d, J=.9 Hz). | | | | |
| 32. | 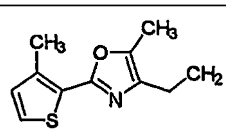 | OEt | OH | 415 | 90 |
| | $^1H$ : 1.15 (3H, t, J=7.0 Hz), 2.34 (3H, s), 2.55 (3H, s), 2.89-3.08 (4H, m), 3.40-3.45 (1H, m), 3.55-3.60 (1H, m), 4.00-4.04 (1H, m), 4.19 (2H, t, J=6.66 Hz), 6.83 (2H, d, J=8.55 Hz), 6.88 (1H, d, J=5.01 Hz), 7.14 (2H, d, J=8.58 Hz),7.23 (1H, d, J=5.01 Hz). | | | | |

(continued)

| Ex. No | $R^1$ | $G_1$ | $R_4$ | Mol.Wt | % yield |
|---|---|---|---|---|---|
| 33. | 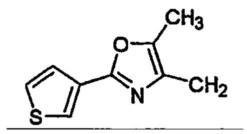 | OEt | OH | 387 | 73 |
| | $^1H$ : 1.16 (3H, t, J=6.96 Hz), 2.4 (3H, s), 2.90-2.97 (1H, dd, J=7.62& 7.65 Hz), 3.03-3.09 (1H, dd, J=4.68&4.35 Hz), 3.39-3.44 (1H, m), 3.57-3.62 (1H, m), 4.01-4.05 (1H, m), 4.94 (2H, s), 6.93 (2H, d, J=8.5 Hz), 7.18 (2H, d, J=8.5 Hz), 7.35-7.38 (1H, m), 7.59-7.61 (1H, m), 7.91-7.92 (1H, m). | | | | |
| 34. | 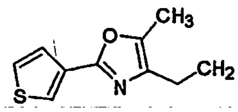 | OEt | OH | 401 | 73 |
| | $^1H$ : 1.15 (3H, t, J=7.0 Hz), 2.34 (3H, s), 2.89-3.08 (4H, m), 3.40-3.45 (1H, m), 3.55-3.60 (1H, m), 4.00-4.04 (1H, m), 4.19 (2H, t, J=6.66 Hz), 6.83 (2H, d, J=8.55 Hz), 6.88 (1H, d, J=5.01 Hz), 7.14 (2H, d, J=8.58 Hz), 7.23 (1H, d, J=5.01 Hz), 7.86 (1H, d, J=2.58 Hz). | | | | |
| 35. | 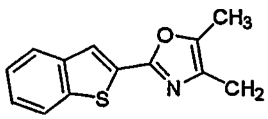 | OEt | OH | 437 | 88 |
| | $^1H$ : 1.18 (3H, t, J=6.99 Hz), 2.45 (3H, s), 2.92-2.99 (1H, m), 3.08-3.14(1H, m), 3.44-3.50 (1H, m), 3.56-3.61 (1H, m), 4.04-4.08 (1H, m), 4.98 (2H, s), 6.94(2H, d, J=8.55 Hz), 7.18 (2H, d, J=8.52 Hz), 7.37-7.4 (2H, m), 7.79-7.86(3H, m). | | | | |
| 36. | 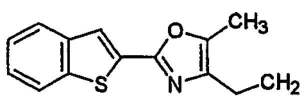 | OEt | OH | 451 | 65 |
| | $^1H$ : 1.16 (3H, t, J=7.0 Hz), 2.38 (3H, s), 2.89-3.06 (4H, m), 3.42-3.57 (2H, m), 4.04-4.06 (1H, m), 4.22 (2H, t, J=6.55 Hz), 6.82 (2H, d, J=8.58 Hz), 7.13 (2H, d, J=8.58 Hz), 7.34-7.4 (2H, m), 7.78-7.86 (3H, m). | | | | |
| 37. | 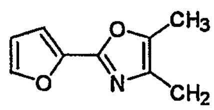 | OEt | OH | 371 | 76 |
| | $^1H$ : 1.16 (3H, t, J=6.99 Hz), 2.41 (3H, s), 2.9-2.98 (1H, m), 3.04-3.10 (1H, m), 3.40-3.45 (1H, m), 3.57-3.62 (1H, m), 4.04-4.06 (1H, m), 4.96 (2H, s), 6.52 (1H, dd, J=1.68 & 3.42 Hz), 6.92 (2H, d, J=8.58 Hz), 6.98 (1H, d, J=3.39 Hz), 7.17 (2H, d, J=8.55 Hz), 7.54 (1H, d, J=1.17 Hz). | | | | |
| 38. | 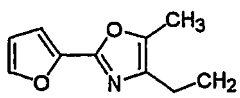 | OEt | OH | 385 | 68 |
| | $^1H$: 1.16 (3H, t, J=6.96 Hz), 2.35 (3H, s), 2.92-2.97 (3H, m), 3.02-3.12(1H, m), 3.41-3.44 (1H, m), 3.56-3.59 (1H, m), 4.0-4.04 (1H, m), 4.19(2H, t, J=6.64 Hz) 6.50 (1H, dd, J=1.64 & 3.36 Hz), 6.8 (2H, d, J=8.52 Hz), 6.94 (1H, d, J=3.39 Hz), 7.13 (2H, d, J=8.55 Hz), 7.51(1H, d, J=1.1 Hz). | | | | |
| 39. | 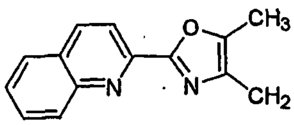 | OEt | OH | 432 | 67 |
| | $^1H$ : 1.18 (3H, t, J=7.0 Hz), 2.53 (3H, s), 2.92-2.99 (1H, m), 3.07-3.13 (1H, m), 3.44-3.49 (1H, m), 3.57-3.62 (1H, m), 4.04-4.08 (1H, m), 5.05 (2H, s), 6.96 (2H, d, J=8.52 Hz), 7.18 (2H, d, J=8.52 Hz), 7.59 (1H, t, J=7.42 Hz), 7.76 (1H, t, J=7.4 Hz), 7.84 (1H, d, J=8.04 Hz), 8.22-8.28 (3H, m). | | | | |

(continued)

| Ex. No | $R^1$ | $G_1$ | $R_4$ | Mol.Wt | % yield |
|---|---|---|---|---|---|
| 40. | 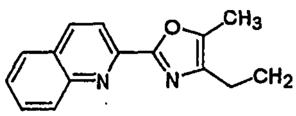 | OEt | OH | 446 | 67 |
| | $^1$H : 1.16 (3H, t, J=6.97 Hz), 2.44 (3H, s), 2.88-2.95 (1H, m), 3.04 (2H, t, J=6.4 Hz), 3.05-3.07 (1H, m), 3.45-3.57 (2H, m), 4.01-4.05 (1H, m), 4.28 (2H, t, J=6.5 Hz), 6.8 (2H, d, J=8.52 Hz), 7.11 (2H, d, J=8.49 Hz), 7.57 (1H, t, J=7.29 Hz), 7.75 (1H, t, J=7.7 Hz), 7.83 (1H, d, J=8.13 Hz), 8.17-&27 (3H, m). | | | | |
| 41. | 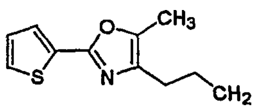 | OEt | OH | 415 | 32 |
| | $^1$H (DMSO-D6) : 0.94 (3H, t, J=6.75 Hz), 1.93-1.97 (2H, m), 2.2 (3H, s), 2.49-2.91 (4H, m), 3.13 (1H, t, J=7.7 Hz), 3.53-3.7 (2H, m), 3.87 (2H, t, J=5.5 Hz), 6.7 (2H, d, J=8.76 Hz), 7.1-7.15 (3H, m), 7.55 (1H, d, J=2.89 Hz), 7.68 (1H, d, J=4.6 Hz). | | | | |
| 42. | 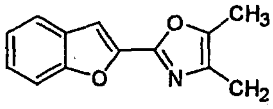 | OEt | OH | 421 | 59 |
| | $^1$H: 1.03 (3H, t, J=6.94 Hz), 2.46 (3H, s), 2.47-2.81 (2H, m), 3.44-3.53 (2H, m), 3.93-3.94 (1H, m), 4.98 (2H, s), 6.94 (2H, d, J=8.55 Hz), 7.15, (2H, d, J=8.52 Hz), 7.33-7.76 (5H, m), 12.59 (1H, s). | | | | |
| 43. | 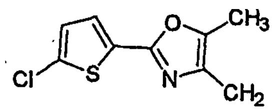 | OEt | OH | 421.5 | 82 |
| | $^1$H: 1.18 (3H, t, J=7.00 Hz), 2.39 (3H, s), 2.91-3.13 (2H, m), 3.44-3.61 (2H, m), 4.05 (1H, t, J=5.82 Hz), 4.92 (2H, s), 6.90-6.93 (3H, m), 7.16 (2H, d, J=8.58 Hz), 7.38 (1H, d, J=3.96 Hz). | | | | |
| 44. | 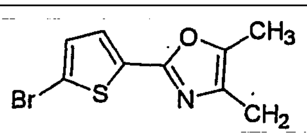 | OEt | OH | 466 | 66 |
| | $^1$H: 1.18 (3H, t, J=7.00 Hz), 2.35 (3H, s), 2.91-3.13 (2H, m), 3.14-3.44 (2H, m), 4.06 (1H, t, J=5.8 Hz), 4.92 (2H, s), 6.92 (2H, d, J=8.55 Hz), 7.05 (1H, d, J=3.93 Hz), 7.16 (2H, d, J=8.58 Hz), 7.36 (1H, d, J=3.93 Hz). | | | | |
| 45. | 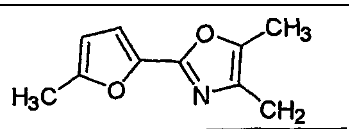 | OEt | OH | 385 | 64 |
| | $^1$H: 1.16 (3H, t, J=6.99 Hz), 2.41 (6H, s), 2.97-3.11 (2H, m), 3.41-3.46 (2H, m), 4.04 (1H, t, J=5.94 Hz), 4.96 (2H, s), 6.13 (1H, d, J=2.68 Hz), 6.90-6.93 (3H, m), 7.16 (2H, d, J=8.50 Hz). | | | | |
| 46. | 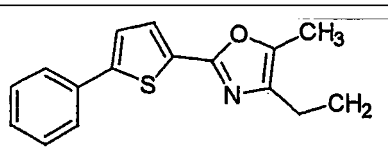 | OEt | OH | 477 | 88 |
| | $^1$H: 1.16 (3H, t, J=6.39 Hz), 2.89 (3H, s), 2.93-3.05 (4H, m), 3.41-3.58 (2H, m), 4.01-4.05 (1H, m), 4.20 (2H, t, J=6.6 Hz), 6.81 (2H, d, J=8.59 Hz), 7.14 (2H, d, J=8.55 Hz), 7.26-7.42 (4H, m), 7.54 (1H, d, J=3.86 Hz), 7.62 (2H, d, J=7.26 Hz). | | | | |

(continued)

| Ex. No | $R^1$ | $G_1$ | $R_4$ | Mol.Wt | % yield |
|---|---|---|---|---|---|
| 47. | 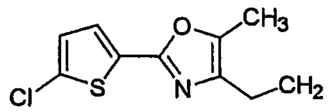 | OEt | OH | 435.5 | 98 |
| | $^1H$: 1.16 (3H, t, J=6.95 Hz), 2.33 (3H, s), 2.90-3.10 (4H, m), 3.40-3.63 (2H, m), 4.02-4.05 (1H, m), 4.17 (2H, t, J=6.90 Hz), 6.80 (2H, d, J=8.61 Hz), 6.89 (1H, d, J=3.78 Hz), 7.13 (2H, d, J=8.52 Hz), 7.33 (1H, d, J=3.95 Hz). | | | | |
| 48. | 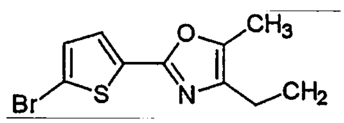 | OEt | OH | 479 | 72 |
| | $^1H$: 1.16 (3H, t, J=6.94 Hz), 2.33 (3H, s), 2.90-3.10 (4H, m), 3.42-3.59 (2H, m), 4.01-4.11 (1H, m), 4.17 (2H, t, J=6.49 Hz), 7.03 (1H, d, J=3.93 Hz), 6.80 (2H, d, J=8.52 Hz), 7.13 (2H, d, J=8.52 Hz), 7.31 (1H, d, J=3.93 Hz). | | | | |
| 49. | 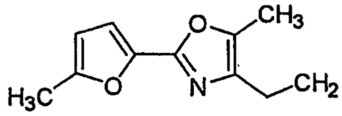 | OEt | OH | 399 | 48 |
| | $^1H$: 1.16 (3H, t, J=6.94 Hz), 2.34 (3H, s), 2.38 (3H, s), 2.92-3.04 (4H, m), 3.39-3.44 (2H, m), 4.02 (1H, t, J=5.98 Hz), 4.19 (2H, t, J=6.61 Hz), 6.09 (1H, d, J=2.53 Hz), 6.81(3H, m), 7.13 (2H, d, J=8.54 Hz). | | | | |
| 50. | 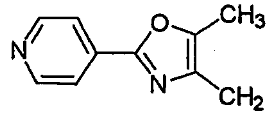 | OEt | OH | 382 | 50 |
| | $^1H$: 1.02 (3H, t, J=6.9 Hz), 2.48 (3H, s), 2.81-2.91 (2H, m), 3.24-3.29 (1H, m), 3.53-3.90 (1H, m), 3.91 (1H, m), 4.98 (2H, s), 6.92 (2H, d, J=8.5 Hz), 7.14 (2H, d, J=8.5 Hz), 7.83 (2H, d, J=4.6 Hz), 8.72(2H, d, J=5.94 Hz) | | | | |
| 51. | 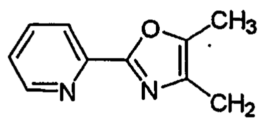 | OEt | OH | 410 | 35 |
| | $^1H$: 1.18 (3H, t, J=6.99 Hz), 2.46 (3H, s),2.92-3.14 (2H, dd, J1=7.3 J2=4.1 Hz), 3.48(2H, m), 4.09 (1H, m), 5.01 (2H, s), 6.96 (2H, d, J=8.6 Hz), 7.19 (2H, d, J=8.5 Hz), 7.78 (1H, m), 7.81 (1H, t, J=7.7), 8.1 (1H, d, J=7.9 Hz), 8.7 (1H, d, J=4.4 Hz) | | | | |
| 52. | 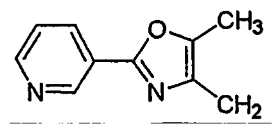 | OEt | OH | 382 | 72 |
| | $^1H$: 1.2 (3H, t, J=7.0 Hz), 2.47 (3H, s), 2.9 (1H, d, J=7.0 Hz), 3.1 (1H, d, J=4.4), 3.5 (2H, m), 4.1 (1H, m), 5.0 (2H, s), 7.0 (2H, d, J=8.61 Hz), 7.2 (2H, d, J=8.5 Hz), 7.59 (1H, d, J=7.62 Hz), 8.48 (1H, d, J=8.0 Hz), 8.7 (1H, s), 9.26 (1H, s). | | | | |

Preparation 4

(2S)-Ethoxy-3-{4-[2-(5-methyl-2-phenyl-oxazol-4-yl)-ethoxy]-phenyl}-propan-1-ol (compound No 64).

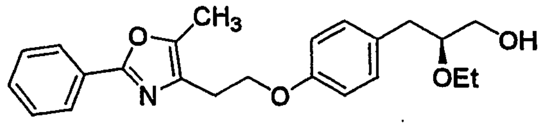

Lithium aluminium hydride (465 mg) was added to an ice cold solution of Ethyl(2S)-ethoxy-3-{4-[2-(5-methyl-2-phenyl-oxazol-4-yl)-ethoxy]-phenyl}-propanoate (2.7 g) in tetrahydrofuran (30 mL) in portions over a period of 15 minutes and the reaction mixture was stirred for further 15 minutes at the same temperature. The reaction was quenched by carefully adding saturated solution of sodium sulfate in water dropwise. Solids were filtered off and washed with hot ethyl acetate. Combined filtrate was dried over sodium sulfate and evaporated. Crude product was chromatographed over silicagel using 5 to 25 % ethyl acetate in petroleum ether to yield 2.6 g of title compound.

In like manner following compounds in the table 3&4 were prepared following the procedure described in preparation 4.

Table 3:

| 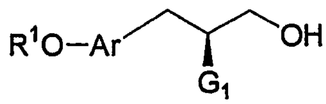 | | | | | |
|---|---|---|---|---|---|
| Ex. No | $R^1$ | Ar | $G_1$ | Mol. Wt | % yield |
| 53. | 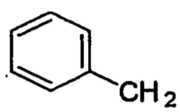 | 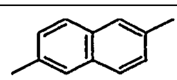 | OEt | 336 | 40 |
| | $^1H$: 1.0 (3H, t, J=6.9 Hz), 2.0 (1H, t, J=5.5 Hz); 2.9 (1H, m), 3.0 (1H, m), 3.5, (2H, m), 3.6 (2H, m), 5.1 (2H, s), 7.2 - 7.5 (9H, complex), 7.7 (2H, t, J=9.4 Hz). | | | | |
| 54. | 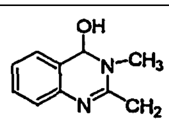 | 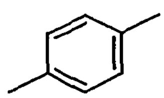 | OEt | 370 | 56 |
| | $^1H$: 1.2 (3H, t, J=7.11 Hz), 2.7 (1H, dd, J=13.8 & 6.8 Hz), 2.8 (1H, dd, J=13.8 & 5.92 Hz), 3.2 (3H, s), 3.4 - 3.6 (5H, m), 4.0 (1H, dd, J=9.16 & 3.64 Hz), 4.1 (1H, t, J=8.95 Hz), 4.9 (1H, m), 6.66 (1H, d, J=7.98 Hz), 6.7 (2H, dd, J=6.7 & 1.96 Hz), 6.8 (1H, t, J=7.09 Hz), 7.11 (2H, d, J=8.58 Hz), 7.2 (1H, m). 7.9 (1H, dd, J=7.8 & 1.3 Hz). | | | | |
| 55. | 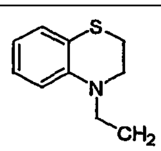 | 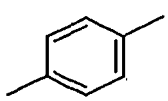 | OEt | 373 | 97 |
| | $^1H$: 1.1 (3H, t, J=6.9 Hz), 2.7 (1H, dd, J=14.0 & 6.9 Hz), 2.8 (1H, dd, J=14.0 & 6.9 Hz), 3.0 (2H, m), 3.4-3.6 (5H, m), 3.7 (2H, t, J =5.8 Hz), 3.8 (2H, m), 4.1 (2H, t, J=5.9 Hz), 6.6 (1H, m), 6.7 (1H, d, J=8.2 Hz), 6.8 (2H, dd, J=8.6 & 1.95 Hz), 7.0 (2H, m), 7.1 (2H, dd, J=8.6& 1.95 Hz). | | | | |
| 56. | 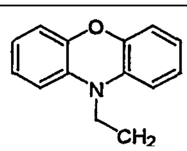 | 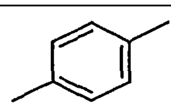 | OEt | 405 | 62 |
| | $^1H$: 1.1 (3H, t, J=7.0 Hz), 2.7 (1H, dd, J=14.0 & 7.0 Hz), 2.78 (1H, dd, J=14.7 & 5.9 Hz), 3.4- 3.6 (5H, complex), 3.9 (2H, t, J=6.6 Hz), 4.1 (2H, t, J=6.6 Hz), 6.6 (6H, complex), 6.8 (4H, complex), 7.1 (2H,d, J=8.6 Hz). | | | | |

(continued)

| Ex. No | $R^1$ | Ar | $G_1$ | Mol. Wt | % yield |
|---|---|---|---|---|---|
| 57. | 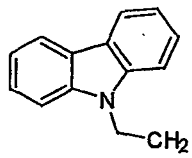 | 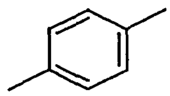 | OEt | 389 | 72 |
| | $^1H$: 1.1 (3H, t, J=7.0 Hz), 1.9 (1H, dd, J=5.3 &1.8 Hz, OH), 2.6 (1H, dd, J=13.5 & 6.8 Hz), 2.7 (1H, dd, J=14.0 & 6.0 Hz), 3.4 - 3.6 (5H, complex), 4.3 (2H, t, J=6.0 Hz), 4.7 (2H, t, J=6.0 Hz), 6.7 (2H, d, J =8.6 Hz), 7.0 (2H, d, J=8.6 Hz), 7.2 (2H, m), 7.5 (4H, m), 8.1 (2H, d, J=7.7 Hz). | | | | |
| 58. | 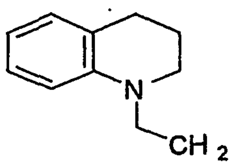 | 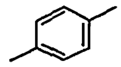 | OEt | 355 | 98 |
| | $^1H$: 1.17 (3H, t, J=6.99 Hz), 1.97 (3H, m), 2.77 (4H, m), 3.4- 3.6 (7H, complex), 3.7 (2H, t, J=6.15 Hz), 4.12 (2H, t, J=6.1 Hz), 6.6 (2H, dd, J=12.13 & 4.66 Hz), 6.7 (2H, d, J=2.79 Hz), 6.95 (1H, d, J=7.14 Hz), 7.05 (1H, d, J=1.44 Hz), 7.0 - 7.2 (2H, m). | | | | |
| 59. | 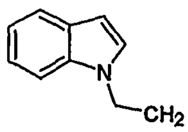 | 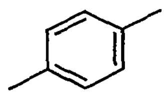 | OEt | 339 | 95 |
| | $^1H$: 1.1 (3H, t, J=7.0 Hz), 2.6 (1H, dd, J=14.7 & 6.8 Hz), 2.7 (1H, dd, J=14.8 & 6.0 Hz), 3.4 -3.6 (5H, complex), 4.2 (2H, t, J=5.6 Hz), 4.5 (2H, t, J=5.6 Hz), 6.5 (1H, d, J=3.0 Hz), 6.7 (2H, dd, J=8.6 & 1.9 Hz), 7.1 (3H, m), 7.2 (2H,m), 7.4 (1H, d, J=8.2 Hz), 7.6 (1H, d, J=7.8 Hz). | | | | |
| 60. | 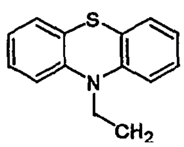 | 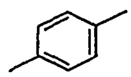 | OEt | 421 | 76 |
| | $^1H$: 1.1 (3H, t, J=6.99 Hz), 2.7 (1H, dd, J=13.8 & 6.97 Hz), 2.8 (1H, dd, J=13.75 & 5.8 Hz), 3.4 -3.6 (5H, m), 4.3 (4H, m), 6.8 (2H, m), 6.9 (4H, m), 7.1 (6H, m). | | | | |
| 61. | 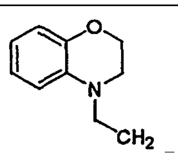 | 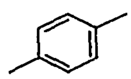 | OEt | 357 | 56 |
| | $^1H$: 1.2 (3H, t, J=6.99 Hz), 2.7 (1H, dd, J=13.8 & 6.69 Hz), 2.8 (1H, dd, J=13.8 & 5.9 Hz) 3.6 (7H, m), 3:7 (2H, t, J=5.64Hz), 4.16 (2H, t, J=5.7Hz), 4.2 (2H, t, J= 4.4Hz), 6.7 (2H, m), 6.8 (4H, m), 7.2 (2H, d, J=8.58 Hz). | | | | |
| 62. | 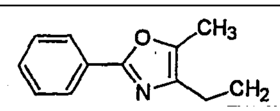 | 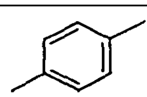 | OH | 353 | 20 |
| | $^1H$: 2.37 (3H, s), 2.7 (2H, m), 2.99 (1H, t, J=6.69), 3.54 (1H, m), 3.4-3.7 (2H, m), 3.9 (1H, m), 4.2(2H, t, J=6.69), 6.8 (2H, dd, J=1.95 & 6.63), 7.1 (2H, d, J=8.55), 7.4 (3H, m), 7.98 (2H, m). | | | | |

(continued)

| Ex. No | $R^1$ | Ar | $G_1$ | Mol. Wt | % yield |
|---|---|---|---|---|---|
| 63. | 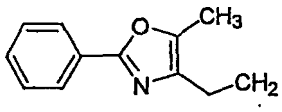 | 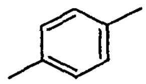 | SPh | 445 | 20 |
| | $^1H$: 2.44 (3H, s), 2.8 (2H, dd, J=7.35 & 2.46 Hz), 3.13 (2H, t, J=5.89 Hz), 3.37 (1H, m), 3.57 (2H, m), 4.31 (2H, t, J=6.1 Hz), 6.82 (2H, d, J=8.6 Hz), 7.1 (2H, d, J=8.55 Hz), 7.28 (2H, m), 7.39 (2H, m), 7.52 (4H, m), 8.19 (2H, m). | | | | |
| 64. | 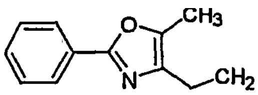 | 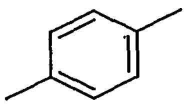 | OEt | 381 | 84 |
| | $^1H$: 1.1 (3H, t, J=6.9 Hz), 2.3 (3H, s), 2.5 (1H, dd, J=13.5 & 6.7 Hz), 2.7 (1H, dd, J=12.9 & 6.9 Hz), 2.9 (2H, t, J=6.69), 3.5 (5H, m), 4.2 (2H, t, J=6.69), 6.8 (2H, d, J=8.55 Hz), 7.1 (2H, d, J=8.5 Hz), 7.2 - 7.4 (3H, m), 7.9 (2H, m). | | | | |
| 65. | 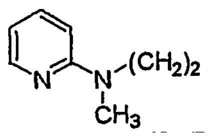 | 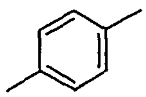 | OEt | 330 | 96 |
| | $^1H$: 1.17 (3H, t, J=6.9 Hz), 1.58 (1H, broad-s), 2.68-2.7 (1H, m), 2.7-2.85 (1H, m), 3.13 (3H, s), 3.48-3.58 (5H, m), 3.97 (2H, t, J= 5.85 Hz), 4.15 (2H, t, J=5.64 Hz), 6.50-6.56 (2H, m), 6.86 (2H, d, J=8.64 Hz), 7.07 (2H, d, J=8.64 Hz); 7.44-7.45 (1H, m), 8.13-8.16 (1H, m). | | | | |
| 66. | 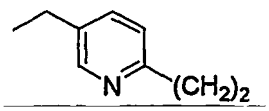 | 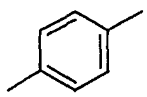 | OEt | 329 | 82 |
| | $^1H$: 1.17 (3H, t, J=7.0 Hz), 1.24 (3H, t, J=7.6 Hz), 2.59-2.69 (3H, m), 2.77-2.85 (1H, m), 3.22 (2H, t, J= 6.7 Hz), 3.49-3.58 (5H, m), 4.31 (2H, t, J= 6.57 Hz), 6.83 (2H, d, J=8.55 Hz), 7.1 (2H, d, J=8.58 Hz), 7.17 (1H, d, J=7.92 Hz); 7.45 (1H, m), 8.4 (1H, d, J=1.98 Hz). | | | | |
| 67. | 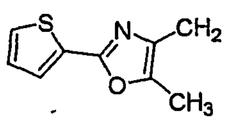 | 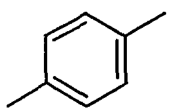 | OEt | 373 | 90 |
| | $^1H$: 1.17 (3H, t, J=6.9 Hz), 2.4(3H, s), 2.69-2.81 (2H, m), 3.45-3.62 (5H, m) 4.96 (2H, s), 6.9 (2H, d, J=8.47 Hz), 7.08-7.09 (1H, m), 7.14 (2H, d, J=8.47 Hz), 7.38-7.40 (1H, m), 7.62-7.64 (1H, m) | | | | |
| 68. | 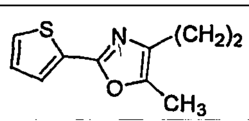 | 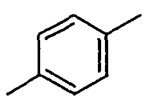 | OEt | 387 | 97 |
| | $^1H$: 1.17 (3H, t, J=7.02 Hz), 2.35 (3H, s), 2.67-2.79 (2H, m), 2.94 (2H, t, J=6.63 Hz), 3.42-3.57 (5H, m), 4.2 (2H, t, J=6.63 Hz), 6.82 (2H, d, J=8.58 Hz), 7.06-7.10 (3H, m), 7.35-7.37 (1H, dd, J=1.17 & 5.04 Hz), 7.57- 7.58 (1H, dd, J=1.11 & 3.66 Hz). | | | | |

(continued)

| Ex. No | $R^1$ | Ar | $G_1$ | Mol. Wt | % yield |
|---|---|---|---|---|---|
| 69. | 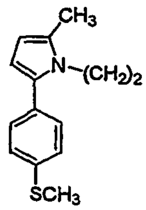 | 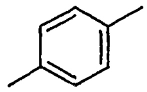 | OEt | 425 | 95 |
| | $^1$H: 1.17 (3H, t, J=6.9 Hz), 2.36 (3H, s), 2.51 (3H, s), 2.6-2.8 (2H, m), 3:4-3.6 (5H, m) 3.92 (2H, t, J=6.47 Hz), 4.29 (2H, t, J=6.6 Hz), 5.96-5.98 (1H, m), 6.0 (1H, d, J=3.4 Hz), 6.62 (2H, d, J=8.5 Hz), 7.05 (2H, d, J=8.5 Hz), 7.25-7.34 (4H, m). | | | | |
| 70. | 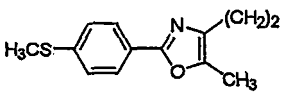 | 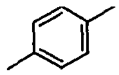 | OEt | 427 | 92 |
| | $^1$H: 1.17 (3H, t, J=6.99 Hz), 2.36 (3H, s), 2.52 (3H, s), 2.67-2.69 (1H, m), 2.77-2.79 (1H, m), 2.96 (2H, t, J=6.6 Hz), 3.43-4.13 (5H, m), 4.21 (2H, t, J=6.6 Hz), 6.82 (2H, d, J=8.5 Hz), 7.08 (2H, d, J=8.5 Hz), 7.28 (2H, d, J=8.4 Hz), 7.88 (2H, d, J=8.4 Hz). | | | | |
| 71. | 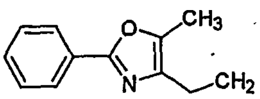 | 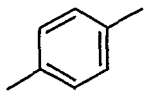 | $NH_2$ | 352 | 70 |
| | $^1$H: 2.37 (3H, s), 2.83 (2H, s), 2.96 (2H, t, J=6.21 Hz), 3.36-3.65 (3H, m), 4.22 (2H, t, J=6.35 Hz), 6.89-6.91 (2H, m), 7.13-7.19 (2H, m), 7.45-7.48 (3H, m), 7.93-7.96 (2H,m). | | | | |
| 72. | 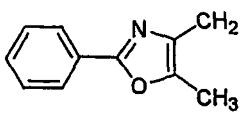 | 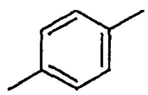 | NHBoc | 438 | 100 |
| | $^1$H: 1.41 (9H, s), 2.26 (1H, broad-s), 2.43 (3H, s), 2.78 (2H, d, J=7.11 Hz), 3.52-3.58 (1H, m), 3.63-3.7 (1H, m), 3.82 (1H, broad-s), 4.97 (2H, s), 6.96 (2H, d, J=8.58 Hz), 7.13 (2H, d, J=8.58 Hz), 7.41-7.46 (3H, m), 7.98-8.03 (2H, m). | | | | |
| 73. | 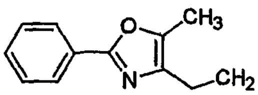 | 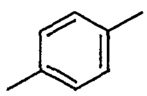 | NHBoc | 452 | 60 |
| | DMSO-$d_6$, $^1$H: 1.28 (9H, s), 2.34 (3H, s), 2.68-2.72 (1H, m), 2.91 (2H, t, J=6.52 Hz), 3.02-3.47 (4H, m), 4.15 (2H, t, J=6.58 Hz.), 6.8 (1H, d, J=8.55 Hz), 7.07 (2H, d, J=8.55 Hz), 7.45-7.52 (3H, m), 7.88-7.91 (2H, m). | | | | |
| 74. | 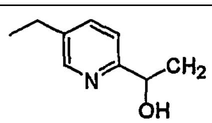 | 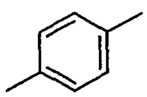 | OEt | 345 | 77 |
| | $^1$H: 1.17 (3H, t, J=6.9 Hz), 1.24-1.32 (3H, m), 2.66-2.71 (4H, m), 3.49-3.57 (5H, m), 4.15 (2H, d, J=6.18 Hz), 5.06-5.10 (1H, t, J=5.7 Hz), 6.84 (2H, d, J=8.64 Hz), 7.09 (2H, d, J=8.61 Hz), 7.39 (1H, d, J=8.04 Hz ), 7.54-7.57 (1H, m), 8.42 (1H, s) | | | | |
| 75. | 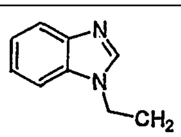 | 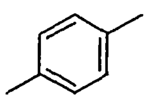 | OEt | 340 | 99 |
| | $^1$H: 1.15 (3H, t, J=6.99 Hz), 2.68 (1H, d, J=6.78 Hz), 2.76 (1H, d, J=6.03 Hz), 3.39-3.57 (5H, m), 4.39 (2H, t, J=5.1 Hz), 4.57 (2H, t, J=5.1 Hz), 6.75 (2H, d, J=8.61 Hz), 7.0 (2H, d, J=8.58 Hz), 7.28-7.33 (2H, m), 7.47 (1H, d, J=6.96 Hz), 7.82 (1H, d, J=6.93 Hz), 8.04 (1H, s) | | | | |

(continued)

| Ex. No | $R^1$ | Ar | $G_1$ | Mol. Wt | % yield |
|---|---|---|---|---|---|
| 76. | 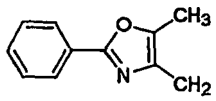 | 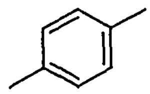 | OEt | 367 | 97 |
| | $^1$H: 1.17 (3H, t, J=6.99 Hz), 2.4 (3H, s), 2.6-2.8 (2H, m), 3.4 - 3.6 (5H, m), 4.9 (2H, s), 6.94 (2H, d, J= 8.64 Hz), 7.12 (2H, d, J=8.58 Hz), 7.42 -7.46 (3H, m), 8.0 - 8.03 (2H, m) | | | | |
| 77. | 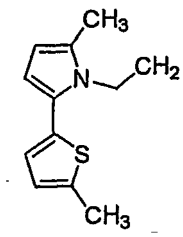 | 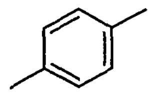 | OEt | 399 | 96 |
| | $^1$H: 1.07 (3H, t, J=6.9 Hz), 2.26 (3H, s), 2.48 (3H, s), 2.62-2.77 (2H, m), 3.41-3.49 (5H, m), 4.05 (2H, t, J=5.7 Hz), 4.29 (2H, t, J=5.6 Hz), 5.81 (1H, d, J=2.9 Hz), 6.02 (1H, d, J=3.4 Hz), 6.75-6.78 (3H, m), 6.89 (1H, d, J=3.4 Hz), 7.1 (2H, d, J=8.5 Hz) | | | | |
| 78. | 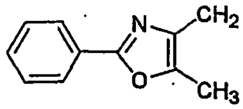 | 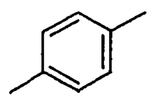 | OH | 339 | 68 |
| | $^1$H: 1.9 (1H, s), 2.02 (1H, s), 2.43 (3H, s), 2.66-2.80 (2H, m), 3.5 (1H, m), 3.68 (1H, m), 3.9 (1H, m), 4.98 (2H, s), 6.9 (2H, d, J=8.5 Hz), 7.1 (2H, d, J=8.5 Hz), 7.42-7.46 (3H, m), 7.9-8.03 (2H, m) | | | | |
| 79. | 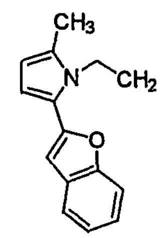 | 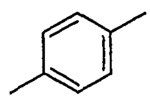 | OEt | 419 | 86 |
| | $^1$H : 1.16 (3H, t, J=6.99 Hz), 2.39 (3H, s), 2.64-2.77 (2H, m), 3.47-3.53 (5H, m), 4.23 (2H, t, J=6.07 Hz), 4.54 (2H, t, J=6.06 Hz), 5.99 (1H, d, J=3.57 Hz), 6.56 (1H, d, J=3.6 Hz), 6.69 (1H, s), 6.73 (2H, d, J=8.61 Hz), 7.05 (2H, d, J=8.55 Hz), 7.2-7.5 (4H, m) | | | | |
| 80. | 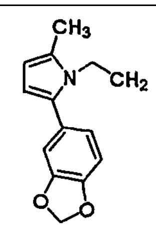 | 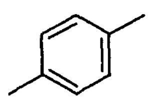 | OEt | 423 | 93 |
| | $^1$H : 1.17 (3H, t, J=7.0 Hz), 2.35 (3H, s), 2.66-2.77 (2H, m), 3.45-3.57 (4H, m), 3.58-3.74 (1H, m), 3.92 (2H, t, J=6.54 Hz), 4.25 (2H, t, J=6.52 Hz), 5.93 (1H, d, J=3.12 Hz), 5.99 (2H, s), 6.04 (1H, d, J=3.39 Hz), 6.64 (2H, d, J=8.58 Hz), 6.84-6.88 (3H, m), 7.04 (2H, d, J=8.55 Hz) | | | | |
| 81. | 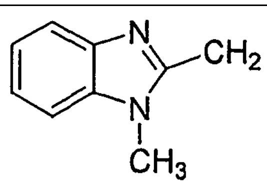 | 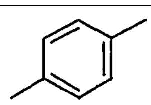 | OEt | 340 | 40 |
| | $^1$H : 1.17 (3H, t, J=6.78 Hz), 2.68-2.77 (2H, m), 3.44-3.61 (5H, m), 3.89 (3H, s), 5.36 (2H, s), 6.99 (2H, d, J=8.64 Hz), 7.12 (2H, d, J=8.61 Hz), 7.26-7.79 (4H, m) | | | | |

(continued)

| Ex. No | $R^1$ | Ar | $G_1$ | Mol. Wt | % yield |
|---|---|---|---|---|---|
| 82. | 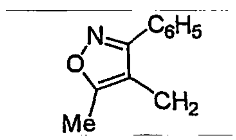 | 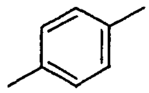 | OEt | 367 | 100 |
| | $^1H$ : 1.19 (3H, t, J=6.97 Hz), 2.49 (3H, s), 2.67-2.83 (2H, m), 3.44-3.62 (5H, m), 4.83 (2H, s), 6.88 (2H, d, J=8.55 Hz), 7.15 (2H, d, J=8.49 Hz), 7.26-7.74 (5H, m) | | | | |

Table 4:

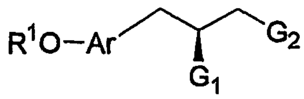

| Ex. No | $R^1$ | Ar | $G_1$ | $G_2$ | Mol. Wt | % yield |
|---|---|---|---|---|---|---|
| 83. | 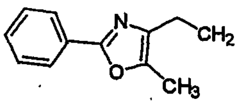 | 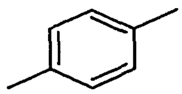 | Oet | $CH_2OH$ | 394 | 87 |
| | $^1H$: 1.17 (3H, t, J=7.00 Hz), 1.66-1.69 (2H, m), 2.37 (3H, s), 2.59-2.66 (2H, m), 2.97 (2H, t, J=.6.75 Hz), 3.43-3.48 (1H, m), 3.57-3.62 (1H, m), 3.72-3.74 (3H, m), 4.21 (2H, t, J= 6.7 Hz), 6.83 (2H, d, J=8.6 Hz), 7.06 (2H, d, J=8.6 Hz), 7.39-7.45 (3H, m), 7.95-7.99 (2H, m). | | | | | |

Preparation 5

1-Ethoxy-(2S)-ethoxy-3-[4-{2-(5-methyl-2-phenyl-oxazol-4-yl)-ethoxy}-phenyl]-propane (compound No 84).

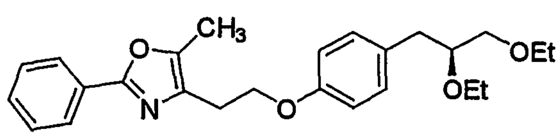

To a stirred suspension of powdered sodium hydroxide (250 mg) in dimethylsulfoxide (10 mL), (2S)-Ethoxy-3-{4-[2-(5-methyl-2-phenyl-oxazol-4-yl)-ethoxy]-phenyl}-propan-1-ol (compound No 64) (1.15 g) was added and stirred at ambient temperature for 20 minutes. Reaction mixture was cooled in an ice bath and ethyl iodide (0.5 g) was added and stirred for further 30 minutes at the same temperature followed by 17 hours at ambient temperature in nitrogen atmosphere. Reaction mixture was poured in ice cold water and extracted with diethyl ether (3X50mL). The combined organic extract was washed with water (100 mL), brine (100 mL), dried over sodium sulfate and evaporated under reduced pressure. Crude product was chromatographed over silicagel using 5% ethyl acetate in petroleum ether to yield 0.6 g of title compound.

Preparation 6

2-((2S)-Ethoxy-3-{4-[2-(5-methyl-2-phenyl-oxazol-4-yl)-ethoxy]-phenyl}-propoxy)-benzoic acid (compound No 89).

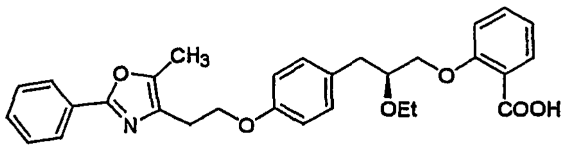

Step 1: Preparation of Methyl-2-((2S)-ethoxy-3-{4-[2-(5-methyl-2-phenyl-oxazol-4-yl)-ethoxy]-phenyl}-propoxy)-benzoate.

To a solution of (2S)-Ethoxy-3-{4-[2-(5-methyl-2-phenyl-oxazol-4-yl)-ethoxy]-phenyl}-propyl methane sulfonate (compound No 91) (0.9 g) in toluene (10 mL) potassium carbonate (0.5 g) was added followed by methyl salicylate (0.25 mL) and the reaction mixture was refluxed for 3 hours. Reaction mixture was cooled to ambient temperature and poured in ice cold water. It was extracted with ethyl acetate (3X50 mL). The combined organic extract was washed with water (100 mL), brine (100 mL), dried over sodium sulfate and evaporated under reduced pressure to yield 818 mg of product.

Step 2: 2-((2S)-Ethoxy-3-{4-[2-(5-methyl-2-phenyl-oxazol-4-yl)-ethoxy]-phenyl}-propoxy)-benzoic acid.

To a solution of Methyl-2-((2S)-ethoxy-3-{4-[2-(S-methyl-2-phenyl-oxazol-4-yl)-ethoxy]-phenyl}-propoxy)-benzoate. (518 mg) in methanol (10 mL) was added another solution of sodium hydroxide (241 mg) in water (5 mL) and the reaction mixture was stirred at ambient temperature for 72 hours. Solvents were evaporated under reduced pressure. Residue was dissolved in water (50 mL), acidified with 1N HCl and extracted with diethyl ether (3X50 mL). The combined organic extract was washed with water (50 ml), brine (50 mL), dried over sodium sulfate and evaporated under reduced pressure. Crude product was recrystalized from a mixture of diisopropyl ether and petroleum ether to yield 345 mg of product.

<u>Preparation 7</u>

((2S)-Ethoxy-3-{4-[2-(5-methyl-2-phenyl-oxazol-4-yl)-ethoxy]-phenyl}-propoxy)-acetic acid (compound No 87)

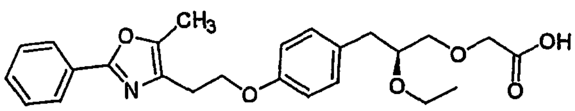

Step 1: Preparation of Ethyl- ((2S)-ethoxy-3-{4-[2-(5-methyl-2-phenyl-oxazol-4-yl)-ethoxy]-phenyl}-propoxy)-acetate.

To a stirred suspension of 50% sodium hydride (189 mg) in tetrahydro furan (10 mL) was added a solution of (2S)-Ethoxy-3-{4-[2-(5-methyl-2-phenyl-oxazol-4-yl)-ethoxy]-phenyl}-propan-1-ol (1.0 g) in 5 mL tetrahydrofuran at a temperature below 10˚C and stirred at ambient temperature for 2 hours. Reaction mixture was again cooled below 10 ˚C and to it was added ethyl bromoacetate (1.75 mL) and stirred at ambient temperature for 15 hours. Reaction mixture was poured into ice cold water (50 mL) and extracted with diethyl ether (3X50 mL). The combined organic extract was washed with water (100 mL), brine (100 mL), dried over sodium sulfate and evaporated under reduced pressure. Crude product was chromatographed over silicagel using 7% ethyl acetate in petroleum ether to yield 350 mg of title compound and 300 mg of (2S)-Ethoxy-3-{4-[2-(5-methyl-2-phenyl-oxazol-4-yl)-ethoxy]-phenyl}-propyl bromoacetate (compound no. 90)

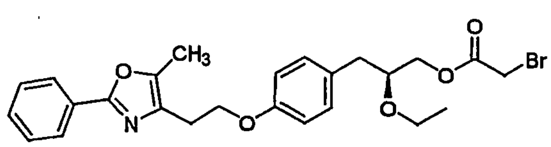

Step 2: Preparation of ((2S)-Ethoxy-3-{4-[2-(5-methyl-2-phenyl-oxazol-4-yl)-ethoxy]-phenyl}-propoxy)-acetic acid.

Title compound was prepared from Ethyl- ((2S)-ethoxy-3-{4-[2-(5-methyl-2-phenyl-oxazol-4-yl)-ethoxy]-phenyl}-propoxy)-acetate following procedure similar to that described in preparation 6, step 2.

<u>Preparation 8</u>

(2S)-Ethoxy-3-{4-[2-(5-methyl-2-phenyl-oxazol-4-yl)-ethoxy]-phenyl}-propyl-methanesulfonate. (compound No 91)

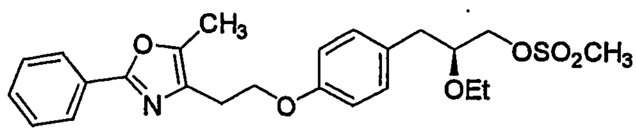

To a solution of (2S)-Ethoxy-3-{4-[2-(5-methyl-2-phenyl-oxazol-4-yl)-ethoxy]-phenyl}-propan-1-ol. (compound 64) (5.4 g) in dichloromethane (80 mL) was added triethyl amine (3.0 nL) and cooled to 10° C. To this was added methanesulfonyl chloride (1.1 mL) dropwise and the reaction mixture was stirred at ambient temperature for 3 hours. Reaction mixture was diluted with dichloromethane (100mL) and washed with water (100 mL). The organic layer was dried over sodium sulfate and evaporated under reduced pressure to yield 6.0 g of title compound.

In like manner following compounds in table 5 were prepared following the procedure described in preparation 5-8 by using appropriate reagents and reaction conditions.

Table 5:

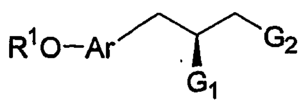

| Ex No | $R^1$ | Ar | $G_1$ | $G_2$ | Mol.Wt | % Yield |
|---|---|---|---|---|---|---|
| 84. | 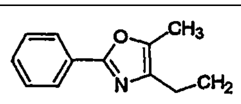 | 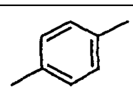 | OEt | OEt | 409 | 57 |
| | $^1$H: 1.1 (3H, t, J=6.99 Hz), 1.2 (3H, t, J=6.99 Hz), 2.4 (3H, s), 2.7 (2H, t, J=6.6 Hz), 3.0 (2H, t, J=6.69 Hz) 3.5 (7H, complex), 4.2 (2H, t, J=6.69 Hz), 6.8 (2H, dd, J=1.87 & 6.65 Hz), 7.1 (2H, d, J=8.55 Hz), 7.4 (3H, m) 7.9 (2H, m). | | | | | |
| 85. | 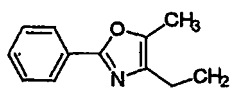 | 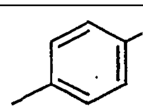 | OEt | 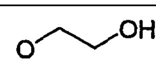 | 425 | 29 |
| | $^1$H: 1.1 (3H, t, J=7.01 Hz), 2.37 (3H, s), 2.7 (2H, dd, J=2.58 & 6.45 Hz), 2.99 (2H, t, J=6.69 Hz) 3.4-3.5 (7H, complex), 3.7 (2H, m), 4.2 (2H, t, J=6.70 Hz), 6.8 (2H, dd, J=2.0 & 6.64 Hz), 7.1 (2H, d, J=8.61 Hz), 7.4 (3H, m), 7.99 (2H, m). | | | | | |
| 86. | 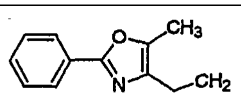 | 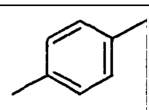 | OEt | 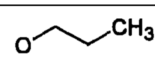 | 423 | 67 |
| | $^1$H: 0.9 (3H, t, J=7.41 Hz), 1.1 (3H, t, J=7.0 Hz), 1.59 (2H, m), 2.3 (3H, s), 2.6-2.8 (2H, m), 2.98 (2H, t, J=6.7 Hz), 3.3 (5H, m), 3.5 (2H, m), 4.2 (2H, t, J=6.7 Hz), 6.8 (2H, dd, J=6.6 & 2.0 Hz), 7.1 (2H, d, J=8.6 Hz), 7.4 (3H, m), 7.9 (2H, m). | | | | | |

(continued)

| Ex No | $R^1$ | Ar | $G_1$ | $G_2$ | Mol.Wt | % Yield |
|---|---|---|---|---|---|---|
| 87. | 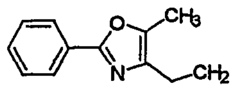 | 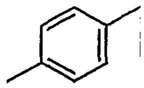 | OEt | 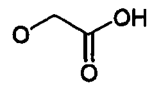 | 439 | 80 |
| | $^1$H: 1.2 (3H, t, J=7.0 Hz), 2.4 (3H, s), 2.7 (1H, dd, J=13.8 & 7.2 Hz), 2.8 (1H, dd, J=13.8 & 5.7 Hz), 3.0 (2H, t, J=6.6 Hz), 3.4 (1H, m), 3.5 - 3.7 (4H,m), 4.0 (2H, s), 4.2 (2H, t, J=6.6 Hz), 6.8 (2H, d, J=8.5 Hz), 7.0 (2H, d, J=8.5 Hz), 7.4 (3H, m), 7.9 (2H, m). | | | | | |
| 88. | 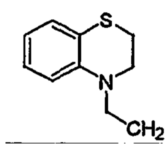 | 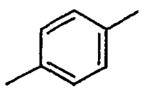 | OEt | OEt | 401 | 88 |
| | $^1$H: 1.12 (3H, t, J=7.0 Hz), 1.19 (3H, t, J=7.0 Hz), 2.75 (2H, m), 3.0 (2H, m), 3.3 8 (2H, dd, J=4.5 & 1.17 Hz), 3.4 (5H, m), 3.7 (2H, t, J=5.8 Hz), 3.8 (2H, m), 4.1 (2H, t, J=5.8 Hz), 6.6 (1H, m), 6.7 (1H, d, J=8.2 Hz), 6.8 (2H, d, J=8.6 Hz), 7.0 (2H, m), 7.1 (2H, d, J=8.6 Hz). | | | | | |
| 89. | 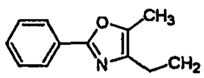 | 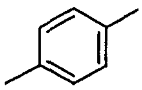 | OEt | 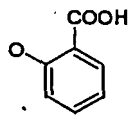 | 501 | 67 |
| | $^1$H: 1.22 (3H, t, J=6.99 Hz), 2.37 (3H, s), 2.79 (1H, dd, J=13.9 & 7.8 Hz), 2.97 (3H, m), 3.61 (2H, m), 3.87 (1H, m), 4.0 (1H, dd, J=10.66 & 7.36 Hz), 4.17 (1H, dd, J=9.61 & 3.21 Hz), 4.2 (2H, t, J=6.7 Hz), 6.88 (3H, m), 7.09 (3H, m), 7.41 (4H, m), 7.96 (2H, dd, J=7.53 & 2.19 Hz), 8.16 (1H, d, J=6.03 & 3.0 Hz). | | | | | |
| 90. | 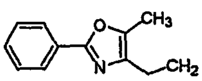 | 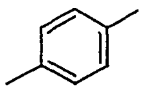 | OEt | 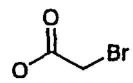 | 502 | 50 |
| | $^1$H: 1.1 (3H, t, J=6.99 Hz), 2.3 (3H, s), 2.7 (2H, m), 2.9 (2H, t, J=6.7 Hz), 3.4 -3.7 (3H, complex), 3.8 (2H, s), 4.0 (1H, m), 4.2 (3H, m), 6.8 (2H, d, J=8.6 Hz), 7.1 (2H, d, J=8.6 Hz), 7.4 (3H, m), 7.9 (2H, m). | | | | | |
| 91. | 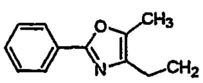 | 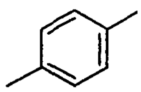 | OEt | $-OSO_2CH_3$ | 459 | 84 |
| | $^1$H: 1.1(3H, t, J=7.0 Hz), 2.3 (3H, s), 2.8 (2H, m), 2.9 (2H, t, J=6.7 Hz), 3.0 (3H, s), 3.5 (2H, m), 3.6 (1H, m), 4.0 (1H, dd, J=10.9 & 5.6 Hz), 4.2 (3H, m), 6.8 (2H, d, J=8.6 Hz), 7.1 (2H, d, J=8.5 Hz), 7.4 (3H, m), 7.9 (2H, dd, J=7.9 & 2.2 Hz). | | | | | |
| 92. | 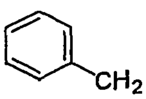 | 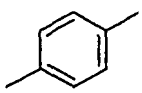 | OEt | $-OSO_2CH_3$ | 364 | 100 |
| 93. | 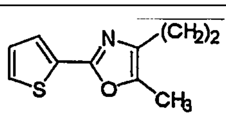 | 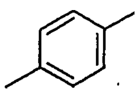 | OEt | $OCH_3$ | 401 | 87 |
| | $^1$H: 1.13 (3H, t, J=6.99 Hz), 2.35 (3H, s), 2.74 (2H, d, J=6.42 Hz), 2.94 (2H, t, J=6.57 Hz), 3.33 (3H, s), 3.42-3.58 (5H,m), 4.20 (2H, t, J=6.61 Hz), 6.8 (2H, d,J=8.32Hz), 7.06-7.13 (3H, m), 7.35-7.36 (1H, m), 7.57-7.58 (1H, m). | | | | | |

(continued)

| Ex No | $R^1$ | Ar | $G_1$ | $G_2$ | Mol.Wt | % Yield |
|---|---|---|---|---|---|---|
| 94. | 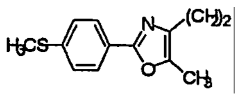 | 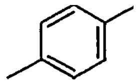 | OEt | OEt | 455 | 88 |
| | $^1$H: 1.11 (3H, t, J=6.99 Hz), 1.17.(3H, t, J=7.0 Hz), 2.39 (3H, s), 2.51 (3H, s), 2.70-2.75 (2H, m), 3.04 (2H, t, J=6 Hz), 3.33-3.55 (7H, m), 4.25 (2H, t, J=6.0 Hz), 6.79 (2H, d, J=8.55 Hz), 7.10 (2H, d, J=8.52 Hz), 7.28 (2H, d, J=8.46 Hz), 8.0 (2H, d, J=8.18 Hz). | | | | | |
| 95. | 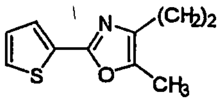 | 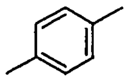 | OEt | OEt | 415 | 50 |
| | $^1$H: 1.12 (3H, t, J=7.0 Hz), 1.25 (3H, t, J=7.0 Hz), 2.35 (3H, s), 2.72-2.8 (2H, m), 2.95 (2H, t, J=6.6 Hz), 3.35-3.38 (2H, m), 3.44-3.56 (5H, m), 4.20 (2H, t, J=6.6 Hz), 6.8 (2H, d, J=8.6 Hz), 7.07-7.10 (1H, m), 7.1 (2H, d, J=8.6 Hz), 7.36-7.38 (1H, m), 7.59-7.60 (1H, m) | | | | | |
| 96. | 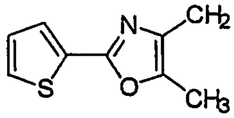 | 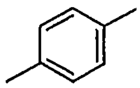 | OEt | OEt | 401 | 37 |
| | $^1$H: 1.11 (3H, t, J=7.0 Hz), 1.17-1.22 (3H, t, J=7.0 Hz), 2.41 (3H, s), 2.74-2.77 (2H, m), 3.36-3.59 (7H, m), 4.95 (2H, s), 6.93 (2H, d, J=8.6 Hz), 7.08-7.11 (1H, m), 7.16 (2H, d, J=8.64 Hz), 7.39-7.41 (1H, m), 7.64-7.66 (1H, m). | | | | | |
| 97. | 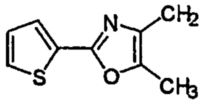 | 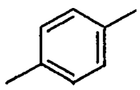 | OEt | $-OSO_2CH_3$ | 451 | 78 |
| | $^1$H: 1.15 (3H, t, J=6.99 Hz), 2.4 (3H, s), 2.77-2.82 (2H, m), 3.03 (3H, s), 3.46-3.61 (3H, m), 4.05-4.26 (2H, m), 4.94 (2H, s), 6.94 (2H, d , J=8.6 Hz), 7.08-7.11 (1H, m), 7.15 (2H, d, J=8.6 Hz), 7.39-7.40 (1H, m), 7.62-7.64 (1H, m). | | | | | |
| 98. | 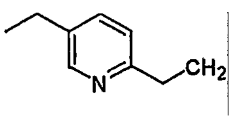 | 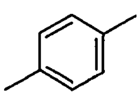 | OEt | $-OSO_2CH_3$ | 407 | 100 |
| | $^1$H: 1.15 (3H, t, J=6.9 Hz), 1.24 (3H, t, J=7.57 Hz), 2.66-2.80 (4H, m), 3.05 (3H, s), 3.32 (2H, t, J=6.4 Hz), 3.49-3.57 (4H, m), 4.02-4.1 (1H, m), 4.34 (2H, t, J=6.4 Hz), 6.84 (2H, d, J=8.53 Hz), 7.1 (2H, d, J=8.5 Hz), 7.25 (1H, d , J=7.97 Hz), 7.65 (1H, m), 8.44 (1H, d, J=1.95 Hz). | | | | | |
| 99. | 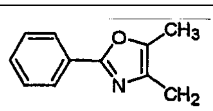 | 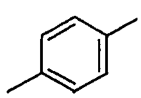 | NHBoc | $-OSO_2CH_3$ | 516 | 85 |
| | $^1$H: 1.42 (9H, s), 2.43 (3H, s), 2.75-2.86 (2H, m), 3.01 (3H, s), 4.05-4.13 (2H, m), 4.22-4.25 (1H, m), 4.97 (2H, s), 6.98 (2H, d, J=8.58 Hz), 7.14 (2H, d, J=8.58 Hz), 7.41-7.46 (3H, m), 8.0-8.03 (2H m). | | | | | |
| 100. | 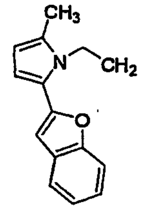 | 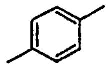 | OEt | $-OSO_2CH_3$ | 497 | 74 |
| | $^1$H : 1.14 (3H, t, J=6.99 Hz), 2.4 (3H, s), 2.75 (2H, t, J=6.93 Hz), 3.02 (3H, s), 3.45-3.58 (2H, m), 3.56-3.67 (2H, m), 4.0-4.1 (1H, m), 4.24 (2H, t, J=5.91 Hz), 4.54 (2H, t, J=6.0 Hz), 5.99 (1H, d, J=3.6 Hz), 6.56 (1H, d, J=3.6 Hz), 6.69 (1H, s), 6.74 (2H, d, J=8.49 Hz), 7.06 (2H, d, J=8.49 Hz), 7.20-7.51 (4H, m) | | | | | |

(continued)

| Ex No | $R^1$ | Ar | $G_1$ | $G_2$ | Mol.Wt | % Yield |
|---|---|---|---|---|---|---|
| 101. | 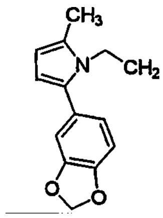 | 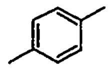 | OEt | $-OSO_2CH_3$ | 501 | 62 |
| | $^1H$ : 1.14 (3H, t, J=6.97 Hz), 2.35 (3H, s), 2.76 (2H, t, J=6.48 Hz), 3.03 (3H, s), 3.48-3.56 (4H, m), 3.92 (2H, t, J=6.48 Hz), 4.20 (1H, t, J=5.46 Hz), 4.25 (2H, t, J=6.31 Hz), 5.92 (1H, d, J=3.3 Hz), 5.99 (2H, s), 6.03 (1H, d, J=3.39 Hz), 6.64 (2H, d, J=8.58 Hz), 6.81-6.84 (3H, m), 7.05 (2H, d, J=8.55 Hz) | | | | | |
| 102. | 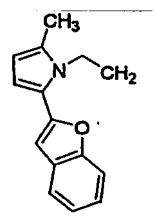 | 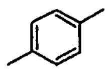 | OEt | 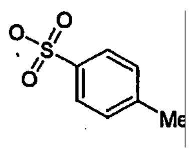 | 573 | 77 |
| | $^1H$ : 1.03 (3H, t, J=6.97 Hz), 2.39 (3H, s), 2.42 (3H, s), 2.65 (2H, d, J=6.39 Hz), 3.33-3.44 (4H, m), 3.92 (2H, t, J=5.28 Hz), 4.19-4.21 (1H, m), 4.54 (2H, t, J=6.01 Hz), 5.98 (1H, d, J=3.6 Hz), 6.56 (1H, d, J=3.6 Hz), 6.67-6.70 (3H, m), 6.97 (2H, d, J=8.46 Hz), 7.21-7.54 (6H, m), 7.75 (2H, d, J=8.22 Hz) | | | | | |
| 103. | 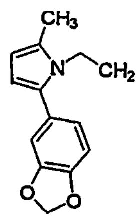 | 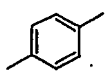 | OEt | 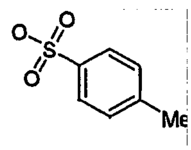 | 577 | 85 |
| | $^1H$ : 1.03 (3H, t, J=6.99 Hz), 2.35 (3H, s), 2.43 (3H, s), 2.65 (2H, d, J=6.39 Hz), 3.33-3.56 (3H, m), 3.89-3.94 (4H, m), 4.25 (2H, t, J=6.48 Hz), 5.92 (1H, d, J=3.33 Hz), 5.99 (2H, s), 6.03 (1H, d, J=3.36 Hz) , 6.59 (2H, d, J=8.55 Hz), 6.84-6.90 (3H, m), 6.97 (2H, d, J=8.52 Hz), 7.31 (2H, d, J=8.4 Hz), 7.76 (2H, d, J=8.28 Hz) | | | | | |
| 104. | 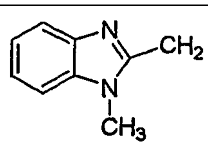 | 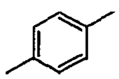 | OEt | OEt | 368 | 92 |
| | $^1H$ : 1.50 (3H, t, J=6.99 Hz), 1.19 (3H, t, J=6.95 Hz), 2.75 (2H, t, J=6.18 Hz), 3.35-3.57 (7H, m), 3.89 (3H, s), 5.36 (2H, s), 6.98 (2H, d, J=8.53 Hz), 7.15 (2H, d, J=8.5 Hz), 7.26-7.37 (4H, m) | | | | | |
| 105. | 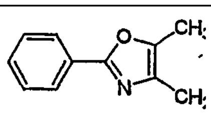 | 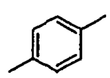 | OEt | *O-n*-Pr | 409 | 40 |
| | $^1H$ : 1.13 (3H, t, J=7.0 Hz), 1.27 (3H, t, J=7.06 Hz), 1.53-1.65 (2H, m), 2.43 (3H, s), 2.69-2.84 (2H, m), 3.35-3.45 (5H, m), 3.55-3.61 (2H, m), 4.98 (2H, s), 6.93 (2H, d, J=8.67 Hz), 7.15 (2H, d, J=8.64 Hz), 7.14-7.46 (3H, m), 8.0-8.04 (2H, m) | | | | | |
| 106. | 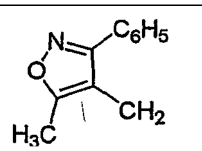 | 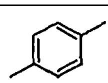 | OEt | OEt | 395 | 98 |
| | $^1H$ : 1.15 (3H, t, J=6.97 Hz), 1.18 (3H, t, J=7.0 Hz), 2.48 (3H, s), 2.78 (2H, d, J=6.8 Hz), 3.39-3.59 (7H, m), 4.83 (2H, s), 6.87 (2H, d, J=8.49 Hz), 7.17 (2H, d, J=8.49 Hz), 7.42-7.74 (5H, m), | | | | | |

(continued)

| Ex No | $R^1$ | Ar | $G_1$ | $G_2$ | Mol.Wt | % Yield |
|---|---|---|---|---|---|---|
| 107. | 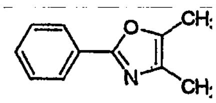 | 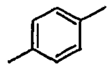 | OEt | 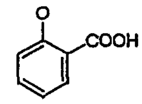 | 487 | 85 |
| | $^1H$ : 1.23 (3H, t, J=7.0 Hz), 2.4 (3H, s), 2.8-3.0 (2H, m), 3.5-3.6 (2H, m), 3.6-3.7 (1H, m), 3.9 (1H, m), 4.0-4.1 (1H, m), 4.9 (2H, s), 6.85 (1H, d, J=8.31 Hz), 6.97 (2H, d, J=8.52 Hz), 7.10-7.15 (3H, m), 7.42-7.49 (4H, m ), 8.0-8.15 (2H, m), 8.17 (1H, d, J =6.15 Hz) | | | | | |
| 108. | 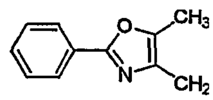 | 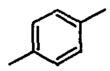 | OEt | $-OSO_2CH_3$ | 446 | 98 |
| | $^1H$ : 1.15 (3H, t, J=6.99 Hz), 2.4 (3H, s), 2.7 - 2.8 (2H, m), 3.0 (3H, s), 3.4 - , 3.6 (2H, m), 3.6 - 3.7 (1H, m), 4.0 - 4.2 (2H , m), 4.9 (2H, s), 6.95 (2H, d, J=8.61), 7.14 (2H, d, J=8.61 Hz), 7.41 - 7.46 (3H, m), 8.0 -8.03 (2H, m) | | | | | |
| 109. | 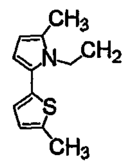 | 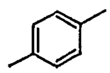 | OEt | $-OSO_2CH_3$ | 477 | 90 |
| | $^1H$ : 1.14 (3H, s), 2.48 (3H, s), 2.74 (3H, s), 2.79 (2H, m), 3.03 (3H, s), 3.43-3.57 (3H, m), 4.05 (3H, t, J=6.4 Hz), 4.33 (1H, m), 4.5 (2H, m), 5.9 (1H, d, J=2.8 Hz), 6.1 (1H, d, J=3.2 Hz), 6.7 (2H, m), 6.8 (1H, d, J=3.1 Hz), 7.09 (2H, d, J=8.2 Hz), 7.25 (1H, s) | | | | | |
| 110. | 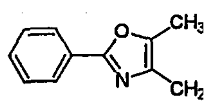 | 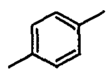 | OEt | OEt | 395 | 76 |
| | $^1H$ : 1.13 (3H, t, J=6.9 Hz), 1.19 (3H, t, J=6.9 Hz), 2.43 (3H, s), 2.77 (2H, m), 3.37-3.63 (7H, m), 4.97 (2H, s), 6.9 (2H, d, J=8.6 Hz), 7.17 (2H, d, J=8.6 Hz), 7.42-7.47 (3H, m), 8.0-8.03 (2H, m) | | | | | |
| 111. | 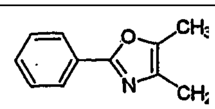 | 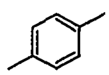 | OEt | 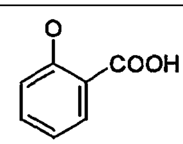 | 443 | 51 |
| | $^1H$ : 1.15 (3H, t, J=7.0 Hz), 2.47 (3H, s), 2.89-2:92 (2H, m), 3.5 (1H, m), 3.65 (1H, m), 3.8 (1H, m), 3.9 (2H, m), 5.0 (2H, s), 6.87-6.96 (5H, m), 7.15 (2H, m), 7.25 (2H, m), 7.5 (3H, m), 8.05 (2H, m) | | | | | |

Preparation 9

2-Ethoxy-l-(4-[2-(5-methyl-2-phenyl-oxazol-4-yl)-ethoxy]-phenyl)-3-hydroxypentane. (Compound.No.114)

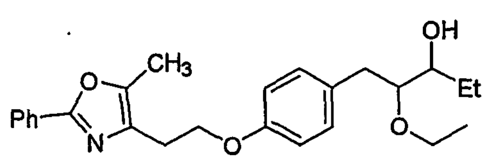

$K_2CO_3$ (0.645 g) was added to a solution of 4-(pentane 2-Ethoxy-3-hydroxy)-phenol (700 mg) in toluene (5 mL) at 20-30 ˚C. The reaction was stirred at reflux temp. for 1 hour. To the reaction mixture was added 2-(2-phenyl-5-methyl-oxazole-4-yl)ethyl methane sulfonate (878 mg).Reaction mixture was stirred for 36 hour at reflux temperature.

Reaction mixture was poured in to water (25 mL) and extracted with ethyl acetate (2 x 25 mL). Combined organic layer was washed with water (2 x 50 mL) & brine (50 mL), dried over sodium sulfate and evaporated under reduced pressure. Crude product was chromatographed over silicagel using pet.ether:ethyl acetate (9:1) as an eluent to afford pure 157 mg product.

Preparation 10

2-Ethoxy-1-{4-[2-(5-methyl-2-phenyl-oxazol-4-yl)-ethoxy]-phenyl}-3-ethoxy pentane (Compound. No. 115)

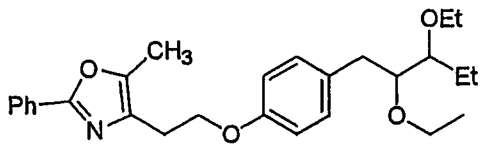

$K_2CO_3$ (0.368 g) was added to a solution of 4-(pentane 2,3-diethoxy)-phenol (403 mg) in toluene (5 mL) at 20-30 ˚C. The reaction mixture was stirred at reflux temperature for 1 hour. To the reaction mixture was added 2-(2-phenyl-5-methyloxazole-4-yl)ethyl methane sulfonate (500 mg). Reaction mixture was stirred for 36 hours at reflux temperature. Reaction mixture was poured in to water (25 mL) and extracted with ethyl acetate (2 x 25 mL). Combined organic layer was washed with water (2 x 50 mL), brine (50 mL) dried over sodium sulfate and evaporated under reduced pressure to yield the crude title compound (206 mg). Crude product was chromatographed over silicagel using pet.ether: ethyl acetate (9:1) as an eluent to afford pure 90 mg product.

In like manner following compounds in table 6 were prepared by a method similar to that described in preparation 9-10.

Table 6:

| 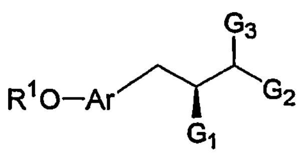 | | | | | | | |
|---|---|---|---|---|---|---|---|
| Ex. No | $R^1$ | Ar | $G_1$ | $G_2$ | $G_3$ | Mol. Wt | % yield |
| 112. | 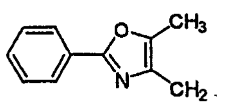 | 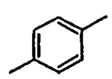 | OEt | OH | Et | 395 | 68 |
| | $^1H$: 0.94 (3H, t, J=7.39 Hz), 1.02 (3H, t, J=7.29 Hz), 1.11 (3H, t, J=6.99 Hz), 1.51-1.53 (2H, m), 2.43 (3H, s), 2.72-2.75 (2H; m), 3.28-3.47 (4H, m), 4.97 (2H, s), 6.94 (2H, d, J=8.5 Hz), 7.15 (2H, d, J=8.43 Hz), 7.42-7.46 (3H, m), 7.99-8.03 (2H, m) | | | | | | |
| 113. | 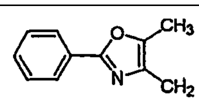 | 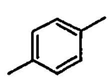 | OEt | OEt | Et | 423 | 26 |
| | $^1H$ - 0.96 (3H, t, J=3.6 Hz), 1.05 (3H, t, J=3.4 Hz), 1.21 (3H, t, J=7.14 Hz), 1.5 (2H, m), 2.4 (3H, s), 2.68 (2H, m), 3.39-3.47 (2H, m), 3.56-3.59 (4H, m), 4.9 (2H, s), 6.93 (2H, d, J=8.54 Hz), 7.16 (2H, d, J=6.8 Hz), 7.42-7.46 (3H, m), 8.00-8.03 (2H, m) | | | | | | |

(continued)

| Ex. No | $R^1$ | Ar | $G_1$ | $G_2$ | $G_3$ | Mol. Wt | % yield |
|---|---|---|---|---|---|---|---|
| 114. | 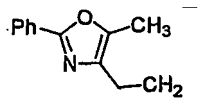 | 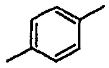 | OEt | OH | Et | 409 | 21.59 |
| | $^1$H : 0.93 (3H, t, J=7.41 Hz), 1.10 (3H, t, J=6.99 Hz) , 1.47-1.52 (2H, m), 2.37 (3H, s), 2.73-2.79 (2H, m ), 2.97 (2H, t, J=6.69 Hz), 3.26-3.47 (4H, m), 4.22 (2H, t, J=6.70 1Hz), 6.8 (2H, d, J=8.55 Hz ), 7.11 (2H, d, J=8.55 Hz), 7.39-7.45 (3H, m), 7.95-7.99 (2H, m) | | | | | | |
| 115. | 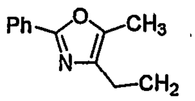 | 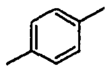 | OEt | OEt | Et | 437 | 26.51 |
| | $^1$H : 0.91-0.96 (3H, m), 1.01-1.06 (3H, m), 1.18-1.61 (3H, m), 1.55-1.61 (2H, m), 2.37 (3H, s), 2.61-2.81 (2H, m), 2.97 (2H, t, J=6.7 Hz), 3.1-3.2 (1H, m), 3.2-3.3 (1H, m), 3.35-3.45 (2H, m), 3.5-3.6 (2H, m), 4.22 (2H, t, J=6.70 Hz ), 6.82 (2H, d, J=8.55 Hz), 7.12 (2H, d, J=8.52 Hz), 7.39-7.45 (3H, m), 7.96-7.99 (2H, m) | | | | | | |

Preparation 11

(2S)-Ethoxy-3-{4-[2-(5-methyl-2-phenyl-oxazol-4-yl)-ethoxy]-phenyl}-propyl azide. (compound No 116)

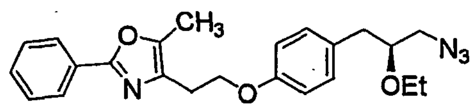

To a solution of (2S)-Ethoxy-3-{4-[2-(5-methyl-2-phenyl-oxazol-4-yl)-ethoxy]-phenyl}-propyl-methanesulfonate. (compound 91) (6.5 g) in dimethylformamide (30 mL), sodium azide (5.3 g) was added and the reaction mixture was heated at 90°C for four hours. Reaction mixture was cooled to 25 °C and poured into water and extracted with ethyl acetate (3X100 mL). The combined organic extract was washed with water (100 mL), brine (100 mL), dried over sodium sulfate and evaporated under reduced pressure. Crude product was triturated with methanol (30 mL) to yield 4.5 g of title compound.

In like manner following compounds in table 7 were prepared by a procedure similar to that described for preparation 11.

Table 7:

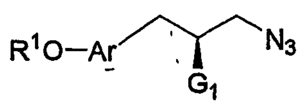

| Ex. No | R | Ar | $G_1$ | Mol. Wt | % yield |
|---|---|---|---|---|---|
| 116. | 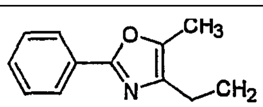 | 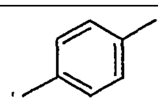 | OEt | 406 | 71 |
| | $^1$H: 1.2 (3H, t, J=7.0 Hz), 2.3 (3H, s), 2.7 (1H, dd, J=13.5 & 6.6 Hz), 2.8 (1H, dd, J=13.5 & 6.2 Hz), 2.9 (2H, t, J=6.7 Hz), 3.1 (2H, m), 3.5 (3H, m), 4.2 (2H, t, J=6.7 Hz), 6.8 (2H, d, J=9.5 Hz), 7.1 (2H, d, J=8.5 Hz), 7.4 (3H, m), 7.9 (2H, m). | | | | |

(continued)

| Ex. No | R | Ar | $G_1$ | Mol. Wt | % yield |
|---|---|---|---|---|---|
| 117. | 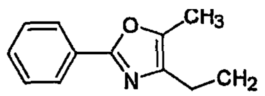 | 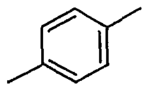 | OH | 378 | 49 |
| | $^1$H: 2.38 (3H, s), 2.73 (2H, dd, J=6.8 & 2.6 Hz), 2.97 (2H, t, J=6.8 Hz), 3.2 (1H, dd, J=12.4 & 6.8 Hz), 3.3 (1H, dd, J=12.4& 3.6 Hz), 3.9 (1H, m), 4.22 (2H, t, J=6.57Hz), 6.83 (2H, d, J=8.64 Hz), 7.15 (2H, d, J=11.5 Hz), 7.41 (3H, m), 7.97 (2H, dd, J=7.59 & 2.25 Hz). | | | | |
| 118. | 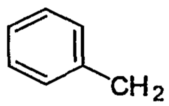 | 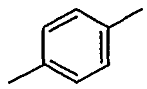 | OEt | 311 | 93 |
| | $^1$H: 1.1 (3H, t, J=6.9 Hz), 2.7 (1H, dd, J=13.8 & 6.8 Hz), 2.8 (1H, dd, J=14.0 & 6.1 Hz), 3.1 (2H, m), 3.5 (3H, m), 5.0 (2H, s), 6.9 (2H, d, J=8.5 Hz), 7.1 (2H, d, J=8.5 Hz), 7.3 - 7.4 (5H, m). | | | | |
| 119. | 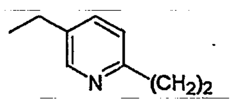 | 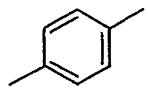 | OEt | 1354 | 96 |
| | $^1$H: 1.17 (3H, t, J=6.9 Hz), 1.24 (3H, t, J=7.6 Hz), 2.61-2.72 (3H, m), 2.78-2.80 (1H, m), 3.13-3.24 (4H, m), 3.50-3.58 (3H, m), 4.31 (2H, t, J= 6.7 Hz), 6.85 (2H, d , J=8.5 Hz), 7.1 (2H, d, J=8.5 Hz), 7.17 (1H, d, J=7.87 Hz), 7.45 (1H, m), 8.38 (1H, d, J=2.09Hz). | | | | |
| 120. | 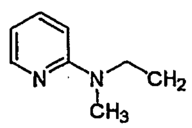 | 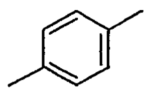 | OEt | 355 | 82 |
| | $^1$H: 1.18 (3H, t, J=6.99 Hz), 2.69-2.8 (2H, m), 3.14 (3H, s), 3.14-3.19 (2H, m), 3.52-3.57 (3H, m), 3.97 (2H, t, J=5.65 Hz), 4.16 (2H, t, J=5.65 Hz), 6.50-6.56 (2H, m), 6.83 (2H, d, J=8.64 Hz), 7.08 (2H, d, J=8.64 Hz), 7.44-7.46 (1H, m); 8.14-8.16 (1H, m). | | | | |
| 121. | 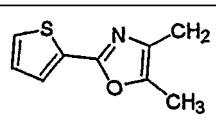 | 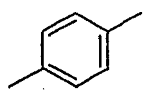 | OEt | 398 | 95 |
| | $^1$H: 1.18 (3H, t, J=6.99 Hz), 2.4 (3H, s), 2.72-2.88 (2H, m), 3.17-3.2 (2H, m), 3.48-3.6 (3H, m), 4.94 (2H, s), 6.9 (2H, d, J=8.6 Hz), 7.053-7.151 (3H, m), 7.38-7.40 (1H, m), 7.62-7.64 (1H, m) | | | | |
| 122. | 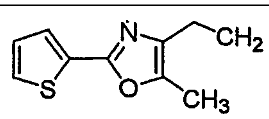 | 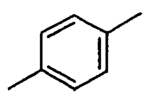 | OEt | 412 | 83 |
| | $^1$H: 1.18 (3H, t, J=7.00 Hz), 2.35 (3H, s), 2.69-2.80 (2H, m), 2.94 (2H, t, J=6.66 Hz), 3.16-3.18 (2H, m), 3.52-3.58 (3H, m), 4.2 (2H, t, J=6.64 Hz), 6.81 (2H, d, J=8.6 Hz), 7.06-7.09 (3H, m), 7.353-7.373 (1H, dd, J=1.14 & 5.07 Hz), 7.57-7.58 (1H, m). | | | | |
| 123. | 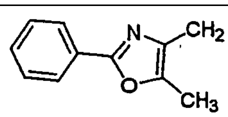 | 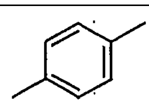 | OH | 363 | 53 |
| | $^1$H: 1.96 (1H, d, J=4.17 Hz), 2.44 (3H, s), 2.7-2.8 (2H, m), 3.25-3.41 (3H, m), 4.98 (2H, s), 6.97 (2H, d, J=8.6 Hz), 7.15 (2H, d, J=8.76 Hz), 7.4-7.45 (3H, m), 8.0-8.03 (2H, m). | | | | |
| 124. | 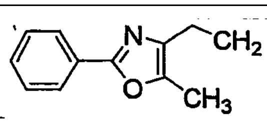 | 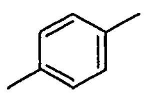 | $NH_2$ | 377 | 20 |
| | $^1$H: 2.38 (3H, s), 2.86 (2H, d, J=5.64 Hz), 2.96 (2H, t, J=6.44 Hz), 3.48-3.53 (2H, m), 3.63-3.67 (1H, m), 4.23 (2H, t, J=6.48 Hz), 6.93 (2H, d, J=8.31 Hz), 7.15 (2H, d, J=8.35 Hz), 7.45-7.48 (3H, m), 7.93-7.96 (2H m). | | | | |

(continued)

| Ex. No | R | Ar | $G_1$ | Mol. Wt | % yield |
|---|---|---|---|---|---|
| 125. | 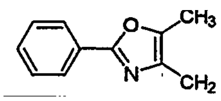 | 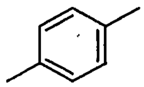 | $NH_2$ | 363 | 40 |
| | DMSO-$d_6$, $^1$H: 2.43 (3H, s), 2.69 (2H, d, J=6.21 Hz), 3.25-3.30 (2H, m), 3.43-3.49 (1H, m), 4.96 (2H, s), 6.98 (2H, d, J=8.58 Hz), 7.15 (2H, d, J=8.58 Hz), 7.51-7.53 (3H, m), 7.91-7.94 (2H, m). | | | | |
| 126. | 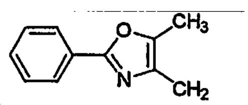 | 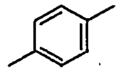 | NHBoc | 463 | 93 |
| | $^1$H: 1.42 (9H, s), 2.43 (3H, s), 2.69-2.80 (2H, m), 3.27-3.44 (2H, m), 3.92 (1H, broad s), 4.97 (2H, s), 6.97 (2H, d, J=8.64 Hz), 7.12 (2H, d, J=8.52 Hz), 7.42-7.46 (3H, m), 8.0-8.03 (2H, m). | | | | |
| 127. | 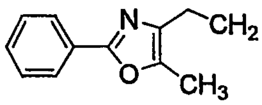 | 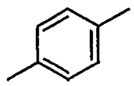 | NHBoc | 477 | 65 |
| | $^1$H: 1.41 (9H, s), 2.37 (3H, s), 2.66-2.78 (2H, m), 2.97 (2H, t, J=6.66 Hz), 3.24-3.41 (2H, m), 3.89 (1H, broad s), 4.24 (2H, t, J=6.68 Hz), 6.84 (2H, d, J=8.61 Hz), 7.07 (2H, d, J=8.57 Hz), 7.40-7.446 (3H, m), 7.95-7.99 (2H, m) | | | | |
| 128. | 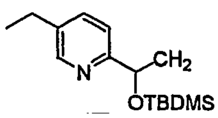 | 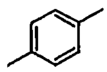 | OEt | 484 | 65 |
| | $^1$H: 0.05 (3H, s), 0.10 (3H, s), 0.91 (9H, s), 1.17 (3H, t, J=6.9 Hz), 1.26 (3H, t, J=7.6 Hz), 2.6-2.7 (4H, m), 3.17 (2H, m), 3.4-3.9 (3H, m), 3.95-3.98 (1H, m), 4.25-4.28 (1H, m), 5.155-5.18 (1H, m), 6.82 (2H, d, J=8.64 Hz), 7.06 (2H, d, J=8.61 Hz), 7.50 -7.54 (2H, m), 8.3 (1H, s) | | | | |
| 129. | 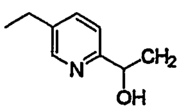 | 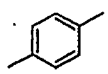 | OEt | 370 | 90 |
| | $^1$H: 1.17 (3H, t, J=6.9Hz), 1.26 (3H, t, J=7.6 Hz), 2.66- 2.72 (4H, m), 3.17 (2H, m), 3.49-3.57 (3H, m), 4.15 (2H, d, J=5.82 Hz), 5.08 (1H, t, J=5.79 Hz), 6.84 (2H, d, J=8.5 Hz), 7.08 (2H, d, J=8.52 Hz), 7.38 (1H, d, J=7.95 Hz), 7.54-7.57 (1H, m), 8.41 (1H, s) | | | | |
| 130. | 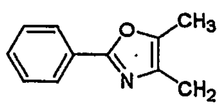 | 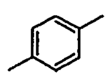 | OEt | 392 | 97 |
| | $^1$H: 1.18 (3H, t, J=6.99 Hz), 2.4 (3H, s), 2.7 - 2.85 (2H, m), 3.2 (2H, m), 3.45 -3.65 (3H, m), 4.9 (2H, s), 6.95 (2H, d, J=8.6), 7.14 (2H, d, J=8.61 Hz), 7.41 - 7.46 (3H, m), 8.0 -8.03 (2H, m) | | | | |
| 131. | 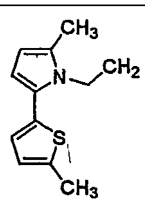 | 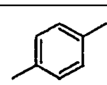 | OEt | 424 | 96 |
| | $^1$H: 1.18 (3H, t, J=6.9 Hz), 2.3 (3H, s), 2.48 (3H, s), 2.65-2.69 (2H, m), 3.16 (2H, m), 3.47-3.57 (3H, m), 4.05 (2H, t, J=6.2 Hz), 4.3 (2H, t, J=6.3 Hz), 5.9 (1H, d, J=3.1Hz), 6.1 (1H, d, J=3.2), 6.7 (3H ,m), 6.8 (1H, d, J=3.3 Hz), 7.04 (2H, d, J=8.4 Hz) | | | | |

(continued)

| Ex. No | R | Ar | $G_1$ | Mol. Wt | % yield |
|---|---|---|---|---|---|
| 132. | 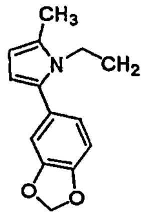 | 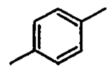 | OEt | 448 | 88 |
| | $^1H$ : 1.17 (3H, t, J=6.96 Hz), 2.3 (3H, s), 2.68-2.78 (2H, dd, J=6.12&6.45 Hz), 3.12- 3.16 (2H, m), 3.49-3.56 (3H, m), 3.92 (2H, t, J=6.39 Hz), 4.25 (2H, t, J=6.3 Hz), 5.92 (1H, d, J=2.79 Hz), 6.0 (1H, d, J=3.27 Hz), 5.98 (2H, s), 6.63 (2H, d, J=8.46 Hz), 6.85 (3H, m), 7.03 (2H, d, J=8.4 Hz). | | | | |
| 133. | 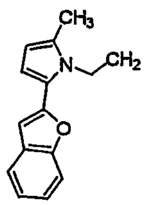 | 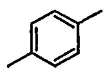 | OEt | 444 | 92 |
| | $^1H$: 1.16 (3H, t, J=6.9 Hz), 2.3 (3H, s), 2.8-2.7 (2H, m), 3.14-3.16 (2H, m), 3.48-3.54 (3H, m), 4.23 (2H, t, J=6.04 Hz), 4.54 (2H, t, J=6.04 Hz), 5.98 (1H, d, J=3.0 Hz), 6.56 (1H, d, J=3.6 Hz) , 6.69-6.75 (3H, m), 7.03 (2H, d, J=8.64 Hz), 7.2-7.5 (4H, m). | | | | |

Preparation 12

(2S)-Ethoxy-3-{4-[2-(5-methyl-2-phenyl-oxazol-4-yl)-ethoxy]-phenyl}-propylamine (compound No 134)

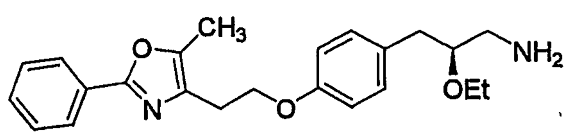

To a slurry of 10 % palladium on charcoal (450 mg) in ethyl acetate, a solution of (2S)-Ethoxy-3-{4-[2-(5-methyl-2-phenyl-oxazol-4-yl)-ethoxy]-phenyl}-propylazide (compound 116) (4.5 g) in ethyl acetate (15 mL) was added and the mixture was stirred in hydrogen atmosphere for 17 hours. Catalyst was filtered and the filtrate was evaporated under reduced pressure to yield 3.2 g of title compound.

Preparation 13

(2S)-Ethoxy-3-{4-[2-(5-methyl-2-phenyl-oxazol-4-yl)-ethoxy]-phenyl}-propyl amine (compound No 134).

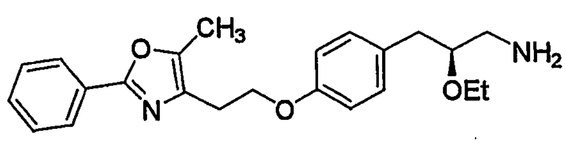

To a solution of N-tert-Butoxycarbonyl-(2S)-Ethoxy-3-{4-[2-(5-methyl-2-phenyl-oxazol-4-yl)-ethoxy]-phenyl}-propyl amine (compound 152) (500 mg) in dichloro methane (10 mL) was added trifluoroacetic acid (0.3 mL) and the

reaction mixture was stirred at ambient temperature for 16 hours. Reaction mixture was diluted with dichloromethane (25 mL) and washed with aqueous solution of sodium bicarbonate (50 mL). The organic extract was dried over calcium carbonate and evaporated under reduced pressure to yield 300 mg of title compound.

Preparation 14

(2S)-Ethoxy-3-(4-{2-[2-methyl-5-(5-methyl-thiophen-2-yl)-pyrrol-1-yl]-ethoxy}-phenyl)-propylamine (compound.No. 146).

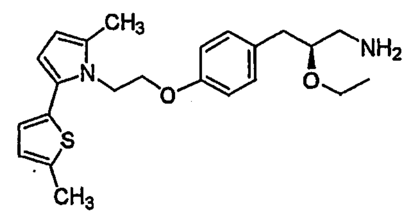

Lithium aluminium hydride (236 mg) was added to an ice cold solution of (2S)-Ethoxy-3-{4-[2-(2-methyl-5-(5-methyl-thiophene-2-yl)-pyrrol-1-yl)-ethoxy]-phenyl}-1-azidopropane
(2.4 g) in tetrahydrofuran (25 mL) in portions over a period of 15 minutes and the reaction mixture was stirred for further 3 hours at the same temperature. A saturated solution of sodium sulfate in water was added dropwise with care until crystalline white solid separated. Solids were filtered off and washed with hot ethyl acetate. Combined filtrate was dried over sodium sulfate and evaporated. Crude product was chromatographed over silicagel using 5 to 25 % ethyl acetate in petroleum ether to yield 1.9 g of title compound

Preparation 15

N-{(2S)-Ethoxy-3-[4-(5-methyl-2-phenyl-oxazol-4-ylmethoxy)-phenyl]-propyl}-methanesulfonamide. (Compound.No. 140)

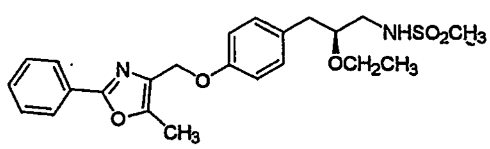

To a solution of (2S)-Ethoxy-3-[4-(5-methyl-2-phenyl-oxazol-4-ylmethoxy)-phenyl]-propylamine (200 mg) in dichloromethane (5 mL) was added triethyl amine (55 mg) and cooled to 10 °C. To this was added methanesulfonyl chloride (0.042 mL) dropwise and the reaction mixture was stirred at ambient temperature for 3 hours. Reaction mixture was diluted with dichloromethane (10 mL) and washed with water (10 mL). The organic layer was dried over sodium sulfate and evaporated under reduced pressure to yield crude 227 mg of product. Crude product was chromatographed over silicagel using 5 to 25 % ethyl acetate in petroleumether to yield 141 mg of title compound

In like manner compounds in the table 8 were prepared following the procedure described in preparations 12-15

Table 8:

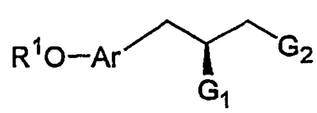

(continued)

| Ex.. No | $R^1$ | Ar | $G_1$ | $G_2$ | Mol. Wt | % Yield |
|---|---|---|---|---|---|---|
| 134. | 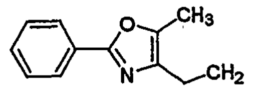 | 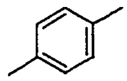 | OEt | $NH_2$ | 380 | 76 |
| | $^1H$: 1.1(3H, t, J=6.91 Hz), 2.3 (3H, s), 2.6-2.8 (4H, m), 2.9 (2H, t, J=6.69 Hz), 3.4-3.5 (3H, m), 4.2 (2H, t, J=6.69 Hz), 6.8 (2H, d, J=8.5 Hz), 7.0 (2H, d, J=8.4 Hz), 7.4 (3H, m), 7.9 (2H, m). | | | | | |
| 135. | 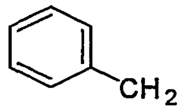 | 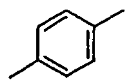 | OEt | $NH_2$ | 285 | 68 |
| | $^1H$: 1.2 (3H, t, J=13.7 Hz), 2.7 (1H, dd, J=13.7 & 6.9 Hz), 2.8 (1H, dd, J=14.2 & 5.8 Hz), 3.4 - 3.6 (5H, m), 5.0 (2H, s), 6.8 (2H, d, J=8.6 Hz), 7.1 (2H, d, J=8.6 Hz), 7.3- 7.4 (5H, m). | | | | | |
| 136. | 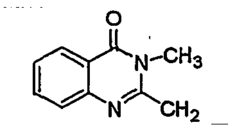 | 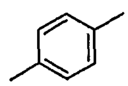 | OEt | $NH_2$ | 367 | 75 |
| | $^1H$: 1.1 (3H, t, J=7.0 Hz), 2.7 (3H, m), 3.4 - 3.5 (4H, m), 3.7 (3H, s), 5.1 (2H, s), 6.9 (2H, d, J=8.6 Hz), 7.1 (2H, d, J=8.6 Hz), 7.5 (1H, m), 7.7 (2H, m), 8.3 (1H, dd, J=8.0 & 0.8 Hz). | | | | | |
| 137. | 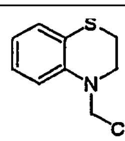 | 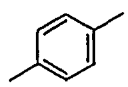 | OEt | $NH_2$ | 372 | 80 |
| | DMSO-$d_6$ $^1H$: 1.0 (3H, t, J=6.9 Hz), 2.6 - 2.8 (4H, complex), 3.0 (2H, m), 3.4-3.8 (7H, m), 4.1 (2H, t, J=5.5 Hz), 6.5 (1H, t, J=7.3 Hz), 6.7 (1H, d, J=8.2 Hz), 6.8 - 7.0 (4H, m), 7.1 (2H, d, J=8.4 Hz). | | | | | |
| 138. | 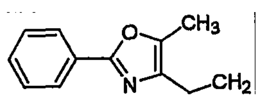 | 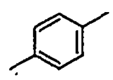 | OEt | 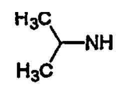 | 422 | 82 |
| | DMSO-$d_6$ $^1H$: 1.0 - 1.1 (9H, m), 2.3 (3H, s), 2.7 (3H, m), 2.9 (3H, m), 3.23 (1H, m), 3.4-3.6 (3H, complex), 4.1 (2H, t, J=6.0 Hz), 6.8 (2H, d, J=8.1 Hz), 7.1 (2H, d, J=8.1 Hz), 7.5 (3H, m), 7.8 (2H, m). | | | | | |
| 139. | 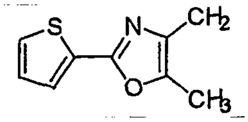 | 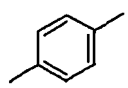 | OEt | $NH_2$ | 462 | 50 |
| | $^1H$: 1.06 (3H, t, J= 7.0 Hz), 2.4 (3H, s), 2.5-2.85 (4H, m), 3.35-3.51 (2H, m), 3.51-3.6(1H, m), 4.94 (2H, s), 6.97 (2H, d, J= 8.28 Hz), 7.14-7.2 (3H, m), 7.64(1H, d, J= 2.7 Hz), 7.7(1H, d, J=4.9 Hz). | | | | | |
| 140. | 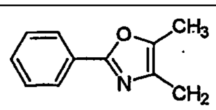 | 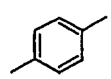 | OEt | $NHSO_2M$ | 444 | 58 |
| | $^1H$ : 1.18 (3H, t, J=6.99 Hz), 2.13 (3H, s), 2.6-2.8 (2H, m), 2.93 (3H, s), 2.9-3.1 (2H, m), 3.2-3.4 (1H, m), 3.4-3.6 (2H, m), 4.97 (2H, s), 6.95 (2H, d, J=8.64 Hz), 7.11 (2H, d, J=8.61 Hz), 7.41-7.46 (3H, m), 8.0-8.3 (2H, m) | | | | | |
| 141. | 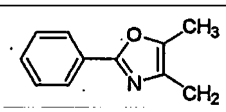 | 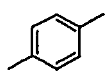 | OEt | I $NH_2$ | 366 | 49 |
| | $^1H$ : 1.2 (3H, t, J=6.9 Hz), 2.45 (3H, s), 2.68 (2H, d, J=5.3 Hz), 2.70-2.84 (2H, m), 3.53-3.62 (2H, m), 3.74-4.05 (1H, m), 5.0 (2H, s), 6.9 (2H, d, J=8.0 Hz), 7.1 (2H, d, J=8.58 Hz), 7.42-7.45 (3H, m), 7.99-8.03 (2H, m) | | | | | |

(continued)

| Ex.. No | $R^1$ | Ar | $G_1$ | $G_2$ | Mol. Wt | % Yield |
|---|---|---|---|---|---|---|
| 142. | 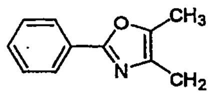 | 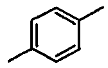 | OEt | NHEt | 394 | 100 |
| | $^1H$ : 0.85 (3H, t, J=7.29 Hz), 1.12 (3H, t, J=7.21 Hz), 2.43 (3H, s), 2.59-2.83 (6H, m), 3.47-3.65 (3H, m), 4.97 (2H, s), 6.93 (2H, d, J=8.61 Hz), 7.11 (2H, d, J=8.61 Hz), 7.42-8.03 (5H, m) | | | | | |
| 143. | 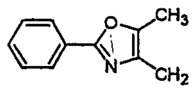 | 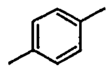 | OEt | 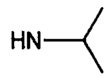 | 408 | 85 |
| | $^1H$: 1.09 (3H, t, J=6.8 Hz), 1.17 (6H, d, J=5.52 Hz), 2.77-2.92 (4H, m), 2.43 (3H, s), 3.25 (1H, t, J=6.16 Hz), 3.43-3.92 (2H, m), 4.96 (2H, s), 6.97 (2H, d, J=8.25 Hz), 7.17 (2H, d, J=8.19 Hz), 7.50-7.92 (5H, m) | | | | | |
| 144. | 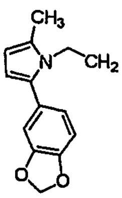 | 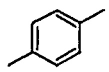 | OEt | $NH_2$ | 422 | 78 |
| | $^1H$ : 1.07 (3H, t, J=6.6 Hz), 2.27 (3H, s), 2.6-2.7 (4H, m), 3.44 (2H, q, J=7.4 &7.13 Hz), 3.45-3.49 (1H, m), 4.19-4.22 (4H, m), 5.80 (1H, d, J=3.08 Hz), 5.89 (1H, d, J=3.34 Hz), 6.03 (2H, s), 6.69 (2H, d, J=8.1 Hz), 6.8-6.9 (3H, m), 7.06 (2H, d, J=8.12 Hz) | | | | | |
| 145. | 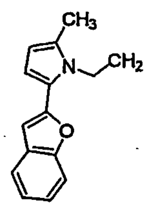 | 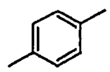 | OEt | $NH_2$ | 418 | 97 |
| | $^1H$ : 1.03 (3H, t, J=6.0 Hz), 2.34 (3H, s), 2.5-2.7 (4H, m), 3.39-3.57 (3H, m), 4.21 (2H, t, J=4.7 Hz ), 4.50 (2H, t, J=4.7 Hz) , 5.94 (1H, d, J=3.42 Hz), 6.52 (1H, d, J=3.5 Hz), 6.76 (2H, d, J=8.4 Hz), 6.9 (1H, s), 7.0 (2H, d, J=8.3 Hz), 7.21-7.23 (2H, m), 7.50-7.59 (2H, m) | | | | | |
| 146. | 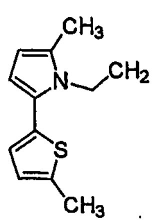 | 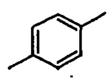 | OEt | $NH_2$ | 398 | 100 |
| | DMSO-$d_6$ H: 1.07 (3H, t, J=6.9 Hz), 2.26 (3H, s), 2.48 (3H, s), 2.62-2.77 (4H, m), 3.41-3.49 (3H, m), 4.05 (2H, t, J=5.6 Hz), 4.29 (2H, t, J=5.6 Hz), 5.81 (1H, d, J=2.9 Hz), 6.02 (1H, d, J=3.4 Hz), 6.75-6.78 (3H, m), 6.89 (1H, d, J=3.4 Hz), 7.1 (2H, d, J=8.5 Hz) | | | | | |
| 147. | 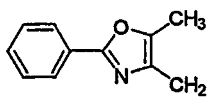 | 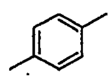 | OEt | $N(CH_3)_2$ | 394 | 20 |
| | $^1H$: 1.10-1.15 (3H, m), 2.23 (6H, s), 2.28-2.35 (2H, m), 2.43 (3H, s), 2.70-2.77 (2H, m), 3.40-3.55 (3H, m), 4.97 (2H, s), 6.93 (2H, d, J=8.28 Hz), 7.16 (2H, m), 7.42-7.44 (3H, m), 8.0-8.03 (3H, m) | | | | | |

(continued)

| Ex.. No | $R^1$ | Ar | $G_1$ | $G_2$ | Mol. Wt | % Yield |
|---|---|---|---|---|---|---|
| 148. | 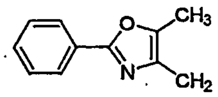 | 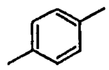 | OEt | NEtBoc | 494 | 64 |
| | $^1H$ : 0.8-1.2 (15H, m), 2.43 (3H, s), 2.65 (2H, m), 3.0 (1H, m), 3.28-3.37 (4H, m), 3.59 (2H, m), 4.97 (2H, s), 6.93 (2H, d, J=8.28 Hz), 7.16 (2H, m), 7.42-7.46 (3H, m), 8.0-8.03 (2H, m) | | | | | |

Preparation 16

N-((2S)-Ethoxy-3-{4-[2-(5-methyl-2-phenyl-oxazol-4-yl)-ethoxy]-phenyl}-propyl)-acetamide(compound No 151)

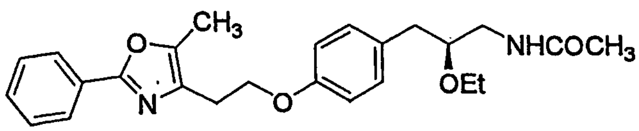

To a solution of (2S) Ethoxy-3-{4-[2-(5-methyl-2-phenyl-oxazol-4-yl)-ethoxy]-phenyl}-propylamine (compound No. 134) (100 mg) in dichloromethane (5 mL), triethylamine (53 mg) was added followed by acetic anhydride (40 mg) at 10 °C and stirred at the same temperature for 2 hours. The reaction mixture was poured in ice cold water and extracted with diethyl ether (3X50 mL). The combined organic extract was washed with water (50 mL), brine (50mL), dried over sodium sulfate and evaporated under reduced pressure to yield 70 mg of title compound.

Preparation 17

N-tertButoxy carbonyl-(2S)-ethoxy 3-(4-hydroxy-phenyl) propylamine. (compound No 150).

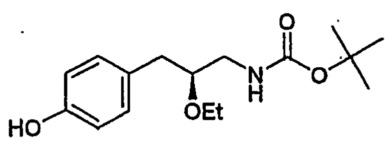

To a solution of 3-(4-Benzyloxy-phenyl)-N-tert butoxycarbonyl-(2S)-ethoxy-propylamine.(compound 149) (10.7 g) in methanol (100 mL) were added a slurry of 10 % palladium on charcoal (1.0 g) in methanol and ammonium formate (7.0 g) and the mixture was refluxed in nitrogen atmosphere for 2 hours. Catalyst was filtered and the filtrate was concentrated in vacuum. Water was added to the residue and extracted with ethyl acetate (3 X 100 mL). The combined extract was washed with water (100 mL), brine (100 mL), dried over sodium sulfate and evaporated under reduced pressure to yield 8.0 g of title compound.

Preparation 18

N-tert-Butoxycarbonyl-(2S)-Ethoxy-3-{4-[2-(5-methyl-2-phenyl-oxazol-4-yl)-ethoxy]-phenyl}-propyl amine (compound 152)

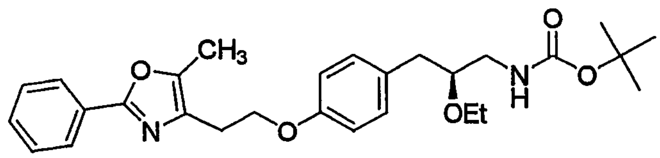

A mixture of 2-(5- methyl-2-phenyl-oxazol-4-yl)-ethyl methane sulfonate (1.0 g), N-tertbutoxy carbonyl-(2S)-ethoxy-3-(4-hydroxy-phenyl)-propylamine.(compound No 150) (1.0 g) and potassium carbonate (1.0 g) in dimethyl formamide (15 mL) was stirred at 75 °C for 16 hours. Reaction mixture was cooled to 25 °C, poured in to ice cold water and extracted with ethyl acetate (3 X 50 mL). The organic layer was washed with water (100 mL), brine (100 mL), dried over sodium sulfate and evaporated under reduced pressure. Crude product was chromatographed over silicagel using 7 % ethyl acetate in petroleum ether to yield 1.3 g of the title compound.

In like manner compounds in the table 9 were prepared following the procedure described in preparations 16-18 using suitable acylating agents.

Table 9:

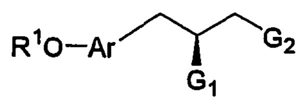

| EX. No | $R^1$ | Ar | $G_1$ | $G_2$ | Mol. Wt | % Yield |
|---|---|---|---|---|---|---|
| 149. | 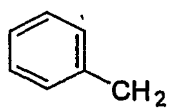 | 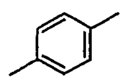 | OEt | NHBoc | 385 | 61 |
| | $^1$H: 1.1 (3H, t, J=6.9 Hz), 1.4 (9H, s), 2.6 (1H, dd, J=14.0 & 6.3 Hz), 2.7 (1H, dd, J=14.0 & 6.1 Hz), 3.0 (1H, m), 3.3 (1H, m), 3.4 - 3.5 (3H, m), 5.0 (2H, s), 6.8 (2H, d, J=8.6 Hz), 7.1 (2H, d, J=8.6 Hz), 7.3 - 7.4 (5H, m). | | | | | |
| 150. | H | 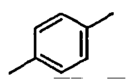 | OEt | NHBoc | 295 | 96 |
| | $^1$H: 1.1 (3H, t, J=6.4 Hz), 1.4 (9H, s), 2.6 (1H, dd, J=13.8 & 6.8 Hz), 2.7 (1H, dd, J=13. 8 & 6.7 Hz), 3.0 (1H, m), 3.3 (1H, m), 3.4 - 3.5 (3H, m), 6.7 (2H, d, J=8.3 Hz), 7.0 (2H, d, J=8.3 Hz). | | | | | |
| 151. | 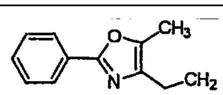 | 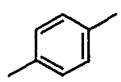 | OEt | $NHCOCH_3$ | 422 | 64 |
| | $^1$H: 1.1 (3H, t, J=7.0 Hz), 1.9 (3H, s), 2.3 (3H, s), 2.6 (2H, m), 2.9 (2H, t, J=6.7 Hz), 3.1 (1H, m), 3.5 (4H, m), 4.2 (2H, t, J=6.7 Hz), 6.8 (2H, d, J=-8.6 Hz), 7.0 (2H, d, J=8.5 Hz), 7.4 (3H, m), 8.0 (2H, dd, J=7.9 & 2.3 Hz). | | | | | |
| 152. | 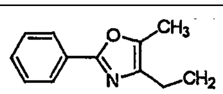 | 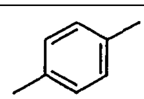 | OEt | NHBoc | 480 | 83 |
| | $^1$H: 1.1 (3H, t, J=7.0 Hz), 1.4 (9H, s), 2.37 (3H, s), 2.6 - 2.8 (2H, m), 2.95 (2H, t, J=6.7 Hz), 3.0 (1H, m), 3.3 (1H, m), 3.4-3.6 (3H, complex), 4.2 (2H, t, J=6.7 Hz), 6.8 (2H, d, J=8.6 Hz), 7.1 (2H, d, J=8.6 Hz), 7.4 (3H, m), 7.9 (2H, m). | | | | | |
| 153. | 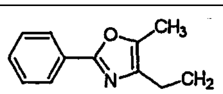 | 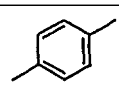 | OEt | NHCbz | 514 | 64 |
| | $^1$H: 1.1 (3H, t, J=7.0 Hz), 2.37 (3H, s), 2.7-2.8 (2H, m), 2.9 (2H, t, J=6.6 Hz), 3.1 (1H, m), 3.25 - 3.6 (4H, complex), 4.2 (2H, t, J=6.6 Hz), 5.0 (2H, s), 6.8 (2H, d, J=8.3 Hz), 7.0 (2H, d, J=8.3 Hz), 7.3- 7.5 (8H, m), 7.9 (2H, m). | | | | | |

(continued)

| EX. No | $R^1$ | Ar | $G_1$ | $G_2$ | Mol. Wt | % Yield |
|---|---|---|---|---|---|---|
| 154. | 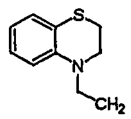 | 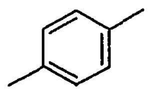 | OEt | NHBoc | 472 | 73 |
| | $^1H$: 1.1 (3H, t, J=7.0 Hz), 1.4 (9H, s), 2.6 (1H, dd, J=14.0 & 6.0 Hz), 2.75 (1H, dd, J=14.1 & 6.0 Hz), 3.0 (3H, m), 3.3 (1H, m), 3.4 -3.55 (3H, m), 3.72 (2H, t, J=5.8 Hz), 3.8 (2H, m), 4.1 (2H, t, J=5.8 Hz), 6.6 (1H, t, J=7.5 Hz), 6.7 (1H, d, J=8.0 Hz), 6.8 (2H, d, J=8.5 Hz), 6.95- 7.0 (2H, m), 7.1 (2H, d, J=8.5 Hz). | | | | | |
| 155. | 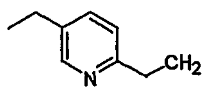 | 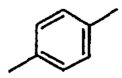 | OEt | NHBoc | 428 | 76 |
| | $^1H$: 1.13 (3H, t, J=6.99 Hz), 1.24 (3H, t, J=7.62 Hz), 1.43 (9H, s), 2.64 (2H, q , J=7.62 Hz), 2.72-3.3 (2H, m), 3.21 (2H, t, J=6.69 Hz), 3.44-3.50 (3H, m), 4.31 (2H, t, J=6.68 Hz), 4.81 (1H, broad-s), 6.82 (2H, d , J=8.61 Hz), 7.06 (2H, d , J=8.61 Hz), 7.18 (1H, d , J=7.89 Hz), 7.43-7.46 (1H, m), 8.38 (1H, d , J=2.01 Hz). | | | | | |
| 156. | 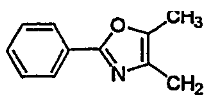 | 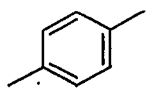 | OEt | NHBoc | 466 | 55 |
| | $^1H$ - 1.13 (3H, t, J=6.9 Hz), 1.4 (9H, s), 2.43 (3H, s), 2.6-2.8 (2H, m), 3.02-3.04 (1H, m), 3.2-3.5 (4H, m), 4.9 (2H, s), 6.93 (2H, d, J=8.6 Hz), 7.12 (2H, d, J=8.6 Hz), 7.42-7.47 (3H, m), 8.0-8.03 (2H, m). | | | | | |
| 157. | 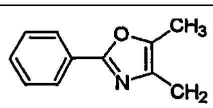 | 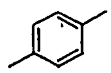 | OEt | I $NHCOCF_3$ | 462 | 83 |
| | $^1H$ : 1.18 (3H, t, J=6.99 Hz), 2.43 (3H, s), 2.6-2.9 (2H, m), 3.15 (1H, m), 3.4-3.6 (4H, m), 4.97 (2H , s), 6.95 (2H, d, J=8.64 Hz), 7,10 (2H, d, J=8.61 Hz), 7.41-7.46(3H, m), 8.0-8.3 (2H, m) | | | | | |
| 158. | 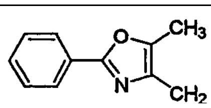 | 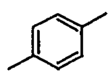 | OEt | NHCOOEt, | 438 | 55 |
| | $^1H$ : 1.14 (3H, t, J=6.99 Hz), 1.23 (3H, t, J=6.63 Hz), 2.43 (3H, s), 2.68-2.77 (2H, dd, J=6.39&6.15 Hz), 3.42-3.45 (1H, m), 3.46-3.52 (4H, m), 4.07-4.15 (2H, m), 4.97 (2H, s), 6.94 (2H, d, J=8.63 Hz), 7.12 (2H, d, J=8.58 Hz), 7.42-8.03 (5H, m) | | | | | |
| 159. | 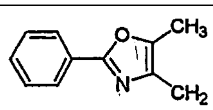 | 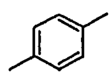 | OEt | I NHCbz | 500 | 42 |
| | $^1H$ : 1.12 (3H, t, J=6.99 Hz), 2.43 (3H, s), 2.67-2.77 (2H, dd, J=6.39&6.06 Hz), 2.8-3.3 (1H , m), 3.40-3.53 (4H, m), 4.96 (2H, s), 5.10 (2H, s), 6.93 (2H,d, J=8.46 Hz), 7.12 (2H, d, J=8.4 Hz), 7.26-8.03 (10H, m), | | | | | |
| 160. | 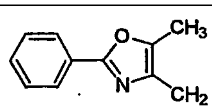 | 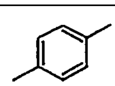 | OEt | $NHCOCH_3$ | 408 | 37 |
| | $^1H$ : 1.2 (3H, t, J=6.9 Hz), 2.1 (3H, s), 2.43 (3H, s), 2.69-2.77 (2H, m), 2.78-3.44 (1H, m), 3.45-3.53 (4H, m), 4.97 (2H, s), 5.73 (1H, s), 6.93 (2H, d, J=8.61 Hz), 7.12 (2H, d, J=8.58 Hz), 7.42-7.46 (3H, m), 8.00-8.03 (2H, m) | | | | | |

Preparation 19

(2S)-Ethoxy-1-ethylsulfanyl-3-{4-[2-(5-methyl-2-phenyl-oxazol-4-yl)-ethoxy]-phenyl}-propane.(compound No 161)

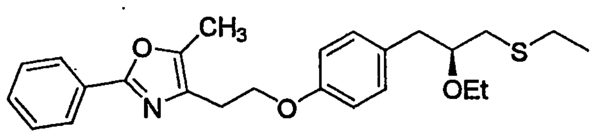

To a stirred mixture of sodium metal (150 mg) and ethanethiol (0.49 mL) in tetrahydro furan (10 mL) was added a solution of (2S)-Ethoxy-3-{4-[2-(5-methyl-2-phenyl-oxazol-4-yl)-ethoxy]-phenyl}-propyl- methanesulfonate (compound No 91) (0.6 g), in 5 mL of tetrahydrofuran dropwise over a period of 10 minutes and the reaction mixture was stirred at ambient temperature for 15 hours. Reaction mixture was poured in to ice cold water and extracted with ethyl acetate (3 X 50 mL). The organic layer was washed with water (100 mL), brine (100 mL), dried over sodium sulfate and evaporated under reduced pressure. Crude product was chromatographed over silica gel using 10-15 % ethyl acetate in petroleum ether as eluent to yield 420 mg of the title compound.

Preparation 20

(2S)-Ethoxy-1-ethyl sulfonyl-3-{4-[2-(5-methyl-2-phenyl-oxazol-4-yl)-ethoxy]-phenyl}-propane. (compound No 162)

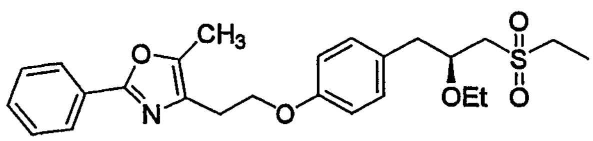

To an ice cold solution of (2S)-Ethoxy-1-ethyl sulfanyl-3-{4-[2-(5-methyl-2-phenyl-oxazol-4-yl)-ethoxy]-phenyl}-propane.(compound No 161) (250 mg) in acetone (10 mL), oxone (900 mg) was added and the reaction mixture was stirred at the same temperature for 2 hours. Solvent was evaporated under reduced pressure, residue was added to water and extracted with ethyl acetate (3 X 50 mL). The organic layer was washed with water (50 mL), brine (50 mL), dried over sodium sulfate and evaporated under reduced pressure. Crude product was chromatographed over silicagel using 15 % ethyl acetate in petroleum ether as eluent to yield 85 mg of title compound.

Preparation 21

(3S)-Ethoxy-4-[4-(5-methyl-2-phenyl-oxazol-4-ylmethoxy)-phenyl]-butyronitrile. (Compound.No. 165)

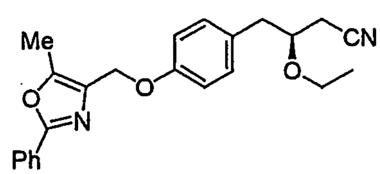

NaCN (0.247 g) was added to a solution of (2S)-Ethoxy-3-{4-(5-methyl-2-phenyl-oxazol-4-ylmethoxy]-phenyl}-propyl-methane sulfonate (1.5 g) in DMF(7.5 mL) at 20-30 ˚C. The reaction mixture was stirred at 85-90 ˚C for 18 hours. Reaction mixture was poured in to water (20 mL) and product was extracted with ethyl acetate (2 x 20 mL). Combined extract was washed with water (2 x 40 mL), brine (40 mL) dried over sodium sulfate and evaporated under reduced pressure to yield 1.2 g of title compound.

Preparation 22

(2S)-Ethoxy-1H-tetrazole-3-[4-(5-methyl-2-phenyl-oxazol-4-ylmethoxy)-phenyl]-propane. (Compound. No. 166)

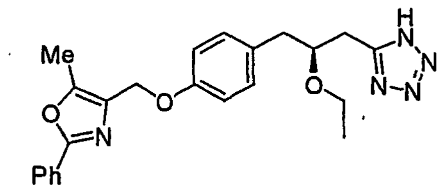

$(Bu)_3SnN_3$ (1.27 g) was added to (3S)-Ethoxy-4-[4-(5-methyl-2-phenyl-oxazol-4-ylmethoxy)-phenyl]-butyronitrile (Compound.No.165) (1.2 g) in xylene (15 mL) at 20-30 °C. The reaction was stirred at reflux temp. for 18 h. The reaction was cooled to 20-30 °C. The reaction mixture was diluted with ethyl acetate (25 mL), washed with 10 % HCl (20 mL), water (3 x 25 mL), brine (25 mL), organic layer was dried over sodium sulfate and evaporated under reduced pressure to yield the crude title compound (1.1g). Crude product was chromatographed over silicagel using pet.ether: ethyl acetate (9:1) as an eluent to afford pure product 700 mg in 52 % yield.

In like manner compounds in the table 10 were prepared following the procedure described in preparations 19-22 using suitable acylating agents.

Table 10:

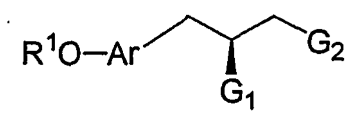

| Ex. No | $R^1$ | Ar | $G_1$ | $G_2$ | Mol.Wt | % Yield |
|---|---|---|---|---|---|---|
| 161. | 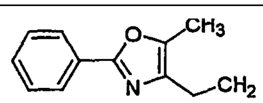 | 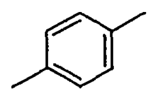 | OEt | SEt | 425 | 76 |
| | $^1H$: 1.13 (3H, t, J=6.9 Hz), 1.2 (3H, t, J=7.45 Hz), 2.37 (3H, s), 2.58 (4H, m), 2.8 (2H, t, J=7.12 Hz), 2.97 (2H, t, J=6.69 Hz), 3.41 (1H, m), 3.53 (2H, dd, J=6.6 & 2.72 Hz), 4.22 (2H, t, J=6.57 Hz), 6.81 (2H, d J=8.28 Hz), 7.11 (2H, d, J=8.46 Hz), 7.43 (3H, m), 7.97 (2H, dd, J=6.67 & 2.28 Hz). | | | | | |
| 162 | 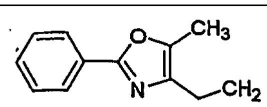 | 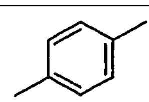 | OEt | $S(O)_2Et$ | 457 | 32 |
| | $^1H$: 1.21 (3H, t, J=7.0 Hz), 1.33 (3H, t, J=7.47 Hz), 2.39 (3H, s), 2.7 (1H, dd, J=13.9 & 7.15 Hz), 2.88 (7H, complex), 3.6 (2H, m), 4.0 (1H, m), 4.23 (2H, t, J=6.48 Hz), 6.83 (2H, d, J=8.58 Hz), 7.0 (2H, d, J=8.5 Hz), 7.44 (3H, m), 8.0 (2H; dd, J=6.0 & 2.77 Hz). | | | | | |
| 163. | 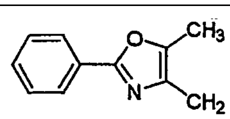 | 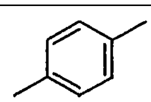 | OEt | SEt | 411 | 89 |
| | $^1H$: 1.13 (3H, t, J=6.9 Hz), 1.23 (3H, t, J=7.2 Hz), 2.4 (3H, s), 2.53-2.62 (4H, m), 2.80-2.85 (2H, m), 3.4-3.5 (1H, m), 3.5-3.6 (2H, m), 4.97 (2H, s), 6.9 (2H, d, J=8.6 Hz), 7.14 (2H, d, J=8.6 Hz), 7.42-7.46 (3H, m), 7.99-8.03 (2H, m) | | | | | |
| 164. | 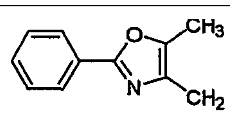 | 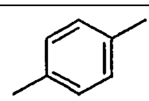 | OEt | SOEt | 427 | 98 |
| | $^1H$: 1.17 (3H, m), 1.2 (3H, m), 2.56 (3H, s), 2.73-2.83 (6H, m), 3.5 (2H, m), 4.1 (1H, m), 5.2 (2H, s), 6.9 (2H, d, J=8.0 Hz), 7.15 (2H, m), 7.54-7.64 (3H, m), 8.3 (2H, d, J=7.7 Hz) | | | | | |

(continued)

| Ex. No | $R^1$ | Ar | $G_1$ | $G_2$ | Mol.Wt | % Yield |
|---|---|---|---|---|---|---|
| 165. | 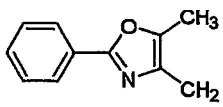 | 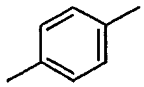 | OEt | CN | 376 | 95 |
| | $^1H$: 1.19 (3% t, J=6.9 Hz), 2.40-2.46 (5H, m, 2.80 (1H, d, J=6.72 Hz), 2.91 (1H, d, J=6.06 Hz), 3.51-3.69 (2H, m), 3.69-3.731 (1H, m), 4.97 (2H, s), 6.94-6.99 (2H, m), 7.14 (2H, d, J=8.58.Hz), 7.41-7.47 (3H, m), 8.00-8.03 (2H, m) | | | | | |
| 166. | 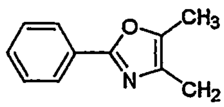 | 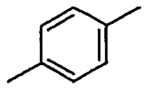 | OEt | 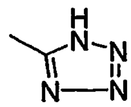 | 419 | 97 |
| | $^1H$: 1.22-1.56 (3H, m), 2.45 (3H, s), 3.02-3.04 (3H, m), 3.17 (1H, d, J=3.75 Hz), 3.50 (1H, d, J=6.99 Hz), 3.71 (2H, m), 4.98 (2H, s), 6.96 (2H, d, J=8.67 Hz), 7.07 (2H, d, J=8.64 Hz), 7.42 -7.45 (3H, m), 8.0-8.03 (2H, m) | | | | | |

Preparation 23

Bisulphate salt of 2S)-Ethoxy-3-{4-[2-(5-methyl-2-phenyl-oxazol-4-yl)-ethoxy]-phenyl}-propyl amine (compound.No.167)

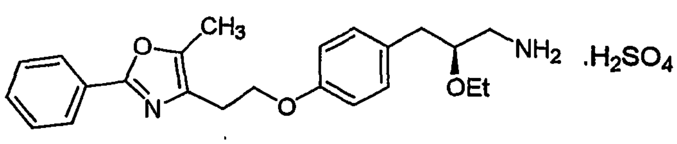

To (2S)-Ethoxy-3-{4-[2-(5-methyl-2-phenyl-oxazol-4-yl)-ethoxy]-phenyl}-propyl amine. (compound.No.134) (300 mg), a chilled solution of acetone (3 mL) containing sulfuric acid (77 mg) was added and stirred at 0 ˚C for 30 minutes. Solvent was evaporated under a flow of nitrogen and the residue was stirred with diisopropyl ether to afford product (138 mg).

Preparation 24

Oxalic acid salt of (2S)-Ethoxy-3-{4-[5-methyl-2-phenyl-oxazol-4-yl methoxy]-phenyl}-propylamine (compound.No. 173)

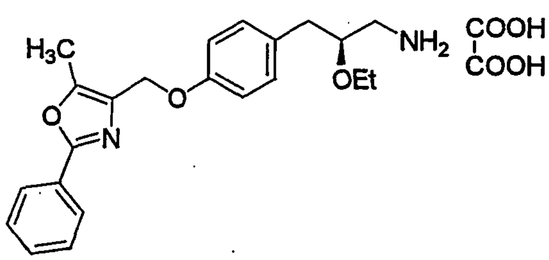

To a solution of (2S)-Ethoxy-3-{4-[5-methyl-2-phenyl-oxazol-4-yl methoxy]-phenyl}-propylamine (compound No 141) (200 mg), in isopropyl alcohol (5 mL), oxalic acid dihydrate (64 mg) was added and stirred at 28 ˚C for 30 minutes. Solid separated was filtered and dried to afford the title compound (140 mg).
In like manner compounds in the table 11 were prepared following the procedure described for the preparation of 23-24.

Table 11:

| Compound No. | Free-base No. | Salt prepared | Melting Point (˚C)* |
|---|---|---|---|
| 167. | 134 | $H_2SO_4$ | 175 |
| 168. | 137 | Oxalic acid | 115 |
| 169. | 138 | Oxalic acid | 122 |
| 170. | 136 | Oxalic acid | 193 |
| 171. | 124 | Oxalic acid | 150-160 |
| 172. | 71 | Oxalic acid | 190 |
| 173. | 141 | Oxalic acid | 135 |
| 174. | 141 | $H_2SO_4$ | 90 |
| 175. | 146 | Oxalic acid | 117 |
| 176. | 143 | Oxalic acid | 134 |
| 177. | 144 | Oxalic acid | 111 |
| 178. | 145 | Oxalic acid | 126 |
| * The melting points were uncorrected and may vary in the range of $\pm$ 4˚C. | | | |

The compounds of the present invention lowered triglyceride, total cholesterol, LDL, VLDL and increased HDL and lowered serum glucose levels. This was demonstrated by *in vivo* animal experiments.

A) Demonstration of *in vivo* efficacy of compounds:

i) Serum triglyceride and total cholesterol lowering activity in Swiss albino mice:

Male Swiss albino mice (SAM) were bred in Zydus animal house. All these animals were maintained under 12 hour light and dark cycle at 25$\pm$1 ˚C. Animals were given standard laboratory chow (NIN, Hyderabad, India) and water ad libitum. SAM of 20-30 g body weight range were used.
The test compounds were administered orally to Swiss albino mice at 0.001 to 50 mg / kg/ day dose for 6 days. The compound was administered after suspending it in 0.25 % CMC or dissolving it in water, when compound is water-soluble. Control mice were treated with vehicle (0.25% of Carboxymethylcellulose; dose 10 ml/kg).
The blood samples were collected on 0$^{th}$ day and in fed state 1 hour after drug administration on 6$^{th}$ day of the treatment. The blood was collected in non heparinised capillary and the serum was analyzed for triglyceride and total cholesterol (Wieland, O. Methods of Enzymatic analysis. Bergermeyer, H., O., Ed., 1963. 211-214; Trinder, P. Ann. Clin. Biochem. 1969. 6: 24-27). Measurement of serum triglyceride and total cholesterol was done using commercial kits (Zydus-Cadila, Pathline, Ahmedabad, India).

Formula for calculation:

Percentage reduction in triglycerides/total cholesterol were calculated according to the formula :

$$\text{Percentage reduction (\%)} = 1 - \left[\frac{TT/OT}{TC/OC}\right] X\ 100$$

OC = Zero day control group value OT = Zero day treated group value
TC = Test day control group TT = Test day treated group

Table 1:

| Triglyceride lowering activity in Swiss albino mice: | | |
|---|---|---|
| Example No. | Dose (mg/kg/day) | % Triglyceride lowering |
| 110 | 3 | 78 |
| 134 | 3 | 54 |
| 130 | 3 | 51 |
| 80 | 3 | 50 |
| 28 | 3 | 78 |

ii) Cholesterol lowering activity in hypercholesterolemic rat models

Male Sprague Dawley rats stock bred in Zydus animal house were maintained under 12 hour light and dark cycle at 25±1 °C. Rats of 100-150 g body weight range were used for the experiment. Animals were made hypercholesterolemic by feeding 1 % cholesterol and 0.5 % sodium cholate mixed with standard laboratory chow (NIN, Hyderabad, India) and water ad libitum for 5 days. The animals were maintained on the same diet throughout the experiment [Petit D., Bonnefis M. T., Rey C and Infante R., Effects of ciprofibrate on liver lipids and lipoprotein synthesis in normal and hyperlipidemic rats, Atherosclerosis, 74, 215-225(1988)].

The test compounds were administered orally at a dose 0.03 to 50 mg/ kg/ day for 4 days, after suspending it in 0.25 % CMC or dissolving it in water when compound is water-soluble. Control group was treated with vehicle alone (0.25% of Carboxymethylcellulose; dose 10 ml/kg).

The blood samples were collected in fed state on $0^{th}$ and 1 hour after drug administration on $6^{th}$ day of the treatment. The blood was collected from the retro-orbital sinus through non-heparinised capillary and the serum samples were analyzed for triglyceride and total cholesterol using commercial kits (Zydus-Cadila, Pathline, Ahmedabad, India). LDL and HDL by commercial kits (Point Scientific, USA). LDL and VLDL cholesterol were calculated from the data obtained for total cholesterol, HDL and triglyceride.

The reduction in VLDL cholesterol is calculated according to the formula. VLDL cholesterol in mg/dl = Total cholesterol - HDL cholesterol - LDL cholesterol

Table 2:

| Example No. | Dose (mg/kg/day) | Total cholesterol reduction (%) |
|---|---|---|
| 141 | 3 | 61 |
| 90 | 3 | 56 |
| 27 | 3 | 44 |

iii) Serum glucose lowering activity in db/db mice models

Homozygous animal $C_{57}$BL/KsJ-db/db mice are obese, hyperglycemic, hyperinsulinemic and insulin resistant (J. Clin. Invest., 85, 962-967, 1990), whereas heterozygous are lean and normoglycemic. The homozygous animals very closely mimic the human type II diabetes when blood sugar levels are not sufficiently controlled. Since this type of model resembles human type II diabetes mellitus, the compounds of the invention were tested for their antidiabetic activity in this model.

The compounds of the present invention showed serum glucose and triglycerides lowering activities.
Male $C_{57}$ BL/KsJ-db/db mice of 8 to 14 weeks age, having body weight range of 40 to 60 grams, procured from the Jackson Laboratory, USA, were used in the experiment.

Test compounds were suspended on 0.25% carboxymethyl cellulose or dissolved in water when the compound is water soluble and administered to test group containing 6 animals at a dose of 0.001 mg to 50 mg/kg through oral gavage daily for 6 days. The control group received vehicle (dose 10 ml/kg). On the $6^{th}$ day, one hour after the drug dosing, blood was collected from retro-orbital sinus and the serum was analyzed for glucose and triglycerides were measured using commercial kits (Zydus-Cadila, Pathline, Ahmedabad, India). The serum glucose and triglyceride lowering activities of the test compound was calculated according of the formula:

Serum glucose lowering activity (%) =

$$1 - \left[ \frac{TT/OT}{TC/OC} \right] X\ 100$$

OC = Zero day control group value OT = Zero day treated group value
TC = Test day control group TT = Test day treated group

| Example No. | Dose (mg/kg/day) | Serum Glucose reduction (%) | Plasma TG reduction (%) |
|---|---|---|---|
| 84 | 3 | 47 | 47 |
| 64 | 3 | 56 | 74 |
| 44 | 3 | 61 | 44 |

No adverse effects were observed for any of the mentioned compounds of invention. The compounds of the present invention showed good serum glucose, lipid and cholesterol lowering activity in the experimental animals used. These compounds are useful for the testing / prophylaxis of diseases caused by hyperlipidemia, hypercholesterolemia, hyperinsulinemia, hyperglycemia such as NIDDM, cardiovascular diseases, stroke, hypertension, obesity since such diseases are interlinked to each other.

## Claims

1. A compound of the general formula (I),

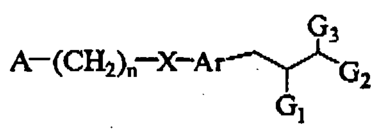

(I)

its tautomeric forms, its stereoisomers, its pharmaceutically acceptable salts, its pharmaceutically acceptable solvates, wherein
'A' represents a substituted or unsubstituted group selected from heteroaryl, heterocyclyl groups; 'n' is an integer from 1-3; 'X' represents oxygen; 'Ar' represents a substituted or unsubstituted phenyl group;
$G_1$ represents $OR_1$, $SR_1$, $S(O)R_3$, $S(Q)_2R_3$, $N_3$, CN, COOH, tetrazolyl groups; $G_2$ represents $OR_1$, $NR_1R_2$, $SR_1$, S (O)$R_3$, $S(O)_2R_3$, $N_3$, CN, COOH, tetrazolyl groups; $R_1$, $R_2$ represent hydrogen, substituted or unsubstituted groups selected from linear or branched ($C_1$-$C_8$)alkyl, acyl, alkoxycarbonyl, aryloxycarbonyl, aralkyloxycarbonyl groups; $R_3$ represents substituted or unsubstituted groups selected from alkyl, aryl groups; with the proviso that, when $G_2$ represents $NR_1R_2$, $G_1$ does not represent -OH group;
$G_3$ represents hydrogen or ($C_1$-$C_8$)alkyl groups.

2. A compound according to claim 1, **characterised in that** the substituents on 'A', $R_1$, $R_2$, and $R_3$ may be the same or different and are independently selected from hydroxyl, oxo, halo, thio, nitro, amino, cyano, formyl, or substituted or unsubstituted groups selected from alkyl, haloalkyl, perhaloalkyl, alkoxy, aryl, aryloxy, aralkyl, aralkoxy, heterocylyl, heteroaryl, acryl, acyloxy, acylamino, monosubstituted or disubstituted amino, carbonylamino, hydroxyalkyl, alkylthio, thioalkyl groups.

3. A compound according to claim 1, **characterised in that** suitable substituents on any substituent of 'A' may be same or different and are independently selected from hydroxyl, oxo, halo, thio, nitro, amino, cyano, formyl, or substituted or unsubstituted groups selected from amidino, guanidino, hydrazino, alkyl, haloalkyl, perhaloalkyl,

alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulphonylamino, alkylsulphonyloxy, alkoxycarbonylamino, aryloxycarbonylamino, aralkyloxycarbonylamino, aminocarbonylamino, alkylaminocarbonylamino, alkoxyamino, hydroxyl amino, sulphonyl derivatives, sulphonyl derivatives, sulphonic acid and its derivatives, phosphonic acid and its derivatives.

4. A compound according to claim 1, **characterised in that** the substituents on the group represented by 'Ar' represents substituted or unsubstituted linear or branched alkyl, alkoxy, thioalkyl, halogen, haloalkyl, haloalkoxy, acyl, amino, acylamino, thio or carboxylic or sulphonic acids and their derivatives, phosphonic acid and their derivatives.

5. Compounds according to claim 1, selected from
3-(6-Benzyloxy-naphthalen-2-yl)-2-ethoxy-propan-1-ol;
(2S)-Ethoxy-3-{4-[2-(5-methyl-2-phenyl-oxazol-4-yl)-ethoxy]-phenyl}-propan-1-ol;
(2S)-Ethoxy-3-{4-(4-hydroxy-3-methyl-3,4-dihydro-quinazolin-2yl-methoxy)-phenyl}-propan-1-ol;
2-Hydroxy-3-{4-[2-(5-methyl-2-phenyl-oxazol-4-yl)-ethoxy]-phenyl}-propan-1-ol;
3-{4-[2-(2,3-Dihydro-benzo[1,4]thiazin-4-yl)-ethoxy]-phenyl}-(2S)-ethoxy-propan-1-ol;
(2S)-Ethoxy-3-[4-(2-(phenoxazin-10-yl)-ethoxy)-phenyl]-propan-1-ol;
3-[4-(2-(Carbazol-9-yl)-ethoxy)-phenyl]-(2S)-ethoxy-propan-1-ol;
3-{4-[2-(3,4-Dihydro-2H-quinolin-1-yl)-ethoxy]-phenyl}-(2S)-ethoxy-propan-1-ol;
(2S)-Ethoxy-3-[4-(2-(indol-1-yl)-ethoxy)-phenyl]-propan-1-ol;
(2S)-Ethoxy-3-[4-(2-(phenothiazin-10-yl)-ethoxy)-phenyl]-propan-1-ol;
3-{4-[2-(2,3-Dihydro-benzo[1,4]oxazin-4-yl}-ethoxy]-phenyl}-(2S)-ethoxy-propan-1-ol;
3-{4-[2-(5-Methyl-2-phenyl-oxazol-4-yl)-ethoxy]-phenyl}-2-phenylsulfanyl-propan-1-ol;
(2S)-Ethoxy-3-{4-[2-(methyl-pyridin-2-yl-amino)-ethoxy]-phenyl}-propan-1-ol and its pharmaceutically acceptable salts;
(2S)-Ethoxy-3-{4-[2-(5-ethyl-pyridin-2-yl)-ethoxy]-phenyl}-propan-1-ol and its pharmaceutically acceptable salts;
(2S)-Ethoxy-3-[4-(5-methyl-2-thiophen-2-yl-oxazol-4-ylmethoxy)-phenyl]-propan-1-ol;
(2S)-Ethoxy-3-{4-[2-(5-methyl-2-thiophen-2-yl-oxazol-4-yl)-ethoxy]-phenyl}-propan-1-ol;
(2S)-Ethoxy-3-(4-{2-[2-methyl-5-(4-methylsulphanyl-phenyl)-pyrrol-1-yl]-ethoxy}-phenyl)-propan-1-ol;
(3S)-Ethoxy-4-{4-[2-(5-methyl-2-phenyl-oxazol-4-yl)-ethoxy]-phenyl}-butan-1-ol;
(2S)-Ethoxy-3-(4-{2-[5-methyl-2-(4-methylsulfanyl-phenyl)-oxazol-4-yl]-ethoxy}-phenyl)-propan-1-ol;
(2S)-Amino-3-{4-[2-(5-methyl-2-phenyl-oxazol-4-yl)-ethoxy]-phenyl}-propan-1-ol and its pharmaceutically acceptable salts;
(2S)-tert-butoxycarbonylamino-3-[4-(5-methyl-2-phenyl-oxazol-4-yl-methoxy)-phenyl]- propan-1-ol:
(2S)-tert-butoxycarbonylamino-3-{4-[2-(5-methyl-2-phenyl-oxazol-4-yl)-ethoxy]-phenyl}-propan-1-ol;
(2S)-Ethoxy-3-(4-{2-[2-methyl-5-(benzofuran-2-yl)-pyrrol-1-yl]-ethoxy}-phenyl)-propan-1-ol;
(2S)-Ethoxy-3-{4-{2-[2-methyl-5-(benzo[1,3]dioxol-5-yl)-pyrrol-1-yl]-ethoxy}-phenyl)-propan-1-ol;
(2S)-Ethoxy-3-[4-(1-methyl-1H-benzoimidazol-2-yl methoxy)-phenyl]-propan-1-ol;
(2S)-Ethoxy-3-[4-(5-methyl-3-phenyl-isoxazol-4-ylmethoxy)-phenyl]-propan-1-ol;
(2S)-Ethoxy-3-{4-[2-(5-ethyl-pyridin-2-yl)-2-hydroxy-ethoxy]-phenyl}-propan-1-ol;
(2S)-Ethoxy-3-[4-(2-benzoimidazol-1-yl-ethoxy)-phenyl]-propan-1-ol;
(2S)-Ethoxy-3-[4-(5-methyl-2-phenyl-oxazol-4-ylmethoxy)-phenyl]-propan-1-ol;
(2S)-Ethoxy-3-(4-{2-[2-methyl-5-(5-methyl-thiophen-2-yl)-pyrrol-1-yl]-ethoxy}-phenyl)-propan-1-ol;
(2S)-Ethoxy-3-[4-(5-methyl-2-phenyl-oxazol-4-ylmethoxy)-phenyl]-propan-1,2-diol;
1-Ethoxy-(2S)-ethoxy-3-[4-{2-(5-methyl-2-phenyl-oxazol-4-yl)-ethoxy}-phenyl]-propane;
2-((2S)-Ethoxy-3-{4-[2-(5-methyl-2-phenyl-oxazol-4-yl)-ethoxy]-phenyl}-propoxy)-ethanol;
2-((2S)-Ethoxy-3-{4-[2-(5-methyl-2-phenyl-oxazol-4-yl)-ethoxy]-phenyl}-propoxy)-benzoic acid and its pharmaceutically acceptable salts;
(2S)-Ethoxy-3-{4-[2-(5-methyl-2-phenyl-oxazol-4-yl)-ethoxy]-phenyl}-propyl bromo acetate;
1-Ethoxy-(2S)-ethoxy-3-[4-{2-(-3,4-Dihydro-2H-benzo[1,4]thiazin-1yl)ethoxy}phenyl]- propane;
1-Propoxy-(2S)-ethoxy-3-[4-{2-(5-methyl-phenyl-oxazol-4-yl)ethoxy}-phenyl]- propane;
2-((2S)-Ethoxy-3-[4-(5-methyl-2-phenyl-oxazol-4-ylmethoxy)-phenyl]-propoxy)-benzoic acid and its pharmaceutically acceptable salts;
1-Ethoxy-(2S)-ethoxy-3-[4-(5-methyl-2-phenyl-oxazol-4-ylmethoxy)-phenyl]- propane;
(2S)-Ethoxy-3-[4-(5-methyl-2-phenyl-oxazol-4-ylmethoxy)-phenyl]-1-phenoxy propane;

(2S)-Ethoxy-1-ethyl sulfinyl-3-{4-(5-methyl-2-phenyl-oxazol-4-ylmethoxy)-phenyl}-propane;
(2S)-Ethoxy-1-ethyl sulfanyl-3-{4-(5-methyl-2-phenyl-oxazol-4-ylmethoxy)-phenyl}-propane;
(2S)-Ethoxy-3-[4-(5-methyl-2-phenyl-oxazol-4-ylmethoxy)-phenyl]-1-isopropoxy propane;
(3S)-Ethoxy-4-[4-(5-methyl-2-phenyl-oxazol-4-ylmethoxy)-phenyl]-butyronitrile;
(2S)-Ethoxy-1H-tetrazole-3-[4-(5-methyl-2-phenyl-oxazol-4-ylmethoxy)-phenyl]-propane;
2-Ethoxy-1-[4-(5-methyl-2-phenyl-oxazol-4-ylmethoxy)-phenyl]-pentane-3-ol;
2,3-Diethoxy-1-[4-(5-methyl-2-phenyl-oxazol-4-ylmethoxy)-phenyl]-pentane;
2-Ethoxy-1-{4-[2-(5-methyl-2-phenyl-oxazol-4-yl)-ethoxy]-phenyl}-pentane-3-ol;
2,3-Diethoxy-1-{4-[2-(5-methyl-2-phenyl-oxazol-4-yl)-ethoxy]-phenyl}-pentane;
((2S)-Ethoxy-3-{4-[2-(5-methyl-2-phenyl-oxazol-4-yl)-ethoxy]-phenyl}-propoxy)-acetic acid and its pharmaceutically acceptable salts;
3-(4-Benzyloxy-phenyl)-(2S)-ethoxy-propyl-methanesulphonate;
(2S)-ethoxy-3-[4-(5-methyl-2-phenyl-oxazol-4-ylmethoxy)-phenyl]-propyl methane sulphonate;
(2S)-Ethoxy-3-{4-[2-(2-methyl-5-(5-methyl-thiophen-2-yl)-pyrrol-1-yl)-ethoxy]-phenyl}-propyl-methane sulphonate;
(2S)-Ethoxy-3-{4-[2-(5-methyl-2-phenyl-oxazol-4-yl)-ethoxy]-phenyl}-propyl-methane sulphonate;
(2S)-ethoxy-3-[4-(5-methyl-2-thiophen-2-yl-oxazol-4-ylmethoxy)-phenyl]}-propyl methanesulphonate;
(2S)-ethoxy-3-{4-[2-(5-ethyl-pyridin-2-yl)-ethoxy]-phenyl}-propyl methanesulphonate;
(2S)-Ethoxy-1-methoxy-3-{4-[2-(5-methyl-2-thiophen-2-yl-oxazol-4-yl)-ethoxy]-phenyl}-propane;
1-Ethoxy-(2S)-ethoxy-3-(4-{2-[5-methyl-2-(4-methylsulfanyl-phenyl)-oxazol-4-yl]-ethoxy}-phenyl)-propane;
1-Ethoxy-(2S)-ethoxy-3-{4-[2-(5-methyl-2-thiophen-2-yl-oxazol-4-yl)-ethoxy]-phenyl}-propane;
(2S)-Ethoxy-3-[4-(5-methyl-2-thiophen-2-yl-oxazol-4-ylmethoxy)-phenyl]-1-ethoxy propane;
(2S)-Ethoxy-3-{4-[2-(2-methyl-5-benzofuran-2-yl-pyrrol-1-yl)-ethoxy]-phenyl}-propyl-methanesulphonate;
(2S)-Ethoxy-3-{4-[2-(2-methyl-5-benzo[1,3]dioxol-5-yl-pyrrol-1-yl)-ethoxy]-phenyl}-propyl-methanesulphonate;
(2S)-Ethoxy-3-{4-[2-(2-methyl-5-benzofuran-2-yl-pyrrol-1-yl)-ethoxy]-phenyl}-propyl-(4-methyl phenyl)-sulphonate;
(2S)-Ethoxy-3-{4-[2-(2-methyl-5-benzo[1,3]dioxol-5-yl-pyrrol-1-yl)-ethoxy]-phenyl}-propyl-(4-methyl phenyl)-sulphonate;
1-Ethoxy-(2S)-ethoxy-3-[4-(1-methyl-1H-benzoimidazol-2-ylmethoxy)-phenyl]-propane;
(2S)-Ethoxy-3-{4-(5-methyl-2-phenyl-oxazol-4-ylmethoxy)-phenyl}-1-propoxy propane;
1-Ethoxy-(2S)-ethoxy-3-[4-(5-methyl-3-phenyl-isoxazol-4-yl methoxy)-phenyl]-propane;
(2S)-tert-Butoxycarbonylamino-3-[4-(5-methyl-2-phenyl-oxazol-4-ylmethoxy)-phenyl]- propyl methanesulphonate;
(2S)-Ethoxy-3-{4-[2-(5-methyl-2-phenyl-oxazol-4-yl)-ethoxy]-phenyl}-propyl amine and its pharmaceutically acceptable salts;
(2S)-Ethoxy-3-[4-{3-methyl-3H-quinazolin-4-on-2yl methoxy}phenyl]propyl amine and its pharmaceutically acceptable salts;
((2S)-Ethoxy-3-{4-[2-(5-methyl-2-phenyl-oxazol-4-yl)-ethoxy]-phenyl}-propyl)-isopropyl-amine and its pharmaceutically acceptable salts;
3-{4-[2-(2,3-Dihydro-benzo[1,4]thiazin-4-yl)-ethoxy]-phenyl}-(2S)-ethoxy-propyl amine and its pharmaceutically acceptable salts;
3-(4-Benzyloxy-phenyl)-(2S)-ethoxy-propyl amine and its pharmaceutically acceptable salts;
(2S)-Ethoxy-3-[4-(5-methyl-2-thiophen-2-yl-oxazol-4-ylmethoxy)-phenyl]-propylamine and its pharmaceutically acceptable salts.
(2S)-Ethoxy-3-[4-(5-methyl-2-phenyl-oxazol-4-ylmethoxy)-phenyl]-propylamine and its pharmaceutically acceptable salts;
N-tert-Butoxycarbonyl-(2S)-ethoxy-3-[4-(5-methyl-2-phenyl-oxazol-4-ylmethoxy)-phenyl]-propyl amine;
(2S)-Ethoxy-3-{4-[2-(2-methyl-5-(5-methyl-thiophen-2-yl)-pyrrol-1-yl)-ethoxy]-phenyl}-propylamine and its pharmaceutically acceptable salts;
N-((2S)-Ethoxy-3-{4-(5-methyl-2-phenyl-oxazol-4-ylmethoxy)-phenyl}-propyl)-methane sulphonamide;
(2S)-Ethoxy-3-[4-(5-methyl-2-phenyl-oxazol-4-ylmethoxy)-phenyl]-propylamine and its pharmaceutically acceptable salts;
[(2S)-Ethoxy-3-[4-(5-methyl-2-phenyl-oxazol-4-ylmethoxy)-phenyl]-propyl]-ethyl-amine and its pharmaceutically acceptable salts;
[(2S)-Ethoxy-3-[4-(5-methyl-2-phenyl-oxazol-4-ylmethoxy)-phenyl]-propyl]-isopropyl-amine and its pharmaceutically acceptable salts;
(2S)-Ethoxy-3-{4-[2-(2-methyl-5-benzo[1,3]dioxol-5-yl-pyrrol-1-yl)-ethoxy]-phenyl}-propylamine and its pharmaceutically acceptable salts;
(2S)-Ethoxy-3-{4-[2-(2-methyl-5-benzofuran-2-yl-pyrrol-1-yl)-ethoxyl-phenyl}-propylamine and its pharmaceutically acceptable salts;
N-[(2S)-Ethoxy-3-[4-(5-methyl-2-phenyl-oxazol-4-ylmethoxy)-phenyl]-propyl]-2,2,2-trifluoxo-acetamide;

N-Ethoxycarbonyl-((2S)-ethoxy-3-{4-(5-methyl-2-phenyl-oxazol-9-ylmethoxy)-phenyl}-propyl)amine;
N-Benzyloxycarbonyl-((2S)-ethoxy-3-{4-(5-methyl-2-phenyl-oxazol-4-ylmethoxy)-phenyl}-propyl)amine;
N-[(2S)-Ethoxy-3-[4-(5-methyl-2-phenyl-oxazol-4-ylmethoxy)-phenyl]-propyl]-acetamide;
(2S)-Hydroxy-3-{4-[2-(5-methyl-2-phenyl-oxazol-4-yl)-ethoxy]-phenyl}-propyl azide;
3-(4-Benzyloxy-phenyl)-(2S)-ethoxy-propyl azide;
(2S)-Ethoxy-3-{4-[2-(5-methyl-2-phenyl-oxazol-4-yl)-ethoxy]-phenyl}-propyl azide;
(2S)-Ethoxy-3-{4-[2-(5-ethyl-pyridin-2-yl)-ethoxy]-phenyl}-propyl azide and its pharmaceutically acceptable salts;
(2S)-Ethoxy-3-[4-(5-methyl-2-phenyl-oxazol-4-ylmethoxy)-phenyl]- propyl azide;
(2S)-Ethoxy-3-{4-[2-(2-methyl-5-(5-methyl-thiophen-2-yl)-pyrrol-1-yl)-ethoxy]-phenyl}-propyl azide;
(2S)-Ethoxy-3-{4-[2-(5-ethyl-pyridine-2-yl)-2-(tert-butyldimethyl-silanyloxy)-ethoxy]-phenyl}- propyl azide and its pharmaceutically acceptable salts;
(2S)-Ethoxy-3-{4-[2-(5-ethyl-pyridine-2-yl)-2-hydroxy-ethoxy]-phenyl}- propyl azide and its pharmaceutically acceptable salts;
(2S)-Ethoxy-3-{4-[2-(methyl-pyridin-2-yl-amino)-ethoxy]-phenyl}- propyl azide and its pharmaceutically acceptable salts;
(2S)-Ethoxy-3-[4-(5-methyl-2-thiophen-2-yl-oxazol-4-ylmethoxy)-phenyl]- propyl azide;
(2S)-Ethoxy-3-{4-[2-(5-methyl-2-thiophen-2-yl-oxazol-4-yl)-ethoxy]-phenyl}-propyl azide;
(2S)-Hydroxy-3-[4-(5-methyl-2-phenyl-oxazol-4-yl)-methoxy)-phenyl]- propyl azide;
(2S)-Amino-3-{4-[2-(5-methyl-2-phenyl-oxazol-4-yl)-ethoxy]-phenyl}- propyl azide and its pharmaceutically acceptable salts;
(2S)-Amino-3-{4-[2-(5-methyl-2-phenyl-oxazol-4-yl)-methoxy]-phenyl}- propyl azide and its pharmaceutically acceptable salts;
(2S)-tert-butoxycarbonylamino-3-[4-(5-methyl-2-phenyl-oxazol-4-ylmethoxy)-phenyl]-propyl azide;
(2S)-tert-butoxycarbonylamino-3-[4-(5-methyl-2-phenyl-oxazol-4-yl-ethoxy)-phenyl]- propyl azide;
(2S)-Ethoxy-3-{4-[2-(2-methyl-5-benzo[1,3]dioxo-5-yl-pyrrol-1-yl)-ethoxy]-phenyl}- propyl azide;
(2S)-Ethoxy-3-{4-[2-(2-methyl-5-benzofuran-2-yl-pyrrol-1-yl)-ethoxy]-phenyl}- propyl azide;
N-Benzyloxycarbonyl-(2S)-ethoxy-3-{4-[2-(5-methyl-2-phenyl-oxazol-4-yl)-ethoxy]-phenyl}-propyl amine;
N-tert-Butoxycarbonyl-(2S)-Ethoxy-3-{4-[2-(5-methyl-2-phenyl-oxazol-4-yl)-ethoxy]-phenyl}- propyl amine;
N-tert-Butoxycarbonyl-3-{4-[2-(2,3-dihydro-benzo[1,4]thiazin-4-yl)-ethoxy]-phenyl}-(2S)-ethoxy-propyl amine;
N-((2S)-Ethoxy-3-{4-[2-[5-methyl-2-phenyl-oxazol-4-yl)-ethoxy]-phenyl}-propyl)-acetamide;
3-(4-Benzyloxy-phenyl)-N-tert-butoxycarbonyl-(2S)-ethoxy-propyl amine;
N-tert-Butoxycarbonyl-(2S)-ethoxy-3-(4-hydroxy-phenyl)- - propyl amine;
N-tert-Butoxycarbonyl-(2S)-ethoxy-3-{4-[2-(5-ethyl-pyridin-2-yl)-ethoxy]-phenyl}-propyl amine;
(2S)-Ethoxy-1-ethylsulphanyl-3-{4-[2-(5-methyl-2-phenyl-oxazol-4-yl)-ethoxy]-phenyl}-propane;
(2S)-Ethoxy-1-ethylsulphonyl-3-{4-[2-(5-methyl-2-phenyl-oxazol-9-yl)-ethoxy]-phenyl}-propane;

**6.** A process for the preparation of compounds of formula (I) as claimed in claim 1 comprising any of the steps below alone or in combination:

a)

i. converting a compound of formula (III) to a compound of formula (Ia)

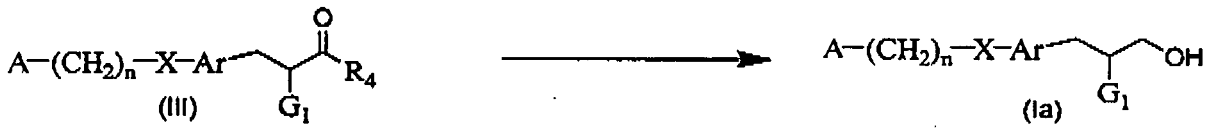

ii. converting the compound of formula (Ia) obtained above to compounds of formula (1b) , if desired

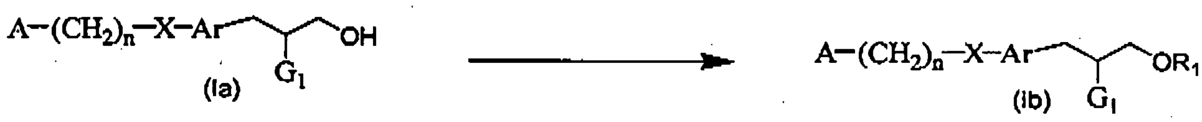

iii. converting the compound of formula (1b) obtained above to compounds of formula (1c), if desired

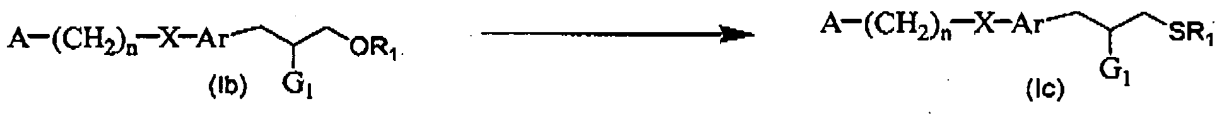

(Ib) (Ic)

iv. converting the compound of formula (1b) obtained above to compounds of formula (1d), if desired

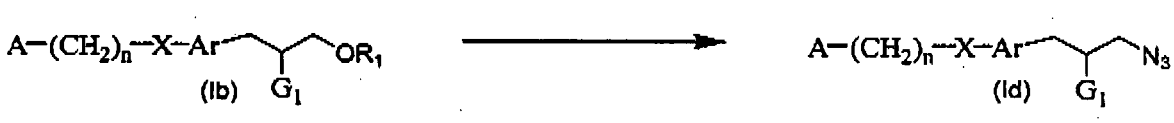

(Ib) (Id)

v. converting the compound of formula (1b) obtained above to compounds of formula (1e), if desired

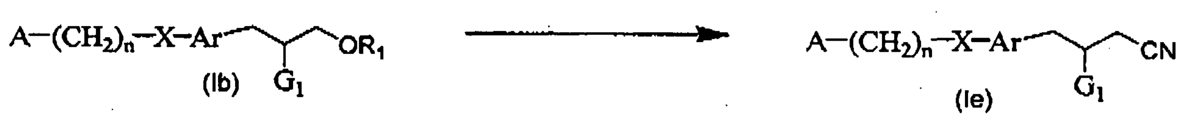

(Ib) (Ie)

b)

i. converting the compound of formula (III) to compounds of formula (1f)

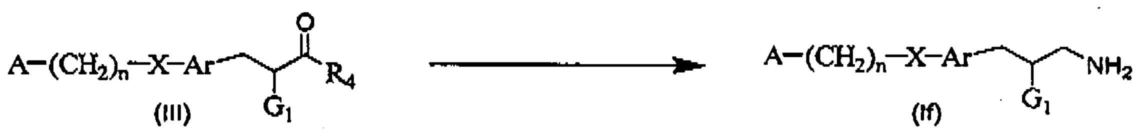

(III) (If)

ii. converting compound of formula (1f) obtained above to compound of formula (1g), if desired

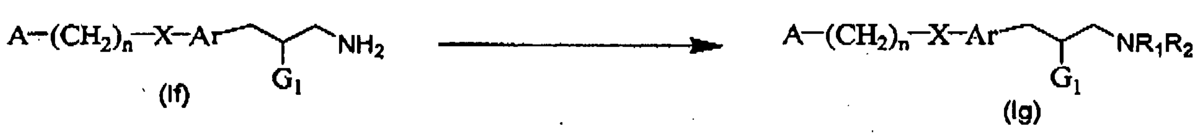

(If) (Ig)

iii. alternatively, converting compound of formula (1d) obtained above, to compound of formula (1f), if desired

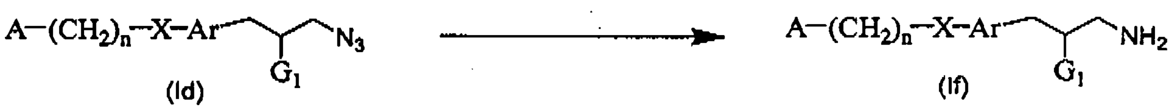

(Id) (If)

wherein compounds of formula (1b), (1c), (1d), (1e), (1f) & (1g) all represent compounds of formula (I) where A, X, Ar, $G_1$, $R_1$, $R_2$ are as defined in claim 1 and $G_2$ represents OH, $OR_1$, $SR_1$, $N_3$, CN, $NH_2$, $NR_1R_2$ respectively.

**7.** A process for the preparation of compound of formula (I), as claimed in claim 1, comprising any of the steps below alone or in combination:

i. reacting a compound of formula (IV), with compounds of formula (V)

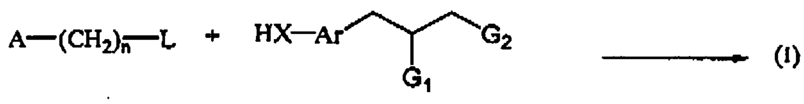

(IV) (V) (I)

ii. reacting a compound of formula (IV) with compound of formula (Va) to obtain compound of formula (Ig)

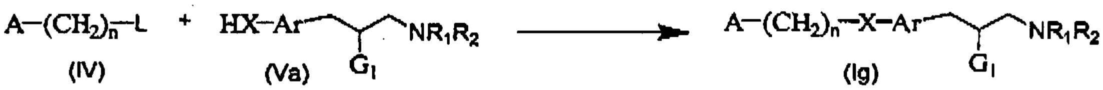

(IV) (Va) (Ig)

iii. reacting a compound of formula (IV) with compound of formula (Vb) to obtain compound of formula (Ib)

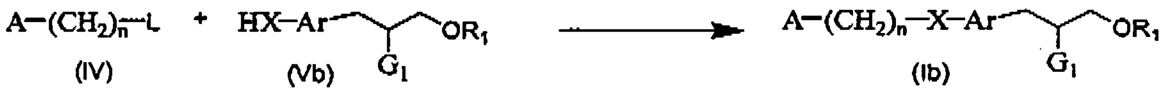

(IV) (Vb) (Ib)

iv. converting the compound of formula (Ib) to compound of formula (Ia)

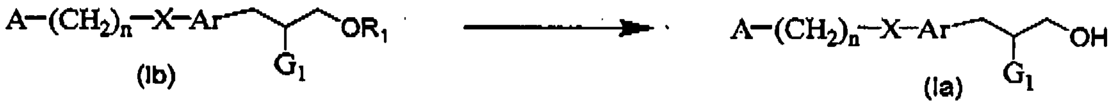

(Ib) (Ia)

wherein compounds of formula (1b) (1a), (1f) all represent compounds of formula (I) where A, X, Ar, $G_1$, $R_1$, $R_2$ are as defined in claim 1, 'L' represents a leaving group selected from halogen, mesylate, tosylate & triflate and $G_2$ represents OH, $OR_1$, $NR_1R_2$.

**8.** A compound of formula (IIIa), its tautomeric forms, its stereoisomers, its pharmaceutically acceptable salts, its pharmaceutically acceptable solvates,
wherein 'A' represents 4-oxazolyl group substituted with one or two substituents selected from substituted or unsubstituted linear or branched ($C_1$-$C_{12}$) alkyl, substituted or unsubstituted single or fused heteroaryl or heterocyclic groups with the proviso that one of the substituents on 'A' is always a heteroaryl or heterocyclic group; 'Ar' represents an unsubstituted phenyl group; $G_1$ represents $OR_1$, where $R_1$ represents substituted or unsubstituted groups selected from linear or branched ($C_1$-$C_{12}$) alkyl groups; $R_4$ represents OH or alkoxy groups; 'n' is an integer from 1-3; X represents O.

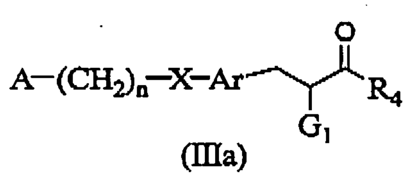

(IIIa)

**9.** A compound according to claim 8, **characterised in that** the substitutions on the substituents on 'A' are selected from hydroxyl, oxo, halo, thio, nitro, amino, cyano, formyl, or substituted or unsubstituted groups selected from amidino, guanidino, hydrazino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocyclyloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, arylthio, alkylsulfonylamino, alkylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfenyl derivatives, sulfonyl derivatives, sulfonic acid and its derivatives, phosphonic acid and its derivatives.

**10.** The compounds of claim 8 selected from
Ethyl (2S)-ethoxy-3-[4-(5-methyl-2-thiophen-2-yl-oxazol-4-ylmethoxy)-phenyl]-propanoate;
Ethyl (2S)-ethoxy-3-{4-[2-(5-methyl-2-thiophen-2-yl-oxazol-4-yl)-ethoxy]-phenyl}-propanoate;
Ethyl (2S)-ethoxy-3-{4-[5-methyl-2-(5-methyl-thiophen-2-yl)-oxazol-4-ylmethoxy]-phenyl}-propanoate;
Ethyl (2S)-ethoxy-3-(4-{2-[5-methyl-2-(5-methyl-thiophen-2-yl)-oxazol-4-yl]-ethoxy}-phenyl)-propanoate;
Ethyl (2S)-ethoxy-3-{4-[5-methyl-2-(3-methyl-thiophen-2-yl)-oxazol-4-ylmethoxy]-phenyl}-propanoate;
Ethyl (2S)-ethoxy-3-(4-{2-[5-methyl-2-(3-methyl-thiophen-2-yl)-oxazol-4-yl]-ethoxy}-phenyl)-propanoate;
Ethyl (2S)-ethoxy-3-[4-(5-methyl-2-thiophen-3-yl-oxazol-4-ylmethoxy)-phenyl]-propanoate;
Ethyl (2S)-ethoxy-3-{4-[2-(5-methyl-2-thiophen-3-yl-oxazol-4-yl)-ethoxy]-phenyl}-propanoate;
Ethyl 3-[4-(2-benzo[b]thiophen-2-yl-5-methyl-oxazol-4-ylmethoxy)-phenyl]-(2S)-ethoxy-propanoate;
Ethyl 3-{4-[2-(2-benzo[b]thiophen-2-yl-5-methyl-oxazol-4-yl)-ethoxy]-phenyl}-(2S)-ethoxy-propanoate;
Ethyl (2S)-ethoxy-3-[4-(2-furan-2-yl-5-methyl-oxazol-4-ylmethoxy)-phenyl]-propanoate;
Ethyl (2S)-ethoxy-3-{4-[2-(2-furan-2-yl-5-methyl-oxazol-4-yl)-ethoxy]-phenyl}-propanoate;
Ethyl (2S)-ethoxy-3-[4-(5-methyl-2-quinolin-2-yl-oxazol-4-ylmethoxy)-phenyl]-propanoate and its pharmaceutically acceptable salts;
Ethyl (2S)-ethoxy-3-{4-[2-(5-methyl-2-quinolin-2-yl-oxazol-4-yl)-ethoxy]-phenyl}-propanoate and its pharmaceutically acceptable salts;
Ethyl (2S)-ethoxy-3-{4-[3-(5-methyl-2-thiophen-2-yl-oxazol-4-yl)-propoxy]-phenyl}-propanoate;
Ethyl (2S)-ethoxy-3-{4-[5-methyl-2-(5-phenyl-thiophen-2-yl)-oxazol-4-ylmethoxy]-phenyl}-propanoate;
Ethyl (2S)-ethoxy-3-{4-[5-methyl-2-(5-chloro-thiophen-2-yl)-oxazol-4-ylmethoxy]-phenyl}-propanoate;
Ethyl (2S)-ethoxy-3-{4-[5-methyl-2-(5-bromo-thiophen-2-yl)-oxazol-4-ylmethoxy]-phenyl}-propanoate;
Ethyl (2S)-ethoxy-3-{4-[5-methyl-2-(5-methyl-furan-2-yl)-oxazal-4-ylmethoxy]-phenyl}-propanoate;
Ethyl (2S)-ethoxy-3-(4-{2-[5-methyl-2-(5-phenyl-thiophen-2-yl)-oxazol-4-yl]-ethoxy}-phenyl)-propanoate;
Ethyl (2S)-ethoxy-3-(4-{2-[5-methyl-2-(5-chloro-thiophen-2-yl)-oxazol-4-yl]-ethoxy}-phenyl)-propanoate;
Ethyl (2S)-ethoxy-3-(4-{2-[5-methyl-2-(5-bromo-thiophen-2-yl)-oxazol-4-yl]-ethoxy}-phenyl)-propanoate;
Ethyl (2S)-ethoxy-3-[4-(5-methyl-2-pyridin-2-yl-oxazol-4-ylmethoxy)-phenyl]- propanoate and its pharmaceutically acceptable salts;
Ethyl (2S)-ethoxy-3-[4-(5-methyl-2-pyridin-4-yl-oxazol-4-ylmethoxy)-phenyl]- propanoate and its pharmaceutically acceptable salts;
Ethyl (2S)-ethoxy-3-[4-{2-(5-methyl-2-pyridin-3-yl-oxazol-4-yl)-ethoxy}-phenyl]-propanoate and its pharmaceutically acceptable salts;
Ethyl (2S)-ethoxy-3-[4-(5-methyl-2-pyridin-3-yl-oxazol-4-ylmethoxy)-phenyl]-propanoate and its pharmaceutically acceptable salts;
(2S)-Ethoxy-3-[4-(5-methyl-2-thiophen-2-yl-oxazol-4-ylmethoxy)-phenyl]-propanoic acid and its pharmaceutically acceptable salts;
(2S)-Ethoxy-3-{4-[2-(5-methyl-2-thiophen-2-yl-oxazol-4-yl)-ethoxy]-phenyl}-propanoic acid and its pharmaceutically acceptable salts;
(2S)-Ethoxy-3-{4-[5-methyl-2-(5-methyl-thiophen-2-yl)-oxazol-4-ylmethoxy]-phenyl}- propanoic acid and its pharmaceutically acceptable salts;
(2S)-Ethoxy-3-(4-{2-[5-methyl-2-(5-methyl-thiophen-2-yl)-oxazol-4-yl]-ethoxy}-phenyl)-propanoic acid and its pharmaceutically acceptable salts;
(2S)-Ethoxy-3-{4-[5-methyl-2-(3-methyl-thiophen-2-yl)-oxazol-4-ylmethoxy]-phenyl}-propanoic acid and its pharmaceutically acceptable salts;
(2S)-Ethoxy-3-(4-{2-[5-methyl-2-(3-methyl-thiophen-2-yl)-oxazol-4-yl]-ethoxy}-phenyl)-propanoic acid and its pharmaceutically acceptable salts;
(2S)-Ethoxy-3-[4-(5-methyl-2-thiophen-3-yl-oxazol-4-ylmethoxy)-phenyl]-propanoic acid and its pharmaceutically acceptable salts;
(2S)-Ethoxy-3-{4-[2-(5-methyl-2-thiophen-3-yl-oxazol-4-yl)-ethoxy]-phenyl}-propanoic acid and its pharmaceutically acceptable salts;
3-[4-(2-Benzo[b]thiophen-2-yl-5-methyl-oxazol-4-ylmethoxy)-phenyl]-(2S)-ethoxy-propanoic acid and its pharmaceutically acceptable salts;
3-{4-[2-(2-Benzo[b]thiophen-2-yl-5-methyl-oxazol-4-yl)-ethoxy]-phenyl}-(2S)-ethoxy-propanoic acid and its pharmaceutically acceptable salts;
(2S)-Ethoxy-3-[4-(2-furan-2-yl-5-methyl-oxazol-4-ylmethoxy)-phenyl]-propanoic acid and its pharmaceutically acceptable salts;
(2S)-Ethoxy-3-{4-[2-(2-furan-2-yl-5-methyl-oxazol-4-yl)-ethoxy]-phenyl}-propanoic acid and its pharmaceutically acceptable salts;
(2S)-Ethoxy-3-[4-(5-methyl-2-quinolin-2-yl-oxazol-4-ylmethoxy)-phenyl]-propanoic acid and its pharmaceutically acceptable salts;
(2S)-Ethoxy-3-{4-[2-(5-methyl-2-quinolin-2-yl-oxazol-4-yl)-ethoxy]-phenyl}-propanoic acid and its pharmaceutically acceptable salts;
(2S)-Ethoxy-3-{4-[3-(5-methyl-2-thiophen-2-yl-oxazol-4-yl)-propoxy]-phenyl}-propanoic acid and its pharmaceutically acceptable salts;
(2S)-Ethoxy-3-{4-[5-methyl-2-Benzofuran-2-yl)-oxazol-4-ylmethoxy]-phenyl}- propanoic acid and its pharmaceutically acceptable salts;
(2S)-Ethoxy-3-{4-[5-methyl-2-(5-chloro-thiophen-2-yl)-oxazol-4-ylmethoxy]-phenyl}- propanoic acid and its pharmaceutically acceptable salts;
(2S)-Ethoxy-3-{4-[5-methyl-2-(5-bromo-thiophen-2-yl)-oxazol-4-ylmethoxy]-phenyl}- propanoic acid and its pharmaceutically acceptable salts;
(2S)-Ethoxy-3-{4-[5-methyl-2-(5-methyl-furan-2-yl)-oxazol-4-ylmethoxy]-phenyl}- propanoic acid and its pharmaceutically acceptable salts;
(2S)-Ethoxy-3-(4-{2-[5-methyl-2-(5-phenyl-thiophen-2-yl)-oxazol-4-yl]-ethoxy}-phenyl)- propanoic acid and its pharmaceutically acceptable salts;
(2S)-Ethoxy-3-(4-{2-[5-methyl-2-(5-chloro-thiophen-2-yl)-oxazol-4-yl]-ethoxy}-phenyl)- propanoic acid and its pharmaceutically acceptable salts;
(2S)-Ethoxy-3-(4-{2-[5-methyl-2-(5-bromo-thiophen-2-yl)-oxazol-4-yl]-ethoxy}-phenyl)- propanoic acid and its pharmaceutically acceptable salts;
(2S)-Ethoxy-3-(4-{2-[5-methyl-2-(5-methyl-furan-2-yl)-oxazol-4-yl]-ethoxy}-phenyl)- propanoic acid and its pharmaceutically acceptable salts;
2(S)-Ethoxy-3-[4-(5-methyl-2-pyridin-2-yl-oxazol-4-ylmethoxy)-phenyl]- propanoic acid and its pharmaceutically acceptable salts;
2(S)-Ethoxy-3-[4-(5-methyl-2-pyridin-4-yl-oxazol-4-ylmethoxy)-phenyl]- propanoic acid and its pharmaceutically acceptable salts;
2(S)-Ethoxy-3-[4-(5-methyl-2-pyridin-3-yl-oxazol-4-ylmethoxy)-phenyl]- propanoic acid and its pharmaceutically acceptable salts;

**11.** The compounds as claimed in any one of claims 8 to 10, suitable as intermediates for the preparation of compounds of formula (I).

**12.** A process for the preparation of compound of formula (IIIa) as claimed in any one of claims 8 to 10 comprising

i. reacting a compound of formula (IVa) wherein 'A' represents 4-oxazolyl group substituted with one or two substitutents selected from substituted or unsubstituted linear or branched ($C_1$-$C_{12}$) alkyl, substituted or unsubstituted single or fused heteroaryl or heterocyclic groups with the proviso that one of the substituents on "A" is always a heteroaryl or heterocyclic group and with the further proviso that when the heteroaryl is pyryl group, such group is unsubstituted; 'n' is an integer from 1-3; and 'L' represents a leaving group selected from halogen, mesylate, tosylate & triflate, with a compound of formula (Vc) wherein X represents oxygen or sulphur; 'Ar' represents unsubstituted phenyl; $G_1$ represents $OR_1$ or $SR_1$, where $R_1$ represents hydrogen, perfluoro($C_1$-$C_{12}$) alkyl, substituted or unsubstituted groups selected from linear or branched ($C_1$-$C_{12}$)alkyl, cyclo($C_1$-$C_{12}$)alkyl, aryl, ar ($C_1$-$C_{12}$) alkyl, heteroaryl, heteroar ($C_1$-$C_{12}$)alkyl, heterocyclyl, alkoxyalkyl, aryloxyalkyl, alkoxycarbonyl, aryloxycarbonyl, cycloalkyloxycarbonyl, alkylaminocarbonyl, arylaminocarbonyl or acyl groups; $R_4$ represents OH, alkoxy or aryloxy, aralkoxy or $NR_1R_2$ groups, where $R_1$ & $R_2$ may be same or different and independently represent hydrogen, substituted or unsubstituted groups selected from linear or branched ($C_1$-$C_8$)alkyl, ($C_3$-$C_7$) cycloalkyl, acyl, aryl, heteroaryl, heterocyclyl, aminocarbonyl, aralkyl, alkylaminocarbonyl, arylaminocarbonyl, aralkylaminocarbonyl, heteroarylaminocarbonyl, heteroaralkylaminocarbonyl, heterocyclylaminocarbonyl, alkoxycarbonyl, aryloxycarbonyl, aralkyloxycarbonyl, heteroaryloxycarbonyl, heteroaraloxycarbonyl, heterocycloxycarbonyl groups or $SO_2R_3$ wherein $R_3$ represents substituted or unsubstituted groups selected from alkyl, aryl, polyhaloalkyl, heterocyclyl, heteroaryl groups,

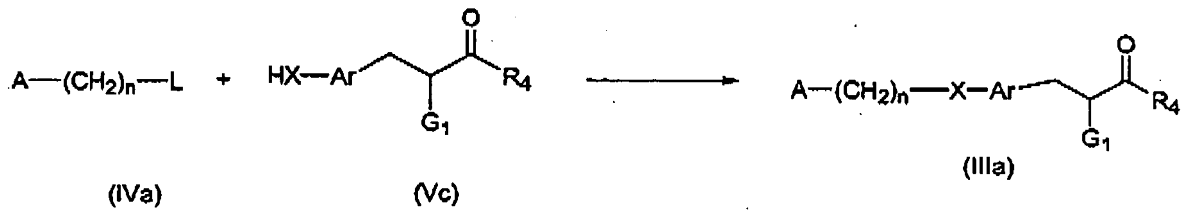

(IVa) (Vc) (IIIa)

ii. optionally hydrolysing the compound of formula (IIIa) wherein $R_4$ represents alkoxy, aryloxy, aralkoxy or $NR_1R_2$ groups, where $R_1$ & $R_2$ are as defined earlier, to a further compound of formula (IIIa) wherein $R_4$ represents OH.

**13.** A pharmaceutical composition which comprises compounds of formula (I) or (IIIa), as claimed in any preceding claims and a pharmaceutically acceptable carrier, diluent, excipients or solvate.

**14.** A pharmaceutical composition according to claim 13, in the form of a tablet, capsule, powder, granule, syrup, solution or suspension.

**15.** A pharmaceutical composition according to claim 13 or claim 14, in combination with sulfonyl urea, biguanide, angiotensin II inhibitor, aspirin, α-glycosidase inhibitor, insulin secretagogue, insulin, β-sitosterol inhibitor, HMG CoA reductase inhibitor, fibrate, nicotinic acid, cholestyramine, cholestipol or probucol, which may be administered together or within such a period as to act synergistically together to a patient in need thereof.

**16.** The use of a compound of formula (I) or (IIIa), as defined in any of claims 1 to 5 or 9 to 11 for the preparation of a medicament for use in the reduction of plasma glucose, triglycerides, total cholesterol, LDL, VLDL or free fatty acids in the plasma of a patient, while optionally elevating HDL cholesterol levels.

**17.** The use according to claim 16, wherein the compound of formula (I) or (IIIa) is for the preparation of a medicament to be given in combination with HMG CoA reductase inhibitor, fibrate, nicotinic acid, cholestyramine, cholestipol or probucol, which additional ingredient may be administered together with the medicament or within such a period as to act synergistically together with the medicament.

**18.** The use of a compound of formula (I) or (IIIa) as defined in any of claims 1 to 5 or 9 to 11 for the preparation preventing or treating diseases caused by hyperlipidaemia, hypercholesteremia, hyperglycemia, obesity, impaired glucose tolerance, leptin resistance, insulin resistance or diabetic complications.

**19.** The use of a compound of formula (I) or (IIIa) as defined in any of claims 1 to 5 or 9 to 11 for the preparation of a medicament for treating a disease wherein insulin resistance is the underlying pathophysiological mechanism, which includes type 2 diabetes, impaired glucose tolerance, dyslipidaemia, hypertension, obesity, atherosclerosis, hyperlipidaemia, coronary artery disease, cardiovascular disorders, renal diseases, microalbuminuria, glomerulonephritis, glomerulosclerosis, nephrotic syndrome, hypertensive nephrosclerosis, diabetic retinopathy, diabetic nephropathy, endothelial cell dysfunction, psoriasis, polycystic ovarian syndrome (PCOS), dementia, end-stage renal disease, osteoporosis, inflammatory bowel diseases, myotonic dystrophy, pancreatitis, arteriosclerosis, xanthoma or cancer.

**20.** The use according to any of claims 16 to 19 wherein the medicament contains a pharmaceutically acceptable carrier, diluent or excipients or solvate.

## Patentansprüche

**1.** Verbindung der allgemeinen Formel (I),

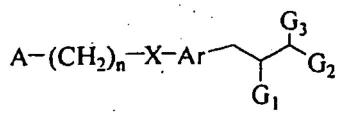

(I)

ihre tautomeren Formen, ihre Stereoisomeren, ihre pharmazeutisch verträglichen Salze, ihre pharmazeutisch verträglichen Solvate,
worin
"A" eine substituierte oder unsubstituierte Gruppe, ausgewählt aus Heteroaryl-, Heterocyclyl-Gruppen, darstellt; "n" eine ganze Zahl von 1 bis 3 ist; "X" Sauerstoff darstellt; "Ar" eine substituierte oder unsubstituierte Phenyl-Gruppe darstellt;
$G_1$ $OR_1$, $SR_1$, $S(O)_2R_3$, $N_3$, CN, COOH, Tetrazolyl-Gruppen darstellt; $G_2$ $OR_1$, $NR_1R_2$, $SR_1$, $S(O)R_3$, $S(O)_2R_3$, $N_3$, CN, COOH, Tetrazolyl-Gruppen darstellt; $R_1$, $R_2$ Wasserstoff, substituierte oder unsubstituierte Gruppen, ausgewählt aus linearen oder verzweigten ($C_1$-$C_8$)-Alkyl-, Acyl-, Alkoxycarbonyl-, Aryloxycarbonyl-, Aralkyloxycarbonyl-Gruppen, darstellen; $R_3$ unsubstituierte oder substituierte Gruppen, ausgewählt aus Alkyl-, ArylGruppen, darstellt; mit der Maßgabe, dass, wenn $G_2$ $NR_1R_2$ darstellt, $G_1$ keine -OH-Gruppe darstellt;
$G_3$ Wasserstoff oder ($C_1$-$C_9$)-Alkyl-Gruppen darstellt.

**2.** Verbindung gemäß Anspruch 1, **dadurch gekennzeichnet, dass** die Substituenten "A", $R_1$, $R_2$ und $R_3$ gleich oder unterschiedlich sein können und unabhängig ausgewählt sind aus Hydroxyl, Oxo, Halogen, Thio, Nitro, Amino, Cyano, Formyl oder substituierten oder unsubstituierten Gruppen, ausgewählt aus Alkyl-, Halogenalkyl-, Perhalogenalkyl-, Alkoxy-, Aryl-, Aryloxy-, Aralkyl-, Aralkoxy-, Heterocyclyl-, Heteroaryl-, Acyl-, Acyloxy-, Acylamino-, monosubstituierten oder disubstituierten Amino-, Carbonylamino-, Hydroxyalkyl-, Alkylthio-,Thialkyl-Gruppen.

**3.** Verbindung gemäß Anspruch 1, **dadurch gekennzeichnet, dass** geeignete Substituenten an einem beliebigen Substituenten von "A" gleich oder unterschiedlich sein können und unabhängig ausgewählt sind aus Hydroxyl, Oxo, Halogen, Thio, Nitro, Amino, Cyano, Formyl oder substituierten oder unsubstituierten Gruppen, ausgewählt aus Amidino, Guanidino, Hydrazino, Alkyl, Halogenalkyl, Perhalogenalkyl, Alkoxy, Halogenalkoxy, Perhalogenalkoxy, Alkenyl, Alkinyl, Cycloalkyl, Cycloalkenyl, Bicycloalkyl, Bicycloalkenyl, Alkoxy, Alkenoxy, Cycloalkoxy, Aryl, Aryloxy, Aralkyl, Aralkoxy, Heterocyclyl, Heteroaryl, Heterocyclylalkyl, Heteroaralkyl, Heteroaryloxy, Heteroaralkoxy, Heterocyclyloxy, Heterocyclylalkoxy, Heterocyclylalkoxyacyl, Acyl, Acyloxy, Acylamino, monosubstituiertem oder disubstituiertem Amino, Arylamino, Aralkylamino, Carbonsäure und ihren Derivaten, zum Beispiel Estern und Amiden, Carbonylamino, Hydroxyalkyl, Aminoalkyl, Alkoxyalkyl, Aryloxyalkyl, Aralkoxyalkyl Alkylthio, Thioalkyl, Arylthio, Alkylsulfonylamino, Alkylsulfonyloxy, Alkoxycarbonylamino, Aryloxycarbonylamino, Aralkyloxycarbonylamino, Aminocarbonylamino, Alkylaminocarbonylamino, Alkoxyamino, Hydroxylamino, Sulfenylderivaten, Sulfonylderivaten, Sulfonsäure und ihren Derivaten, Phosphonsäure und ihren Derivaten.

**4.** Verbindung gemäß Anspruch 1, **dadurch gekennzeichnet, dass** die Substituenten an der Gruppe, die durch "Ar" dargestellt wird, substituiertes oder unsubstituiertes lineares oder verzweigtes Alkyl, Alkoxy, Thioalkyl, Halogen, Halogenalkyl, Halogenalkoxy, Acyl, Amino, Acylamino, Thio oder Carbon- oder Sulfonsäuren und ihre Derivate, Phophonsäure und ihre Derivate darstellen.

**5.** Verbindungen gemäß Anspruch 1, ausgewählt aus
3-(6-Benzyloxynaphthalin-2-yl)-2-ethoxypropan-1-ol;
(2S)-Ethoxy-3-{4-[2-(5-methyl-2-phenyloxazol-4-yl)-ethoxy]-phenyl}-propan-1-ol;
(2S)-Ethoxy-3-{4-(4-hydroxy-3-methyl-3,4-dihydrochinazolin-2-yl-methoxy)-phenyl}-propan-1-ol;
2-Hydroxy-3-{4-[2-(5-methyl-2-phenyloxazol-4-yl)-ethoxy]-phenyl}-propan-1-ol;
3-{4-[2-(2,3-Dihydro-benzo[1,4]thiazin-4-yl)-ethoxy]-phenyl}-(2S)-ethoxy-propan-1-ol;
(2S)-Ethoxy-3-[4-(2-(phenoxazin-10-yl)-ethoxy)-phenyl]-propan-1-ol;
3-[4-(2-(Carbazol-9-yl)-ethoxy)-phenyl]-(2S)-ethoxy-propan-1-ol;
3-{4-[2-(3,4-Dihydro-2H-chinolin-1-yl)-ethoxy]-phenyl}-(2S)-methoxy-propan-1-ol;
(2S)-Ethoxy-3-[4-(2-(indol-1-yl)-ethoxy)-phenyl]-propan-1-ol;
(2S)-Ethoxy-3-[4-(2-(phenathiazin-10-yl)-ethoxy)-phenyl]-propan-1-ol;
3-{4-[2-(2,3-Dihydro-benzo[1,4]oxazin-4-yl)-ethoxy]-phenyl}-(2S)-ethoxy-propan-1-ol;
3-{4-[2-(5-Methyl-2-phenyloxazol-4-yl)-ethoxy]-phenyl}-2-phenylsulfanyl-propan-1-ol;
(2S)-Ethoxy-3-{4-[2-(methylpyridin-2-yl-amino)-ethoxy]-phenyl}-propan-1-ol und seinen pharmazeutisch verträglichen Salzen;
(2S)-Ethoxy-3-{4-[2-(5-ethylpyridin-2-yl)-ethoxy]-phenyl}-propan-1-ol und seinen pharmazeutisch verträglichen Salzen;
(2S)-Ethoxy-3-[4-(5-methyl-2-thiophen-2-yl-oxazol-4-yl-methoxy)-phenyl]-propan-1-ol;
(2S)-Ethoxy-3-{4-[2-(5-methyl-2-thiophen-2-yl-oxazol-4-yl)-ethoxy]-phenyl}-propan-1-ol;
(2S)-Ethoxy-3-(4-{2-[2-methyl-5-(4-methylsulfanylphenyl)-pyrrol-1-yl]-ethoxy}-phenyl)-propan-1-ol;
(3S)-Ethoxy-4-{4-[2-(5-methyl-2-phenyl-oxazol-4-yl)-ethoxy]-phenyl}-butan-1-ol;
(2S)-Ethoxy-3-(4-{2-[5-methyl-2-(4-methylsulfanylphenyl)-oxazol-4-yl]-ethoxy}-phenyl)-propan-1-ol;
(2S)-Amino-3-{4-[2-(5-methyl-2-phenyl-oxazol-4-yl)-ethoxy]-phenyl}-propan-1-ol und seinen pharmazeutisch Salzen;
(2S)-tert-Butoxycarbonylamino-3-[4-(5-methyl-2-phenyl-oxazol-4-yl-methoxy)-phenyl]-propan-1-ol;
(2S)-tert-Butoxycarbonylamino-3-{4-[2-(5-methyl-2-phenyl-oxazol-4-yl)-ethoxy]-phenyl}-propan-1-ol;
(2S)-Ethoxy-3-(4-{2-[2-methyl-5-(benzofuran-2-yl)-pyrrol-1-yl]-ethoxy}-phenyl)-propan-1-ol;
(2S)-Ethoxy-3-(4-{2-[2-methyl-5-(benzo[1,3]dioxol-5-yl)-pyrrol-1-yl]-ethoxy}-phenyl)-propan-1-ol;
(2S)-Ethoxy-3-[4-(1-methyl-1H-benzoimidazol-2-yl-methoxy)-phenyl]-propan-1-ol;
(2S)-Ethoxy-3-[4-(5-methyl-3-phenyl-isoxazol-4-yl-methoxy)-phenyl]-propan-1-ol;
(2S)-Ethoxy-3-{4-[2-(5-ethylpyridin-2-yl)-2-hydroxy-ethoxy]-phenyl}-propan-1-ol;
(2S)-Ethoxy-3-[4-(2-benzoiidazol-1-yl-ethoxy)-phenyl]-propan-1-ol;
(2S)-Ethoxy-3-[4-(5-methyl-2-phenyl-oxazol-4-yl-methoxy)-phenyl]-propan-1-ol;
(2S)-Ethoxy-3-(4-{2-[2-methyl-5-(5-methyl-thiophen-2-yl)-pyrrol-1-yl]-ethoxy}-phenyl)-propan-1-ol;
(2S)-Ethoxy-3-[4-(5-methyl-2-phenyl-oxazol-4-yl-methoxy)-phenyl]-propan-1,2-diol;
1-Ethoxy-(2S)-ethoxy-3-[4-{2-(5-methyl-2-phenyl-oxazol-4-yl)-ethoxy}-phenyl]-propan;
2-((2S)-Ethoxy-3-{4-[2-(5-methyl-2-phenyl-oxazol-4-yl)-ethoxy]-phenyl}-propoxy)-ethanol;
2-((2S)-Ethoxy-3-{4-[2-(5-methyl-2-phenyl-oxazol-4-yl)-ethoxy]-phenyl}-propoxy-benzoesäure und ihren pharmazeutisch verträglichen Salzen;
(2S)-Ethoxy-3-{4-[2-(5-methyl-2-phenyl-oxazol-4-yl)-ethoxy]-phenyl}-propylbromacetat;
1-Ethoxy-(2S)-ethoxy-3-[4-{2-(-3,4-dihydro-2H-benzo[1,4]thiazin-1-yl)-ethoxy}-phenyl]-propan;
1-Propoxy-(2S)-ethoxy-3-[4-{2-(5-methyl-2-phenyl-oxazol-4-yl)-ethoxy}-phenyl]-propan;
2-((2S)-Ethoxy-3-[4-(5-methyl-2-phenyl-oxazol-4-yl-methoxy)-phenyl]-propoxy)-benzoesäure und ihren pharmazeutisch verträglichen Salzen;
1-Ethoxy-(2S)-ethoxy-3-[4-(5-methyl-2-phenyl-oxazol-4-yl-methoxy)-phenyl]-propan;
(2S)-Ethoxy-3-[4-(5-methyl-2-phenyl-oxazol-2-yl-methoxy)-phenyl]-1-phenoxypropan;
(2S)-Ethoxy-1-ethylsulfinyl-3-{4-(5-methyl-2-phenyl-oxazol-4-yl-methoxy)-phenyl}-propan;
(2S)-Ethoxy-1-ethylsulfanyl-3-{4-(5-methyl-2-phenyl-oxazol-4-yl-methoxy)-phenyl}-propan;
(2S)-Ethoxy-3-[4-(5-methyl-2-phenyl-oxazol-4-yl-methoxy)-phenyl]-1-isopropoxypropan;
(3S)-Ethoxy-4-[4-(5-methyl-2-phenyl-oxazol-4-yl-methoxy)-phenyl]-butyronitril;
(2S)-Ethoxy-1H-tetrazol-3-[4-(5-methyl-2-phenyl-oxazol-4-yl-methoxy)-phenyl]-propan;
2-Ethoxy-1-[4-(5-methyl-2-phenyl-oxazol-4-yl-methoxy)-phenyl]-pentan-3-ol;
2,3-Diethoxy-1-[4-(5-methyl-2-phenyl-oxazol-4-yl-methoxy)-phenyl]-pentan;
2-Ethoxy-1-{4-[2-(5-methyl-2-phenyl-oxazol-4-yl)-ethoxy]-phenyl}-pentan-3-ol;
2,3-Diethoxy-1-{4-[2-(5-methyl-2-phenyl-oxazol-4-yl)-ethoxy]-phenyl}-pentan;
((2S)-Ethoxy-3-{4-[2-(5-methyl-2-phenyl-oxazol-4-yl)-ethoxy]-phenyl}-propoxy)-essigsäure und ihren pharmazeutisch verträglichen Salzen;
3-(4-Benzyloxy-phenyl)-(2S)-ethoxy-propyl-methansulfonat;
(2S)-Ethoxy-3-[4-(5-methyl-2-phenyl-oxazol-4-yl-methoxy)-phenyl]-propylmethan-sulfonat;
(2S)-Ethoxy-3-{4-[2-(2-methyl-5-(5-methyl-thiophen-2-yl)-pyrrol-1-yl)-ethoxy]-phenyl}-propylmethansulfonat;
(2S)-Ehoxy-3-{4-[2-(5-methyl-2-phenyl-oxazol-4-yl)-ethoxy]-phenyl}-propylmethansulfonat;
(2S)-Ethoxy-3-[4-(5-methyl-2-thiophen-2-yl-oxazol-4-yl-methoxy)-phenyl]}-propylmethansulfonat
(2S)-Ethoxy-3-{4-[2-(5-ethyl-pyridin-2-yl)-ethoxy]-phenyl}-propylmethansulfonat;
(2S)-Ethoxy-1-methoxy-3-{4-[2-(5-methyl-2-thiophen-2-yl-oxazo)-4-yl)-ethoxy]-phenyl}-propan;
1-Ethoxy-(2S)-ethoxy-3-(4-{2-[5-methyl-2-(4-methylsulfanyl-phenyl)-oxazol-4-yl]-ethoxy}-phenyl)-propan;
1-Ethoxy-(2S)-ethoxy-3-{4-[2-(5-methyl-2-thiophen-2-yl-oxazol-4-yl)-ethoxy]-phenyl}-propan;
(2S)-Ethoxy-3-[4-(5-methyl-2-thiophen-2-yl-oxazol-4-yl-methoxy)-phenyl]-1-ethoxy propan;
(2S)-Ethoxy-3-{4-[2-(2-methyl-5-benzofuran-2-yl-pyrrol-1-yl)-ethoxy]-phenyl}-propylmethansulfonat
(2S)-Ethoxy-3-{4-[2-(2-methyl)-2-benzo[1,3]dioxol-4-yl-pyrrol-1-yl)-ethoxy]-phenyl}-propylmethansulfonat;
(2S)-Ethoxy-3-{4-[2-(2-methyl-5-benzofuran-2-yl-pyrrol-1-yl)-ethoxy]-phenyl}-propyl-(4-methylphenyl)-sulfonat;
(2S)-Ethoxy-3-{4-[2-(2-methyl-5-benzo[1,3]dioxol-5-yl-pyrrol-1-yl)-ethoxy]-phenyl}-propyl-(4-methylphenyl)-sulfonat;

1-Ethoxy-(2S)-ethoxy-3-[4-(1-methyl-1H-benzoimidazol-2-yl-methoxy)-phenyl]-propan;
(2S)-Ethoxy-3-{4-(5-methyl-2-phenyl-oxazol-4-yl-methoxy)-phenyl}-1-propoxy-propan;
1-Ethoxy-(2S)-ethoxy-3-[4-(5-methyl-3-phenyl-isoxazol-4-yl-methoxy)-phenyl]-propan;
(2S)-tert-Butoxycarbonylamino-3-[4-(5-methyl-2-phenyl-oxazol-4-yl-methoxy)-phenyl]-propyl-methansulfonat;
(2S)-Ethoxy-3-{4-[2-(5-methyl-2-phenyl-oxazol-4-yl)-ethoxy]-phenyl}-propylamin und seinen pharmazeutisch verträglichen Salzen;
(2S)-Ethoxy-3-[4-{3-methy)-3H-chinazolin-4-on-2-yl-methoxy}-phenyl]-propylamin und seinen pharmazeutisch verträglichen Salzen;
((2S)-Ethoxy-3-{4-[2-(5-methyl-2-phenyl-oxazol-4-yl)-ethoxy]-phenyl}-propyl)-isopropylamin und seinen pharmazeutisch verträglichen Salzen;
3-{4-[2-(2,3-Dihydro-benzo[1,4]thiazin-4-yl)-ethoxy]-phenyl}-(2S)-ethoxy-propylamin und seinen pharmazeutisch verträglichen Salzen;
3-(4-Benzyloxy-phenyl)-(2S)-ethoxy-propylamin und seinen pharmazeutisch verträglichen Salzen;
(2S)-Ethoxy-3-[4-(5-methyl-2-thiophen-2-yl-oxazol-4-yl-methoxy)-phenyl]-propylamin und seinen pharmazeutisch verträglichen Salzen;
(2S)-Ethoxy-3-[4-(5-methyl-2-phenyl-oxazol-4-yl-methoxy)-phenyl]-propylamin und seinen pharmazeutisch verträglichen Salzen;
N-tert-Butoxycarbonyl-(2S)-ethoxy-3-[4-(5-rnethyl-2-phenyl-oxazol-4-yl-methoxy)-phenyl]-propylamin;
(2S)-Ethoxy-3-{4-[2-(2-methyl-5-(5-methyl-thiophen-2-yl)-pyrrol-1-yl)-ethoxy]-phenyl}-propylamin und seinen pharmazeutisch verträglichen Salzen;
N-((2S)-Ethoxy-3-{4-(5-methyl-2-phenyl-oxazol-4-yl-methoxy)-phenyl}-propyl)-methansulfonamid;
(2S)-Ethoxy-3-[4-(5-methyl-2-phenyl-oxazol-4-yl-methoxy)-phenyl]-propylamin und seinen pharmazeutisch verträglichen Salzen;
[(2S)-Ethoxy-3-[4-(5-methyl-2-phenyl-oxazol-4-yl-methoxy)-phenyl]-propyl]-ethylamin und seinen pharmazeutisch verträglichen Salzen;
[(2S)-Ethoxy-3-[4-(5-methyl-2-phenyl-oxazol-4-yl-methoxy)-phenyl]-propyl]-isopropylamin und seinen pharmazeutisch verträglichen Salzen;
(2S)-Ethoxy-3-{4-[2-(2-methyl-5-benzo[1,3]dioxol-5-yl-pyrrol-1-yl)-ethoxy]-phenyl}-propylamin und seinen pharmazeutisch verträglichen Salzen;
(2S)-Ethoxy-3-{4-[2-(2-methyl-5-benzofuran-2-yl-pyrrol-1-yl)-ethoxy]-phenyl}-propylamin und seinen pharmazeutisch verträglichen Salzen;
N-[(25)-Ethoxy-3-[4-(5-methyl-2-phenyl-oxazol-4-yl-methoxy)-phenyl]-propyl]-2,2,2-trifluoracetamid,
N-Ethoxycarbonyl-((2S)-ethoxy-3-{4-(5-methyl-2-phenyl-oxazol-4-yl-methoxy)-phenyl}-propyl)-amin;
N-Benzyloxycarbonyl-((2S)-ethoxy-3-{4-(5-methyl-2-phenyl-oxazol-4-yl-methoxy)-phenyl}-propyl)-amin;
N-[(2S)-Ethoxy-3-[4-(5-methyl-2-phenyl-oxazol-4-yl-methoxy)-phenyl]-propyl]-acetamid;
(2S)-Hydroxy-3-{4-[2-(5-methyl-2-phenyl-oxazol-4-yl)-ethoxy]-phenyl}-propylazid;
3-(4-Benzyloxy-phenyl)-(2S)-ethoxy-propylazid;
(2S)-Ethoxy-3-{4-{2-(5-methyl-2-phenyl-oxazol-4-yl)-ethoxy]-phenyl}-propylazid;
(2S)-Ethoxy-3-{4-[2-(S-ethyl-pyridin-2-yl)-ethoxy]-phenyl}-propylazid und seinen pharmazeutisch verträglichen Salzen;
(2S)-Ethoxy-3-[4-(5-methyl-2-phenyl-oxazol-4-yl-methoxy)-phenyl]-propylazid;
(2S)-Ethoxy-3-{4-[2-(2-methyl-5-(5-methyl-thiophen-2-yl)-pyrrol-1-yl)-ethoxy]-phenyl}-propylazid;
(2S)-Ethoxy-3-{4-[2-(5-ethyl-pyridin-2-yl)-2-.(tert-butyldimethyl-silanyloxy)-ethoxy]-phenyl}-propylazid und seinen pharmazeutisch verträglichen Salzen;
(2S)-Ethoxy-3-{4-[2-(5-ethylpyridin-2-yl)-2-hydroxy-ethoxy]-phenyl}-propylazid und seinen pharmazeutisch verträglichen Salzen;
(2S)-Ethoxy-3-{4-[2-(methyl-pyridin-2-yl-amino)-ethoxy]-phenyl}-propylazid und seinen pharmazeutisch verträglichen Salzen;
(2S)-Ethoxy-3-[4-(5-methyl-2-thiophen-2-yl-oxazol-4-yl-methoxy)-phenyl]-propylazid;
(2S)-Ethoxy-3-{4-[2-(5-methyl-2-thiophen-2-yl-oxazol-4-yl)-ethoxy]-phenyl}-propylazid;
(2S)-Hydroxy-3-[4-(5-methyl-2-phenyl-oxazol-4-yl)-methoxy)-phenyl]-propylazid;
(2S)-Amino-3-{4-[2-(5-methyl-2-phenyl-oxazol-4-yl)-ethoxy]-phenyl}-propylazid und seinen pharmazeutisch verträglichen Salzen;
(2S)-Amino-3-{4-[2-(5-methyl-2-phenyl-oxazol-4-yl)-methoxy]-phenyl}-propylazid und seinen pharmazeutisch verträglichen Salzen;
(2S)-tert-Butoxycarbonylamino-3-[4-(5-methyl-2-phenyl-oxazol-4-yl-methoxy)-phenyl]-propylazid;
(2S)-tert-Butoxycarbonylamino-3-[4-(5-methyl-2-phenyl-oxazol-4-yl-ethoxy)-phenyl]-propylazid;
(2S)-Ethoxy-3-{4-[2-(2-methyl-5-benzo[1,3]dioxol-5-yl-pyrrol-1-yl)-ethoxy]-phenyl}-propylazid;

(2S)-Ethoxy-3-{4-[2-(2-methyl-5-benzofuran-2-yl-pyrrol-1-yl)-ethoxy]-phenyl}-propylazid;
N-Benzyloxycarbonyl-(2S)-ethoxy-3-{4-[2-(5-methyl-2-phenyl-oxazol-4-yl)-ethoxy]-phenyl}-propylamin;
N-tert-Butoxycarbonyl-(2S)-ethoxy-3-{4-[2-(5-methyl-2-phenyl-oxazol-4-yl)-ethoxy]-phenyl}-propylamin;
N-tert-Butoxycarbonyl-3-{4-[2-(2,3-dihydro-benzo[1,4]thiazin-4-yl)-ethoxy]-phenyl}-(2S)-ethoxy-propylamin;
N-((2S)-Ethoxy-3-{4-[2-(5-methyl-2-phenyl-oxazol-4-yl)-ethoxy]-phenyl}-propyl)-acetamid;
3-(4-Benzyloxy-phenyl)-N-tert-butoxycarbonyl-(2S)-ethoxy-propylamin;
N-tert-Butoxycarbonyl-(2S)-ethoxy-3-(4-hydroxy-phenyl)-propylamin;
N-tert-Butoxycarbonyl-(2S)-ethoxy-3-{4-[2-(5-ethyl-pyridin-2-yl)-ethoxy]-phenyl}-propylamin;
(2S)-Ethoxy-1-ethylsulfanyl-3-{4-[2-(5-methyl-2-phenyl-oxazol-4-yl)-ethoxy]-phenyl}-propan;
(2S)-Ethoxy-1-ethylsulfonyl-3-{4-[2-(5-methyl-2-phenyl-oxazol-4-yl)-ethoxy]-phenyl]-propan.

**6.** Verfahren zur Herstellung von Verbindungen der Formel (I), wie sie in Anspruch 1 beansprucht sind, umfassend einen beliebigen der unten aufgeführten Schritte allein oder in Kombination:

a)

i. Umwandeln einer Verbindung der Formel (III) in eine Verbindung der Formel (Ia)

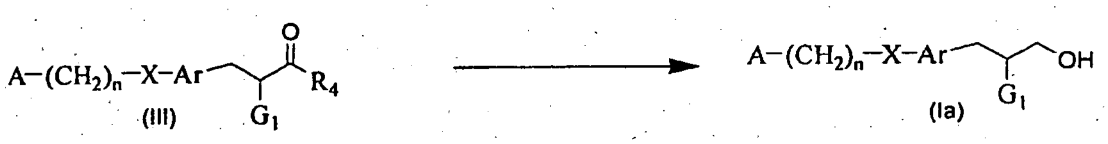

(III) (Ia)

ii. Umwandeln der Verbindung der Formel (Ia), die oben erhalten wurde, in Verbindungen der Formel (Ib), wenn gewünscht

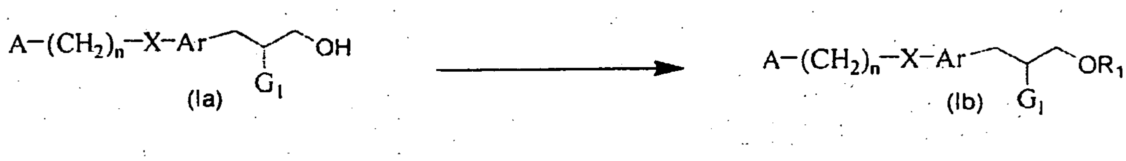

(Ia) (Ib)

iii. Umwandeln der Verbindung der Formel (Ib), die oben erhalten wurde, in Verbindungen der Formel (Ic), wenn gewünscht

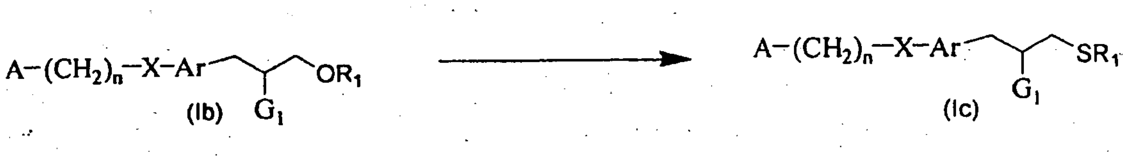

(Ib) (Ic)

iv. Umwandeln der Verbindung der Formel (Ib), die oben erhalten wurde, in Verbindungen der Formel (Id), wenn gewünscht

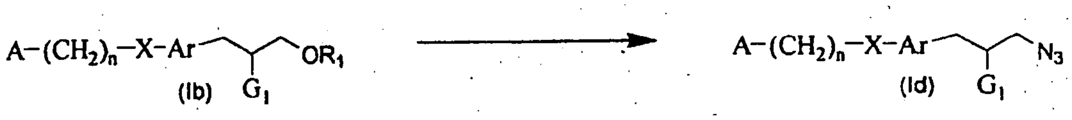

(Ib) (Id)

v) Umwandeln der Verbindung der Formel (Ib), die oben erhalten wurde, in Verbindungen der Formel (Ie), wenn gewünscht

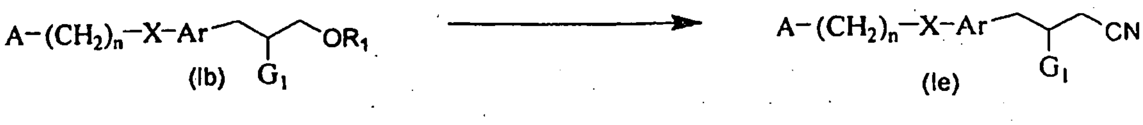

(Ib) (Ie)

b)

i. Umwandeln der Verbindung der Formel (III) in Verbindungen der Formel (If)

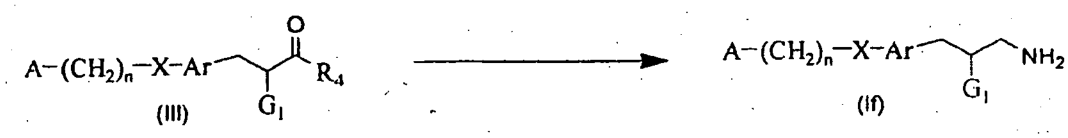

(III) (If)

ii. Umwandeln der Verbindung der Formel (If), die oben erhalten wurde, in eine Verbindung der Formel (Ig), wenn gewünscht

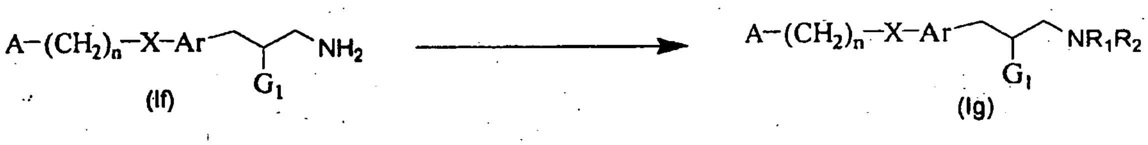

(If) (Ig)

iii. alternativ Umwandeln einer Verbindung der Formel (Id), die oben erhalten wurde, in eine Verbindung der Formel (If, wenn gewünscht

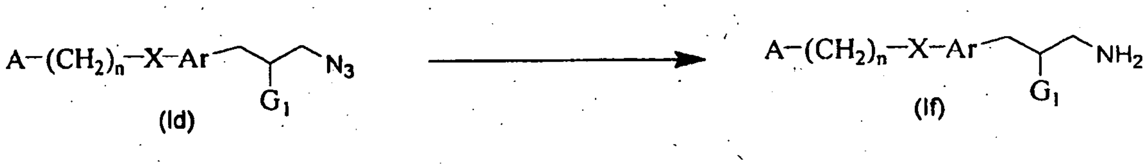

(Id) (If)

wobei Verbindungen der Formel (1b), (1c), (1d), (1e), (1f) und (1g) alle Verbindungen der Formel (I) darstellen, worin A, X, Ar, $G_1$, $R_1$, $R_2$ wie in Anspruch 1 definiert sind und $G_2$ OH, $OR_1$, $SR_1$, $N_3$, CN, $NH_2$ bzw. $NR_1R_2$ darstellt.

**7.** Verfahren zur Herstellung einer Verbindung der Formel (I), wie sie in Anspruch 1 beansprucht ist, umfassend einen beliebigen der unten angeführten Schritte allein oder in Kombination:

i. Umsetzen einer Verbindung der Formel (IV) mit Verbindungen der Formel (V)

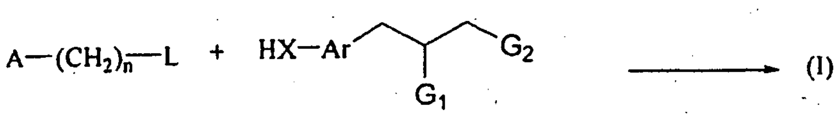

(IV) (V)

ii. Umsetzen einer Verbindung der Formel (IV) mit einer Verbindung der Formel- (Va) unter Erhalt einer Verbindung der Formel (Ig)

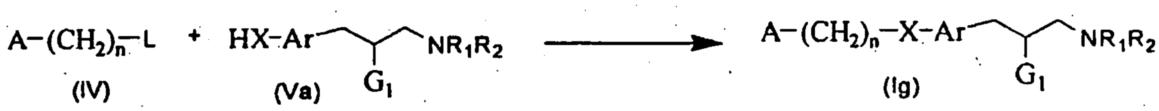

(IV) (Va) (Ig)

iii. Umsetzen einer Verbindung der Formel (IV) mit einer Verbindung der Formel (Vb) unter Erhalt einer Verbindung der Formel (Ib)

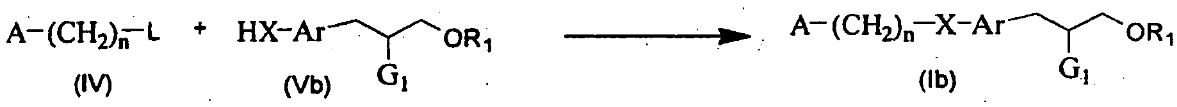

(IV) (Vb) (Ib)

iv. Umwandeln der Verbindung der Formel (Ib) in eine Verbindung der Formel (Ia)

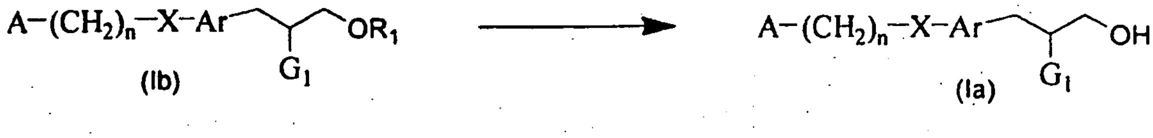

(Ib) (Ia)

worin Verbindungen der Formeln (1b), (1a), (1f) alle Verbindungen der Formel (I) darstellen, worin A, X, Ar, $G_1$, $R_1$, $R_2$ wie in Anspruch 1 beansprucht sind, "L" eine Abgangsgruppe, ausgewählt aus Halogen, Mesylat, Tosylat und Triflat, darstellt und $G_2$ OH, $OR_1$, $NR_1R_2$ darstellt.

**8.** Verbindung der Formel (IIIa), ihre tautomeren Formen, ihre Steroisomere, ihre pharmazeutisch verträglichen Salze, ihre pharmazeutisch verträglichen Solvate, worin "A" eine 4-Oxazolyl-Gruppe darstellt, substituiert mit einem oder zwei Substituenten, ausgewählt aus substituierten oder unsubstituierten, linearen oder verzweigten ($C_1$-$C_{12}$)-Alkyl-, substituierten oder unsubstituierten einzelnen oder kondensierten Heteroaryl- oder heterocyclischen Gruppen, mit der Maßgabe, dass einer der Substituenten an "A" immer eine Heteroaryl- oder heterocyclische Gruppe ist; "Ar" eine unsubstituierte Phenyl-Gruppe darstellt; $G_1$ $OR_1$ darstellt, worin $R_1$ substituierte oder unsubstituierte Gruppen, ausgewählt aus linearen oder verzweigten ($C_1$-$C_{12}$)-AlkylGruppen, darstellt; $R_4$ OH oder Alkoxy-Gruppen darstellt; "n" eine ganze Zahl von 1-3 ist; X für O steht.

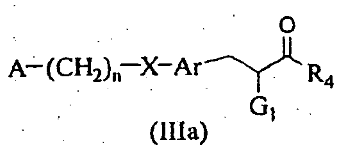

(IIIa)

**9.** Verbindung gemäß Anspruch 8, **dadurch gekennzeichnet, dass** die Substitutionen an den Substituenten an "A" ausgewählt sind aus Hydroxyl, Oxo, Halogen, Thio, Nitro, Amino, Cyano, Formyl oder substituierten oder unsubstituierten Gruppen, ausgewählt aus Amidino, guanidin, Hydrazino, Alkyl, Halogenalkyl, Perhalogenalkyl, Alkoxy, Halogenalkoxy, Perhalogenalkoxy, Alkenyl, Alkinyl, Cycloalkyl, Cycloalkenyl, Bicycloalkyl, Bicycloalkenyl, Alkoxy, Alkenoxy, Cycloalkoxy, Aryl, Aryloxy, Aralkyl, Aralkoxy, Heterocyclyl, Heteroaryl; Heterocyclylalkyl, Heteroaralkyl, Heteroaryloxy, Heteroaralkoxy, Heterocyclyloxy, Heterocyclylalkoxy, Heterocyclylalkoxyacyl, Acyl, Acyloxy, Acylamino, monosubstituiertem oder disubstituiertem Amino, Arylamino, Aralkylamino, Carbonsäure und ihren Derivaten, zum Beispiel Estern und Amiden, Carbonylamino, Hydroxyalkyl, Aminoalkyl, Alkoxyalkyl, Aryloxyalkyl, Aralkoxyalkyl, Alkylthio, Thioalkyl, Arylthio, Alkylsulfonylamino, Alkylsulfonyloxy, Alkoxycarbonylamino, Aryloxycarbonylamino, Aralkyloxycarbonylamino, Aminocarbonylamino, Alkylaminocarbonylamino, Alkoxyamino, Hydroxylamino, Sulfenylderivaten, Sulfonylderivaten, Sulfonsäure und ihren Derivaten, Phosphonsäure und ihren Derivaten.

**10.** Verbindungen gemäß Anspruch 8, ausgewählt aus
Ethyl-(2S)-ethoxy-3-[4-(5-methyl-2-thiophen-2-yl-oxazol-4-yl-methoxy)-phenyl]-propanoat;

Ethyl-(2S)-ethoxy-3-{4-[2-(5-methyl-2-thiophen-2-yl-oxazol-4-yl)-ethoxy]-phenyl}-propanoat;
Ethyl-(2S)-ethoxy-3-{4-[5-methyl-2-(5-methyl-thiophen-2-yl)-oxazol-4-yl-methoxy]-phenyl}-propanoat;
Ethyl-(2S)-ethoxy-3-(4-{2-[5-methyl-2-(5-methyl-thiophen-2-yl)-oxazol-4-yl]-ethoxy}-phenyl)-propanoat;
Ethyl-(2S)-ethoxy-3-{4-[5-methyl-2-(3-methyl-thiophen-2-yl)-oxazol-4-yl-methoxy]-phenyl}-propanoat;
Ethyl-(2S)-ethoxy-3-(4-{2-[5-methyl-2-(3-methyl-thiophen-2-yl)-oxazol-4-yl]-ethoxy}-phenyl)-propanoat;
Ethyl-(2S)-ethoxy-3-[4-(5-methyl-2-thiophen-3-yl-oxazol-4-yl-methoxy)-phenyl]-propanoat;
Ethyl-(2S)-ethoxy-3-{4-[2-(5-methyl-2-thiophen-3-yl-oxazol-4-yl)-ethoxy]-phenyl}-propanoat;
Ethyl-3-[4-(2-benzo[b]thiophen-2-yl-5-methyl-oxazol-4-yl-methoxy)-phenyl]-(2S)-ethoxy-propanoat;
Ethyl-3-{4-[2-(2-benzo[b]thiophen-2-yl-5-methyl-oxazol-4-yl)-ethoxy]-phenyl}-(2S)-ethoxy-propanoat;
Ethyl-(2S)-ethoxy-3-[4-(2-furan-2-yl-5-methyl-oxazol-4-yl-methoxy)-phenyl]-propanoat;
Ethyl-(2S)-ethoxy-3-{4-[2-(2-furan-2-yl-5-methyl-oxazol-4-yl)-ethoxy]-phenyl}-propanoat;
Ethyl-(2S)-ethoxy-3-[4-(5-methyl-2-chinolin-2-yl-oxazol-4-xyl-methoxy)-phenyl]-propanoat und seinen pharmazeutisch verträglichen Salzen;
Ethyl-(2S)-ethoxy-3-{4-[2-(5-methyl-2-chinolin-2-yl-oxazol-4-yl)-ethoxy]-phenyl}-propanoat und seinen pharmazeutisch verträglichen Salzen;
Ethyl-(2S)-ethoxy-3-{4-[3-(5-methyl-2-thiophen-2-yl-oxazol-4-yl)-propxy]-phenyl}-propanoat;
Ethyl-(2S)-ethoxy-3-{4-[5-methyl-2-(5-phenyl-thiophen-2-yl)-oxazol-4-yl-methoxy]-phenyl}-propanoat;
Ethyl-(2S)-ethoxy-3-{4-[5-methyl-2-(5-chlor-thiophen-2-yl)-oxazol-4-yl-methoxy]-phenyl}-propanoat;
Ethyl-(2S)-ethoxy-3-{4-[5-methyl-2-(5-brom-thiophen-2-yl)-oxazol-4-yl-methoxy]-phenyl}-propanoat;
Ethyl-(2S)-ethoxy-3-{4-[5-methyl-2-(5-methyl-furan-2-yl)-oxazol-4-yl-methoxy]phenyl}-propanoat;
Ethyl-(2S)-ethoxy-3-(4-{2-[5-methyl-2-(5-phenyl-thiophen-2-yl)-oxazol-4-yl]-ethoxy}-phenyl)-propanoat;
Ethyl-(2S)-ethoxy-3-(4-{2-[5-methyl-2-(5-chlor-thiophen-2-yl)-oxazol-4-yl]-ethoxy}-phenyl)-propanoat;
Ethyl-(2S)-ethoxy-3-(4-{2-[5-methyl-2-(5-brom-thiophen-2-yl)-oxazol-4-yl]-ethoxy}-phenyl)-propanoat;
Ethyl-(2S)-ethoxy-3-[4-(5-methyl-2-pyridin-2-yl-oxazol-4-yl-methoxy)-phenyl]-propanoat und seinen pharmazeutisch verträglichen Salzen;
Ethyl-(2S)-ethoxy-3-[4-(5-methyl-2-pyridin-4-yl-oxazol-4-yl-methoxy)-phenyl]-propanoat und seinen pharmazeutisch verträglichen Salzen;
Ethyl-(2S)-ethoxy-3-[4-{2-(5-methyl-2-pyridin-3-yl-oxazol-4-yl)-ethoxy}-phenyl]-propanoat und seinen pharmazeutisch verträglichen Salzen;
Ethyl-(2S)-ethoxy-3-[4-(5-methyl-2-pyridin-3-yl-oxazol-4-yl-methoxy)-phenyl]-propanoat und seinen pharmazeutisch verträglichen Salzen;
(2S)-Ethoxy-3-[4-(5-methyl-2-thiophen-2-yl-oxazol-4-yl-methoxy)-phenyl]-propansäure und ihren pharmazeutisch verträglichen Salzen;
(2S)-Ethoxy-3-{4-[2-(5-methyl-2-thiophen-2-yl-oxazol-4-yl)-ethoxy]-phenyl}-propansäure und ihren pharmazeutisch verträglichen Salzen;
(2S)-Ethoxy-3-{4-[5-methyl-2-(5-methyl-thiophen-2-yl)-oxazol-4-yl-methoxy]-phenyl}-propansäure und ihren pharmazeutisch verträglichen Salzen;
(2S)-Ethoxy-3-(4-{2-[5-methyl-2-(5-methyl-thiophen-2-yl)-oxazol-4-yl]-ethoxy}-phenyl)-propansäure und ihren pharmazeutisch verträglichen Salzen;
(2S)-Ethoxy-3-{4-[5-methyl-2-(3-methyl-thiophen-2-yl)-oxazol-4-yl-methoxy]-phenyl}-propansäure und ihren pharmazeutisch verträglichen Salzen;
(2S)-Ethoxy-3-(4-{2-[5-methyl-2-(3-methyl-thiophen-2-yl)-oxazol-4-yl]-ethoxy}-phenyl)-propansäure und ihren pharmazeutisch verträglichen Salzen;
(2S)-Ethoxy-3-[4-(5-methyl-2-thiophen-3-yl-oxazol-4-yl-methoxy)-phenyl]-propansäure und ihren pharmazeutisch verträglichen Salzen;
(2S)-Ethoxy-3-{4-[2-(5-methyl-2-thiophen-3-yl-oxazol-4-yl)-ethoxy]-phenyl}-propansäure und ihren pharmazeutisch verträglichen Salzen;
3-[4-(2-Benzo[b]thiophen-2-yl-5-methyl-oxazol-4-yl-methoxy)-phenyl]-(2S)-ethoxy-propansäure und ihren pharmazeutisch verträglichen Salzen;
3-{4-[2-(2-Benzo[b]thiophen-2-yl-5-methyl-oxazol-4-yl)-ethoxy]-phenyl}-(2S)-ethoxy-propansäure und ihren pharmazeutisch verträglichen Salzen;
(2S)-Ethoxy-3-[4-(2-furan-2-yl-5-methyl-oxazol-4-yl-methoxy)-phenyl]-propansäure und ihren pharmazeutisch verträglichen Salzen;
(2S)-Ethoxy-3-{4-[2-(2-furan-2-yl-5-methyl-oxazol-4-yl)-ethoxy]-phenyl}-propansäure und ihren pharmazeutisch verträglichen Salzen;
(2S)-Ethoxy-3-[4-(5-methyl-2-chinolin-2-yl-oxazol-4-yl-methoxy)-phenyl]-propansäure und ihren pharmazeutisch verträglichen Salzen;
(2S)-Ethoxy-3-{4-[2-(5-methyl-2-chinolin-2-yl-oxazol-4-yl)-ethoxy]-phenyl}-propansäure und ihren pharmazeutisch

verträglichen Salzen;
(2S)-Ethoxy-3-{4-[3-(5-methyl-2-thiophen-2-yl-oxazol-4-yl)-propoxy]-phenyl}-propansäure und ihren pharmazeutisch verträglichen Salzen;
(2S)-Ethoxy-3-{4-[5-methyl-2-benzofuran-2-yl)-oxazol-4-yl-methoxy]-phenyl}-propansäure und ihren pharmazeutisch verträglichen Salzen;
(2S)-Ethoxy-3-{4-[5-methyl-2-(5-chlor-thiophen-2-yl)-oxazol-4-yl-methoxy]-phenyl}-propansäure und ihren pharmazeutisch verträglichen Salzen;
(2S)-Ethoxy-3-{4-[5-methyl-2-(5-brom-thiophen-2-yl)-oxazol-4-yl-methoxy]-phenyl}-propansäure und ihren pharmazeutisch verträglichen Salzen;
(2S)-Ethoxy-3-{4-[5-methyl-2-(5-methyl-furan-2-yl)-oxazol-4-yl-methoxy]-phenyl}-propansäure und ihren pharmazeutisch verträglichen Salzen;
(2S)-Ethoxy-3-(4-{2-[5-methyl-2-(5-phenyl-thiophen-2-yl)-oxazol-4-yl]-ethoxy}-phenyl)-propansäure und ihren pharmazeutisch verträglichen Salzen;
(2S)-Ethoxy-3-(4-{2-[5-methyl-2-(5-chlor-thiophen-2-yl)-oxazol-4-yl]-ethoxy}-phenyl)-propansäure und ihren pharmazeutisch verträglichen Salzen;
(2S)-Ethoxy-3-(4-{2-[5-methyl-2-(5-brom-thiophen-2-yl)-oxazol-4-yl]-ethoxy}-phenyl)-propansäure und ihren pharmazeutisch verträglichen Salzen;
(2S)-Ethoxy-3-[4-{2-[5-methyl-2-(5-methyl-furan-2-yl)-oxazol-4-yl]-ethoxy}-phenyl)-propansäure und ihren pharmazeutisch verträglichen Salzen;
2(S)-Ethoxy-3-{4-(5-methyl-2-pyridin-2-yl-oxazol-4-yl-methoxy)-phenyl]-propansäure und ihren pharmazeutisch verträglichen Salzen;
2(S)-Ethoxy-3-[4-(5-methyl-2-pyridin-4-yl-oxazol-4-yl-methoxy)-phenyl]-propansäure und ihren pharmazeutisch verträglichen Salzen;
2(S)-Ethoxy-3-[4-(5-methyl-2-pyridin-3-yl-oxazol-4-yl-methoxy)-phenyl]-propansäure und ihren pharmazeutisch verträglichen Salzen.

**11.** Verbindungen, wie sie in einem der Ansprüche 8 bis 10 beansprucht sind, die als Intermediate zur Herstellung von Verbindungen der Formel (I) geeignet sind.

**12.** Verfahren zur Herstellung einer Verbindung der Formel (IIIa), wie sie in einem der Ansprüche 8 bis 10 beansprucht ist, umfassend

i. Umsetzen einer Verbindung der Formel (IVa), worin "A" eine 4-Oxazolyl-Gruppe darstellt, substituiert mit einem oder zwei Substituenten, ausgewählt aus substituierten oder unsubstituierten, linearen oder verzweigten ($C_1$-$C_{12}$)-Alkyl-, substituierten oder unsubstituierten einzelnen oder kondensierten Aryl- oder heterocyclischen Gruppen, mit der Maßgabe, dass einer der Substituenten an "A" immer eine Heteroaryl- oder heterocyclische Gruppe ist, und mit der weiteren Maßgabe, dass, wenn das Heteroaryl eine Pyryl-Gruppe ist, eine solche Gruppe unsubstituiert ist; "n" eine ganze Zahl von 1 bis 3 ist; und "L" eine Abgangsgruppe, ausgewählt aus Halogen, Mesylat, Tosylat und Triflat, darstellt, mit einer Verbindung der Formel (Vc), worin X Sauerstoff oder Schwefel darstellt; "Ar" unsubstituiertes Phenyl darstellt; $G_1$ $OR_1$ oder $SR_1$ darstellt, worin $R_1$ Wasserstoff, Perfluor ($C_1$-$C_{12}$)-Alkyl, substituierte oder unsubstituierte Gruppen, ausgewählt, aus linearen oder verzweigten ($C_1$-$C_{12}$)-Alkyl-, Cyclo($C_1$-$C_{12}$)-alkyl-, Aryl-, Ar($C_1$-$C_{12}$)-Alkyl-, Heteroaryl-, Heteroar($C_1$-$C_{12}$)-alkyl-, Heterocyclyl-, Alkoxyalkyl-, Arytoxyalkyl-, Alkoxycarbonyl-, Aryloxycarbonyl-, Cycloalkyloxycarbonyl-, Alkylaminocarbonyl-, Arylaminocarbonyl- oder Acyl-Gruppen, darstellt; $R_4$ OH, Alkoxy- oder Aryloxy-, Aralkoxy- oder $NR_1R_2$-Gruppen darstellt, wobei $R_1$ und $R_2$ gleich oder unterschiedlich sein können und Wasserstoff, substituierte oder unsubstituierte Gruppen darstellen, ausgewählt aus linearen oder verzweigten ($C_1$-$C_8$)-Alkyl-, ($C_3$-$C_7$)-Cycloalkyl-, Acyl-, Aryl-, Heteroaryl-, Heterocyclyl-, Aminocarbonyl-, Aralkyl-, Alkylaminocarbonyl-, Arylaminocarbonyl-, Aralkylaminocarbonyl-, Heteroarylaminocarbonyl-, Heteroaralkylaminocarbonyl-, Heterocyclylaminocarbonyl-, Alkoxycarbonyl-, Aryloxycarbonyl-, Aralkyloxycarbonyl-, Heteroaryloxycarbonyl-, Heteroaralkoxycarbonyl-, Heterocyclooxycarbonyl-Gruppen oder $SO_2R_3$, worin $R_3$ substituierte oder unsubstituierte Gruppen darstellt, ausgewählt aus Alkyl-, Aryl-, Polyhalogenalkyl-, Heterocyclyl-, Hetero-

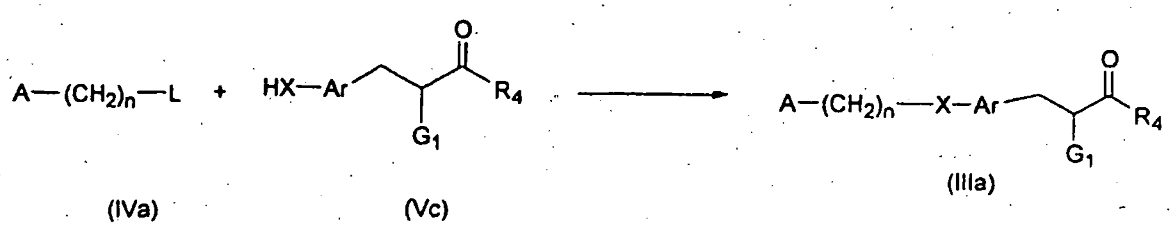

(IVa) (Vc) (IIIa)

ii. gegebenenfalls Hydrolysieren der Verbindung der Formel (IIIa), worin $R_4$ Alkoxy-, Aryloxy-, Aralkoxy- oder $NR_1R_2$-Gruppen darstellt, worin $R_1$ und $R_2$ wie vorstehend definiert sind, zu einer weiteren Verbindung der Formel (IIIa), worin $R_4$ für OH steht.

**13.** Pharmazeutische Zusammensetzung, welche Verbindungen der Formel (I) oder (IIIa), wie sie in einem vorangehenden Anspruch beansprucht sind, und einen pharmazeutisch verträglichen Träger, ein pharmazeutisch verträgliches Verdünnungsmittel, pharmazeutisch verträgliche Exzipientien oder ein pharmazeutisch verträgliches Solvat umfasst.

**14.** Pharmazeutische Zusammensetzung gemäß Anspruch 13 in der Form einer Tablette, einer Kapsel, eines Pulvers, eines Granulats, eines Sirups, einer Lösung oder einer Suspension.

**15.** Pharmazeutische Zusammensetzung gemäß Anspruch 13 oder Anspruch 14 in Kombination mit Sulfonylharnstoff, Biguanid, Angiotensin II-Inhibitor, Aspirin, a-Glycosidase-Inhibitor, Insulin-Sekretagogum, Insulin, β-Sitosterol-Inhibitor, HMG-CoA-Reduktase-Inhibitor, Fibrat, Nikotinsäure, Cholestyramin, Cholestipol oder Probucol, welche zusammen oder innerhalb eines solchen Zeitraums, dass sie zusammen synergistisch wirken, an einen Patienten, der Bedarf dafür hat, verabreicht werden können.

**16.** Verwendung einer Verbindung der Formel (I) oder (IIIa), wie sie in einem der Ansprüche 1 bis 5 oder 9 bis 11 definiert ist, zur Herstellung eines Medikaments zur Verwendung bei der Verringerung von Plasmaglucose, Triglyceriden, Gesamt-Cholesterin, LDL, VLDL oder freien Fettsäuren im Plasma eines Patienten, während gegebenenfalls die HDL-Cholesterin-Spiegel erhöht werden.

**17.** Verwendung gemäß Anspruch 16, wobei die Verbindung der Formel (I) oder (IIIa) zur Herstellung eines Medikaments bestimmt ist, das in Kombination mit HMG-CoA-Reduktase-Inhibitor, Fibrat, Nikotinsäure, Cholestyramin, Cholestipol oder Probucol zu geben ist, wobei das zusätzliche Ingrediens zusammen mit dem Medikament oder innerhalb eines solchen Zeitraums, dass es synergistisch zusammen mit dem Medikament wirkt, verabreicht werden kann.

**18.** Verwendung einer Verbindung der Formel (I) oder (IIIa), wie sie in einem der Ansprüche 1 bis 5 oder 9 bis 11 definiert ist, zur Prävention oder Behandlung von Erkrankungen, verursacht durch Hyperlipidämie, Hypercholesterinämie, Hyperglykämie, Adipositas, verschlechterte Glucose-Toleranz, Leptin-Resistenz, Insulin-Resistenz oder diabetische Komplikationen.

**19.** Verwendung einer Verbindung der Formel (I) oder (IIIa), wie sie in einem der Ansprüche 1 bis 5 oder 9 bis 11 definiert ist, zur Herstellung eines Medikaments zur Behandlung einer Erkrankung, bei der Insulin-Resistenz der zugrunde liegende pathophysioloische Mechanismus ist, umfassend Typ 2-Diabetes, verschlechterte Glucose-Toleranz, Dyslipidämie, Hypertension, Adipositas, Artheriosklerose, Hyperlipidämie, Koronararterien-Erkrankung, kardiovaskuläre Störungen, Nieren-Erkrankungen, Mikroalbuminurie, Glomerulonephritis, Glomerulosklerose, nephrotisches Syndrom, hypertensive Nephrosklerose, diabetische Retinopathie, diabetische Nephropathie, Endothelialzell-Dysfunktion, Psoriasis, polycystisches Ovarial-Syndrom (PCOS), Demenz, Nieren-Erkrankung im Endstadium, Osteoporose, entzündliche Darm-Erkrankungen, myotonische Dystrophie, Pankreatitis, Artheriosklerose, Xanthom oder Krebs.

**20.** Verwendung nach einem der Ansprüche 16 bis 19, wobei das Medikament einen pharmazeutisch verträglichen Träger, ein pharmazeutisch verträgliches Verdünnungsmittel oder pharmazeutisch verträgliche Exzipientien oder ein pharmazeutisch verträgliches Solvat enthält.

## Revendications

1. Composé de la formule générale (I),

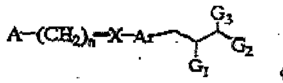

(I)

ses formes tautomères, ses stéréoisomères, ses sels pharmaceutiquement acceptables, ses solvates pharmaceutiquement acceptables où « A » représente un groupe substitué ou non substitué choisi parmi les groupes hétéroaryle, hétérocyclyle ; « n » est un entier de 1-3 ; « X » représente oxygène ; « Ar » représente un groupe phényle substitué ou non substitué ; $G_1$ représente les groupes $OR_1$, $SR_1$, $S(O)R_3$, $S(O)_2R_3$, $N_3$, CN, COOH, tétrazolyle ; $G_2$ représente les groupes $OR_1$, $NR_1R_2$, $SR_1$, $S(O)R_3$, $S(O)_2R_3$, $N_3$, CN, COOH, tétrazolyle ; $R_1$, $R_2$ représentent hydrogène, des groupes substitués ou non substitués choisis parmi les groupes alkyle en $C_1$-$C_8$, acyle, alcoxycarbonyle, aryloxycarbonyle, aralkyloxycarbonyle, linéaires ou ramifiés ; $R_3$ représente des groupes substitués ou non substitués choisis parmi les groupes alkyle, aryle ; à la condition que, lorsque $G_2$ représente $NR_1R_2$, $G_1$ ne représente pas un groupe -OH ; $G_3$ représente hydrogène ou des groupes alkyle en $C_1$-$C_8$.

2. Composé selon la revendication 1, **caractérisé par le fait que** les substituants sur « A », $R_1$, $R_2$ et $R_3$ peuvent être identiques ou différents et sont indépendamment choisis parmi hydroxyle, oxo, halo, thio, nitro, amino, cyano, formyle, ou des groupes substitués ou non substitués choisis parmi alkyle, haloalkyle, perhaloalkyle, alcoxy, aryle, aryloxy, aralkyle, aralcoxy, hétérocyclyle, hétéroaryle, acyle, acyloxy, acylamino, des groupes amino, carbonylamino, hdyroxyalkyle, alkylthio, thioalkyle monosubstitués ou disubstitués.

3. Composé selon la revendication 1, **caractérisé par le fait que** des substituants appropriés sur n'importe quel substituant de « A » peuvent être identiques ou différents et sont indépendamment choisis parmi hydroxyle, oxo, halo, thio, nitro, amino, cyano, formyl, ou des groupes substitués ou non substitués choisis parmi amidino, guanidino, hydrazino, alkyle, haloalkyle, perhaloalkyle, alcoxy, haloalcoxy, perhaloalcoxy, alcényle, alcynyle, cycloalkyle, cycloalcényle, bicycloalkyle, bicycloalcényle, alcoxy, alcénoxy, cycloalcoxy, aryle, aryloxy, aralkyle, aralcoxy, hétérocyclyle, hétéroaryle, hétérocyclylalkyle, hétéroaralkyle, hétéroaryloxy, hétéroaralcoxy, hétérocyclyloxy, hetérocyclylalcoxy, hétérocyclylalcoxyacyle, acyle, acyloxy, acylamino, amino monosubstitué ou disubstitué, arylamino, aralkylamino, acide carboxylique et ses dérivés tels que les esters et les amides, carbonylamino, hydroxyalkyle, aminoalkyle, alcoxyalkyle, aryloxyalkyle, aralcoxyalkyle, alkylthio, thioalkyle, arylthio, alkylsulfonylamino, alkylsulfonyloxy, alcoxycarbonylamino, aryloxycarbonylamino, aralkyloxycarbonylamino, aminocarbonylamino, alkylaminocarbonylamino, alcoxyamino, hydroxyl amino, des dérivés de sulfényle, des dérivés de sulfonyl, acide sulfonique et ses dérivés, acide phosphonique et ses dérivés.

4. Composé selon la revendication 1, **caractérisé par le fait que** les substituants sur le groupe représenté par « Ar » représentent alkyle, alcoxy, thioalkyle, halogène, haloalkyle, haloalcoxy, acyle, amino, acylamino, thio, ou acides carboxyliques ou sulfoniques et leurs dérivés, acide phosphonique et ses dérivés, linéaires ou ramifiés, substitués ou non substitués.

5. Composés selon la revendication 1, choisis parmi
le 3-(6-benzyloxy-naphtalén-2-yl)-2-éthoxy-propan-1-ol ;
le (2S)-éthoxy-3-{4-[2-(5-méthyl-2-phényl-oxazol-4-yl)-éthoxy]-phényl}-propan-1-ol ;
le (2S)-éthoxy-3-{4-(4-hydroxy-3-méthyl-3,4-dihydroquinazolin-2-yl-méthoxy)-phényl}-propan-1-ol ;
le 2-hydroxy-3-{4-[2-(5-méthyl-2-phényl-oxazol-4-yl)-éthoxy]-phényl}-propan-1-ol ;
le 3-{4-[2-(2,3-dihydro-benzo[1,4]thiazin-4-yl)-éthoxy]-phényl}-(2S)-éthoxy-propan-1-ol ;
le (2S)-éthoxy-3-[4-(2-(phénoxazin-10-yl)-éthoxy)-phényl]-propan-1-ol ;
le 3-[4-(2-(carbazol-9-yl)-éthoxy)-phényl]-(2S)-éthoxy-propan-1-ol ;
le 3-{4-[2-(3,4-dihydro-2h-quinolin-1-yl)-éthoxy]-phényl)-(2S)-éthoxy-propan-1-ol ;
le (2S)-éthoxy-3-[4-(2-(indol-1-yl)-éthoxy)-phényl]-propan-1-ol ;
le (2S)-éthoxy-3-[4-(2-(phénothiazin-10-yl)-éthoxy)-phényl]-propan-1-ol ;
le 3-{4-[2-(2,3-dihydro-benzo[1,4]oxazin-4-yl)-éthoxy]-phényl}-(2S)-éthoxy-propan-1-ol ;
le 3-{4-[2-(5-méthyl-2-phényl-oxazol-4-yl)-éthoxy]-phényl}-2-phénylsulfanyl-propan-1-ol;
le (2S)-éthoxy-3-{4-[2-(méthyl-pyridin-2-yl-amino)-éthoxy]-phényl}-propan-1-ol et ses sels pharmaceutiquement

acceptables ;
le (2S)-éthoxy-3-{4-[2-(5-éthyl-pyridin-2-yl)-éthoxy]-phényl}-propan-1-ol et ses sels pharmaceutiquement acceptables ;
le (2S)-éthoxy-3-[4-(5-méthyl-2-thiophén-2-yl-oxazol-4-ylméthoxy)-phényl]-propan-1-ol ;
le (2S)-éthoxy-3-{4-[2-(5-méthyl-2-thiophén-2-yl-oxazol-4-yl)-éthoxy]-phényl}-propan-1-ol ;
le (2S)-éthoxy-3-(4-{2-[2-méthyl-5-(4-méthylsulfanyl-phényl)-pyrrol-1-yl]-éthoxy}-phényl)-propan-1-ol ;
le (3S)-éthoxy-4-{4-[2-(5-méthyl-2-phényl-oxazol-4-yl)-éthoxy]-phényl}-butan-1-ol ;
le (2S)-éthoxy-3-(4-{2-[5-méthyl-2-(4-méthylsulfanyl-phényl)-oxazol-4-yl]-éthoxy}-phényl)-propan-1-ol ;
le (2S)-amino-3-{4-[2-(5-méthyl-2-phényl-oxazol-4-yl)-éthoxy]-phényl}-propan-1-ol et ses sels pharmaceutiquement acceptables ;
le (2S)-tert-butoxycarbonylamino-3-[4-(5-méthyl-2-phényl-oxazol-4-yl-méthoxy)-phényl]-propan-1-ol ;
le (2S)-tert-butoxycarbonylamino-3-{4-[2-(5-méthyl-2-phényl-oxazol-4-yl)-éthoxy]-phényl-propan-1-ol ;
le (2S)-éthoxy-3-(4-{2-[2-méthyl-5-(benzofuran-2-yl)-pyrrol-1-yl]-éthoxy}-phényl)-propan-1-ol ;
le (2S)-éthoxy-3-(4-{2-[2-méthyl-5-(benzo[1,3]dioxol-5-yl)-pyrrol-1-yl]-éthoxy}-phényl)-propan-1-ol ;
le (2S)-éthoxy-3-[4-(1-méthyl-1h-benzoimidazol-2-yl méthoxy)-phényl]-propan-1-ol ;
le (2S)-éthoxy-3-[4-(5-méthyl-3-phényl-isoxazol-4-ylméthoxy)-phényl]-propan-1-ol ;
le (2S)-éthoxy-3-{4-[2-(5-éthyl-pyridin-2-yl)-2-hydroxy-éthoxy]-phényl}-propan-1-ol ;
le (2S)-éthoxy-3-[4-(2-benzoimidazol-1-yl-éthoxy)-phényl]-propan-1-ol ;
le (2S)-éthoxy-3-[4-(5-méthyl-2-phényl-oxazol-4-ylméthoxy)-phényl]-propan-1-ol ;
le (2S)-éthoxy-3-(4-{2-[2-méthyl-5-(5-méthyl-thiophén-2-yl)-pyrrol-1-yl]-éthoxy}-phényl)-propan-1-ol ;
le (2S)-éthoxy-3-[4-(5-méthyl-2-phényl-oxazol-4-ylméthoxy)-phényl]-propan-1,2-diol ;
le 1-éthoxy-(2S)-éthoxy-3-[4-{2-(5-méthyl-2-phényl-oxazol-4-yl)-éthoxy}-phényl]-propane ;
le -2-((2S)-éthoxy-3-{4-[2-(S-méthyl-2-phényl-oxazol-4-yl)-éthoxy]-phényl}-propoxy)-éthanol ;
l'acide 2-((2S)-éthoxy-3-{4-[2-(5-méthyl-2-phényl-oxazol-4-yl)-éthoxy]-phényl}-propoxy)-benzoïque et ses sels pharmaceutiquement acceptables ;
le bromo acétate de (2S)-éthoxy-3-{4-[2-(5-méthyl-2-phényl-oxazol-4-yl)-éthoxy]-phényl}-propyl ;
le 1-éthoxy-(2S)-éthoxy-3-[4-{2-(3,4-dihydro-2h-benzo[1,4] thiazin-1-yl) éthoxy}phényl]-propane ;
le 1-propoxy-(2S)-éthoxy-3-[4-{2-(5-méthyl-2-phényl-oxazol-4-yl)éthoxy)-phényl]-propane ;
l'acide 2-((2S)-éthoxy-3-[4-(5-methyl-2-phényl-oxazol-4-ylméthoxy)-phényl]-propoxy)-benzoïque et ses sels pharmaceutiquement acceptables ;
le 1-éthoxy-(2S)-éthoxy-3-[4-(5-méthyl-2-phényl-oxazol-4-ylméthoxy)-phényl]-propane ;
le (2S)-éthoxy-3-[4-(5-méthyl-2-phényl-oxazol-4-yl-ylméthoxy)-phényl]-1-phénoxy propane ;
le (2S)-éthoxy-1-éthyl sulfinyl-3-{4-(5-méthyl-2-phényl-oxazol-4-ylméthoxy)-phényl}-propane ;
le (2S)-éthoxy-1-éthyl sulfanyl-3-{4-(5-méthyl-2-phényl-oxazol-4-ylméthoxy)-phényl}-propane ;
le (2S)-éthoxy-3-[4-(5-méthyl-2-phényl-oxazol-4-ylméthoxy)-phényl]-1-isopropoxy propane ;
le (3S)-éthoxy-4-[4-(5-méthyl-2-phényl-oxazol-4-ylméthoxy)-phényl]-butyronitrile ;
le (2S)-éthoxy-1H-tétrazole-3-[4-(5-méthyl-2-phényl-oxazol-4-ylméthoxy)-phényl]-propane ;
le 2-éthoxy-1-[4-(5-méthyl-2-phényl-oxazol-4-ylméthoxy)-phényl]-pentane-3-ol ;
le 2,3-diéthoxy-1-[4-(5-méthyl-2-phényl-oxazol-4-ylméthoxy)-phényl]-pentane ;
le 2-éthoxy-1-{4-[2-(5-méthyl-2-phényl-oxazol-4-yl)-éthoxy]-phényl}-pentane-3-ol ;
le 2,3-diéthoxy-1-{4-[2-(5-méthyl-2-phényl-oxazol-4-yl)-éthoxy]-phényl}-pentane ;
l'acide ((2S)-éthoxy-3-{4-[2-(5-méthyl-2-phényl-oxazol-4-yl)-éthoxy]-phényl}-propoxy)-acétique et ses sels pharmaceutiquement acceptables ;
le 3-(4-benzyloxy-phényl)-(2S)-éthoxy-propyl méthanesulfonate ;
le (2S)-éthoxy-3-[4-(5-méthyl-2-phényl-oxazol-4-ylméthoxy)-phényl]-propyl méthanesulfonate ;
le (2S)-éthoxy-3-{4-[2-(2-methyl-5-(5-méthyl-thiophen-2-yl)-pyrrol-1-yl)-éthoxy]-phényl}-propyl méthanesulfonate ;
le (2S)-éthoxy-3-{4-[2-(5-méthyl-2-phényl-oxazol-4-yl)-éthoxy]-phényl}-propyl méthanesulfonate ;
le (2S)-éthoxy-3-[4-(5-méthyl-2-thiophén-2-yl-oxazol-4-ylméthoxy)-phényl]}-propyl méthanesulfonate ;
le (2S)-éthoxy-3-{4-[2-(5-éthyl-pyridin-2-yl)-éthoxy]-phényl}-propyl methanesulfonate ;
le (2S)-éthoxy-1-méthoxy-3-{4-[2-(5-méthyl-2-thiophén-2-yl-oxazol-4-yl)-éthoxy]-phényl}-propane ;
le 1-éthoxy-(2S)-éthoxy-3-(4-{2-[5-méthyl-2-(4-méthylsulfanyl-phényl)-oxazol-4-yl]-éthoxy}-phényl)-propane ;
le 1-éthoxy-(2S)-éthoxy-3-{4-[2-(5-méthyl-2-thiophén-2-yl-oxazol-4-yl)-éthoxy]-phényl}-propane ;
le (2S)-éthoxy-3-[4-(5-méthyl-2-thiophén-2-yl-oxazol-4-ylméthoxy)-phényl]-1-éthoxy propane ;
le (2S)-éthoxy-3-{4-[2-(2-méthyl-5-benzofuran-2-yl-pyrrol-1-yl)-éthoxy]-phényl}-propyl méthanesulfonate ;
le (2S)-éthoxy-3-{4-[2-(2-méthyl-5-benzo[1,3]dioxol-5-yl-pyrrol-1-yl)-éthoxy]-phényl}-propyl méthanesulfonate ;
le (2S)-éthoxy-3-{4-[2-(2-méthyl-5-benzofuran-2-yl-pyrrol-1-yl)-éthoxy]-phényl}-propyl-(4-méthyl phényl)-sulfonate ;
le (2S)-éthoxy-3-{4-[2-(2-méthyl-5-benzo[1,3]dioxol-5-yl-pyrrol-1-yl)-éthoxy]-phényl}-propyl-(4-méthyl phényl)-

-sulfonate ;
le 1-éthoxy-(2S)-éthoxy-3-[4-(1-méthyl-1h-benzoimidazol-2-ylméthoxy)-phényl]-propane ;
le (2S)-éthoxy-3-{4-(5-méthyl-2-phényl-oxazol-4-ylméthoxy)-phényl}-1-propoxy propane ;
le 1-éthoxy-(2S)-éthoxy-3-[4-(5-méthyl-3-phényl-isoxazol-4-yl méthoxy)-phényl]-propane ;
le (2S)-tert-butoxycarbonylamino-3-[4-(5-méthyl-2-phényl-oxazol-4-ylméthoxy)-phényl]-propyl méthanesulfonate ;
le (2S)-éthoxy-3-{4-[2-(5-méthyl-2-phényl-oxazol-4-yl)-éthoxy]-phényl}-propyl amine et ses sels pharmaceutiquement acceptables ;
le (2S)-éthoxy-3-[4-{3-méthyl-3H-quinazolin-4-on-2yl)-méthoxy}phényl]propyl amine et ses sels pharmaceutiquement acceptables ;
le ((2S)-éthoxy-3-{4-[2-(5-méthyl-2-phényl-oxazol-4-yl)-éthoxy]-phényl}-propyl)-isopropyl-amine et ses sels pharmaceutiquement acceptables ;
le 3-{4-[2-(2,3-dihydro-benzo[1,4]thiazin-4-yl)-éthoxy]-phényl}-(2S)-éthoxy-propyl amine et ses sels pharmaceutiquement acceptables ;
le 3-(4-benzyloxy-phényl)-(2S)-éthoxy-propyl amine et ses sels pharmaceutiquement acceptables ;
le (2S)-éthoxy-3-[4-(5-méthyl-2-thiophén-2-yl-oxazol-4-ylméthoxy)-phényl]-propylamine et ses sels pharmaceutiquement acceptables ;
le (2S)-éthoxy-3-[4-(5-méthyl-2-phényl-oxazol-4-ylméthoxy)-phényl]-propylamine et ses sels pharmaceutiquement acceptables ;
le n-tert-butoxycarbonyl-(2S)-éthoxy-3-[4-(5-méthyl-2-phényl-oxazol-4-ylméthoxy)-phényl]-propyl amine ;
le (2S)-éthoxy-3-{4-[2-(2-méthyl-5-(5-méthyl-thiophén-2-yl)-pyrrol-1-yl)-éthoxy]-phényl}-propylamine et ses sels pharmaceutiquement acceptables ;
le n-((2S)-éthoxy-3-{4-(5-méthyl-2-phényl-oxazol-4-ylméthoxy)-phényl}-propyl)-méthane sulfonamide ;
le (2S)-éthoxy-3-[4-(5-méthyl-2-phényl-oxazol-4-ylméthoxy)-phényl]-propylamine et ses sels pharmaceutiquement acceptables ;
le [(2S)-éthoxy-3-[4-(5-méthyl-2-phényl-oxazol-4-ylméthoxy)-phényl]-propyl]-éthyl-amine et ses sels pharmaceutiquement acceptables ;
le [(2S)-éthoxy-3-[4-(5-méthyl-2-phényl-oxazol-4-ylméthoxy)-phényl]-propyl]-isopropyl-amine et ses sels pharmaceutiquement acceptables ;
le (2S)-éthoxy-3-{4-[2-(2-methyl-5-benzo[1,3]dioxol-5-yl-pyrrol-1-yl)-éthoxy]-phényl}-propylamine et ses sels pharmaceutiquement acceptables ;
le (2S)-éthoxy-3-{4-[2-(2-méthyl-5-benzofuran-2-yl-pyrrol-1-yl)-éthoxy]-phényl}-propylamine et ses sels pharmaceutiquement acceptables ;
le n-[(2S)-éthoxy-3-[4-(5-méthyl-2-phényl-oxazol-4-ylméthoxy)-phényl]-propyl]-2,2,2-trifluoro-acétamide ;
le n-éthoxycarbonyl-((2S)-éthoxy-3-{4-(5-méthyl-2-phényl-oxazol-4-ylméthoxy)-phényl}-propyl)amine ;
le n-benzyloxycarbonyl-((2S)-éthoxy-3-{4-(5-méthyl-2-phényl-oxazol-4-ylméthoxy)-phényl}-propyl)amine ;
le n-[(2S)-éthoxy-3-[4-(5-méthyl-2-phényl-oxazol-4-ylméthoxy)-phényl]-propyl]-acétamide ;
le (2S)-hydroxy-3-{4-[2-(5-méthyl-2-phényl-oxazol-4-yl)-éthoxy]-phényl}-propyl azide ;
le 3-(4-benzyloxy-phényl)-(2S)-éthoxy-propyl azide ;
le (2S)-éthoxy-3-{4-[2-(5-méthyl-2-phényl-oxazol-4-yl)-éthoxy]-phényl}-propyl azide ;
le (2S)-éthoxy-3-{4-[2-(5-éthyl-pyridin-2-yl)-éthoxy]-phényl}-propyl azide et ses sels pharmaceutiquement acceptables ;
le (2S)-éthoxy-3-[4-(5-méthyl-2-phényl-oxazol-4-ylméthoxy)-phényl]-propyl azide ;
le (2S)-éthoxy-3-{4-[2-(2-méthyl-5-(5-méthyl-thiophén-2-yl)-pyrrol-1-yl)-éthoxy]-phényl}-propyl azide ;
le (2S)-éthoxy-3-{4-[2-(5-éthyl-pyridine-2-yl)-2-(tert-butyldiméthyl-silanyloxy)-éthoxy]-phényl}-propyl azide et ses sels pharmaceutiquement acceptables ;
le (2S)-éthoxy-3-{4-[2-(5-éthyl-pyridine-2-yl)-2-hydroxy-éthoxy]-phényl}-propyl azide et ses sels pharmaceutiquement acceptables ;
le (2S)-éthoxy-3-{4-[2-(méthyl-pyridin-2-yl-amino)-éthoxy]-phényl}-propyl azide et ses sels pharmaceutiquement acceptables ;
le (2S)-éthoxy-3-[4-(5-méthyl-2-thiophén-2-yl-oxazol-4-ylméthoxy)-phényl]-propyl azide ;
le (2S)-éthoxy-3-{4-[2-(5-méthyl-2-thiophén-2-yl-oxazol-4-yl)-éthoxy]-phényl}-propyl azide ;
le (2S)-hydroxy-3-[4-(5-méthyl-2-phényl-oxazol-4-yl)-méthoxy)-phényl]-propyl azide ;
le (2S)-amino-3-{4-[2-(5-méthyl-2-phényl-oxazol-4-yl)-éthoxy]-phényl}-propyl azide et ses sels pharmaceutiquement acceptables ;
le (2S)-amino-3-{4-[2-(5-méthyl-2-phényl-oxazol-4-yl)-méthoxy]-phényl}-propyl azide et ses sels pharmaceutiquement acceptables ;
le (2S)-tert-butoxycarbonylamino-3-[4-(5-méthyl-2-phényl-oxazol-4-ylméthoxy)-phényl]-propyl azide ;
le (2S)-tert-butoxycarbonylamino-3-[4-(5-méthyl-2-phényl-oxazol-4-yl-éthoxy)-phényl]-propyl azide ;

le (2S)-éthoxy-3-{4-[2-(2-méthyl-5-benzo[1,3]dioxol-5-yl-pyrrol-1-yl)-éthoxy]-phényl}-propyl azide ;
le (2S)-éthoxy-3-{4-[2-(2-méthyl-5-benzofuran-2-yl-pyrrol-1-yl)-éthoxy]-phényl}-propyl azide ;
le n-benzyloxycarbonyl-(2S)-éthoxy-3-{4-[2-(5-méthyl-2-phényl-oxazol-4-yl)-éthoxy]-phényl)-propyl amine ;
le n-tert-butoxycarbonyl-(2S)-éthoxy-3-{4-[2-(5-méthyl-2-phényl-oxazol-4-yl)-éthoxy]-phényl}-propyl amine ;
le n-tert-butoxycarbonyl-3-{4-[2-(2,3-dihydro-benzo[1,4]thiazin-4-yl)-éthoxy]-phényl}-(2S)-éthoxy-propyl amine ;
le n-((2S)-éthoxy-3-{4-[2-(5-méthyl-2-phényl-oxazol-4-yl)-éthoxy]-phényl}-propyl)-acétamide ;
le 3-(4-benzyloxy-phényl)-n-tert-butoxycarbonyl-(2S)-éthoxy-propyl amine ;
le n-tert-butoxycarbonyl-(2S)-éthoxy-3-(4-hyd-roxy-phényl)-propyl amine ;
le n-tert-butoxycarbonyl-(2S)-éthoxy-3-{4-[2-(5-éthyl-pyridin-2-yl)-éthoxy]-phényl}-propyl amine ;
le (2S)-éthoxy-1-éthylsulfanyl-3-{{4-[2-(5-méthyl-2-phényl-oxazol-4-yl)-éthoxy]-phényl}-propane ;
le (2S)-éthoxy-1-éthylsulfonyl-3-{4-[2-(5-méthyl-2-phényl-oxazol-4-yl)-éthoxyl-phényl}-propane.

**6.** Procédé de préparation des composés de formule (I) tel que définis à la revendication 1, comprenant l'une quelconque des étapes ci-dessous seule ou en combinaison :

a)

i. convertir un composé de formule (III) en un composé de formule (1a)

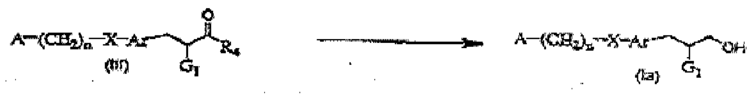

ii. convertir le composé de formule (1a) obtenu ci-dessus en des composés de formule (1b), si souhaité

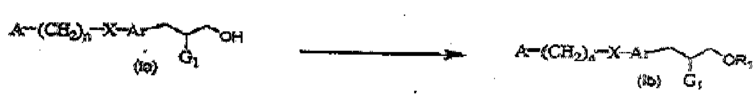

iii. convertir le composé de formule (1b) obtenu ci-dessus en des composés de formule (1c), si souhaité

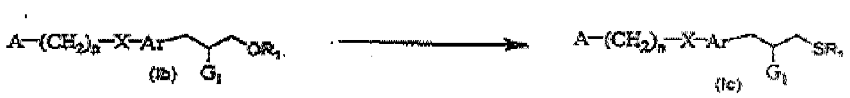

iv. convertir le composé de formule (1b) obtenu ci-dessous en des composés de formule (1d), si souhaité

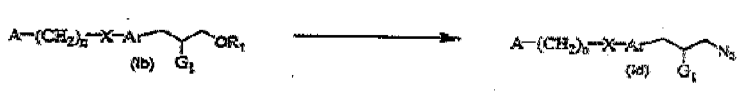

v. convertir le composé de formule (1b) obtenu ci-dessus en des composés de formule (1e), si souhaité

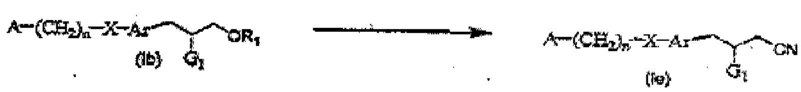

b)

i. convertir le composé de formule (III) en des composés de formule (1f)

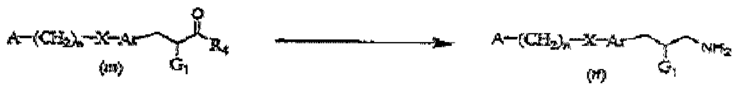

ii. convertir le composé de formule (1f) obtenu ci-dessus en un composé de formule (1g), si souhaité

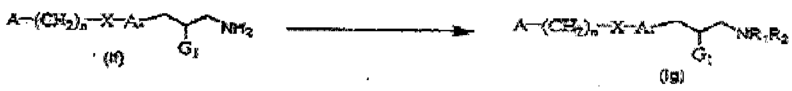

iii. en variante, convertir le composé de formule (1d) obtenu ci-dessus, en un composé de formule (1f), si souhaité

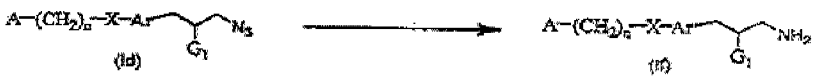

les composés de formule (1b), (1c), (1d), (1e), (1f) & (1g) représentant tous des composés de formule (I) où A, X, Ar, $G_1$, $R_1$, $R_2$ sont tels que définis à la revendication 1 et $G_2$ représente respectivement OH, $OR_1$, $SR_1$, $N_3$, CN, $NH_2$, $NR_1R_2$.

**7.** Procédé pour la préparation d'un composé de formule (I), tel que défini à la revendication 1, comprenant l'une quelconque des étapes ci-dessous seule ou en combinaison :

i. faire réagir un composé de formule (IV), avec des composés de formule (V)

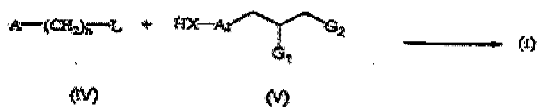

ii. faire réagir un composé de formule (IV) avec un composé de formule (Va) pour obtenir un composé de formule (Ig)

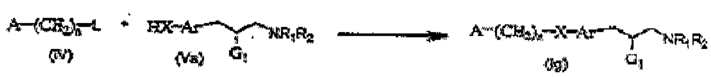

iii. faire réagir un composé de formule (IV) avec un composé de formule (Vb) pour obtenir un composé de formule (Ib)

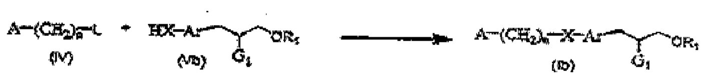

iv. convertir le composé de formule (Ib) en un composé de formule (Ia)

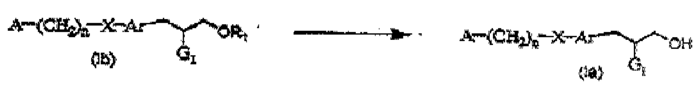

les composés de formule (1b), (1a), (1f) représentant tous des composés de formule (I) où A, X, Ar, $G_1$, $R_1$, $R_2$ sont tels que définis à la revendication 1, « L » représente un groupe partant choisi parmi halogène, mésylate, tosylate & triflate et $G_2$ représente OH, $OR_1$, $NR_1R_2$.

**8.** Composé de formule (IIIa), ses formes tautomères, ses stéréisomères, ses sels pharmaceutiquement acceptables, ses solvates pharmaceutiquement acceptables, où « A » représente un groupe 4-oxazolyle substitué par un ou deux substituants choisis parmi les groupes alkyle en $C_1$-$C_{12}$ linéaires ou ramifiés, substitués ou non substitués, les groupes hétéroaryle ou hétérocycliques individuels ou fusionnés, substitués ou non substitués, à la condition que l'un des substituants sur « A » soit toujours un groupe hétéroaryle ou hétérocyclique ; « Ar » représente un groupe phényle non substitué ; $G_1$ représente $OR_1$, où $R_1$ représente des groupes substitués ou non substitués choisis parmi les groupes alkyle en $C_1$-$C_{12}$ linéaires ou ramifiés ; $R_4$ représente OH ou des groupes alcoxy ; « n » est un entier de 1-3 ; X représente O.

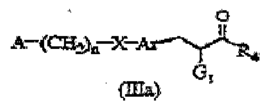

(IIIa)

**9.** Composé selon la revendication 8, **caractérisé par le fait que** les substitutions sur les substituants sur « A » sont choisies parmi hydroxyle, oxo, halo, thio, nitro, amino, cyano, formyle, ou des groupes substitués ou non substitués choisis parmi amidino, guanidino, hydrazino, alkyle, haloalkyle, perhaloalkyle, alcoxy, haloalcoxy, perhaloalcoxy, alcényle, alcynyle, cycloalkyle, cycloalcényle, bicycloalkyle, bicycloalcényle, alcoxy, alcénoxy, cycloalcoxy, aryle, aryloxy, aralkyle, aralcoxy, hétérocyclyle, hétéroaryle, hétérocyclylalkyle, hétéroaralkyle, hétéroaryloxy, hétéroaralcoxy, hétérocyclyloxy, hétérocyclylalcoxy, hétérocyclylalcoxyacyle, acyle, acyloxy, acylamino, amino monosubstitué ou disubstitué, arylamino, aralkylamino, acide carboxylique et ses dérivés tels que les esters et les amides, carbonylamino, hydroxyalkyle, aminoalkyle, alcoxyalkyle, aryloxyalkyle, aralcoxyalkyle, alkylthio, thioalkyle, arylthio, alkylsulfonylamino, alkylsulfonyloxy, alcoxycarbonylamino, aryloxycarbonylamino, aralkyloxycarbonylamino, aminocarbonylamino, alkylaminocarbonylamino, alcoxyamino, hydroxyl amino, des dérivés de sulfényle, des dérivés de sulfonyle, acide sulfonique et ses dérivés, acide phosphonique et ses dérivés.

**10.** Composés selon la revendication 8, choisis parmi
le (2S)-éthoxy-3-[4-(5-méthyl-2-thiophén-2-yl-oxazol-4-ylméthoxy)-phényl]-propanoate d'éthyle ;
le (2S)-éthoxy-3-{4-[2-(5-méthyl-2-thiophén-2-yl-oxazol-4-yl)-éthoxy]-phényl}-propanoate d'éthyle ;
le (2S)-éthoxy-3-{4-[5-méthyl-2-(5-méthyl-thiophén-2-yl)-oxazol-4-ylméthoxy]-phényl}-propanoate d'éthyle ;
le (2S)-éthoxy-3-(4-{2-[5-méthyl-2-(5-méthyl-thiophén-2-yl)-oxazol-4-yl]-éthoxy}-phényl)-propanoate d'éthyle ;
le (2S)-éthoxy-3-{4-[5-méthyl-2-(3-méthyl-thiophén-2-yl)-oxazol-4-ylméthoxy]-phényl}-propanoate d'éthyle ;
le (2S)-éthoxy-3-(4-{2-[5-méthyl-2-(3-méthyl-thiophén-2-yl)-oxazol-4-yl]-éthoxy}-phényl)-propanoate d'éthyle;
le (2S)-éthoxy-3-[4-(5-méthyl-2-thiophén-3-yl-oxazol-4-ylméthoxy)-phényl]-propanoate d'éthyle ;
le (2S)-éthoxy-3-{4-[2-(5-méthyl-2-thiophén-3-yl-oxozol-4-yl)-éthoxy]-phényl}-propanoate d'éthyle ;
le 3-[4-(2-benzo[b]thiophén-2-yl-5-méthyl-oxazol-4-ylméthoxy)-phényl]-(2S)-éthoxy-propanoate d'éthyle ;
le 3-{4-[2-(2-benzo[b]thiophén-2-yl-5-méthyl-oxazol-4-yl)-éthoxyl-phényl]-(2S)-éthoxy-propanoate d'éthyle ;
le (2S)-éthoxy-3-[4-(2-furan-2-yl-5-méthyl-oxazol-4-ylméthoxy)-phényl]-propanoate d'éthyle ;
le (2S)-éthoxy-3-{4-[2-(2-furan-2-yl-5-méthyl-oxazol-4-yl)-éthoxy]-phényl}-propanoate d'éthyle ;
le (2S)-éthoxy-3-[4-(5-méthyl-2-quinolin-2-yl-oxazol-4-ylméthoxy)-phényl]-propanoate d'éthyle et ses sels pharmaceutiquement acceptables ;
le (2S)-éthoxy-3-{4-[2-(5-méthyl-2-quinolin-2-yl-oxazol-4-yl)-éthoxy]-phényl}-propanoate d'éthyle et ses sels pharmaceutiquement acceptables ;
le (2S)-éthoxy-3-{4-[3-(5-méthyl-2-thiophén-2-yl-oxazol-4-yl)-propoxy]-phényl}-propanoate d'éthyle ;
le (2S)-éthoxy-3-{4-[5-méthyl-2-(5-phényl-thiophén-2-yl)-oxazol-4-ylméthoxy]-phényl}-propanoate d'éthyle ;
le (2S)-éthoxy-3-{4-[5-méthyl-2-(5-chloro-thiophén-2-yl)-oxazol-4-ylméthoxy]-phényl}-propanoate d'éthyle ;
le (2S)-éthoxy-3-{4-[5-méthyl-2-(5-bromo-thiophén-2-yl)-oxazol-4-ylméthoxy]-phényl}-propanoate d'éthyle ;
le (2S)-éthoxy-3-{4-[5-méthyl-2-(5-méthyl-furan-2-yl) - oxazol-4-ylméthoxy]-phényl}-propanoate d'éthyle ;
le (2S)-éthoxy-3-(4-{2-[5-méthyl-2-(5-phényl-thiophén-2-yl)-oxazol-4-yl]-éthoxy}-phényl)-propanoate d'éthyle ;
le (2S)-éthoxy-3-(4-{2-[5-méthyl-2-(5-chloro-thiophén-2-yl)-oxazol-4-yl]-éthoxy}-phényl)-propanoate d'éthyle ;
le (2S)-éthoxy-3-(4-{2-[5-méthyl-2-(5-bromo-thiophén-2-yl)-oxazol-4-yl]-éthoxy}-phényl)-propanoate d'éthyle :
le (2S)-éthoxy-3-[4-(5-méthyl-2-pyridin-2-yl-oxazol-1-ylméthoxy)-phényl]-propanoate d'éthyle et ses sels pharmaceutiquement acceptables ;
le (2S)-éthoxy-3-[4-(5-méthyl-2-pyridin-4-yl-oxazol-4-ylméthoxy)-phényl]-propanoate d'éthyle et ses sels pharmaceutiquement acceptables ;
le (2S)-éthoxy-3-[4-{2-(5-méthyl-2-pyridin-3-yl-oxazol-4-yl)-éthoxy}-phényl]-propanoate d'éthyle et ses sels pharmaceutiquement acceptables ;
le (2S)-éthoxy-3-[4-(5-méthyl-2-pyridin-3-yl-oxazol-4-ylméthoxy)-phényl]-propanoate d'éthyle et ses sels pharmaceutiquement acceptables ;
l'acide (2S)-éthoxy-3-[4-(5-méthyl-2-thiophén-2-yl-oxazol-4-ylméthoxy)-phényl]-propanoïque et ses sels pharmaceutiquement acceptables ;
l'acide (2S)-éthoxy-3-{4-[2-(5-méthyl-2-thiophén-2-yl-oxazol-4-yl)-éthoxy]-phényl}-propanoïque et ses sels pharmaceutiquement acceptables ;
l'acide (2S)-éthoxy-3-{4-[5-méthyl-2-(5-méthyl-thiophén-2-yl)-oxazol-4-ylméthoxy]-phényl}-propanoïque et ses sels pharmaceutiquement acceptables ;
l'acide (2S)-éthoxy-3-(4-{2-[5-méthyl-2-(5-méthyl-thiophén-2-yl)-oxazol-4-yl]-éthoxy}-phényl)-propanoïque et ses sels pharmaceutiquement acceptables ;
l'acide (2S)-éthoxy-3-{4-[5-méthyl-2-(3-méthyl-thiophén-2-yl)-oxazol-4-ylméthoxy]-phényl}-propanoïque et ses sels pharmaceutiquement acceptables ;
l'acide (2S)-éthoxy-3-(4-{2-[5-méthyl-2-(3-méthyl-thiophén-2-yl)-oxazol-4-yl]-éthoxy}-phényl)-propanoique et ses sels pharmaceutiquement acceptables ;
l'acide (2S)-éthoxy-3-[4-(5-méthyl-2-thiophén-3-yl-oxazol-4-ylméthoxy) -phényl] -propanoïque et ses sels pharmaceutiquement acceptables ;
l'acide (2S)-éthoxy-3-{4-[2-(5-méthyl-2-thiophén-3-yl-oxazol-4-yl)-éthoxy]-phényl}-propanoïque et ses sels pharmaceutiquement acceptables ;
l'acide 3-[4-(2-benzo[b]thiophén-2-yl-5-méthyl-oxazol-4-ylméthoxy)-phényl]-(2S)-éthoxy-propanoïque et ses sels pharmaceutiquement acceptables ;
l'acide 3-{4-[2-(2-benzo[b]thiophén-2-yl-5-méthyl-oxazol-4-yl)-éthoxy]-phényl}-(2S)-éthoxy-propanoïque et ses sels pharmaceutiquement acceptables ;
l'acide (2S)-éthoxy-3-[4-(2-furan-2-yl-5-méthyl-oxazol-4-ylméthoxy)-phényl]-propanoïque et ses sels pharmaceutiquement acceptables ;
l'acide (2S)-éthoxy-3-{4-[2-(2-furan-2-yl-5-méthyl-oxazol-4-yl)-éthoxy]-phényl}-propanoïque et ses sels pharmaceutiquement acceptables ;
l'acide (2S)-éthoxy-3-[4-(5-méthyl-2-quinolin-2-yl-oxazol-4-ylméthoxy)-phényl]-propanoïque et ses sels pharmaceutiquement acceptables ;
l'acide (2S)-éthoxy-3-{4-[2-(5-méthyl-2-quinolin-2-yl-oxazol-4-yl)-éthoxy]-phényl}-propanoïque et ses sels pharmaceutiquement acceptables ;
l'acide (2S)-éthoxy-3-{4-[3-(5-méthyl-2-thiophén-2-yl-oxazol-4-yl)-propoxy]-phényl}-propanoïque et ses sels pharmaceutiquement acceptables ;
l'acide (2S)-éthoxy-3-{4-[5-méthyl-2-benzofuran-2-yl)-oxazol-4-ylméthoxy]-phényl}-propanoïque et ses sels pharmaceutiquement acceptables ;
l'acide (2S)-éthoxy-3-{4-[5-méthyl-2-(5-chloro-thiophén-2-yl)-oxazol-4-ylméthoxy]-phényl}-propanoïque et ses sels pharmaceutiquement acceptables ;
l'acide (25)-éthoxy-3-{4-[5-méthyl-2-(5-bromo-thiophén-2-yl)-oxazol-4-ylméthoxy]-phényl}-propanoïque et ses sels pharmaceutiquement acceptables ;
l'acide (2S)-éthoxy-3-{4-[5-méthyl-2-(5-méthyl-furan-2-yl)-oxazol-4-ylméthoxy]-phényl}-propanoïque et ses sels pharmaceutiquement acceptables ;
l'acide (2S)-éthoxy-3-(4-(2-[5-méthyl-2-(5-phényl-thiophén-2-yl)-oxazol-4-yl]-éthoxy}-phényl)-propanoïque et ses sels pharmaceutiquement acceptables ;
l'acide (2S)-éthoxy-3-(4-{2-[5-méthyl-2-(5-chloro-thiophén-2-yl)-oxazol-4-yl]-éthoxy}-phényl)-propanoïque et ses sels pharmaceutiquement acceptables ;
l'acide (2S)-éthoxy-3-(4-{2-[5-méthyl-2-(5-bromo-thiophén-2-yl)-oxazol-4-yl]-éthoxy}-phényl)-propanoïque et ses sels pharmaceutiquement acceptables ;
l'acide (2S)-éthoxy-3-(4-{2-[5-méthyl-2-(5-méthyl-furan-2-yl)-oxazol-4-yl]-éthoxy}-phényl)-propanoïque et ses sels pharmaceutiquement acceptables ;
l'acide 2(S)-éthoxy-3-[4-(5-méthyl-2-pyridin-2-yl-oxazol-4-yhnéthoxy)-phényl]-propanoïque et ses sels pharmaceutiquement acceptables ;
l'acide 2(S)-éthoxy-3-[4-(5-méthyl-2-pyridin-4-yl-oxazol-4-ylméthoxy)-phényl]-propanoïque et ses sels pharmaceutiquement acceptables ;
l'acide 2(S)-éthoxy-3-[4-(5-méthyl-2-pyridin-3-yl-oxazol-4-ylméthoxy)-phényl]-propanoïque et ses sels pharmaceutiquement acceptables.

**11.** Composés selon l'une quelconque des revendications 8 à 10, appropriés en tant que produits intermédiaires pour la préparation de composés de formule (I).

**12.** Procédé pour la préparation d'un composé de formule (IIIa) tel que défini à l'une quelconque des revendications 8

à 10, comprenant les opérations consistant à

i. faire réagir un composé de formule (Iva) dans laquelle « A » représente un groupe 4-oxazolyle substitué par un ou deux substituants choisis parmi les groupes alkyle en $C_1$-$C_{12}$ linéaires ou ramifiés, substitués ou non substitués, les groupes hétéroaryle ou hétérocycliques individuels ou fusionnés, substitués ou non substitués, à la condition que l'un des substituants sur « A » soit toujours un groupe hétéroaryle ou hétérocyclique et à la condition supplémentaire que, lorsque l'hétéroaryle est un groupe pyryle, un tel groupe est non substitué ; « n » est un entier de 1-3 ; et « L » représente un groupe partant choisi parmi halogène, mésylate, tosylate & triflate, avec un composé de formule (Vc) dans laquelle X représente oxygène ou soufre ; « Ar » représente un phényle non substitué ; $G_1$ représente $OR_1$ ou $SR_1$, où $R_1$ représente hydrogène, perfluoroalkyle en $C_1$-$C_{12}$, les groupes substitués ou non substitués choisis parmi les groupes alkyle en $C_1$-$C_{12}$, cycloalkyle en $C_1$-$C_{12}$, aryle, ar-alkyle en $C_1$-$C_{12}$, hétéroaryle, hétéroar-alkyle en $C_1$-$C_{12}$, hétérocyclyle, alcoxyalkyle, aryloxyalkyle, alcoxycarbonyle, aryloxycarbonyle, cycloalkyloxycarbonyle, alkylaminocarbonyle, arylaminocarbonyle ou acyle, linéaires ou ramifiés ; $R_4$ représente OH ou des groupes alcoxy ou aryloxy, aralcoxy ou $NR_1R_2$, où $R_1$ & $R_2$ peuvent être identiques ou différents et représentent indépendamment hydrogène, des groupes substitués ou non substitués choisis parmi les groupes alkyle en $C_1$-$C_8$, cycloalkyle en $C_3$-$C_7$, acyle, aryle, hétéroaryle, hétérocyclyle, aminocarbonyle, aralkyle, alkylaminocarbonyle, arylaminocarbonyle, aralkylaminocarbonyle, hétéroarylaminocarbonyle, hétéroaralkylaminocarbonyle, hétérocyclylaminocarbonyle, alcoxycarbonyle, aryloxycarbonyle, aralkyloxycarbonyle, hétéroaryloxycarbonyle, hétéroaralcoxycarbonyle, hétérocycloxycarbonyle, linéaires ou ramifiés, ou $SO_2R_3$ où $R_3$ représente des groupes substitués ou non substitués choisis parmi les groupes alkyle, aryle, polyhaloalkyle, hétérocyclyle, hétéroaryle,

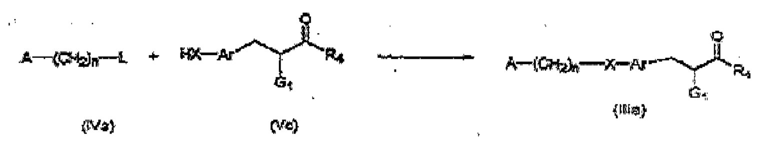

ii. facultativement hydrolyser le composé de la formule (IIIa) dans laquelle $R_4$ représente les groupes alcoxy, aryloxy, aralcoxy ou $NR_1R_2$, où $R_1$ & $R_2$ sont tels que définis précédemment, en un autre composé de formule (IIIa) dans laquelle $R_4$ représente OH.

**13.** Composition pharmaceutique qui comprend des composés de la formule (I) ou (IIIa), tels que définis à l'une quelconque des revendications précédentes, et un support, un diluant, des excipients ou un solvate pharmaceutiquement acceptables.

**14.** Composition pharmaceutique selon la revendication 13, sous la forme d'un comprimé, capsule, poudre, granulé, sirop, solution ou suspension.

**15.** Composition pharmaceutique selon la revendication 13 ou la revendication 14, en combinaison avec de la sulfonyl urée, du biguanide, un inhibiteur de l'angiotensine II, de l'aspirine, un inhibiteur de l'$\alpha$-glycosidase, un sécrétagogue de l'insuline, de l'insuline, un inhibiteur du $\beta$-sitostérol, un inhibiteur de l'HMG-CoA réductase, un fibrate, de l'acide nicotinique, de la cholestyramine, du cholestipol ou du probucol, qui peuvent être administrés ensemble ou en l'espace d'une période de temps telle qu'ils agissent ensemble de façon synergique, à un patient en ayant besoin.

**16.** Utilisation d'un composé de formule (I) ou (IIIa), tel que défini à l'une quelconque des revendications 1 à 5 ou 9 à 11, pour la préparation d'un médicament destiné à être utilisé dans la réduction de la glycémie veineuse, des triglycérides, du cholesterol total, de la LDL, de la VLDL ou des acides gras libres dans le plasma d'un patient, tout en élevant facultativement les taux de cholesterol HDL.

**17.** Utilisation selon la revendication 16, dans laquelle le composé de formule (I) ou (IIIa) est destiné à la préparation d'un médicament devant être donné en combinaison avec un inhibiteur de la HMG CoA réductase, un fibrate, de l'acide nicotinique, de la cholestyramine, du cholestipol ou du probucol, lequel ingrédient supplémentaire peut être administré conjointement avec le médicament ou en l'espace d'une période de temps telle qu'il agit de façon synergique conjointement avec le médicament.

**18.** Utilisation du composé de formule (I) ou (IIIa) tel que défini à l'une quelconque des revendications 1 à 5 ou 9 à 11

pour la préparation d'un médicament pour la prévention ou le traitement de maladies provoquées par une hyperlipidémie, une hypercholestérolémie, une hyperglycémie, l'obésité, une tolérance détériorée au glucose, une résistance à la leptine, une résistance à l'insuline ou des complications diabétiques.

**19.** Utilisation d'un composé de formule (I) ou (IIIa) tel que défini à l'une quelconque des revendications 1 à 5 ou 9 à 11 pour la préparation d' un médicament pour traiter une maladie dans laquelle la résistance à l'insuline est le mécanisme pathophysiologique sous-jacent, qui comprend le diabète de type 2, la tolérance détériorée au glucose, la dyslipidémie, l'hypertension, l'obésité, l'athérosclérose, l'hyperlipidémie, la coronaropathie, les troubles cardiovasculaires, les affections rénales, la microalbuminurie, la glomérulonéphrite, la glomérulosclérose, le syndrome néphrotique, la néphrosclérose hypertensive, la rétinopathie diabétique, la néphropathie diabétique, le dysfonctionnement des cellules endothéliales, le psoriasis, le syndrome des ovaires polykystiques (PCOS), la démence, une insuffisance rénale terminale, l'ostéoporose, une affection abdominale inflammatoire, une dystrophie myotonique, une pancréatite, une artériosclérose, un xanthome ou un cancer.

**20.** Utilisation selon l'une quelconque des revendications 16 à 18, dans laquelle le médicament contient un support, un diluant ou des excipients ou un solvate pharmaceutiquement acceptables.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9736579 A
- WO 9725042 A
- EP 0753298 A
- WO 9802159 A
- WO 0140170 A
- WO 03008362 A
- WO 02092084 A
- WO 0216331 A
- WO 9807699 A
- WO 02100403 A
- US 5232945 A
- WO 03072100 A
- WO 0320269 A
- WO 0216332 A
- WO 0218355 A
- WO 0296895 A
- WO 0296894 A
- WO 0296893 A
- WO 0262774 A
- WO 0250048 A
- WO 0250047 A
- WO 0276957 A
- WO 0251820 A
- WO 0214291 A
- WO 0138325 A
- WO 0116120 A
- WO 0100403 A
- WO 0116111 A
- WO 0179202 A
- WO 0179197 A
- WO 0008002 A
- US 20010008898 A
- JP 2002338555 B
- JP 2001261612 B
- WO 9119702 A
- JP 51149265 B
- EP 0478363 A
- WO 9638415 A
- WO 2004031162 A
- WO 2004004665 A
- WO 03072102 A

### Non-patent literature cited in the description

- **ROSS ; GLOMSET.** *New Engl. J. Med.,* 1976, vol. 295, 369-377
- **STAMPFER et al.** *N. Engl. J. Med.,* 1991, vol. 325, 373-381
- **MILLER.** *Br. Med. J.,* 1981, vol. 282, 1741-1744
- **PICARDO et al.** *Arteriosclerosis,* 1986, vol. 6, 434-441
- **MACIKINNON et al.** *J. Biol. Chem.,* 1986, vol. 261, 2548-2552
- *Exp. Clin. Endocrinol. Diabetes,* 2001, vol. 109 (4), 548-9
- *Nature Reviews: Drug Discovery,* 2002, vol. 1 (4), 276-86
- *Endocrine Reviews,* 1999, vol. 20 (5), 649-688
- *J. Medicinal Chemistry,* 2000, vol. 43 (4), 58-550
- *Cell,* 1999, vol. 55, 932-943
- *Nature,* 2000, vol. 405, 421-424
- *Trends in Pharmacological Sci.,* 2000, 469-473
- *Endocrinology,* 1994, vol. 135, 798-800
- *Cell,* 1995, vol. 83, 803-812
- *Cell,* 1999, vol. 99, 239-242
- *Cell Biology,* 1998, vol. 95, 14751-14756
- *Trends Endocrine. Metabolism,* 1993, vol. 4, 291-296
- *Current Biol.,* 1995, vol. 5, 618-621
- *Cell,* 1994, vol. 79, 1147-1156
- *Cell,* 1996, 377-389
- *Molecular Cell,* 1998, 465-470
- *Carcinogenesis,* 1998, 1949-1953
- *Proc. Natl. Acad. Sci.,* 1997, vol. 94, 237-241
- *Cancer Research,* 1998, vol. 58, 3344-3352
- *Med. Res. Rev.,* 2000, vol. 20 (5), 350-366
- *Science,* 1995, vol. 269, 543-46
- *Proc. Natl. Acad Sci.,* 1996, vol. 93, 5793-5796
- *Journal of Medical Chemistry, American Chemical Society,* 1994, vol. 37, 2537-2551
- **JAQUES et al.** Enantiomers, Racemates and Resolution. Wiley Interscience, 1981
- **R A. SHELDON.** Chirotechnology. Marcel Dekker, Inc, 1993, 173-204
- **A. N. COLLINS ; G. N. SHELDRACK ; J CROSBY.** Chirality in Industry II. John Wiley & Sons, Inc, 1997, 81-98

- **E. L. ELIEL ; S. H. WILEN.** Stereochemistry of Organic Compound. John Wiley & Sons, Inc, 1999, 297-464
- **T. W. GREENE ; P. G. M.. WUTS.** Protective groups in Organic Synthesis. John Wiley & Sons, Inc, 1999, 201-245
- Remington: the Science and Practice of Pharmacy. 1995
- **WIELAND, O.** Methods of Enzymatic analysis. 1963, 211-214
- **TRINDER, P.** *Ann. Clin. Biochem.,* 1969, vol. 6, 24-27
- **PETIT D. ; BONNEFIS M. T. ; REY C ; INFANTE R.** Effects of ciprofibrate on liver lipids and lipoprotein synthesis in normal and hyperlipidemic rats. *Atherosclerosis,* 1988, vol. 74, 215-225
- *J. Clin. Invest.,* 1990, vol. 85, 962-967